# EUROPEAN PATENT APPLICATION

(11) **EP 4 793 274 A1**
(43) Date of publication of application: **19.08.2026**
(21) Application number: 24876687.5
(22) Date of filing: 12.10.2024
(51) Int. Cl.: C07D 401/14, A61K 31/506, A61P 35/00

(54) **SALT AND CRYSTAL FORM OF KIF18A INHIBITOR COMPOUND, PREPARATION METHOD THEREFOR AND USE THEREOF**

(30) Priority: 13.10.2023 CN 202311331874
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); ZANG, Yang, Wuhan, Hubei 430075 (CN); LI, Qun, Wuhan, Hubei 430075 (CN); ZONG, Qiao, Wuhan, Hubei 430075 (CN); DING, Xiaohua, Wuhan, Hubei 430075 (CN); QIAN, Lina, Wuhan, Hubei 430075 (CN)
(74) Representative: Gille Hrabal Partnerschaftsgesellschaft mbB Patentanwälte
(86) International application number: PCT/CN2024/124472
(87) International publication number: WO 2025/077882

(57) **Abstract**

The present invention relates to a salt and a crystal form of a KIF18A inhibitor compound as shown in formula I, a preparation method therefor and a use thereof. The structure of formula I is shown below.

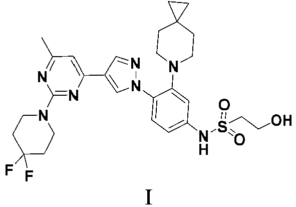

## Description

The present application claims priority to the prior application with the Patent Application No. 202311331874.0, entitled "SALT AND CRYSTAL FORM OF KIF18A INHIBITOR COMPOUND, PREPARATION METHOD THEREFOR AND USE THEREOF" and filed with the China National Intellectual Property Administration on October 13, 2023, which is incorporated herein by reference in its entirety.

### TECHNICAL FIELD

The present disclosure belongs to the field of pharmaceutics, and relates to a salt and a crystal form of a KIF18A inhibitor compound, a preparation method therefor, and use thereof.

### BACKGROUND

Cancer is one of the most prevalent diseases afflicting humans and is a leading cause of death worldwide. However, to date, only a few of the available cancer treatments and therapies have achieved considerable success. Cancer is generally characterized by unregulated cell proliferation. Cellular pathways control the proliferation process through the cell cycle and centrosome cycle, and damage to one or more genes responsible for the cellular pathways can result in loss of normal regulation of cell proliferation. These dysregulated genes can encode a variety of tumor suppressor proteins or oncogene proteins that are involved in a series of events leading to unexamined cell cycle progression and cell proliferation. Various kinases and kinesins have been identified, which play key roles in the regulation and progression of the cell cycle and mitosis in both normally dividing cells and cancer cells.

Kinesins are molecular motors that play important roles in cell division and intracellular vesicle and organelle trafficking. Mitotic kinesins function in multiple aspects of spindle assembly, chromosome segregation, centrosome segregation, and dynamics (O. Rath and F. Kozielski, Nature Review Cancer, 12:527-39, 2012). Human kinesins are categorized into 14 subfamilies based on sequence homology within the so-called "motor domain". The ATPase activity of this domain drives unidirectional movement along microtubules (MTs). The non-motor domains of these proteins are responsible for cargo attachment. The "cargo" may include any one of a variety of different membranous organelles, signal transduction scaffolding systems, and chromosomes. Kinesins use ATP hydrolysis energy to move cargo along polarized microtubules. Therefore, kinesins are often referred to as "plus-end" or "minus-end" directed motors.

The KIF18A gene belongs to the kinesin-8 subfamily and is a plus-end directed motor. KIF18A is thought to influence the dynamics of the plus ends of kinetochore microtubules to control proper chromosome positioning and spindle tension. The deletion of human KIF18A results in spindle elongation, increased metaphase chromosome oscillation, and activation of the mitotic spindle assembly checkpoint in HeLa cervical cancer cells. KIF18A appears to be a viable target for cancer treatment. KIF18A is overexpressed in various types of cancers, including but not limited to colon cancer, breast cancer, lung cancer, pancreatic cancer, prostate cancer, bladder cancer, head cancer, neck cancer, cervical cancer, and ovarian cancer. Furthermore, in cancer cell lines, the deletion or knock-out or inhibition of the KIF18A gene affects the mitotic spindle of cancer cell lines. In particular, it has been found that the inhibition of KIF18A induces mitotic cell arrest, a known weakness that can promote mitotic cell death by apoptosis, mitotic catastrophe, or multiphase-driven lethality, or death following mitotic slippage during the interphase. Therefore, there is a strong interest in finding inhibitors of the KIF18A protein.

Currently, no drugs that treat numerous conditions including cancer through inhibition of KIF18A are available on the market. Therefore, the development of new compounds capable of inhibiting the activity of KIF18A is of great significance for treating diseases.

Chinese Patent Application No. CN202310400928.8 discloses a compound of formula I as follows:

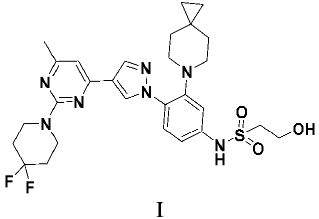

The compound can effectively inhibit the activity of KIF18A. Therefore, studying the salt form and the crystal form of the compound is beneficial to further obtaining a drug with clinical application value.

### SUMMARY

In order to solve the problems in the prior art, in one aspect, the present disclosure provides a crystal form of a compound of formula I, wherein the compound of formula I has a structure shown below:

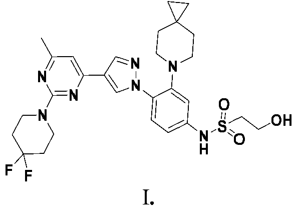

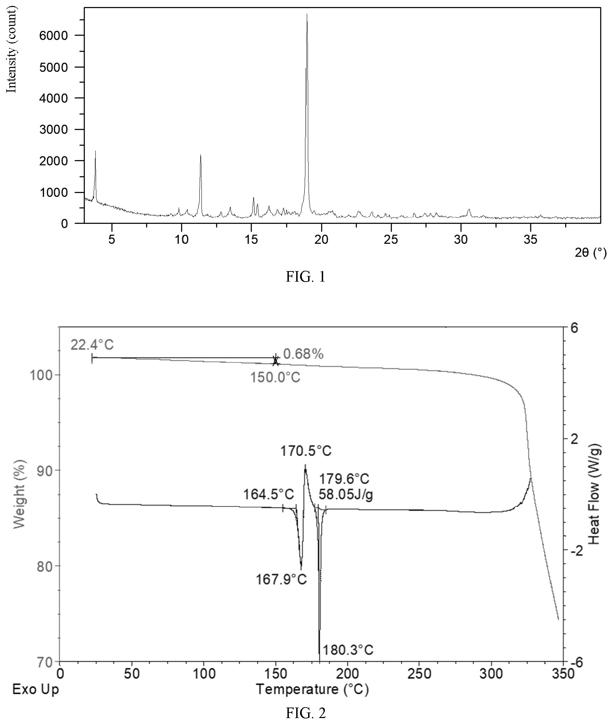

In some embodiments, the present disclosure provides free crystal form A of the compound of formula I, and the free crystal form A has an X-ray powder diffraction pattern comprising diffraction peaks at 2θ±0.2° diffraction angles of 3.8074, 11.3487, 15.1458, 15.4196, 16.2456, and 18.9612; further, the free crystal form A has an X-ray powder diffraction pattern comprising diffraction peaks at 2θ±0.2° diffraction angles of 3.8074, 9.8108, 10.3581, 11.3487, 13.4688, 15.1458, 15.4196, 16.2456, 16.8635, 17.3003, 18.9612, 22.6628, and 30.5503; furthermore, the free crystal form A has an X-ray powder diffraction pattern comprising diffraction peaks at 2θ±0.2° diffraction angles of 3.8074, 9.8108, 10.3581, 11.3487, 12.7984, 13.4688, 15.1458, 15.4196, 16.2456, 16.8635, 17.3003, 18.9612, 20.7248, 22.6628, 23.6322, 30.5503, 26.6666, 27.4293, and 28.2276; still further, the free crystal form A has an X-ray powder diffraction pattern comprising diffraction peaks at 2θ±0.2° diffraction angles of 3.8074, 9.8108, 10.3581, 11.3487, 12.7984, 13.4688, 15.1458, 15.4196, 16.2456, 16.8635, 17.3003, 18.9612, 20.7248, 22.6628, 23.6322, 24.5787, 25.7207, 26.6666, 27.4293, 28.2276, 30.5503, and 35.7206; still further, the free crystal form A has an XRPD pattern substantially as shown in FIG. 1.

In some embodiments, the free crystal form A has one, two, or three of the following characteristics:
(1) a TGA curve of the free crystal form A showing a weight loss of 0-2%, preferably about 0-1% (e.g., 0.1%, 0.2%, 0.3%, 0.4%, 0.5%, 0.6%, 0.7%, or 0.8%) at 150.0±3 °C;
(2) a DSC curve of the free crystal form A having 2 endothermic peaks at 167.9±3 °C and 180.3±3 °C;
(3) a DSC curve of the free crystal form A having 1 exothermic peak at 170.5 °C±3 °C.

In some embodiments, the free crystal form A has one, two, or three of the following characteristics:
(1) a TGA curve of the free crystal form A showing a weight loss of about 0.68% at 150.0±3 °C;
(2) a DSC curve of the free crystal form A having 2 endothermic peaks at 167.9±3 °C and 180.3±3 °C;
(3) a DSC curve of the free crystal form A having 1 exothermic peak at 170.5 °C±3 °C.

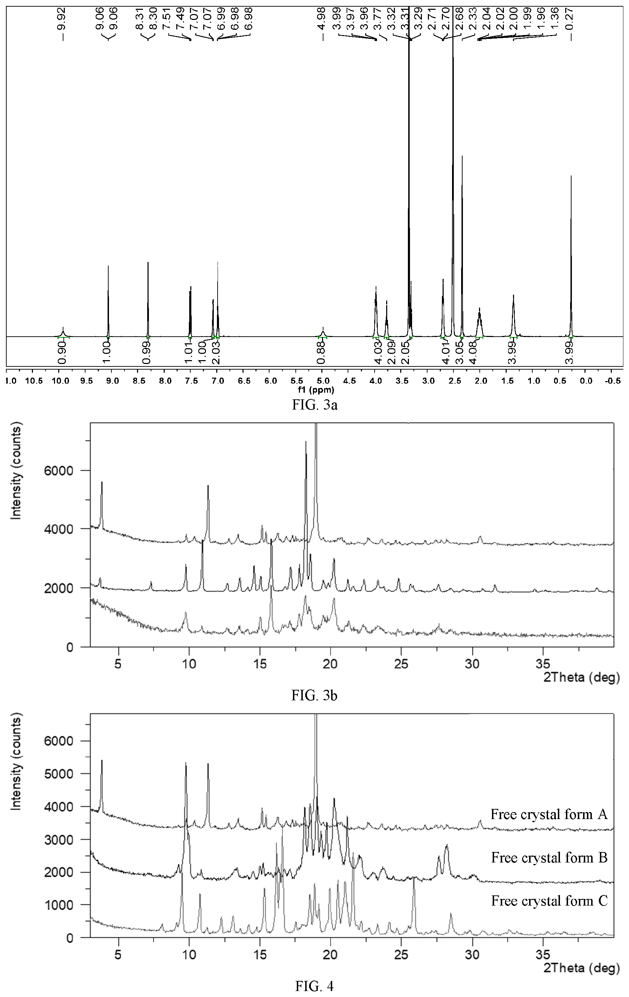

In some embodiments, a TGA/DSC profile of the free crystal form A is substantially as shown in FIG. 2; a ¹H NMR spectrum of the free crystal form A is substantially as shown in FIG. 3a.

In some embodiments, the free crystal form is an anhydrate.

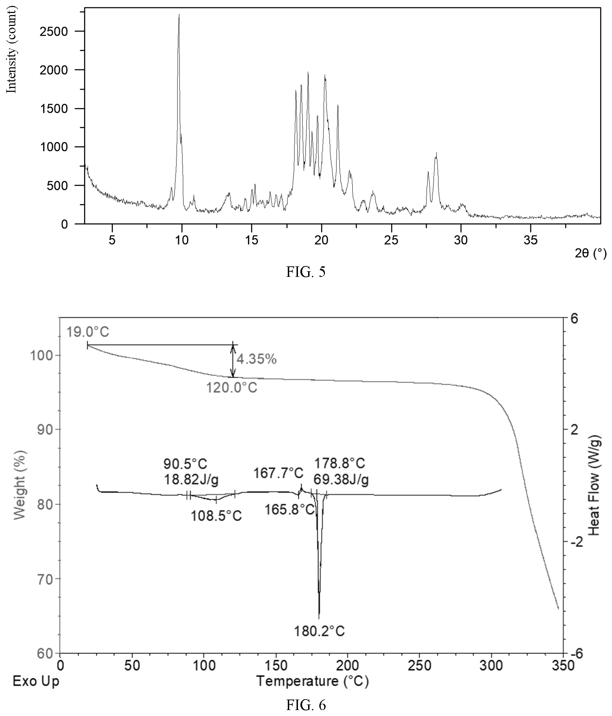

In some embodiments, the present disclosure provides free crystal form B of the compound of formula I, and the free crystal form B has an X-ray powder diffraction pattern comprising diffraction peaks at 2θ±0.2° diffraction angles of 9.7812, 18.1668, 18.5510, 19.0431, 20.2387, and 21.1776; further, the free crystal form B has an X-ray powder diffraction pattern comprising diffraction peaks at 2θ±0.2° diffraction angles of 9.7812, 18.1668, 18.5510, 19.0431, 19.3191, 19.7143, 20.2387, and 21.1776; furthermore, the free crystal form B has an X-ray powder diffraction pattern comprising diffraction peaks at 2θ±0.2° diffraction angles of 9.7812, 9.9884, 18.1668, 18.5510, 19.0431, 19.3191, 19.7143, 20.2387, and 21.1776; furthermore, the free crystal form B has an X-ray powder diffraction pattern comprising diffraction peaks at 2θ±0.2° diffraction angles of 9.2508, 9.7812, 9.9884, 15.0024, 15.2215, 18.1668, 18.5510, 19.0431, 19.3191, 19.7143, 20.2387, 21.1776, 22.0900, 27.6189, and 28.2642; still further, the free crystal form B has an X-ray powder diffraction pattern comprising diffraction peaks at 2θ±0.2° diffraction angles of 9.2508, 9.7812, 9.9884, 10.8644, 13.3822, 14.5151, 15.0024, 15.2215, 16.3081, 16.7223, 17.1474, 18.1668, 18.5510, 19.0431, 19.3191, 19.7143, 20.2387, 21.1776, 22.0900, 23.0450, 23.7619, 25.7395, 27.6189, 28.2642, and 30.0557; still further, the free crystal form B has an XRPD pattern substantially as shown in FIG. 5.

In some embodiments, the free crystal form B has one, two, or three of the following characteristics:
(1) a TGA curve of the free crystal form B showing a weight loss of 2-6%, preferably about 3-5% (e.g., 3.0%, 3.5%, 4.0%, or 4.5%) at 120.0±3 °C;
(2) a DSC curve of the free crystal form B having 3 endothermic peaks at 108.5±3 °C, 165.8±3 °C, and 180.2±3 °C;
(3) a DSC curve of the free crystal form B having 1 exothermic peak at 167.7 °C±3 °C.

In some embodiments, the free crystal form B has one, two, or three of the following characteristics:
(1) a TGA curve of the free crystal form B showing a weight loss of about 4.35% at 120.0±3 °C;
(2) a DSC curve of the free crystal form B having 3 endothermic peaks at 108.5±3 °C, 165.8±3 °C, and 180.2±3 °C;
(3) a DSC curve of the free crystal form B having 1 exothermic peak at 167.7 °C±3 °C.

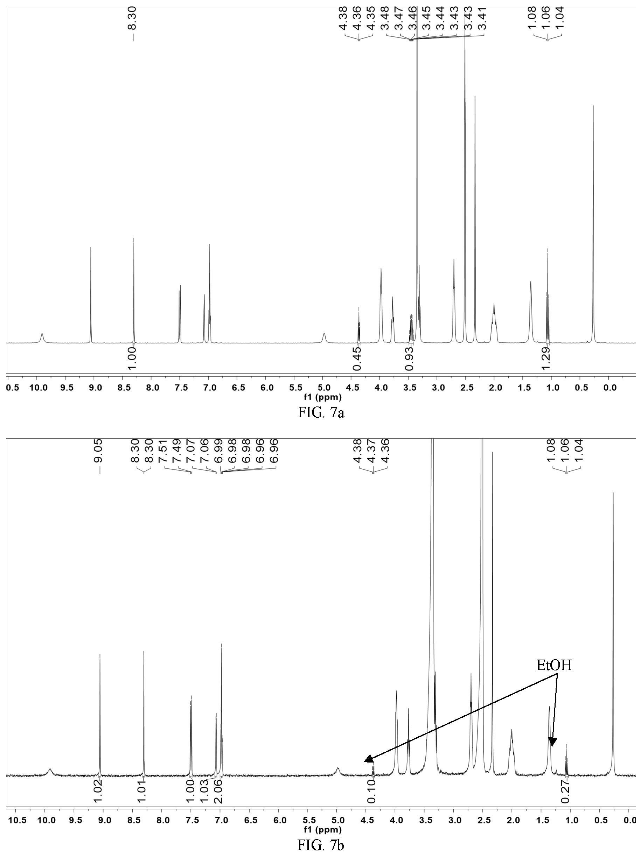

In some embodiments, a TGA/DSC profile of the free crystal form B is substantially as shown in FIG. 6; a ¹H NMR spectrum of the free crystal form B is substantially as shown in FIG. 7a.

In some embodiments, the free crystal form B is a solvate.

In some embodiments, the free crystal form B is an EtOH solvate.

In some embodiments, in the solvate, EtOH and the free crystal form are in a molar ratio of 0.10 (about 0.8 wt%). In some embodiments, the free crystal form B is a co-solvate of EtOH and water.

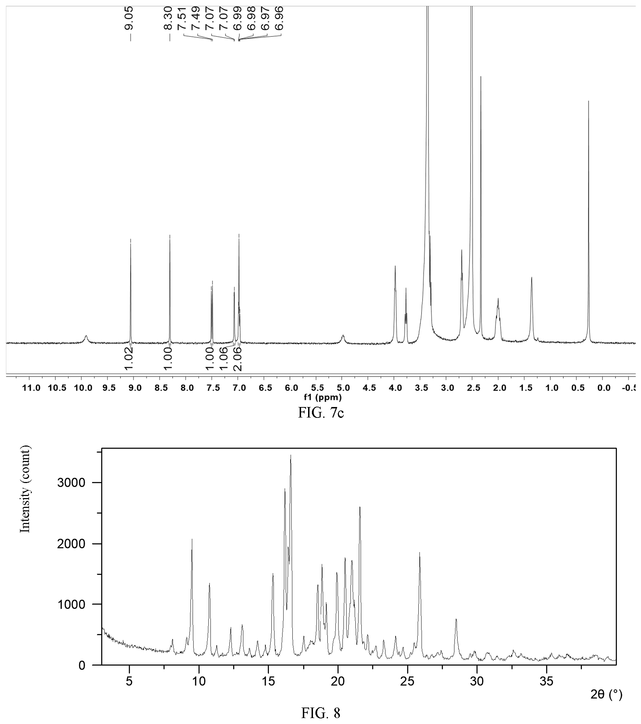

In some embodiments, the present disclosure provides free crystal form C of the compound of formula I, and the free crystal form C has an X-ray powder diffraction pattern comprising diffraction peaks at 2θ±0.2° diffraction angles of 9.5020, 16.1724, 16.5974, 21.5887, and 25.8930; further, the free crystal form C has an X-ray powder diffraction pattern comprising diffraction peaks at 2θ±0.2° diffraction angles of 9.5020, 15.3140, 16.1724, 16.5974, 18.8700, 19.9398, 20.5180, 21.0065, 21.5887, and 25.8930; furthermore, the free crystal form C has an X-ray powder diffraction pattern comprising diffraction peaks at 2θ±0.2° diffraction angles of 9.5020, 10.7612, 15.3140, 16.1724, 16.5974, 18.5313, 18.8700, 19.1799, 19.9398, 20.5180, 21.0065, 21.5887, 25.8930, and 28.4921; still further, the free crystal form C has an X-ray powder diffraction pattern comprising diffraction peaks at 2θ±0.2° diffraction angles of 3.1461, 8.1061, 9.1126, 9.5020, 10.7612, 11.2726, 12.2849, 13.1097, 14.2058, 14.7763, 15.3140, 16.1724, 16.5974, 17.5352, 18.5313, 18.8700, 19.1799, 19.9398, 20.5180, 21.0065, 21.2198, 21.5887, 22.1453, 22.7421, 23.2908, 24.1476, 24.6794, 25.4796, 25.8930, 27.4465, 28.4921, 29.8280, 30.7213, 31.4381, 32.6390, 35.3421, 36.5656, and 38.4547; still further, the free crystal form C has an XRPD pattern substantially as shown in FIG. 8.

In some embodiments, the free crystal form C has one or two of the following characteristics:
(1) a TGA curve of the free crystal form C showing a weight loss of 1-6%, preferably about 1-4% (e.g., 1.0%, 1.5%, 2.0%, 2.5%, 3.0%, 3.5%, or 4.0%) at 150.0±3 °C;
(2) a DSC curve of the free crystal form C having 2 endothermic peaks at 53.0±3 °C and 182.3±3 °C.

In some embodiments, the free crystal form C has one or two of the following characteristics:
(1) a TGA curve of the free crystal form C showing a weight loss of about 2.77% at 150.0±3 °C;
(2) a DSC curve of the free crystal form C having 2 endothermic peaks at 53.0±3 °C and 182.3±3 °C.

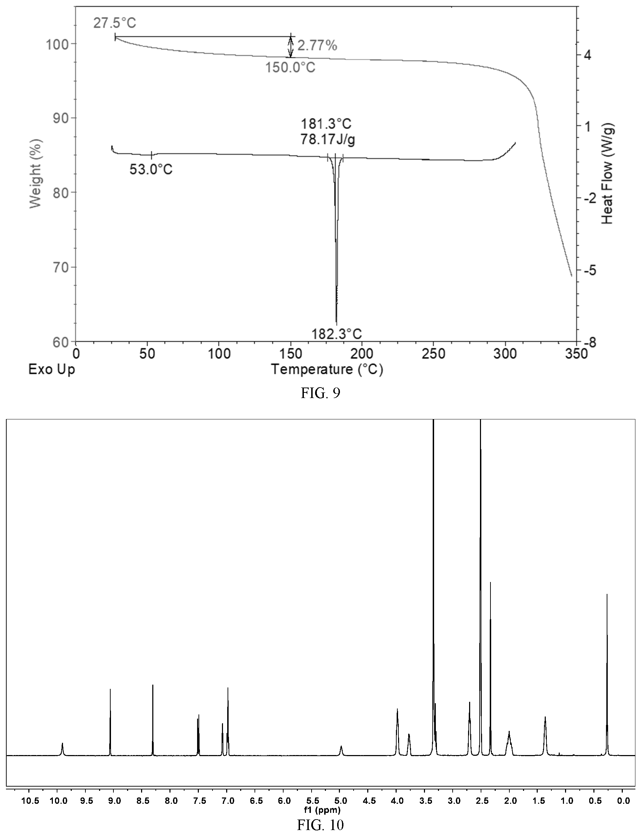

In some embodiments, a TGA/DSC profile of the free crystal form C is substantially as shown in FIG. 9; a ¹H NMR spectrum of the free crystal form C is substantially as shown in FIG. 10.

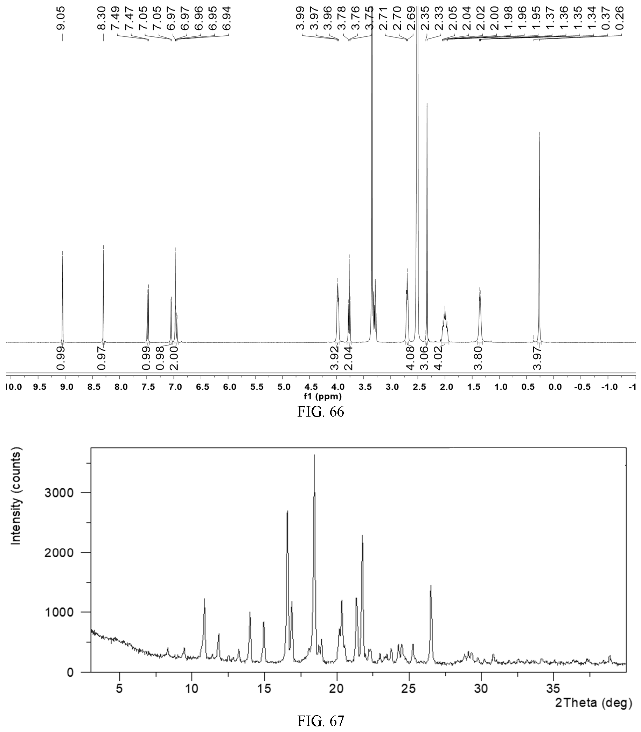

In some embodiments, the present disclosure provides free crystal form D of the compound of formula I, and the free crystal form D has an X-ray powder diffraction pattern comprising diffraction peaks at 2θ±0.2° diffraction angles of 10.8461, 16.5793, 18.4363, 20.3590, 21.3940, 21.7745, and 26.5122; further, the free crystal form D has an X-ray powder diffraction pattern comprising diffraction peaks at 2θ±0.2° diffraction angles of 10.8461, 13.9909, 16.5793, 16.8857, 18.4363, 20.3590, 21.3940, 21.7745, and 26.5122; furthermore, the free crystal form D has an X-ray powder diffraction pattern comprising diffraction peaks at 2θ±0.2° diffraction angles of 10.8461, 13.9909, 14.9577, 16.5793, 16.8857, 18.4363, 20.1677, 20.3590, 21.3940, 21.7745, and 26.5122; furthermore, the free crystal form D has an X-ray powder diffraction pattern comprising diffraction peaks at 2θ±0.2° diffraction angles of 10.8461, 11.8218, 13.9909, 14.9577, 16.5793, 16.8857, 18.4363, 18.7513, 18.9436, 20.1677, 20.3590, 21.3940, 21.7745, 22.2999, 23.7694, 24.2605, 24.4938, 25.2628, 26.5122, 29.7635, and 30.8099; still further, the free crystal form D has an X-ray powder diffraction pattern comprising diffraction peaks at 2θ±0.2° diffraction angles of 8.2985, 9.4241, 10.8461, 11.8218, 12.5232, 13.2208, 13.9909, 14.9577, 16.5793, 16.8857, 18.4363, 18.7513, 18.9436, 20.1677, 20.3590, 21.3940, 21.7745, 22.2999, 23.7694, 24.2605, 24.4938, 25.2628, 26.5122, 29.2296, 29.7635, 30.8099, and 38.8753; still further, the free crystal form D has an XRPD pattern substantially as shown in FIG. 67. In some embodiments, the free crystal form D has one or two or more of the following characteristics:
(1) a TGA curve showing a weight loss of 3.0-8.0%, preferably about 4.0-7.0% (e.g., 5.0%, 5.5%, 6.0%, or 6.5%), and more preferably about 5.79% at 120±3 °C;
(2) a DSC curve having 2 endothermic peaks at 53.9±3 °C and 182.9±3 °C.

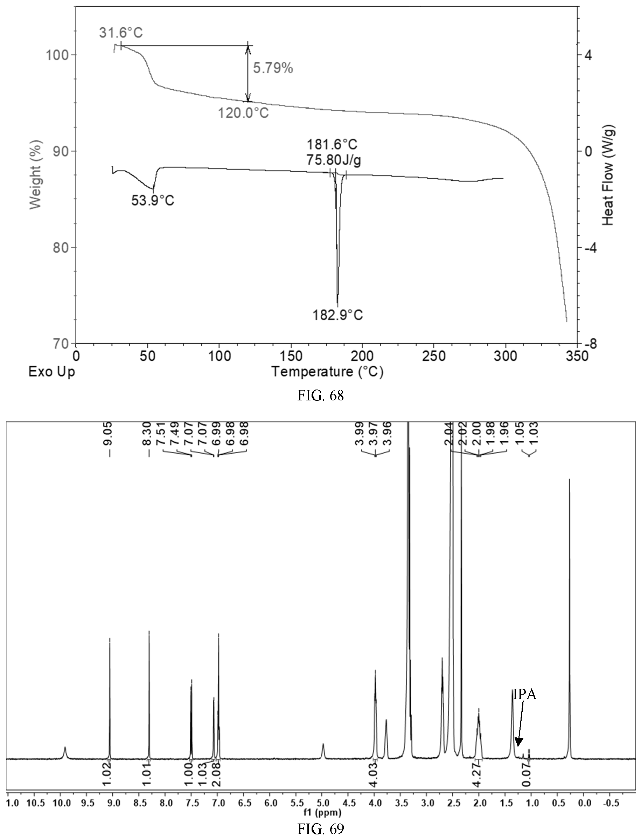

In some embodiments, a TGA/DSC profile of the free crystal form D is substantially as shown in FIG. 68; a ¹H NMR spectrum of the free crystal form D is substantially as shown in FIG. 69.

In some embodiments, the free crystal form D is a hydrate.

In some embodiments, the free crystal form D has a water content of 2.0-3.5%, preferably 2.0-3.0%.

In some embodiments, the free crystal form D is a monohydrate.

In some embodiments, the free crystal form D has the following crystal parameters:
crystal system triclinic
space group P-1
unit cell parameters
a = 8.9217(3) Å, b = 10.9062(5) Å, c = 15.6399(5) Å
α = 95.3240(10)°, β = 97.7830(10)°, γ = 100.4940(10)°

In some embodiments, the free crystal form D has the parameters listed in Table B3-1.

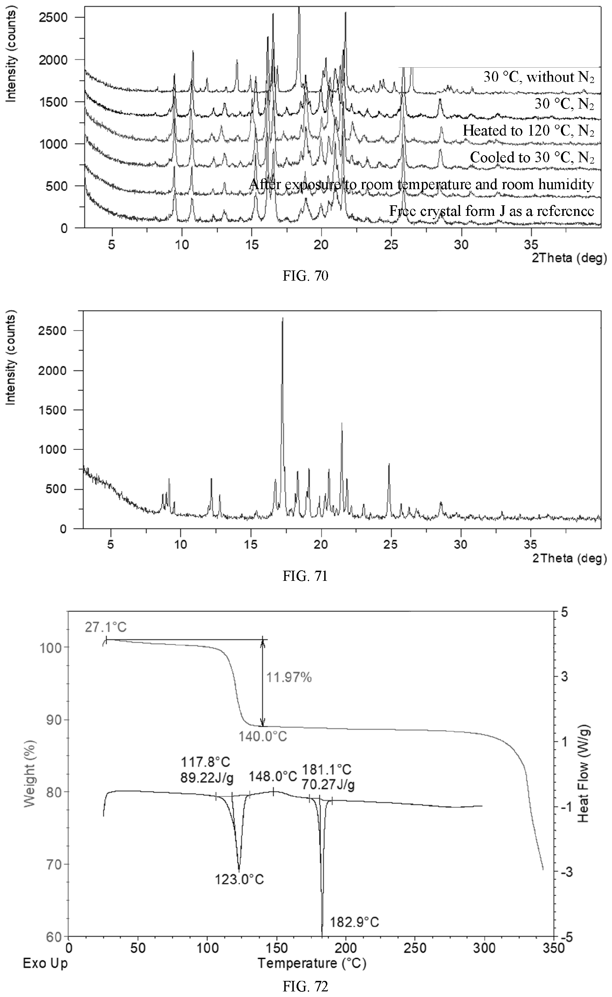

In some embodiments, the present disclosure provides free crystal form E of the compound of formula I, and the free crystal form E has an X-ray powder diffraction pattern comprising diffraction peaks at 2θ±0.2° diffraction angles of 17.2384, 18.3327, 19.1326, 20.5505, 21.4769, and 24.8471; further, the free crystal form E has an X-ray powder diffraction pattern comprising diffraction peaks at 2θ±0.2° diffraction angles of 16.7268, 17.2384, 18.3327, 19.1326, 20.5505, 21.4769, 21.8496, and 24.8471; furthermore, the free crystal form E has an X-ray powder diffraction pattern comprising diffraction peaks at 2θ±0.2° diffraction angles of 9.1459, 12.1780, 16.7268, 17.2384, 18.3327, 18.9674, 19.1326, 20.5505, 21.4769, 21.8496, and 24.8471; furthermore, the free crystal form E has an X-ray powder diffraction pattern comprising diffraction peaks at 2θ±0.2° diffraction angles of 8.6847, 8.9272, 9.1459, 12.1780, 12.7789, 16.7268, 17.2384, 18.1543, 18.3327, 18.9674, 19.1326, 19.8795, 20.2931, 20.5505, 21.4769, 21.8496, 24.8471, and 28.5466; still further, the free crystal form E has an X-ray powder diffraction pattern comprising diffraction peaks at 2θ±0.2° diffraction angles of 8.6847, 8.9272, 9.1459, 9.4934, 12.1780, 12.7789, 15.3545, 16.7268, 17.2384, 18.1543, 18.3327, 18.9674, 19.1326, 19.8795, 20.2931, 20.5505, 21.4769, 21.8496, 22.1726, 23.0433, 24.8471, 25.6946, 26.8264, 28.5466, and 29.5968; still further, the free crystal form E has an XRPD pattern substantially as shown in FIG. 71.

In some embodiments, the free crystal form E has one or two or more of the following characteristics:
(1) a TGA curve showing a weight loss of 9-14%, preferably about 10-13% (e.g., 10.5%, 11.0%, 11.5%, 12.0%, or 12.5%), and more preferably about 11.97% at 140+3 °C;
(2) a DSC curve having 2 endothermic peaks at 123.0±3 °C and 182.9±3 °C;
(3) a DSC curve having 1 exothermic peak at 148.0+3 °C.

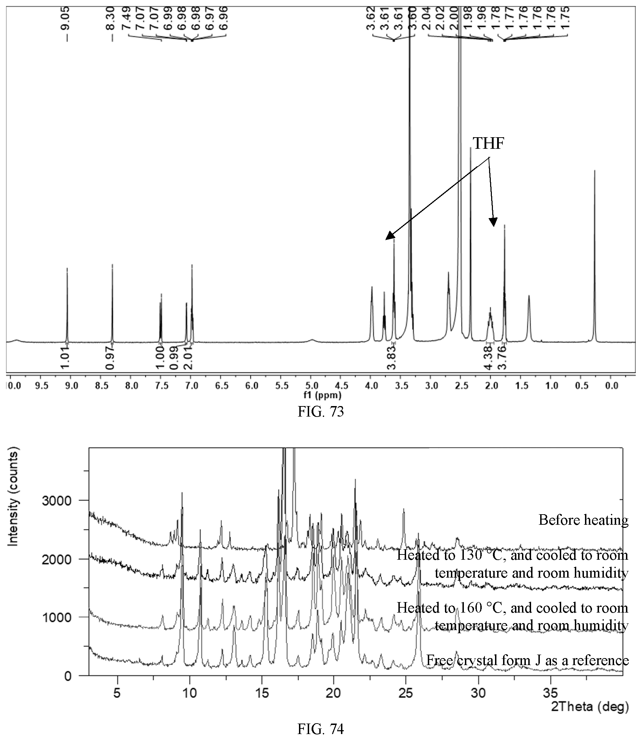

In some embodiments, a TGA/DSC profile of the free crystal form E is substantially as shown in FIG. 72; a ¹H NMR spectrum of the free crystal form E is substantially as shown in FIG. 73.

In some embodiments, the free crystal form E is a solvate.

In some embodiments, the free crystal form E is a THF solvate.

In some embodiments, in the THF solvate of the free crystal form E, THF and the free crystal form are in a molar ratio of 0.95:1.

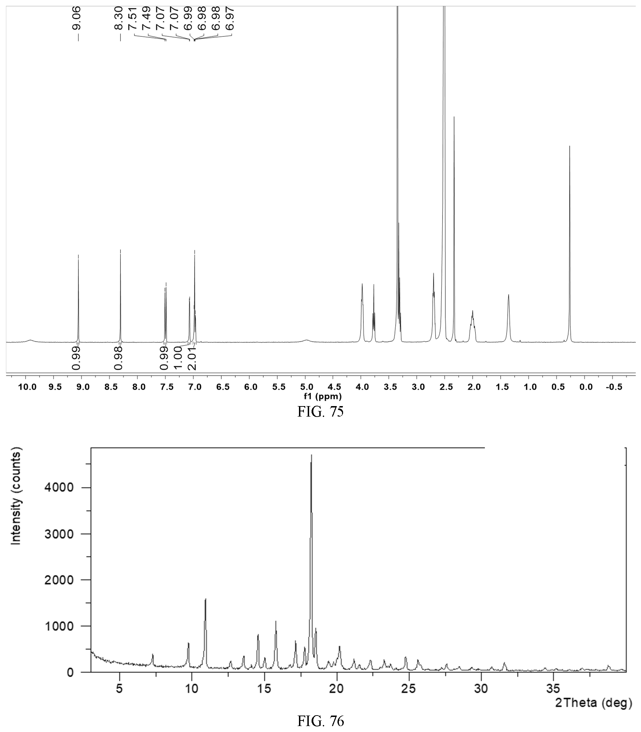

In some embodiments, the present disclosure provides free crystal form F of the compound of formula I, and the free crystal form F has an X-ray powder diffraction pattern comprising diffraction peaks at 2θ±0.2° diffraction angles of 10.9091, 14.5589, 15.7854, 18.2249, and 18.5345; further, the free crystal form F has an X-ray powder diffraction pattern comprising diffraction peaks at 2θ±0.2° diffraction angles of 9.7443, 10.9091, 14.5589, 15.7854, 17.1471, 18.2249, and 18.5345; furthermore, the free crystal form F has an X-ray powder diffraction pattern comprising diffraction peaks at 2θ±0.2° diffraction angles of 9.7443, 10.9091, 14.5589, 15.7854, 17.1471, 17.7727, 18.2249, 18.5345, and 20.2138; furthermore, the free crystal form F has an X-ray powder diffraction pattern comprising diffraction peaks at 2θ±0.2° diffraction angles of 7.2680, 9.7443, 10.9091, 13.5597, 14.5589, 15.7854, 17.1471, 17.7727, 18.2249, 18.5345, 20.2138, and 24.7687; still further, the free crystal form F has an X-ray powder diffraction pattern comprising diffraction peaks at 2θ±0.2° diffraction angles of 7.2680, 9.7443, 10.9091, 12.6503, 13.5597, 14.5589, 15.0095, 15.7854, 17.1471, 17.7727, 18.2249, 18.5345, 19.4299, 20.2138, 21.2140, 21.5620, 22.3451, 23.2974, 23.7177, 24.7687, 25.6111, 27.5720, 28.4674, 29.3309, 30.7039, 31.5840, 34.3936, 35.1042, 36.9389, and 38.7357; still further, the free crystal form F has an XRPD pattern substantially as shown in FIG. 76. In some embodiments, the free crystal form F has one or two or more of the following characteristics:
(1) a TGA curve showing a weight loss of 0.5-2.5%, preferably about 0.5-2.0% (e.g., 0.5%, 1.0%, or 1.5%), and more preferably about 1.19% at 150±3 °C;
(2) a DSC curve having 1 endothermic peak at 181.1±3 °C (peak temperature).

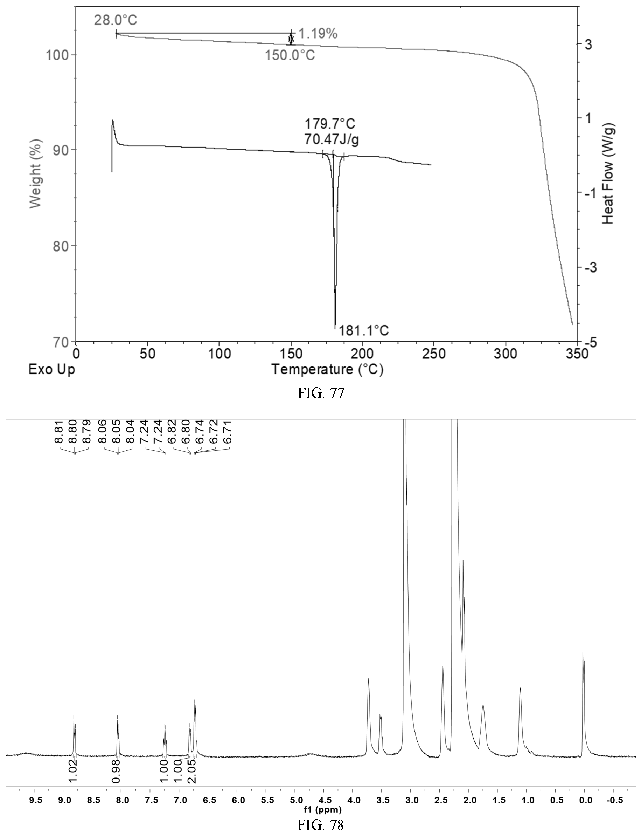

In some embodiments, a TGA/DSC profile of the free crystal form F is substantially as shown in FIG. 77; a ¹H NMR spectrum of the free crystal form F is substantially as shown in FIG. 78.

In some embodiments, the free crystal form F is an anhydrate.

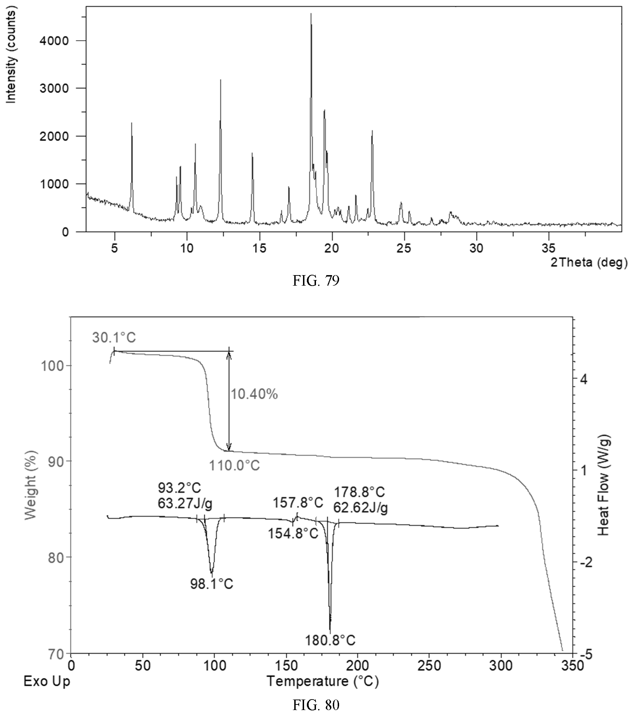

In some embodiments, the present disclosure provides free crystal form G of the compound of formula I, and the free crystal form G has an X-ray powder diffraction pattern comprising diffraction peaks at 206±0.2° diffraction angles of 6.1695, 12.2893, 18.5441, 19.4906, and 22.7762; further, the free crystal form G has an X-ray powder diffraction pattern comprising diffraction peaks at 2θ±0.2° diffraction angles of 6.1695, 10.5406, 12.2893, 14.5010, 18.5441, 19.4906, and 22.7762; furthermore, the free crystal form G has an X-ray powder diffraction pattern comprising diffraction peaks at 2θ±0.2° diffraction angles of 6.1695, 9.2517, 9.4965, 10.5406, 12.2893, 14.5010, 18.5441, 18.8489, 19.4906, and 22.7762; furthermore, the free crystal form G has an X-ray powder diffraction pattern comprising diffraction peaks at 2θ±0.2° diffraction angles of 6.1695, 9.2517, 9.4965, 10.5406, 12.2893, 14.5010, 16.9986, 18.5441, 18.8489, 19.4906, 21.1520, 21.6669, 22.4742, 22.7762, and 24.7980; still further, the free crystal form G has an X-ray powder diffraction pattern comprising diffraction peaks at 2θ±0.2° diffraction angles of 6.1695, 9.2517, 9.4965, 10.2696, 10.5406, 10.9989, 12.2893, 14.5010, 16.5003, 16.9986, 18.5441, 18.8489, 19.4906, 20.6318, 21.1520, 21.6669, 22.4742, 22.7762, 24.7980, 25.3519, 26.0012, 26.8697, and 28.1782; still further, the free crystal form G has an XRPD pattern substantially as shown in FIG. 79.

In some embodiments, the free crystal form G has one or two or more of the following characteristics:
(1) a TGA curve showing a weight loss of 8-13%, preferably about 9-11% (e.g., 9.5%, 10.0%, 10.5%, or 11.0%), and more preferably about 10.40% at 110±3 °C;
(2) a DSC curve having 3 endothermic peaks at 98.1±3 °C, 154.8±3 °C, and 180.8±3 °C;
(3) a DSC curve having 1 exothermic peak at 157.8±3 °C.

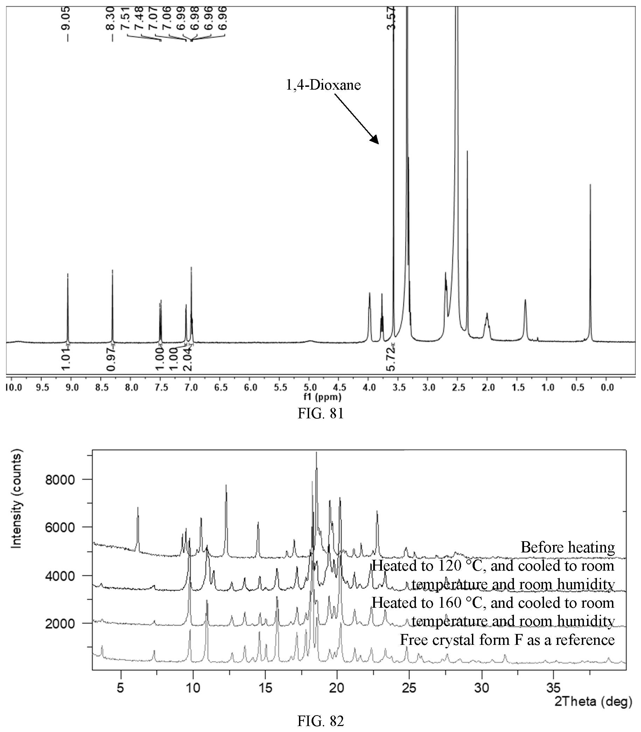

In some embodiments, a TGA/DSC profile of the free crystal form G is substantially as shown in FIG. 80; a ¹H NMR spectrum of the free crystal form G is substantially as shown in FIG. 81.

In some embodiments, the free crystal form G is a solvate.

In some embodiments, the free crystal form G is a 1,4-dioxane solvate; in some embodiments, in the solvate, 1,4-dioxane and the free crystal form are in a molar ratio of 0.04 (about 0.6 wt%) or 0.02 (about 0.3 wt%).

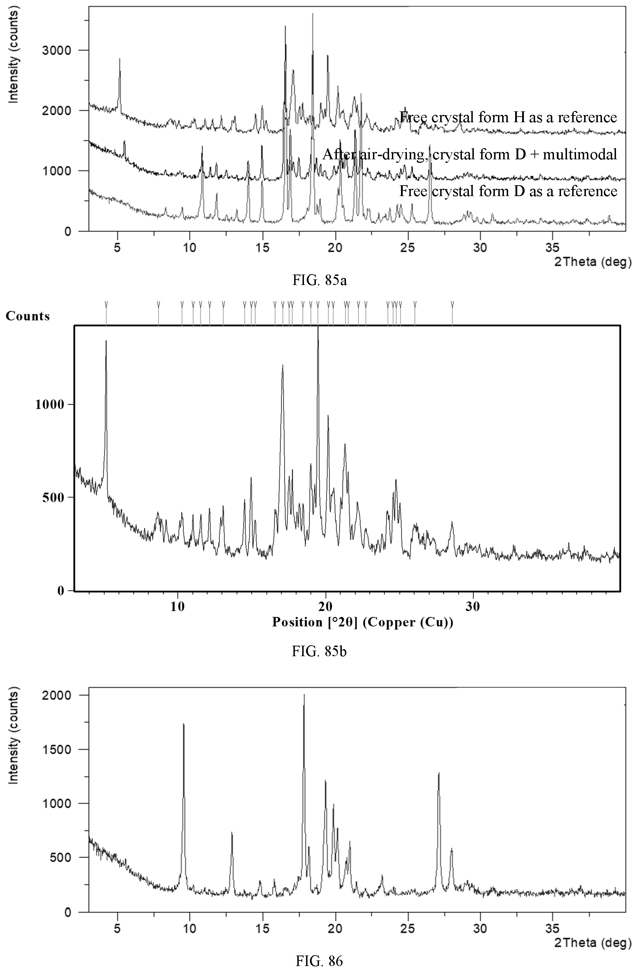

In some embodiments, the present disclosure provides free crystal form H of the compound of formula I, and the free crystal form H has an X-ray powder diffraction pattern comprising diffraction peaks at 2θ±0.2° diffraction angles of 5.1382, 17.0866, 18.9850, 19.4851, 20.1891, and 21.3352; further, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, and 21.3352; furthermore, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, 21.3352, 21.5416, and 24.7905; furthermore, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, 20.5085, 21.3352, 21.5416, 24.5735, and 24.7905; still further, the free crystal form H has an X-ray powder diffraction pattern comprising diffraction peaks at 2θ±0.2° diffraction angles of 5.1382, 8.6958, 10.2939, 11.0299, 11.5587, 12.1517, 13.0908, 14.5022, 14.9595, 15.2308, 16.5672, 17.0866, 17.5191, 17.7454, 18.4679, 18.9850, 19.4851, 20.1891, 20.5085, 21.3352, 21.5416, 22.2233, 22.7317, 24.2236, 24.5735, 24.7905, 25.0488, 26.0331, and 28.5683; still further, the free crystal form H has an XRPD pattern substantially as shown in FIG. 85b.

In some embodiments, the present disclosure provides free crystal form I of the compound of formula I, and the free crystal form I has an X-ray powder diffraction pattern comprising diffraction peaks at 2θ±0.2° diffraction angles of 9.5592, 17.8352, 19.3336, 19.8678, 20.1554, and 27.1065; further, the free crystal form I has an X-ray powder diffraction pattern comprising diffraction peaks at 2θ±0.2° diffraction angles of 9.5592, 12.8988, 17.8352, 19.3336, 19.8678, 20.1554, and 27.1065; furthermore, the free crystal form I has an X-ray powder diffraction pattern comprising diffraction peaks at 2θ±0.2° diffraction angles of 9.5592, 12.8988, 17.8352, 19.3336, 19.8678, 20.1554, 20.9976, and 27.1065; furthermore, the free crystal form I has an X-ray powder diffraction pattern comprising diffraction peaks at 2θ±0.2° diffraction angles of 9.5592, 12.8988, 17.8352, 18.1790, 19.3336, 19.8678, 20.1554, 20.7214, 20.9976, 27.1065, and 28.0012; still further, the free crystal form I has an X-ray powder diffraction pattern comprising diffraction peaks at 2θ±0.2° diffraction angles of 9.5592, 12.8988, 14.7994, 15.8059, 17.8352, 18.1790, 19.3336, 19.8678, 20.1554, 20.7214, 20.9976, 23.2310, 27.1065, and 28.0012; still further, the free crystal form I has an XRPD pattern substantially as shown in FIG. 86.

In some embodiments, the free crystal form I has one or two or more of the following characteristics:
(1) a TGA curve showing a weight loss of 2-7%, preferably about 3-6% (e.g., 3.5%, 4.0%, or 4.5%) at 120±3 °C;
(2) a DSC curve having 3 endothermic peaks at 54.6±3 °C, 168.0±3 °C, and 181.2±3 °C;
(3) a DSC curve having 1 exothermic peak at 170.5±3 °C.

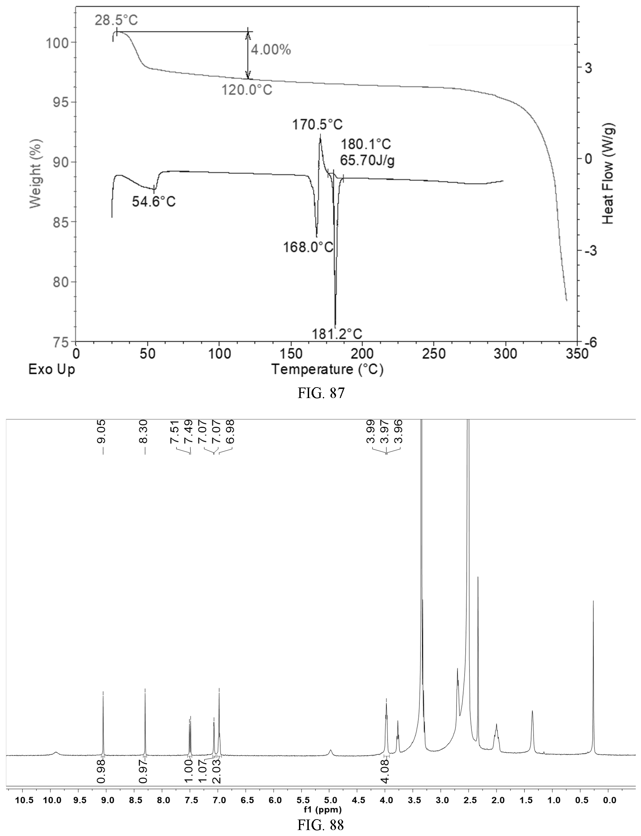

In some embodiments, a TGA/DSC profile of the free crystal form I is substantially as shown in FIG. 87; a ¹H NMR spectrum of the free crystal form I is substantially as shown in FIG. 88.

In some embodiments, the free crystal form I is a hydrate.

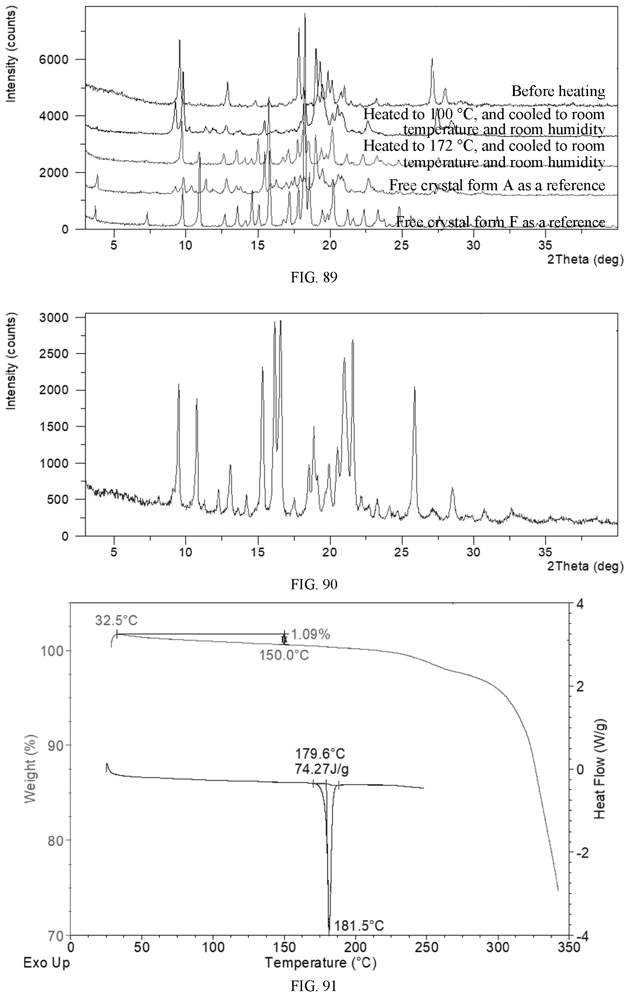

In some embodiments, the present disclosure provides free crystal form J of the compound of formula I, and the free crystal form J has an X-ray powder diffraction pattern comprising diffraction peaks at 2θ±0.2° diffraction angles of 9.4887, 10.7503, 15.3049, 16.1644, 16.5855, 20.9755, 21.5744, and 25.8914; further, the free crystal form J has an X-ray powder diffraction pattern comprising diffraction peaks at 2θ±0.2° diffraction angles of 9.4887, 10.7503, 15.3049, 16.1644, 16.5855, 18.8695, 20.5033, 20.9755, 21.5744, and 25.8914; furthermore, the free crystal form J has an X-ray powder diffraction pattern comprising diffraction peaks at 2θ±0.2° diffraction angles of 9.4887, 10.7503, 15.3049, 16.1644, 16.5855, 18.5225, 18.8695, 19.9313, 20.5033, 20.9755, 21.5744, and 25.8914; furthermore, the free crystal form J has an X-ray powder diffraction pattern comprising diffraction peaks at 2θ±0.2° diffraction angles of 9.4887, 10.7503, 12.2600, 13.0965, 14.1913, 15.3049, 16.1644, 16.5855, 18.5225, 18.8695, 19.1552, 19.9313, 20.5033, 20.9755, 21.5744, 22.1570, 23.2764, 25.8914, and 28.4642; still further, the free crystal form J has an X-ray powder diffraction pattern comprising diffraction peaks at 2θ±0.2° diffraction angles of 9.4887, 10.7503, 12.2600, 13.0965, 14.1913, 15.3049, 16.1644, 16.5855, 17.5096, 18.5225, 18.8695, 19.1552, 19.9313, 20.5033, 20.9755, 21.5744, 22.1570, 23.2764, 24.1115, 24.6829, 25.8914, 27.1763, 28.4642, 29.7254, 30.7261, and 32.6167; still further, the free crystal form J has an XRPD pattern substantially as shown in FIG. 90.

In some embodiments, the free crystal form J has one or two or more of the following characteristics:
(1) a TGA curve showing a weight loss of 0.5-2.5%, preferably about 0.5-1.5% (e.g., 0.5%, 1.0%, or 1.5%), and more preferably about 1.09% at 150±3 °C;
(2) a DSC curve having 1 endothermic peak at 181.5±3 °C.

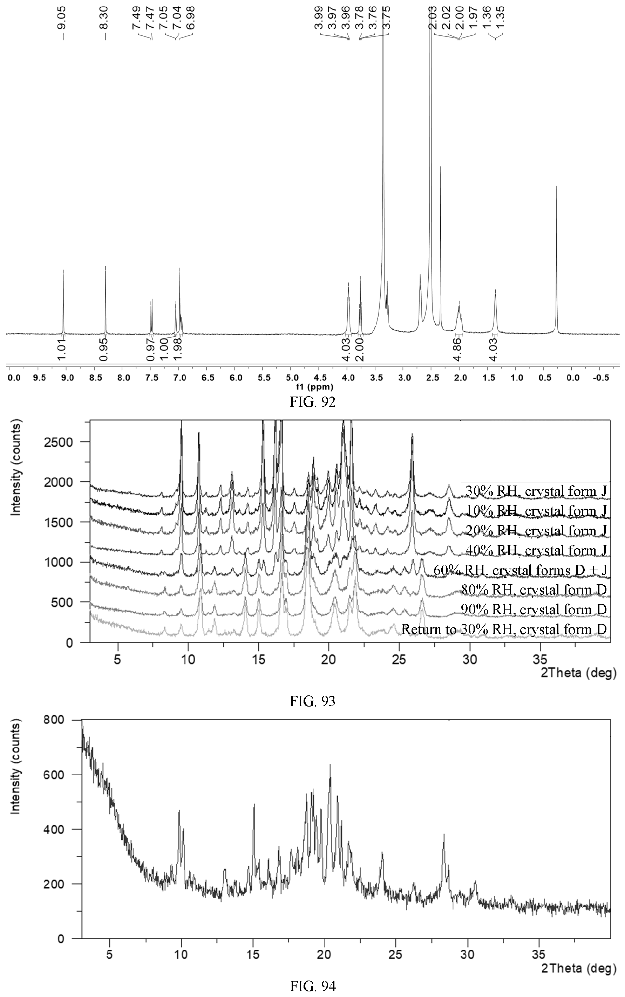

In some embodiments, a TGA/DSC profile of the free crystal form J is substantially as shown in FIG. 91; a ¹H NMR spectrum of the free crystal form J is substantially as shown in FIG. 92.

In some embodiments, the free crystal form J is an anhydrate.

In some embodiments, the present disclosure provides free crystal form K of the compound of formula I, and the free crystal form K has an X-ray powder diffraction pattern comprising diffraction peaks at 2θ±0.2° diffraction angles of 15.0722, 18.7484, 19.0653, 19.7672, 20.4112, and 20.9312; further, the free crystal form K has an X-ray powder diffraction pattern comprising diffraction peaks at 2θ±0.2° diffraction angles of 9.8469, 15.0722, 18.7484, 19.0653, 19.2235, 19.4283, 19.7672, 20.4112, 20.9312, and 21.1881; furthermore, the free crystal form K has an X-ray powder diffraction pattern comprising diffraction peaks at 2θ±0.2° diffraction angles of 9.8469, 10.1208, 15.0722, 18.7484, 19.0653, 19.2235, 19.4283, 19.7672, 20.4112, 20.9312, 21.1881, 21.6963, and 28.3413; furthermore, the free crystal form K has an X-ray powder diffraction pattern comprising diffraction peaks at 2θ±0.2° diffraction angles of 9.8469, 10.1208, 14.6920, 15.0722, 15.4155, 16.0745, 16.7829, 17.6356, 18.1386, 18.7484, 19.0653, 19.2235, 19.4283, 19.7672, 20.4112, 20.9312, 21.1881, 21.6963, 24.0591, 28.3413, 28.6573, and 30.5394; still further, the free crystal form K has an X-ray powder diffraction pattern comprising diffraction peaks at 2θ±0.2° diffraction angles of 9.3256, 9.8469, 10.1208, 13.0145, 14.6920, 15.0722, 15.4155, 16.0745, 16.7829, 17.6356, 18.1386, 18.7484, 19.0653, 19.2235, 19.4283, 19.7672, 20.4112, 20.9312, 21.1881, 21.6963, 24.0591, 26.2506, 28.3413, 28.6573, 30.5394, and 33.0658; still further, the free crystal form K has an XRPD pattern substantially as shown in FIG. 94.

In some embodiments, the free crystal form K has one or two or more of the following characteristics:
(1) a TGA curve showing a weight loss of 6-11%, preferably about 7-10% (e.g., 7.0%, 7.5%, 8.0%, 8.5%, or 9.0%), and more preferably about 8.24% at 150±3 °C;
(2) a DSC curve having 3 endothermic peaks at 72.0±3 °C, 165.2±3 °C, and 180.2±3 °C;
(3) a DSC curve having 1 exothermic peak at 167.1±3 °C.

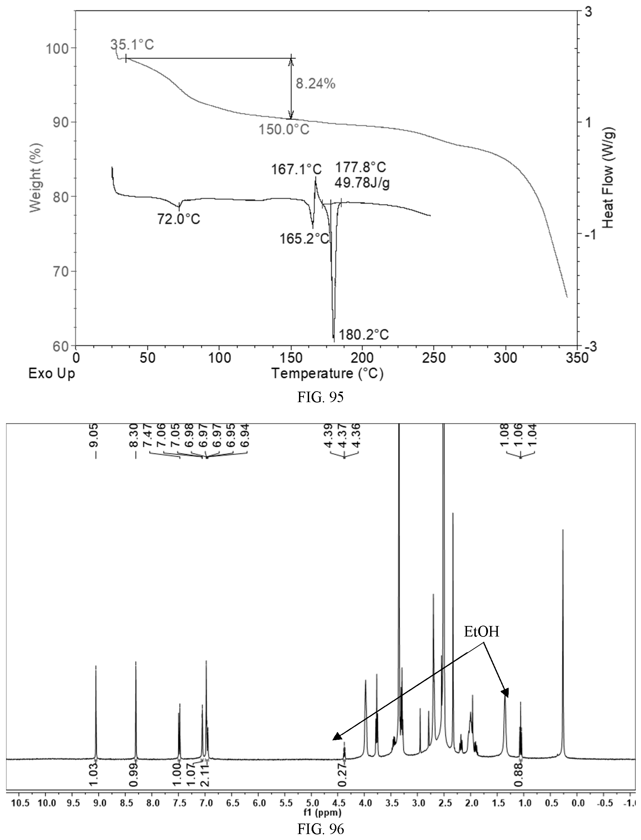

In some embodiments, a TGA/DSC profile of the free crystal form K is substantially as shown in FIG. 95; a ¹H NMR spectrum of the free crystal form K is substantially as shown in FIG. 96.

In some embodiments, the free crystal form K is a solvate.

In some embodiments, the free crystal form K is an EtOH solvate or a co-solvate of EtOH and water.

In some embodiments, in the solvate, EtOH and the free crystal form are in a molar ratio of 0.27.

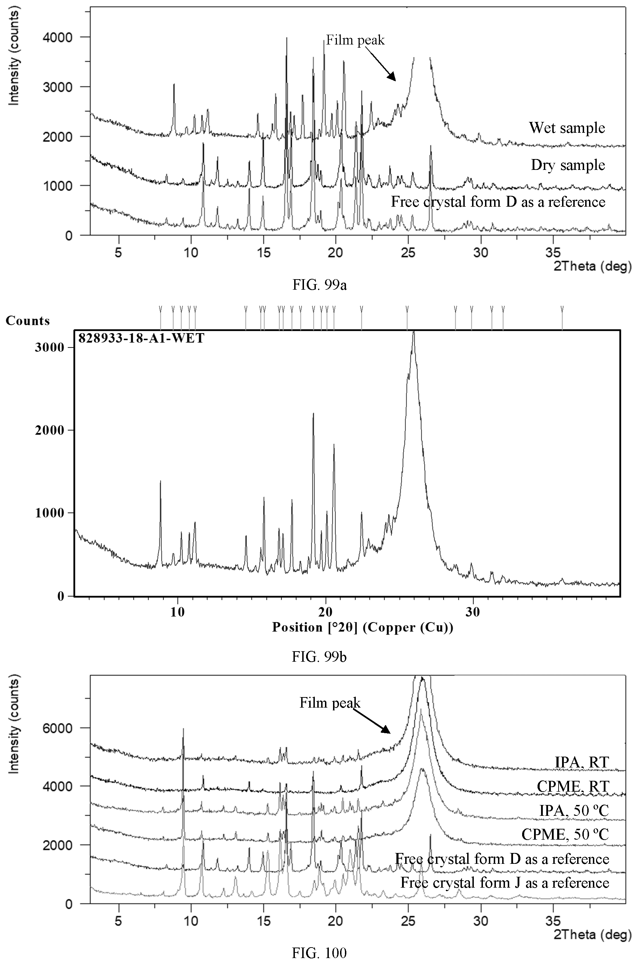

In some embodiments, the present disclosure provides free crystal form L of the compound of formula I, and the free crystal form L has an X-ray powder diffraction pattern comprising diffraction peaks at 2θ±0.2° diffraction angles of 8.8304, 15.8341, 17.7145, 19.1795, 20.5748, and 25.5115; further, the free crystal form L has an X-ray powder diffraction pattern comprising diffraction peaks at 2θ±0.2° diffraction angles of 8.8304, 15.8341, 17.7145, 19.1795, 20.0943, 20.5748, and 25.5115; furthermore, the free crystal form L has an X-ray powder diffraction pattern comprising diffraction peaks at 2θ±0.2° diffraction angles of 8.8304, 15.8341, 17.7145, 19.1795, 20.0943, 20.5748, 22.4409, and 25.5115; furthermore, the free crystal form L has an X-ray powder diffraction pattern comprising diffraction peaks at 2θ±0.2° diffraction angles of 8.8304, 11.1727, 15.8341, 16.8550, 17.1190, 17.7145, 19.1795, 19.7076, 20.0943, 20.5748, 22.4409, and 25.5115; still further, the free crystal form L has an X-ray powder diffraction pattern comprising diffraction peaks at 2θ±0.2° diffraction angles of 8.8304, 9.6805, 10.2446, 10.7612, 11.1727, 14.5973, 15.5971, 15.8341, 16.8550, 17.1190, 17.7145, 18.2884, 19.1795, 19.7076, 20.0943, 20.5748, 22.4409, 25.5115, 28.7935, 29.8794, 31.2614, 32.0233, and 35.9945; still further, the free crystal form L has an XRPD pattern substantially as shown in FIG. 99b.

In another aspect, the present disclosure provides a pharmaceutically acceptable salt of the compound of formula I, which may be selected from a salt formed by the compound of formula I with an acid, wherein the acid may be selected from an inorganic acid and an organic acid. The pharmaceutically acceptable salt of the compound of formula I is selected from hydrochloride, sulfate, maleate, phosphate, p-toluenesulfonate, methanesulfonate, ethanesulfonate, L-aspartate, fumarate, tartrate, and 1,2-ethanedisulfonate of the compound of formula I.

According to a preferred embodiment of the present disclosure, the pharmaceutically acceptable salt of the compound of formula I is hydrochloride, sulfate, maleate, phosphate, p-toluenesulfonate, methanesulfonate, ethanesulfonate, or 1,2-ethanedisulfonate of the compound of formula I.

According to an embodiment of the present disclosure, it will be understood by those skilled in the art that when the compound of formula I forms a salt with an acid, the acid and the compound of formula I may be in a molar ratio of 5:1 to 1:5, e.g., 5:1, 4.5:1, 4:1, 3.5:1, 3:1, 2.5:1, 2:1, 1.5:1, 1:1, 1:1.5, 1:2, 1:2.5, 1:3, 1:3.5, 1:4, 1:4.5, or 1:5. In some other embodiments, the acid and the compound of formula I are in the molar ratio of 1:1, 1.1:1, 1.2:1, 1.3:1, 1.4:1, 1.5:1, 1.6:1, 1.7:1, 1.8:1, 1.9:1, 2.1:1, 2.2:1, 2.3:1, 2.4:1, 2.5:1, 2.6:1, 2.7:1, 2.8:1, 2.9:1, 3.1:1, 3.2:1, 3.3:1, 3.4:1, 3.5:1, 3.6:1, 3.7:1, 3.8:1, 3.9:1, 4.1:1, 4.2:1, 4.3:1, 4.4:1, 4.5:1, 4.6:1, 4.7:1, 4.8:1, or 4.9:1.

In some embodiments, the pharmaceutically acceptable salt of the compound of formula I is hydrochloride of the compound of formula I, wherein in the hydrochloride, hydrochloric acid and the compound of formula I are in a molar ratio of 1:1, 1.1:1, 1.2:1, 1.3:1, or 2:1.

In some embodiments, the pharmaceutically acceptable salt of the compound of formula I is sulfate of the compound of formula I, wherein in the sulfate, sulfuric acid and the compound of formula I are in a molar ratio of 1:1, 1.1:1, 1.2:1, 1.5:1, 1.6:1, or 2:1.

In some embodiments, the pharmaceutically acceptable salt of the compound of formula I is maleate of the compound of formula I, wherein in the maleate, maleic acid and the compound of formula I are in a molar ratio of 1:1.

In some embodiments, the pharmaceutically acceptable salt of the compound of formula I is phosphate of the compound of formula I, wherein in the phosphate, phosphoric acid and the compound of formula I are in a molar ratio of 4:1, 4.3:1, 4.5:1, or 5:1.

In some embodiments, the pharmaceutically acceptable salt of the compound of formula I is p-toluenesulfonate of the compound of formula I, wherein in the p-toluenesulfonate, p-toluenesulfonic acid and the compound of formula I are in a molar ratio of 1:1.

In some embodiments, the pharmaceutically acceptable salt of the compound of formula I is methanesulfonate of the compound of formula I, wherein in the methanesulfonate, methanesulfonic acid and the compound of formula I are in a molar ratio of 1:1 or 1.1:1.

In some embodiments, the pharmaceutically acceptable salt of the compound of formula I is ethanesulfonate of the compound of formula I, wherein in the ethanesulfonate, ethanesulfonic acid and the compound of formula I are in a molar ratio of 1:1 or 1.1:1.

In some embodiments, the pharmaceutically acceptable salt of the compound of formula I is 1,2-ethanedisulfonate of the compound of formula I, wherein in the 1,2-ethanedisulfonate, 1,2-ethanedisulfonic acid and the compound of formula I are in a molar ratio of 1:1, 1.1:1, or 1.2:1.

In another aspect, the present disclosure provides a crystal form of the pharmaceutically acceptable salt of the compound of formula I.

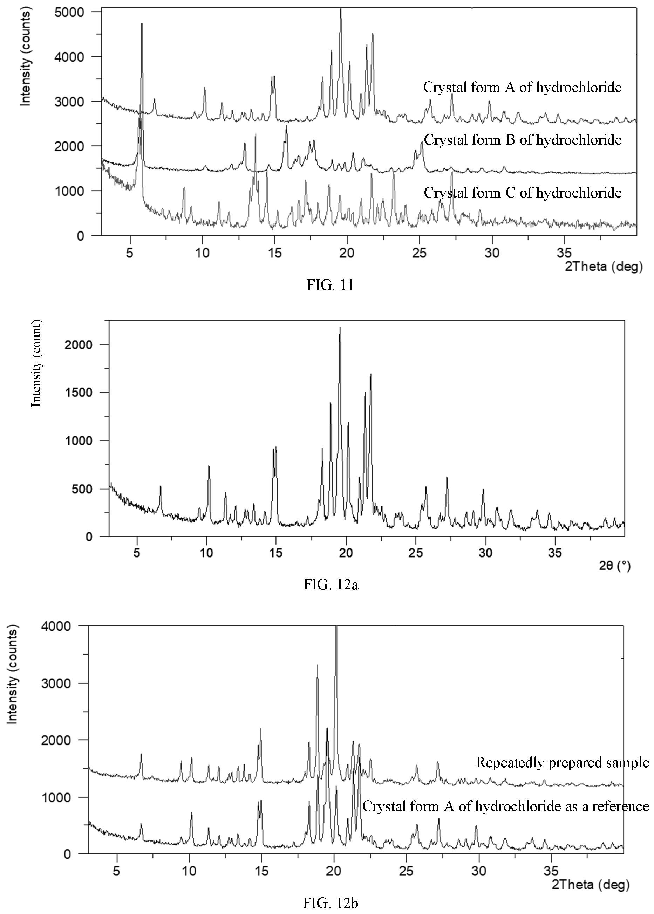

In some embodiments, the present disclosure provides crystal form A of the hydrochloride of the compound of formula I, and the crystal form A of the hydrochloride has an X-ray powder diffraction pattern comprising diffraction peaks at 2θ±0.2° diffraction angles of 18.8832, 19.5324, 20.1382, 21.3379, and 21.7432; further, the crystal form A of the hydrochloride has an X-ray powder diffraction pattern comprising diffraction peaks at 2θ±0.2° diffraction angles of 14.7835, 14.9893, 18.2960, 18.8832, 19.3310, 19.5324, 20.1382, 21.3379, and 21.7432; furthermore, the crystal form A of the hydrochloride has an X-ray powder diffraction pattern comprising diffraction peaks at 2θ±0.2° diffraction angles of 10.1755, 14.7835, 14.9893, 18.2960, 18.8832, 19.3310, 19.5324, 20.1382, 20.9418, 21.3379, 21.7432, 25.7379, 27.2284, and 29.8240; still further, the crystal form A of the hydrochloride has an X-ray powder diffraction pattern comprising diffraction peaks at 2θ±0.2° diffraction angles of 6.6910, 9.4043, 10.1755, 11.3549, 12.0715, 12.7466, 13.3874, 14.1825, 14.7835, 14.9893, 17.2365, 18.2960, 18.8832, 19.3310, 19.5324, 20.1382, 20.9418, 21.3379, 21.7432, 22.5426, 23.5259, 24.0002, 25.3951, 25.7379, 26.6858, 27.2284, 27.7725, 28.5839, 29.1003, 29.8240, 30.7557, 31.8138, 33.7066, 34.5682, 35.3206, 36.1697, 37.1954, 38.5831, and 39.2520; still further, the crystal form A of the hydrochloride has an XRPD pattern substantially as shown in FIG. 12a or 12b.

In some embodiments, the crystal form A of the hydrochloride has one or two of the following characteristics:
(1) a TGA curve of the crystal form A of the hydrochloride showing a weight loss of 4-12%, preferably about 6-10% (e.g., 6.0%, 6.5%, 7.0%, 7.5%, 8.0%, 8.5%, 9.0%, or 9.5%) at 160.0±3 °C;
(2) a DSC curve of the crystal form A of the hydrochloride having one endothermic peak at 165.8±3 °C.

In some embodiments, the crystal form A of the hydrochloride has one or two of the following characteristics:
(1) a TGA curve of the crystal form A of the hydrochloride showing a weight loss of 8.21% at 160.0±3 °C;
(2) a DSC curve of the crystal form A of the hydrochloride having one endothermic peak at 165.8±3 °C.

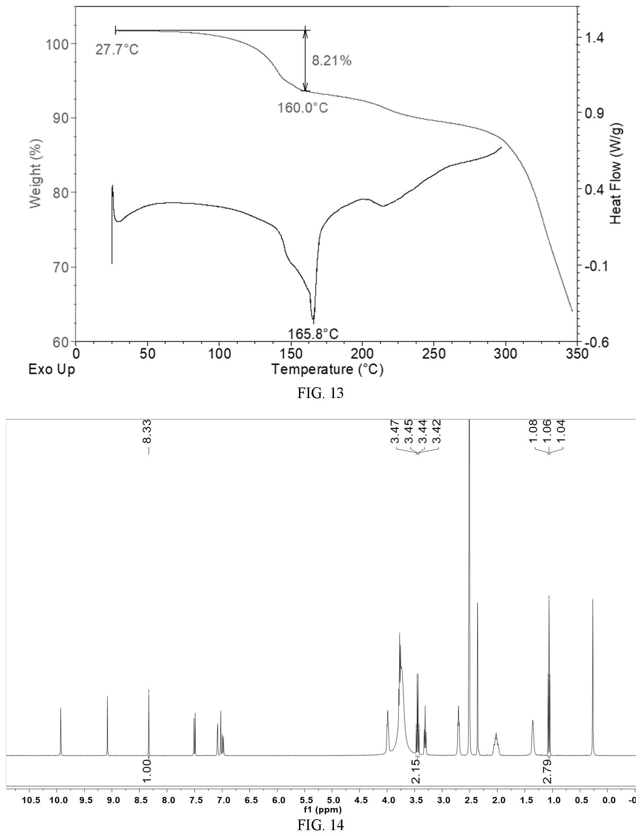

In some embodiments, a TGA/DSC profile of the crystal form A of the hydrochloride is substantially as shown in FIG. 13; a ¹H NMR spectrum of the crystal form A of the hydrochloride is substantially as shown in FIG. 14.

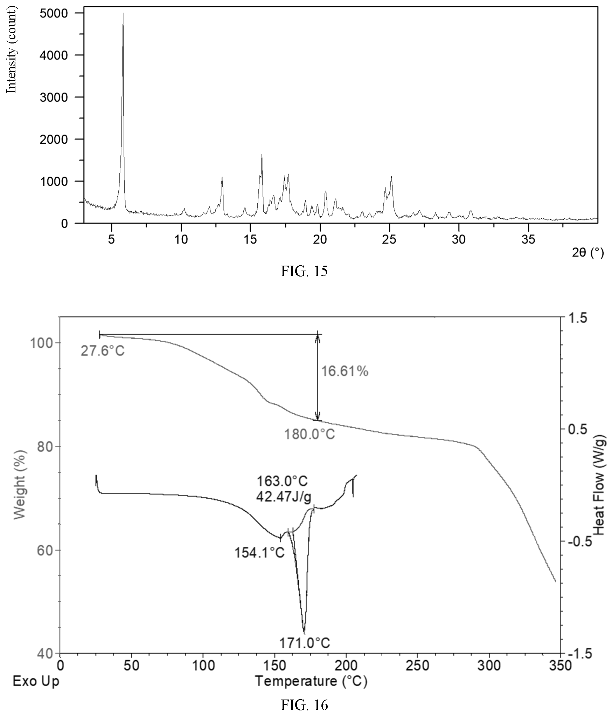

In some embodiments, the present disclosure provides crystal form B of the hydrochloride of the compound of formula I, and the crystal form B of the hydrochloride has an X-ray powder diffraction pattern comprising diffraction peaks at 2θ±0.2° diffraction angles of 5.8284, 12.9418, 15.8305, 17.4248, 17.7208, and 25.1539; further, the crystal form B of the hydrochloride has an X-ray powder diffraction pattern comprising diffraction peaks at 2θ±0.2° diffraction angles of 5.8284, 12.9418, 15.8305, 16.6807, 17.4248, 17.7208, 20.3860, 21.1241, 24.6834, and 25.1539; furthermore, the crystal form B of the hydrochloride has an X-ray powder diffraction pattern comprising diffraction peaks at 2θ±0.2° diffraction angles of 5.8284, 12.9418, 15.6394, 15.8305, 16.6807, 17.4248, 17.7208, 20.3860, 21.1241, 24.6834, and 25.1539; furthermore, the crystal form B of the hydrochloride has an X-ray powder diffraction pattern comprising diffraction peaks at 2θ±0.2° diffraction angles of 5.8284, 12.0258, 12.9418, 14.5910, 15.6394, 15.8305, 16.6807, 17.4248, 17.7208, 18.9434, 19.3954, 19.8130, 20.3860, 21.1241, 24.6834, and 25.1539; still further, the crystal form B of the hydrochloride has an X-ray powder diffraction pattern comprising diffraction peaks at 2θ±0.2° diffraction angles of 5.8284, 10.2084, 12.0258, 12.9418, 14.5910, 15.6394, 15.8305, 16.6807, 17.0761, 17.4248, 17.7208, 18.9434, 19.3954, 19.8130, 20.3860, 21.1241, 23.0260, 23.5341, 24.6834, 25.1539, 26.7017, 27.1748, 28.3119, 29.2775, 30.8075, and 34.0826; still further, the crystal form B of the hydrochloride has an XRPD pattern substantially as shown in FIG. 15.

In some embodiments, the crystal form B of the hydrochloride has one or two of the following characteristics:
(1) a TGA curve of the crystal form B of the hydrochloride showing a weight loss of 10-20%, preferably about 12-18% (e.g., 12.0%, 12.5%, 13.0%, 13.5%, 14.0%, 14.5%, 15.0%, 15.5%, 16.0%, 16.5%, 17.0%, 17.5%, or 18.0%) at 180.0±3 °C;
(2) a DSC curve of the crystal form B of the hydrochloride having 2 endothermic peaks at 154.1±3 °C and 171.0±3 °C.

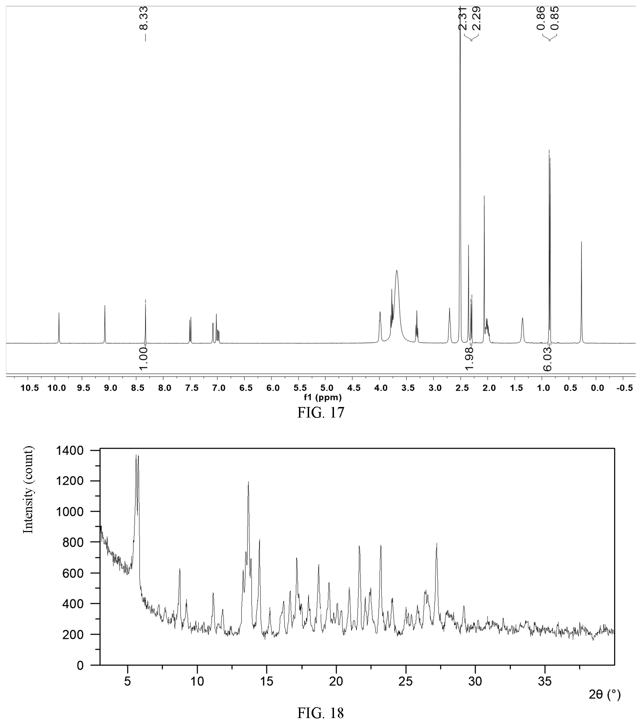

In some embodiments, the crystal form B of the hydrochloride has one or two of the following characteristics:
(1) a TGA curve of the crystal form B of the hydrochloride showing a weight loss of 16.61% at 180.0±3 °C;
(2) a DSC curve of the crystal form B of the hydrochloride having 2 endothermic peaks at 154.1±3 °C and 171.0±3 °C. In some embodiments, a TGA/DSC profile of the crystal form B of the hydrochloride is substantially as shown in FIG. 16; a ¹H NMR spectrum of the crystal form B of the hydrochloride is substantially as shown in FIG. 17.

In some embodiments, the present disclosure provides crystal form C of the hydrochloride of the compound of formula I, and the crystal form C of the hydrochloride has an X-ray powder diffraction pattern comprising diffraction peaks at 2θ±0.2° diffraction angles of 5.6027, 5.7758, 13.6699, 14.4660, 21.6765, 23.2062, and 27.1991; further, the crystal form C of the hydrochloride has an X-ray powder diffraction pattern comprising diffraction peaks at 2θ±0.2° diffraction angles of 5.6027, 5.7758, 13.6699, 14.4660, 17.1465, 18.7028, 21.6765, 23.2062, and 27.1991; furthermore, the crystal form C of the hydrochloride has an X-ray powder diffraction pattern comprising diffraction peaks at 2θ±0.2° diffraction angles of 5.6027, 5.7758, 13.2815, 13.6699, 13.8622, 14.4660, 17.1465, 18.7028, 21.6765, 23.2062, and 27.1991; furthermore, the crystal form C of the hydrochloride has an X-ray powder diffraction pattern comprising diffraction peaks at 2θ±0.2° diffraction angles of 5.6027, 5.7758, 8.7367, 11.1522, 13.2815, 13.6699, 13.8622, 14.4660, 16.6600, 17.1465, 18.7028, 19.4828, 20.9473, 21.6765, 22.4844, 23.2062, 26.3673, and 27.1991; still further, the crystal form C of the hydrochloride has an X-ray powder diffraction pattern comprising diffraction peaks at 2θ±0.2° diffraction angles of 5.6027, 5.7758, 8.7367, 9.2140, 11.1522, 11.8436, 13.2815, 13.6699, 13.8622, 14.4660, 15.2083, 16.1990, 16.6600, 17.1465, 17.9792, 18.7028, 19.4828, 20.0737, 20.3774, 20.9473, 21.6765, 22.0633, 22.4844, 23.2062, 24.0140, 24.9698, 25.8392, 26.3673, 27.1991, 28.0148, 29.1691, and 37.7780; still further, the crystal form C of the hydrochloride has an XRPD pattern substantially as shown in FIG. 18.

In some embodiments, the crystal form C of the hydrochloride has one, two, or three of the following characteristics:
(1) a TGA curve of the crystal form C of the hydrochloride showing a weight loss of 2-10%, preferably about 3-6% (e.g., 3.0%, 3.5%, 4.0%, 4.5%, 5.0%, 5.5%, or 6.0%) at 90.0±3 °C;
(2) a TGA curve of the crystal form C of the hydrochloride showing a weight loss of 2-10%, preferably about 5-8% (e.g., 5.0%, 5.5%, 6.0%, 6.5%, 7.0%, 7.5%, or 8.0%) at 170.0±3 °C;
(3) a DSC curve of the crystal form C of the hydrochloride having 2 endothermic peaks at 77.8±3 °C and 174.6±3 °C.

In some embodiments, the crystal form C of the hydrochloride has one, two, or three of the following characteristics:
(1) a TGA curve of the crystal form C of the hydrochloride showing a weight loss of 4.67% at 90.0±3 °C;
(2) a TGA curve of the crystal form C of the hydrochloride showing a weight loss of 6.00% at 170.0±3 °C;
(3) a DSC curve of the crystal form C of the hydrochloride having 2 endothermic peaks at 77.8±3 °C and 174.6±3 °C.

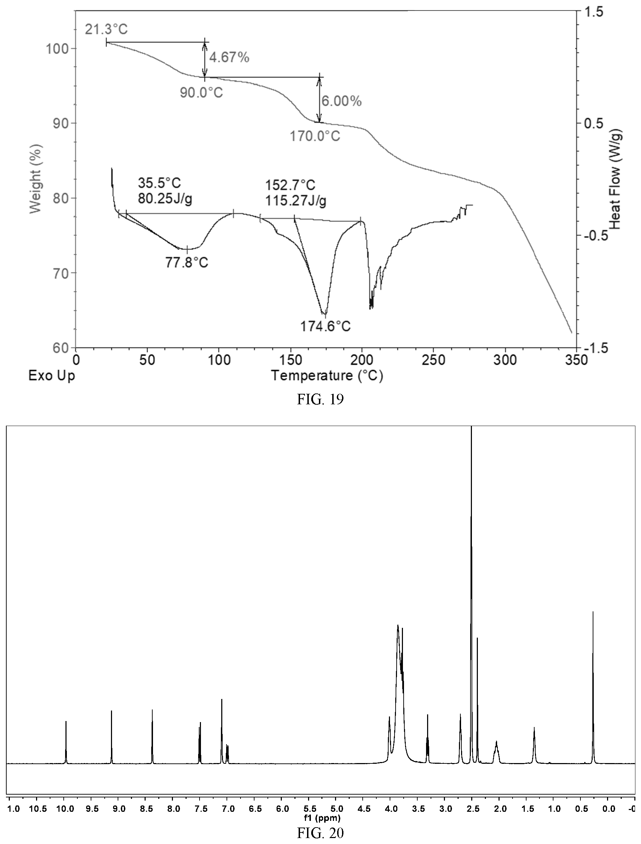

In some embodiments, a TGA/DSC profile of the crystal form C of the hydrochloride is substantially as shown in FIG. 19; a ¹H NMR spectrum of the crystal form C of the hydrochloride is substantially as shown in FIG. 20.

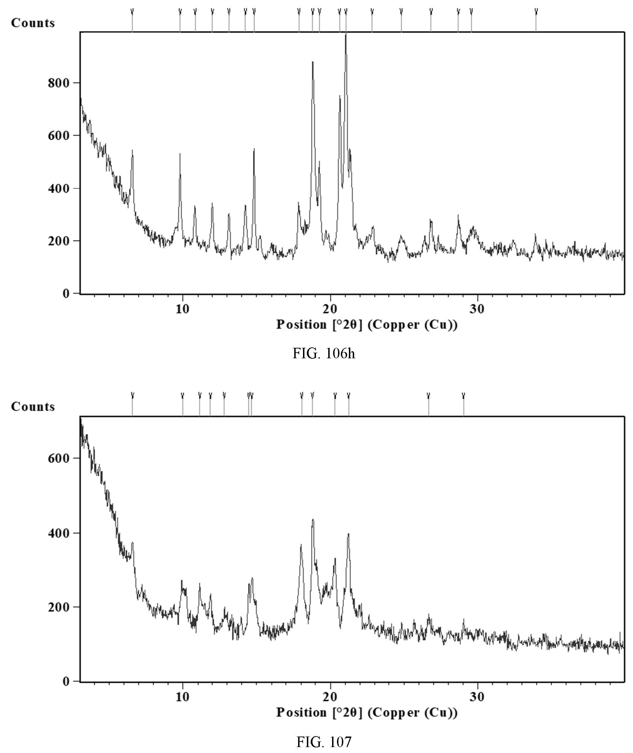

In some embodiments, the present disclosure provides crystal form D of the hydrochloride of the compound of formula I, and the crystal form D of the hydrochloride has an X-ray powder diffraction pattern comprising diffraction peaks at 2θ±0.2° diffraction angles of 14.4539, 14.6720, 18.0560, 18.7688, 20.3323, and 21.2274; further, the crystal form D of the hydrochloride has an X-ray powder diffraction pattern comprising diffraction peaks at 2θ±0.2° diffraction angles of 6.5782, 11.8655, 14.4539, 14.6720, 18.0560, 18.7688, 20.3323, and 21.2274; furthermore, the crystal form D of the hydrochloride has an X-ray powder diffraction pattern comprising diffraction peaks at 2θ±0.2° diffraction angles of 6.5782, 9.9619, 11.8655, 14.4539, 14.6720, 18.0560, 18.7688, 20.3323, and 21.2274; furthermore, the crystal form D of the hydrochloride has an X-ray powder diffraction pattern comprising diffraction peaks at 2θ±0.2° diffraction angles of 6.5782, 9.9619, 11.1481, 11.8655, 14.4539, 14.6720, 18.0560, 18.7688, 20.3323, 21.2274, and 26.6683; still further, the crystal form D of the hydrochloride has an X-ray powder diffraction pattern comprising diffraction peaks at 2θ±0.2° diffraction angles of 6.5782, 9.9619, 11.1481, 11.8655, 12.8040, 14.4539, 14.6720, 18.0560, 18.7688, 20.3323, 21.2274, 26.6683, and 29.0358; still further, the crystal form D of the hydrochloride has an XRPD pattern substantially as shown in FIG. 107.

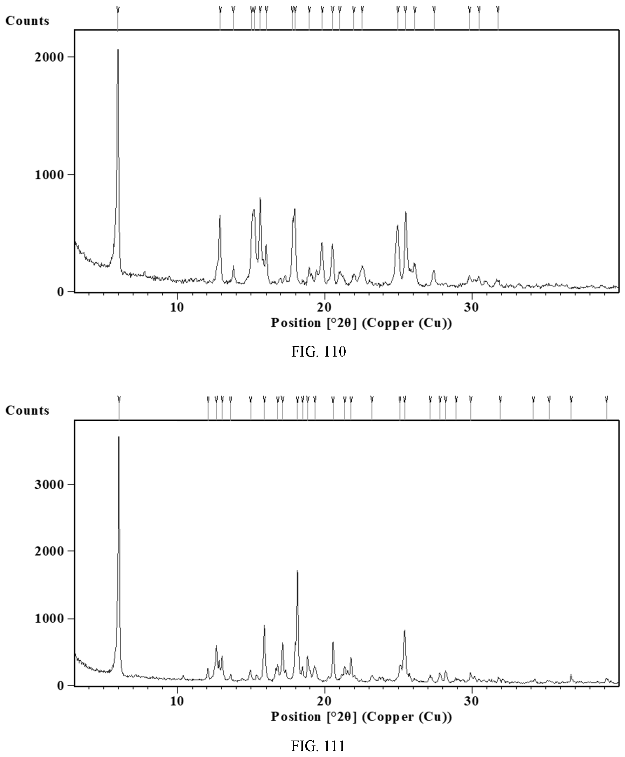

In some embodiments, the present disclosure provides crystal form E of the hydrochloride of the compound of formula I, and the crystal form E of the hydrochloride has an X-ray powder diffraction pattern comprising diffraction peaks at 2θ±0.2° diffraction angles of 5.9750, 12.8980, 15.2093, 15.6063, 17.9714, and 25.4838; further, the crystal form E of the hydrochloride has an X-ray powder diffraction pattern comprising diffraction peaks at 2θ±0.2° diffraction angles of 5.9750, 12.8980, 15.2093, 15.6063, 17.9714, 24.9674, and 25.4838; furthermore, the crystal form E of the hydrochloride has an X-ray powder diffraction pattern comprising diffraction peaks at 2θ±0.2° diffraction angles of 5.9750, 12.8980, 15.0507, 15.2093, 15.6063, 17.8054, 17.9714, 24.9674, and 25.4838; furthermore, the crystal form E of the hydrochloride has an X-ray powder diffraction pattern comprising diffraction peaks at 2θ±0.2° diffraction angles of 5.9750, 12.8980, 15.0507, 15.2093, 15.6063, 16.0284, 17.8054, 17.9714, 19.8405, 20.5435, 22.5488, 24.9674, 25.4838, and 26.1257; still further, the crystal form E of the hydrochloride has an X-ray powder diffraction pattern comprising diffraction peaks at 2θ±0.2° diffraction angles of 5.9750, 12.8980, 13.7925, 15.0507, 15.2093, 15.6063, 16.0284, 17.8054, 17.9714, 18.9298, 19.8405, 20.5435, 21.0218, 21.9935, 22.5488, 24.9674, 25.4838, 26.1257, 27.4268, 29.8322, 30.4635, and 31.7533; still further, the crystal form E of the hydrochloride has an XRPD pattern substantially as shown in FIG. 110.

In some embodiments, the present disclosure provides crystal form F of the hydrochloride of the compound of formula I, and the crystal form F of the hydrochloride has an X-ray powder diffraction pattern comprising diffraction peaks at 2θ±0.2° diffraction angles of 6.0385, 15.9019, 18.1478, 20.5749, and 25.4190; further, the crystal form F of the hydrochloride has an X-ray powder diffraction pattern comprising diffraction peaks at 2θ±0.2° diffraction angles of 6.0385, 12.6362, 15.9019, 17.1363, 18.1478, 20.5749, and 25.4190; furthermore, the crystal form F of the hydrochloride has an X-ray powder diffraction pattern comprising diffraction peaks at 2θ±0.2° diffraction angles of 6.0385, 12.6362, 13.0399, 15.9019, 17.1363, 18.1478, 18.8427, 20.5749, 21.8092, and 25.4190; furthermore, the crystal form F of the hydrochloride has an X-ray powder diffraction pattern comprising diffraction peaks at 2θ±0.2° diffraction angles of 6.0385, 12.6362, 13.0399, 15.9019, 16.8142, 17.1363, 18.1478, 18.4776, 18.8427, 19.3354, 20.5749, 21.3694, 21.8092, 25.1076, and 25.4190; still further, the crystal form F of the hydrochloride has an X-ray powder diffraction pattern comprising diffraction peaks at 2θ±0.2° diffraction angles of 6.0385, 12.0772, 12.6362, 13.0399, 13.5971, 14.9525, 15.9019, 16.8142, 17.1363, 18.1478, 18.4776, 18.8427, 19.3354, 20.5749, 21.3694, 21.8092, 23.2223, 25.1076, 25.4190, 27.1737, 27.8200, 28.2136, 28.9299, 29.9049, 31.9291, 34.1705, 35.2232, 36.7164, and 39.1368; still further, the crystal form F of the hydrochloride has an XRPD pattern substantially as shown in FIG. 111.

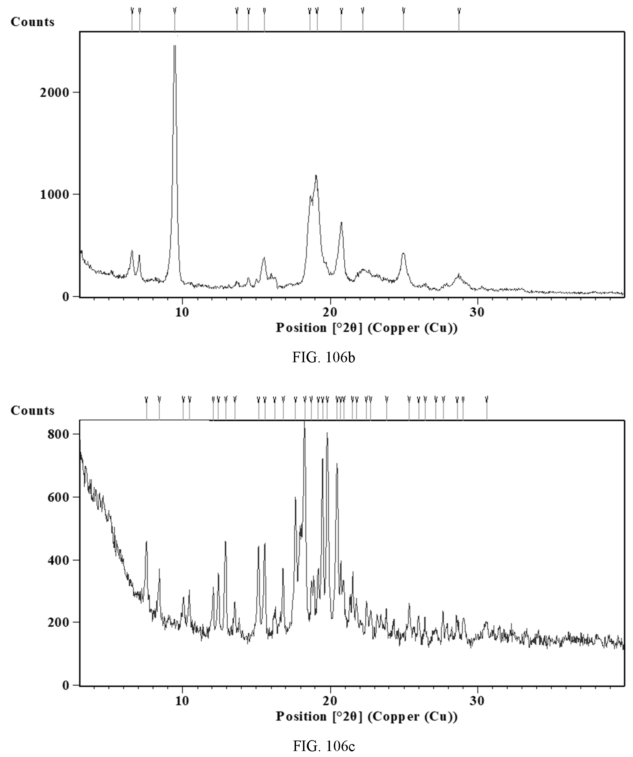

In some embodiments, the present disclosure provides crystal form G of the hydrochloride of the compound of formula I, and the crystal form G of the hydrochloride has an X-ray powder diffraction pattern comprising diffraction peaks at 2θ±0.2° diffraction angles of 9.4719, 18.6112, 19.0898, 20.7520, and 24.9637; further, the crystal form G of the hydrochloride has an X-ray powder diffraction pattern comprising diffraction peaks at 2θ±0.2° diffraction angles of 6.5684, 9.4719, 15.5605, 18.6112, 19.0898, 20.7520, and 24.9637; furthermore, the crystal form G of the hydrochloride has an X-ray powder diffraction pattern comprising diffraction peaks at 2θ±0.2° diffraction angles of 6.5684, 7.0798, 9.4719, 15.5605, 18.6112, 19.0898, 20.7520, and 24.9637; furthermore, the crystal form G of the hydrochloride has an X-ray powder diffraction pattern comprising diffraction peaks at 2θ±0.2° diffraction angles of 6.5684, 7.0798, 9.4719, 15.5605, 18.6112, 19.0898, 20.7520, 22.1933, 24.9637, and 28.7471; still further, the crystal form G of the hydrochloride has an X-ray powder diffraction pattern comprising diffraction peaks at 2θ±0.2° diffraction angles of 6.5684, 7.0798, 9.4719, 13.6772, 14.4725, 15.5605, 18.6112, 19.0898, 20.7520, 22.1933, 24.9637, and 28.7471; still further, the crystal form G of the hydrochloride has an XRPD pattern substantially as shown in FIG. 106b.

In some embodiments, the present disclosure provides crystal form H of the hydrochloride of the compound of formula I, and the crystal form H of the hydrochloride has an X-ray powder diffraction pattern comprising diffraction peaks at 2θ±0.2° diffraction angles of 12.9273, 15.1559, 15.5659, 17.6473, 18.2972, 19.4651, 19.7972, and 20.4603; further, the crystal form H of the hydrochloride has an X-ray powder diffraction pattern comprising diffraction peaks at 2θ±0.2° diffraction angles of 12.9273, 15.1559, 15.5659, 16.8381, 17.6473, 18.2972, 19.4651, 19.7972, 20.4603, and 20.7147; furthermore, the crystal form H of the hydrochloride has an X-ray powder diffraction pattern comprising diffraction peaks at 2θ±0.2° diffraction angles of 7.5428, 12.4381, 12.9273, 15.1559, 15.5659, 16.8381, 17.6473, 18.2972, 19.1721, 19.4651, 19.7972, 20.4603, and 20.7147; furthermore, the crystal form H of the hydrochloride has an X-ray powder diffraction pattern comprising diffraction peaks at 2θ±0.2° diffraction angles of 7.5428, 8.4236, 10.0644, 10.4677, 12.0841, 12.4381, 12.9273, 13.5430, 15.1559, 15.5659, 16.8381, 17.6473, 18.2972, 18.7344, 19.1721, 19.4651, 19.7972, 20.4603, 20.7147, 20.9217, 21.5046, 21.7941, 22.4391, 22.7460, 23.8221, 25.3590, and 27.6629; still further, the crystal form H of the hydrochloride has an X-ray powder diffraction pattern comprising diffraction peaks at 2θ±0.2° diffraction angles of 7.5428, 8.4236, 10.0644, 10.4677, 12.0841, 12.4381, 12.9273, 13.5430, 15.1559, 15.5659, 16.2484, 16.8381, 17.6473, 18.2972, 18.7344, 19.1721, 19.4651, 19.7972, 20.4603, 20.7147, 20.9217, 21.5046, 21.7941, 22.4391, 22.7460, 23.8221, 25.3590, 25.9795, 26.4453, 27.1720, 27.6629, 28.6111, 29.0095, and 30.6017; still further, the crystal form H of the hydrochloride has an XRPD pattern substantially as shown in FIG. 106c.

In some embodiments, the crystal form H of the hydrochloride has one or two or more of the following characteristics:
(1) a TGA curve showing a weight loss of 5-10%, preferably about 6-9% (e.g., 7.0%, 7.5%, or 8.0%), and more preferably about 7.61% at 170±3 °C;
(2) a DSC curve having 2 endothermic peaks at 155.5 °C and 191.5 °C.

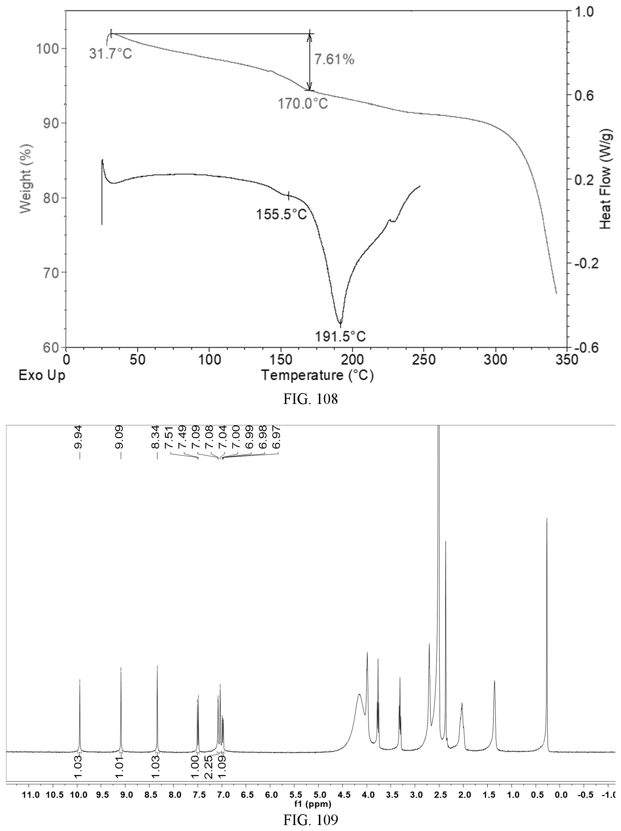

In some embodiments, a TGA/DSC profile of the crystal form H of the hydrochloride is substantially as shown in FIG. 108; a ¹H NMR spectrum of the crystal form H of the hydrochloride is substantially as shown in FIG. 109.

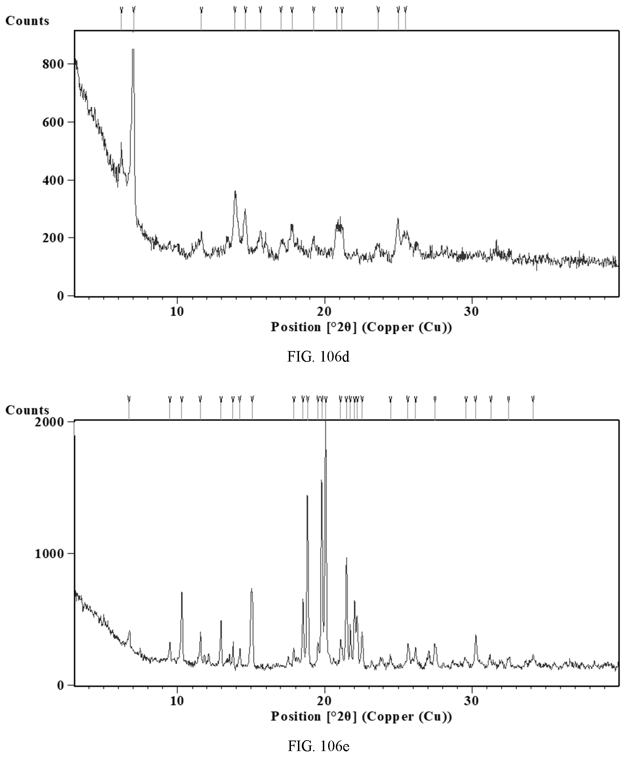

In some embodiments, the present disclosure provides crystal form I of the hydrochloride of the compound of formula I, and the crystal form I of the hydrochloride has an X-ray powder diffraction pattern comprising diffraction peaks at 2θ±0.2° diffraction angles of 6.2065, 7.0236, 13.8915, 14.6038, 20.8063, 21.1874, and 24.9901; further, the crystal form I of the hydrochloride has an X-ray powder diffraction pattern comprising diffraction peaks at 2θ±0.2° diffraction angles of 6.2065, 7.0236, 13.8915, 14.6038, 17.7770, 20.8063, 21.1874, and 24.9901; furthermore, the crystal form I of the hydrochloride has an X-ray powder diffraction pattern comprising diffraction peaks at 2θ±0.2° diffraction angles of 6.2065, 7.0236, 13.8915, 14.6038, 15.6403, 17.7770, 20.8063, 21.1874, 24.9901, and 25.4883; furthermore, the crystal form I of the hydrochloride has an X-ray powder diffraction pattern comprising diffraction peaks at 2θ±0.2° diffraction angles of 6.2065, 7.0236, 11.6337, 13.8915, 14.6038, 15.6403, 17.7770, 19.2571, 20.8063, 21.1874, 24.9901, and 25.4883; still further, the crystal form I of the hydrochloride has an X-ray powder diffraction pattern comprising diffraction peaks at 2θ±0.2° diffraction angles of 6.2065, 7.0236, 11.6337, 13.8915, 14.6038, 15.6403, 17.0255, 17.7770, 19.2571, 20.8063, 21.1874, 23.6537, 24.9901, and 25.4883; still further, the crystal form I of the hydrochloride has an XRPD pattern substantially as shown in FIG. 106d.

In some embodiments, the present disclosure provides crystal form J of the hydrochloride of the compound of formula I, and the crystal form J of the hydrochloride has an X-ray powder diffraction pattern comprising diffraction peaks at 2θ±0.2° diffraction angles of 10.3031, 15.0739, 18.8389, 19.8236, 20.0800, and 21.4914; further, the crystal form J of the hydrochloride has an X-ray powder diffraction pattern comprising diffraction peaks at 2θ±0.2° diffraction angles of 10.3031, 15.0739, 18.5121, 18.8389, 19.8236, 20.0800, 21.4914, and 22.0336; furthermore, the crystal form J of the hydrochloride has an X-ray powder diffraction pattern comprising diffraction peaks at 2θ±0.2° diffraction angles of 10.3031, 12.9670, 15.0739, 18.5121, 18.8389, 19.8236, 20.0800, 21.4914, 21.7526, 22.0336, and 22.2164; furthermore, the crystal form J of the hydrochloride has an X-ray powder diffraction pattern comprising diffraction peaks at 2θ±0.2° diffraction angles of 6.7191, 9.4898, 10.3031, 11.5598, 12.9670, 13.7721, 14.2248, 15.0739, 17.8940, 18.5121, 18.8389, 19.5450, 19.8236, 20.0800, 21.0834, 21.4914, 21.7526, 22.0336, 22.2164, 22.5454, 25.6500, 26.1804, 27.4986, and 30.2463; still further, the crystal form J of the hydrochloride has an X-ray powder diffraction pattern comprising diffraction peaks at 2θ±0.2° diffraction angles of 6.7191, 9.4898, 10.3031, 11.5598, 12.9670, 13.7721, 14.2248, 15.0739, 17.8940, 18.5121, 18.8389, 19.5450, 19.8236, 20.0800, 21.0834, 21.4914, 21.7526, 22.0336, 22.2164, 22.5454, 24.4676, 25.6500, 26.1804, 27.4986, 29.5851, 30.2463, 31.2643, 32.4691, and 34.1280; still further, the crystal form J of the hydrochloride has an XRPD pattern substantially as shown in FIG. 106e.

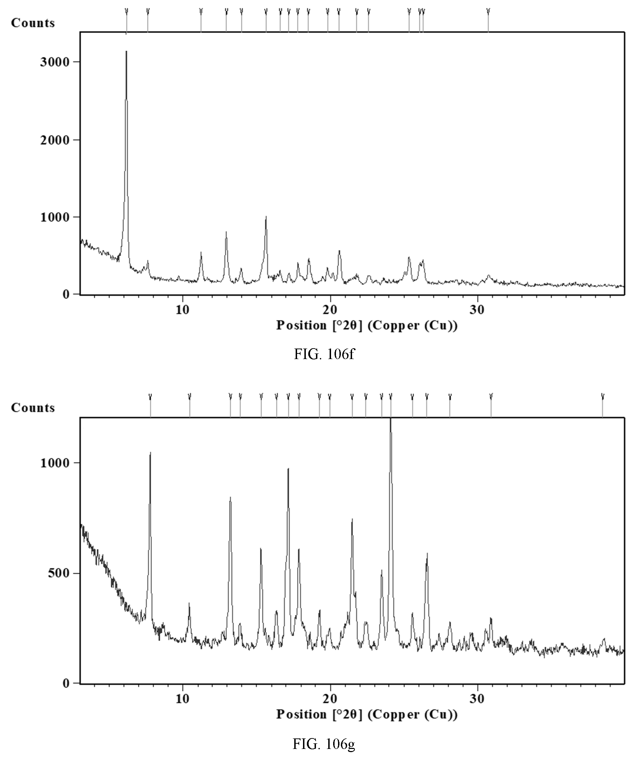

In some embodiments, the present disclosure provides crystal form K of the hydrochloride of the compound of formula I, and the crystal form K of the hydrochloride has an X-ray powder diffraction pattern comprising diffraction peaks at 2θ±0.2° diffraction angles of 6.1719, 11.2365, 12.9493, 15.6381, 20.5819, and 25.3451; further, the crystal form K of the hydrochloride has an X-ray powder diffraction pattern comprising diffraction peaks at 2θ±0.2° diffraction angles of 6.1719, 11.2365, 12.9493, 15.6381, 18.5078, 20.5819, and 25.3451; furthermore, the crystal form K of the hydrochloride has an X-ray powder diffraction pattern comprising diffraction peaks at 2θ±0.2° diffraction angles of 6.1719, 11.2365, 12.9493, 15.6381, 17.8017, 18.5078, 20.5819, 25.3451, 26.0590, and 26.3085; furthermore, the crystal form K of the hydrochloride has an X-ray powder diffraction pattern comprising diffraction peaks at 2θ±0.2° diffraction angles of 6.1719, 7.6126, 11.2365, 12.9493, 13.9646, 15.6381, 17.8017, 18.5078, 19.8165, 20.5819, 25.3451, 26.0590, and 26.3085; still further, the crystal form K of the hydrochloride has an X-ray powder diffraction pattern comprising diffraction peaks at 2θ±0.2° diffraction angles of 6.1719, 7.6126, 11.2365, 12.9493, 13.9646, 15.6381, 16.6189, 17.1911, 17.8017, 18.5078, 19.8165, 20.5819, 21.7916, 22.5977, 25.3451, 26.0590, 26.3085, and 30.7181; still further, the crystal form K of the hydrochloride has an XRPD pattern substantially as shown in FIG. 106f.

In some embodiments, the present disclosure provides crystal form L of the hydrochloride of the compound of formula I, and the crystal form L of the hydrochloride has an X-ray powder diffraction pattern comprising diffraction peaks at 2θ±0.2° diffraction angles of 7.7752, 13.8951, 17.1547, 21.4837, and 24.0925; further, the crystal form L of the hydrochloride has an X-ray powder diffraction pattern comprising diffraction peaks at 2θ±0.2° diffraction angles of 7.7752, 13.8951, 15.3025, 17.1547, 17.8681, 21.4837, 24.0925, and 26.5641; furthermore, the crystal form L of the hydrochloride has an X-ray powder diffraction pattern comprising diffraction peaks at 2θ±0.2° diffraction angles of 7.7752, 13.8951, 15.3025, 17.1547, 17.8681, 21.4837, 23.4738, 24.0925, and 26.5641; furthermore, the crystal form L of the hydrochloride has an X-ray powder diffraction pattern comprising diffraction peaks at 2θ±0.2° diffraction angles of 7.7752, 10.4411, 13.8951, 15.3025, 16.3403, 17.1547, 17.8681, 19.2621, 21.4837, 23.4738, 24.0925, 25.5726, 26.5641, 28.1186, and 30.8899; still further, the crystal form L of the hydrochloride has an X-ray powder diffraction pattern comprising diffraction peaks at 2θ±0.2° diffraction angles of 7.7752, 10.4411, 13.2333, 13.8951, 15.3025, 16.3403, 17.1547, 17.8681, 19.2621, 19.9563, 21.4837, 22.4156, 23.4738, 24.0925, 25.5726, 26.5641, 28.1186, 30.8899, and 38.4894; still further, the crystal form L of the hydrochloride has an XRPD pattern substantially as shown in FIG. 106g.

In some embodiments, the present disclosure provides crystal form M of the hydrochloride of the compound of formula I, and the crystal form M of the hydrochloride has an X-ray powder diffraction pattern comprising diffraction peaks at 2θ±0.2° diffraction angles of 9.8040, 14.8344, 18.8157, 19.2503, 20.6275, and 21.0461; further, the crystal form M of the hydrochloride has an X-ray powder diffraction pattern comprising diffraction peaks at 2θ±0.2° diffraction angles of 6.5546, 9.8040, 14.8344, 18.8157, 19.2503, 20.6275, and 21.0461; furthermore, the crystal form M of the hydrochloride has an X-ray powder diffraction pattern comprising diffraction peaks at 2θ±0.2° diffraction angles of 6.5546, 9.8040, 12.0038, 14.2374, 14.8344, 17.8676, 18.8157, 19.2503, 20.6275, and 21.0461; furthermore, the crystal form M of the hydrochloride has an X-ray powder diffraction pattern comprising diffraction peaks at 2θ±0.2° diffraction angles of 6.5546, 9.8040, 10.8235, 12.0038, 13.1333, 14.2374, 14.8344, 17.8676, 18.8157, 19.2503, 20.6275, 21.0461, 26.8393, and 28.6992; still further, the crystal form M of the hydrochloride has an X-ray powder diffraction pattern comprising diffraction peaks at 2θ±0.2° diffraction angles of 6.5546, 9.8040, 10.8235, 12.0038, 13.1333, 14.2374, 14.8344, 17.8676, 18.8157, 19.2503, 20.6275, 21.0461, 22.8294, 24.8213, 26.8393, 28.6992, 29.5703, and 33.9685; still further, the crystal form M of the hydrochloride has an XRPD pattern substantially as shown in FIG. 106h.

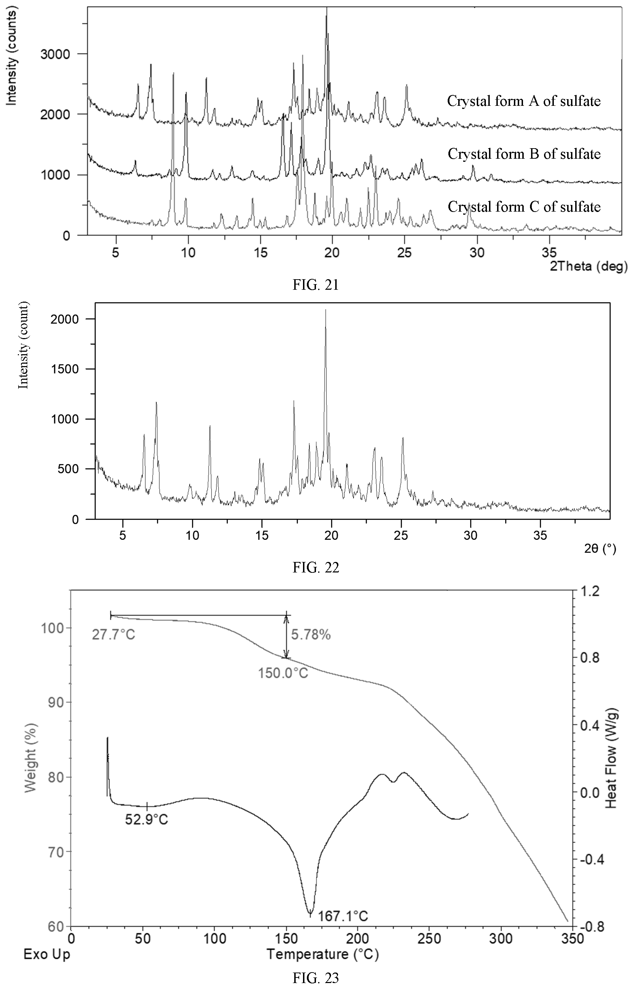

In some embodiments, the present disclosure provides crystal form A of the sulfate of the compound of formula I, and the crystal form A of the sulfate has an X-ray powder diffraction pattern comprising diffraction peaks at 2θ±0.2° diffraction angles of 6.5297, 7.4156, 11.2590, 17.2920, and 19.5870; further, the crystal form A of the sulfate has an X-ray powder diffraction pattern comprising diffraction peaks at 2θ±0.2° diffraction angles of 6.5297, 7.4156, 11.2590, 17.2920, 18.3831, 18.9056, 19.5870, 23.1076, and 25.1201; furthermore, the crystal form A of the sulfate has an X-ray powder diffraction pattern comprising diffraction peaks at 2θ±0.2° diffraction angles of 6.5297, 7.4156, 11.2590, 17.2920, 18.3831, 18.9056, 19.5870, 19.8280, 23.1076, and 25.1201; furthermore, the crystal form A of the sulfate has an X-ray powder diffraction pattern comprising diffraction peaks at 2θ±0.2° diffraction angles of 6.5297, 7.4156, 11.2590, 14.8207, 15.0692, 17.2920, 17.5479, 18.3831, 18.9056, 19.5870, 19.8280, 21.1050, 23.1076, 23.6024, and 25.1201; still further, the crystal form A of the sulfate has an X-ray powder diffraction pattern comprising diffraction peaks at 2θ±0.2° diffraction angles of 6.5297, 7.4156, 9.8272, 11.2590, 11.7996, 13.0075, 14.8207, 15.0692, 17.2920, 17.5479, 18.3831, 18.9056, 19.5870, 19.8280, 20.1191, 21.1050, 21.9720, 23.1076, 23.6024, 25.1201, 27.2805, 28.7070, 29.8446, and 32.6299; still further, the crystal form A of the sulfate has an XRPD pattern substantially as shown in FIG. 22.

In some embodiments, the crystal form A of the sulfate has one or two of the following characteristics:
(1) a TGA curve of the crystal form A of the sulfate showing a weight loss of 2-10%, preferably about 4-7% (e.g., 4.0%, 4.5%, 5.0%, 5.5%, 6.0%, 6.5%, or 7.0%) at 150.0±3 °C;
(2) a DSC curve of the crystal form A of the sulfate having 2 endothermic peaks at 52.9±3 °C and 167.1±3 °C.

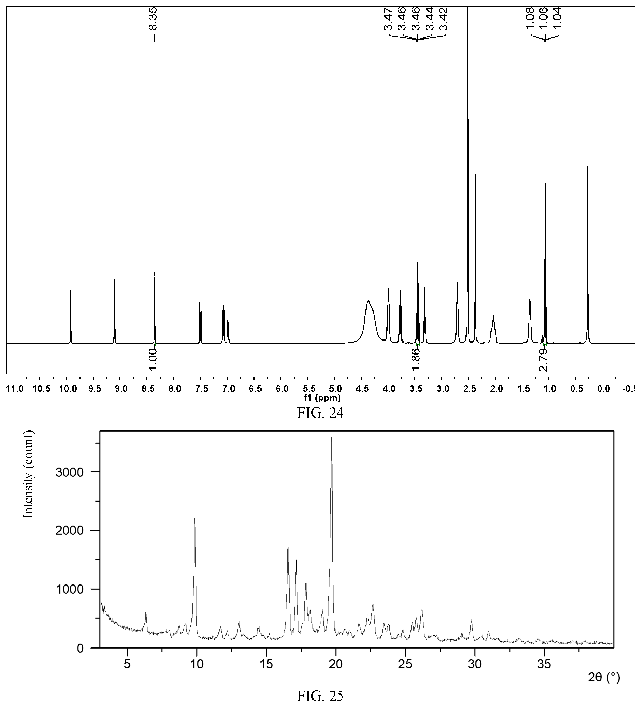

In some embodiments, the crystal form A of the sulfate has one or two of the following characteristics:
(1) a TGA curve of the crystal form A of the sulfate showing a weight loss of 5.78% at 150.0±3 °C;
(2) a DSC curve of the crystal form A of the sulfate having 2 endothermic peaks at 52.9±3 °C and 167.1±3 °C. In some embodiments, a TGA/DSC profile of the crystal form A of the sulfate is substantially as shown in FIG. 23; a ¹H NMR spectrum of the crystal form A of the sulfate is substantially as shown in FIG. 24.

In some embodiments, the present disclosure provides crystal form B of the sulfate of the compound of formula I, and the crystal form B of the sulfate has an X-ray powder diffraction pattern comprising diffraction peaks at 2θ±0.2° diffraction angles of 9.8491, 16.5561, 17.1270, 17.8255, and 19.6977; further, the crystal form B of the sulfate has an X-ray powder diffraction pattern comprising diffraction peaks at 2θ±0.2° diffraction angles of 9.8491, 16.5561, 17.1270, 17.8255, 18.1372, 19.6977, 19.0468, 22.6654, and 26.1622; furthermore, the crystal form B of the sulfate has an X-ray powder diffraction pattern comprising diffraction peaks at 2θ±0.2° diffraction angles of 6.3375, 9.8491, 13.0250, 16.5561, 17.1270, 17.8255, 18.1372, 19.6977, 19.0468, 22.2378, 22.6654, 25.7586, 26.1622, and 29.7168; still further, the crystal form B of the sulfate has an X-ray powder diffraction pattern comprising diffraction peaks at 2θ±0.2° diffraction angles of 6.3375, 8.6916, 9.1864, 9.8491, 11.7001, 12.1619, 13.0250, 14.4700, 15.1807, 16.5561, 17.1270, 17.8255, 18.1372, 19.0468, 19.6977, 21.6775, 22.2378, 22.6654, 23.4512, 23.8100, 24.8186, 25.4720, 25.7586, 26.1622, 27.1217, 29.0464, 29.7168, 30.9706, 33.1687, and 34.5562; still further, the crystal form B of the sulfate has an XRPD pattern substantially as shown in FIG. 25.

In some embodiments, the crystal form B of the sulfate has one or two of the following characteristics:
(1) a TGA curve of the crystal form B of the sulfate showing a weight loss of 0-5%, preferably about 1-3% (e.g., 1.0%, 1.5%, 2.0%, 2.5%, or 3.0%) at 150.0±3 °C;
(2) a DSC curve of the crystal form B of the sulfate having 2 endothermic peaks at 69.7±3 °C and 238.5±3 °C.

In some embodiments, the crystal form B of the sulfate has one or two of the following characteristics:
(1) a TGA curve of the crystal form B of the sulfate showing a weight loss of 1.16% at 150.0±3 °C;
(2) a DSC curve of the crystal form B of the sulfate having 2 endothermic peaks at 69.7±3 °C and 238.5±3 °C.

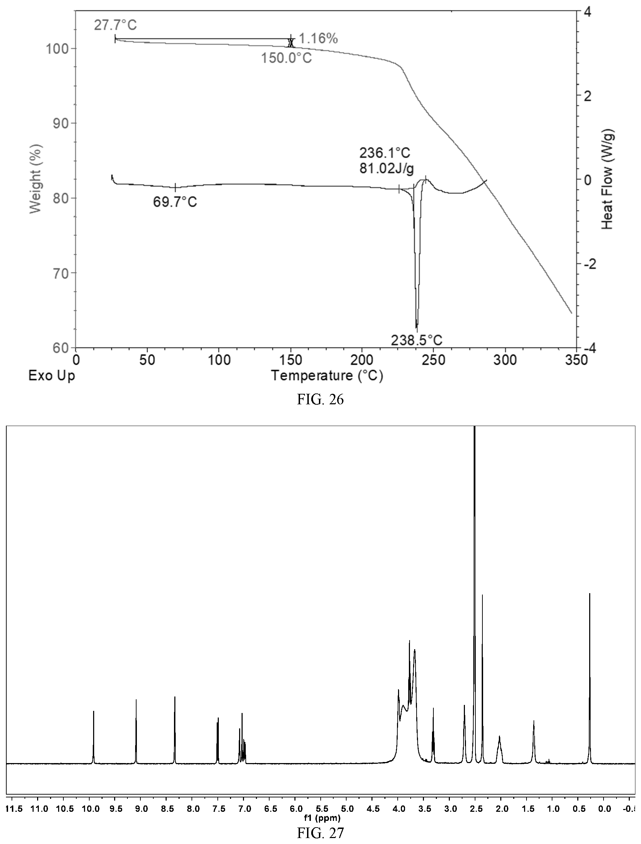

In some embodiments, a TGA/DSC profile of the crystal form B of the sulfate is substantially as shown in FIG. 26; a ¹H NMR spectrum of the crystal form B of the sulfate is substantially as shown in FIG. 27.

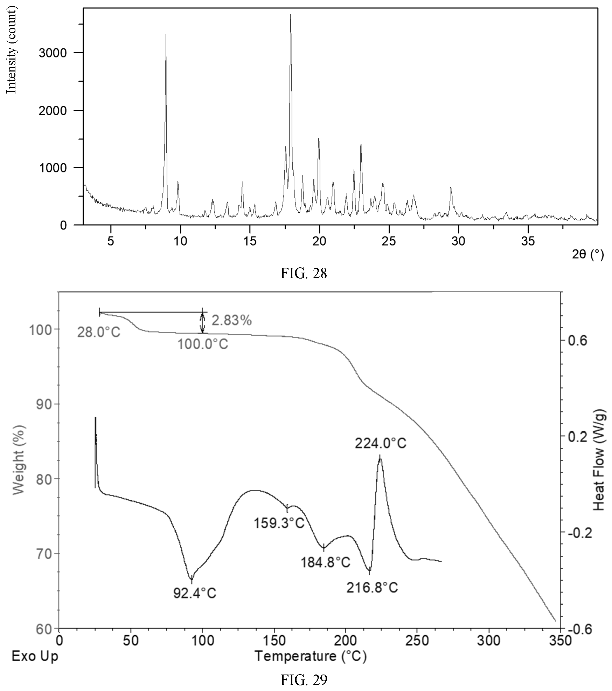

In some embodiments, the present disclosure provides crystal form C of the sulfate of the compound of formula I, and the crystal form C of the sulfate has an X-ray powder diffraction pattern comprising diffraction peaks at 2θ±0.2° diffraction angles of 8.9487, 17.5523, 17.9120, 19.9597, and 22.9905; further, the crystal form C of the sulfate has an X-ray powder diffraction pattern comprising diffraction peaks at 2θ±0.2° diffraction angles of 8.9487, 9.8314, 14.4549, 17.5523, 17.9120, 18.7584, 19.5850, 19.9597, 20.9974, 22.4850, 22.9905, 24.5952, and 29.4160; furthermore, the crystal form C of the sulfate has an X-ray powder diffraction pattern comprising diffraction peaks at 2θ±0.2° diffraction angles of 8.9487, 9.8314, 12.2693, 14.4549, 17.5523, 17.9120, 18.7584, 19.5850, 19.9597, 20.6152, 20.9974, 21.9335, 22.4850, 22.9905, 23.6939, 23.9937, 24.5952, 26.7497, and 29.4160; still further, the crystal form C of the sulfate has an X-ray powder diffraction pattern comprising diffraction peaks at 2θ±0.2° diffraction angles of 7.4762, 8.0099, 8.9487, 9.8314, 11.7697, 12.2693, 13.3788, 14.4549, 14.9774, 15.3199, 16.8293, 17.5523, 17.9120, 18.7584, 19.5850, 19.9597, 20.6152, 20.9974, 21.9335, 22.4850, 22.9905, 23.6939, 23.9937, 24.5952, 24.8904, 25.3759, 26.2847, 26.7497, 28.4461, 29.4160, 31.7091, 33.4595, 34.8478, 36.5099, and 38.0959; still further, the crystal form C of the sulfate has an XRPD pattern substantially as shown in FIG. 28.

In some embodiments, the crystal form C of the sulfate has one, two, or three of the following characteristics:
(1) a TGA curve of the crystal form C of the sulfate showing a weight loss of 1-5%, preferably about 1-4% (e.g., 1.0%, 1.5%, 2.0%, 2.5%, 3.0%, 3.5%, or 4.0%) at 100.0±3 °C;
(2) a DSC curve of the crystal form C of the sulfate having 4 endothermic peaks at 92.4±3 °C, 159.3±3 °C, 184.8±3 °C, and 216.8±3 °C;
(3) a DSC curve of the crystal form C of the sulfate having 1 exothermic peak at 224.0±3 °C.

In some embodiments, the crystal form C of the sulfate has one, two, or three of the following characteristics:
(1) a TGA curve of the crystal form C of the sulfate showing a weight loss of 2.83% at 100.0±3 °C;
(2) a DSC curve of the crystal form C of the sulfate having 4 endothermic peaks at 92.4±3 °C, 159.3±3 °C, 184.8±3 °C, and 216.8±3 °C;
(3) a DSC curve of the crystal form C of the sulfate having 1 exothermic peak at 224.0±3 °C.

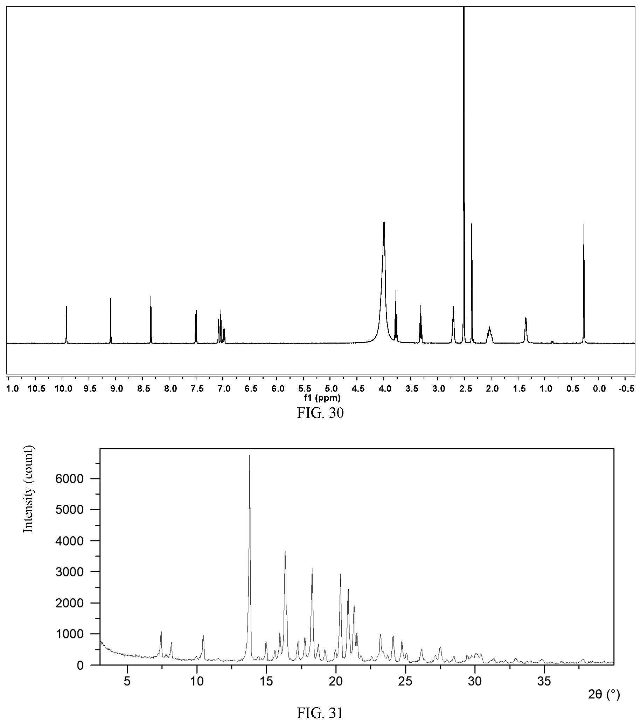

In some embodiments, a TGA/DSC profile of the crystal form C of the sulfate is substantially as shown in FIG. 29; a ¹H NMR spectrum of the crystal form C of the sulfate is as shown in FIG. 30.

In some embodiments, the present disclosure provides crystal form A of the maleate of the compound of formula I, and the crystal form A of the maleate has an X-ray powder diffraction pattern comprising diffraction peaks at 2θ±0.2° diffraction angles of 13.7865, 16.3294, 18.2795, 20.3336, and 20.8824; further, the crystal form A of the maleate has an X-ray powder diffraction pattern comprising diffraction peaks at 2θ±0.2° diffraction angles of 7.4318, 13.7865, 15.9646, 16.3294, 18.2795, 20.3336, 20.8824, 21.3104, 21.5283, 23.1978, and 24.1136; furthermore, the crystal form A of the maleate has an X-ray powder diffraction pattern comprising diffraction peaks at 2θ±0.2° diffraction angles of 7.4318, 10.4550, 13.7865, 15.9646, 16.3294, 17.7554, 18.2795, 20.3336, 20.8824, 21.3104, 21.5283, 23.1978, 24.1136, and 24.7430; still further, the crystal form A of the maleate has an X-ray powder diffraction pattern comprising diffraction peaks at 2θ±0.2° diffraction angles of 7.4318, 8.1664, 10.4550, 11.4963, 13.7865, 14.9748, 15.6098, 15.9646, 16.3294, 17.2388, 17.7554, 18.2795, 18.7232, 19.2252, 19.9626, 20.3336, 20.8824, 21.3104, 21.5283, 21.8250, 22.5862, 23.1978, 23.7175, 24.1136, 24.7430, 25.0797, 26.1594, 27.1361, 27.5054, 28.4773, 29.4248, 30.0997, 30.4233, 31.3563, 32.1988, 32.8832, 34.8721, 36.2350, and 37.8027; still further, the crystal form A of the maleate has an XRPD pattern substantially as shown in FIG. 31.

In some embodiments, the crystal form A of the maleate has one or two of the following characteristics:
(1) a TGA curve of the crystal form A of the maleate showing a weight loss of 0-4%, preferably about 0-3% (e.g., 0.5%, 1.0%, 1.5%, 2.0%, 2.5%, or 3.0%) at 130.0±3 °C;
(2) a DSC curve of the crystal form A of the maleate having 1 endothermic peak at 142.1±3 °C.

In some embodiments, the crystal form A of the maleate has one or two of the following characteristics:
(1) a TGA curve of the crystal form A of the maleate showing a weight loss of 0.42% at 130.0±3 °C;
(2) a DSC curve of the crystal form A of the maleate having 1 endothermic peak at 142.1±3 °C.

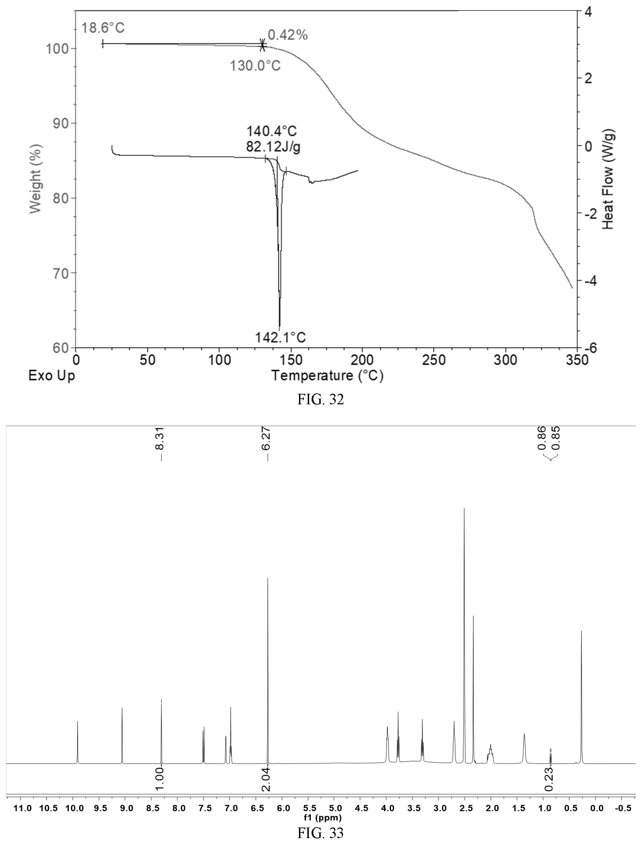

In some embodiments, a TGA/DSC profile of the crystal form A of the maleate is substantially as shown in FIG. 32; a ¹H NMR spectrum of the crystal form A of the maleate is as shown in FIG. 33.

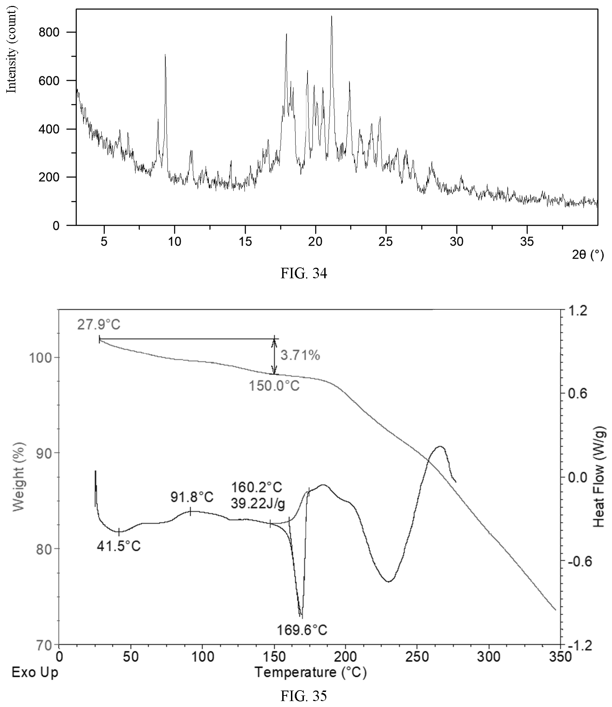

In some embodiments, the present disclosure provides crystal form A of the phosphate of the compound of formula I, and the crystal form A of the phosphate has an X-ray powder diffraction pattern comprising diffraction peaks at 2θ±0.2° diffraction angles of 9.3607, 17.8932, 19.3716, 19.8946, 20.5448, 21.1372, and 22.4294; further, the crystal form A of the phosphate has an X-ray powder diffraction pattern comprising diffraction peaks at 2θ±0.2° diffraction angles of 8.8155, 9.3607, 17.8932, 18.4384, 19.3716, 19.8946, 20.5448, 21.1372, 22.4294, and 24.5486; furthermore, the crystal form A of the phosphate has an X-ray powder diffraction pattern comprising diffraction peaks at 2θ±0.2° diffraction angles of 8.8155, 9.3607, 11.1767, 16.4840, 17.8932, 18.4384, 19.3716, 19.8946, 20.5448, 21.1372, 22.4294, 23.1105, 23.9976, 24.5486, 25.7922, and 26.3981; still further, the crystal form A of the phosphate has an X-ray powder diffraction pattern comprising diffraction peaks at 2θ±0.2° diffraction angles of 3.2059, 8.8155, 9.3607, 11.1767, 13.9605, 16.4840, 17.8932, 18.4384, 19.3716, 19.8946, 20.5448, 21.1372, 22.4294, 23.1105, 23.9976, 24.5486, 25.7922, 26.3981, 26.9789, 28.2812, and 30.2804; still further, the crystal form A of the phosphate has an XRPD pattern substantially as shown in FIG. 34.

In some embodiments, the crystal form A of the phosphate has one, two, or three of the following characteristics:
(1) a TGA curve of the crystal form A of the phosphate showing a weight loss of 1-6%, preferably about 2-5% (e.g., 2.0%, 2.5%, 3.0%, 3.5%, 4.0%, 4.5%, or 5.0%) at 150.0±3 °C;
(2) a DSC curve of the crystal form A of the phosphate having 2 endothermic peaks at 41.5±3 °C and 169.6±3 °C;
(3) a DSC curve of the crystal form A of the phosphate having 1 exothermic peak at 91.8 °C±3 °C.

In some embodiments, the crystal form A of the phosphate has one, two, or three of the following characteristics:
(1) a TGA curve of the crystal form A of the phosphate showing a weight loss of 3.71% at 150.0±3 °C;
(2) a DSC curve of the crystal form A of the phosphate having 2 endothermic peaks at 41.5±3 °C and 169.6±3 °C;
(3) a DSC curve of the crystal form A of the phosphate having 1 exothermic peak at 91.8 °C±3 °C.

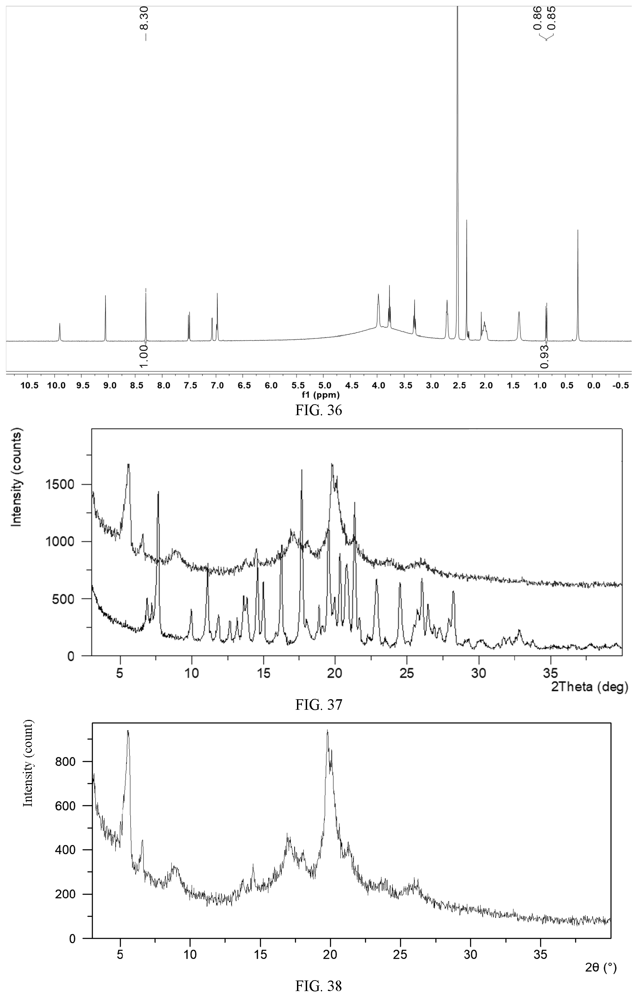

In some embodiments, a TGA/DSC profile of the crystal form A of the phosphate is substantially as shown in FIG. 35; a ¹H NMR spectrum of the crystal form A of the phosphate is as shown in FIG. 36.

In some embodiments, the present disclosure provides crystal form A of the p-toluenesulfonate of the compound of formula I, and the crystal form A of the p-toluenesulfonate has an X-ray powder diffraction pattern comprising diffraction peaks at 2θ±0.2° diffraction angles of 5.6569, 6.5865, 9.0105, 14.4962, 17.1789, and 19.7973; further, the crystal form A of the p-toluenesulfonate has an XRPD pattern substantially as shown in FIG. 38.

In some embodiments, the crystal form A of the p-toluenesulfonate has one or two of the following characteristics:
(1) a TGA curve of the crystal form A of the p-toluenesulfonate showing a weight loss of 2-8%, preferably about 2-6% (e.g., 2.0%, 2.5%, 3.0%, 3.5%, 4.0%, 4.5%, 5.0%, 5.5%, or 6.0%) at 150.0±3 °C;
(2) a DSC curve of the crystal form A of the p-toluenesulfonate having 2 endothermic peaks at 81.6±3 °C and 156.0±3 °C.

In some embodiments, the crystal form crystal form A of the p-toluenesulfonate has one or two of the following characteristics:
(1) a TGA curve of the crystal form A of the p-toluenesulfonate showing a weight loss of 4.15% at 150.0±3 °C;
(2) a DSC curve of the crystal form A of the p-toluenesulfonate having 2 endothermic peaks at 81.6±3 °C and 156.0±3 °C.

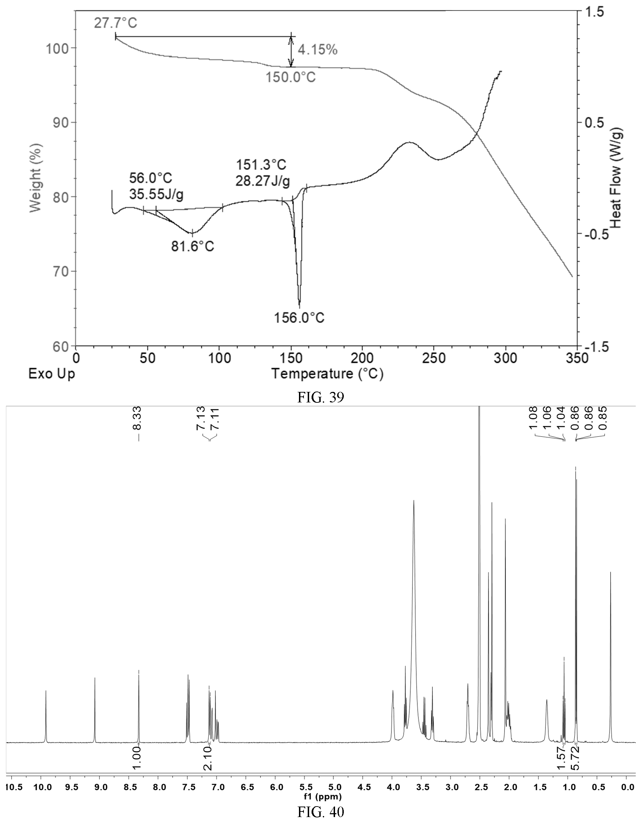

In some embodiments, a TGA/DSC profile of the crystal form A of the p-toluenesulfonate is substantially as shown in FIG. 39; a ¹H NMR spectrum of the crystal form A of the p-toluenesulfonate is as shown in FIG. 40.

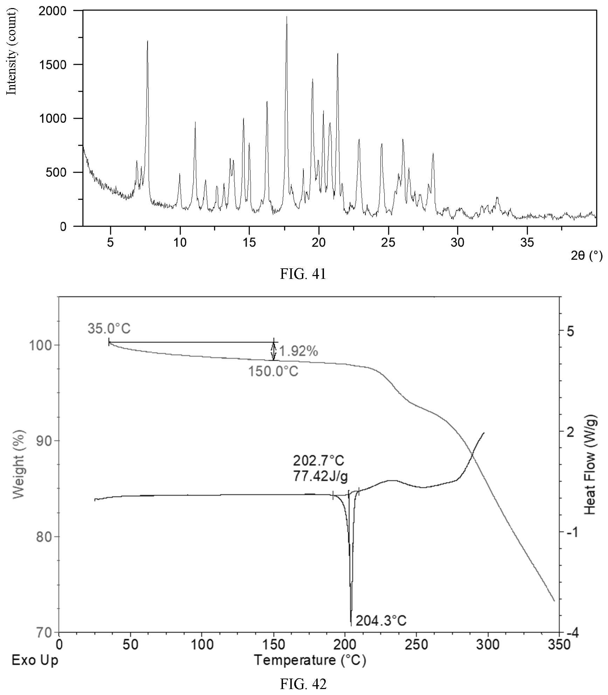

In some embodiments, the present disclosure provides crystal form B of the p-toluenesulfonate of the compound of formula I, and the crystal form B of the p-toluenesulfonate has an X-ray powder diffraction pattern comprising diffraction peaks at 2θ±0.2° diffraction angles of 7.6660, 16.2695, 17.6681, 19.5433, 20.3312, and 21.3520; further, the crystal form B of the p-toluenesulfonate has an X-ray powder diffraction pattern comprising diffraction peaks at 2θ±0.2° diffraction angles of 7.6660, 11.1056, 14.5769, 16.2695, 17.6681, 19.5433, 20.3312, 20.7694, 21.3520, and 26.0500; furthermore, the crystal form B of the p-toluenesulfonate has an X-ray powder diffraction pattern comprising diffraction peaks at 2θ±0.2° diffraction angles of 7.6660, 11.1056, 14.5769, 14.9845, 16.2695, 17.6681, 19.5433, 20.3312, 20.7694, 21.3520, 22.8795, 24.5060, 26.0500, and 28.2460; still further, the crystal form B of the p-toluenesulfonate has an X-ray powder diffraction pattern comprising diffraction peaks at 2θ±0.2° diffraction angles of 6.8890, 7.6660, 9.9665, 11.1056, 11.8448, 12.6617, 13.1706, 13.6098, 13.8415, 14.5769, 14.9845, 16.2695, 17.6681, 18.8718, 19.5433, 19.9700, 20.3312, 20.7694, 21.3520, 21.6902, 22.8795, 24.5060, 26.0500, 26.4583, 27.2822, 28.2460, 29.2107, 30.0879, 31.8132, 32.8460, 33.7653, and 37.7617; still further, the crystal form B of the p-toluenesulfonate has an XRPD pattern substantially as shown in FIG. 41.

In some embodiments, the crystal form B of the p-toluenesulfonate has one or two of the following characteristics:
(1) a TGA curve of the crystal form B of the p-toluenesulfonate showing a weight loss of 1-6%, preferably about 1-4% (e.g., 1.0%, 1.5%, 2.0%, 2.5%, 3.0%, 3.5%, or 4.0%) at 150.0±3 °C;
(2) a DSC curve of the crystal form B of the p-toluenesulfonate having 1 endothermic peak at 204.3±3 °C.

In some embodiments, the crystal form B of the p-toluenesulfonate has one or two of the following characteristics:
(1) a TGA curve of the crystal form B of the p-toluenesulfonate showing a weight loss of 1.92% at 150.0±3 °C;
(2) a DSC curve of the crystal form B of the p-toluenesulfonate having 1 endothermic peak at 204.3±3 °C.

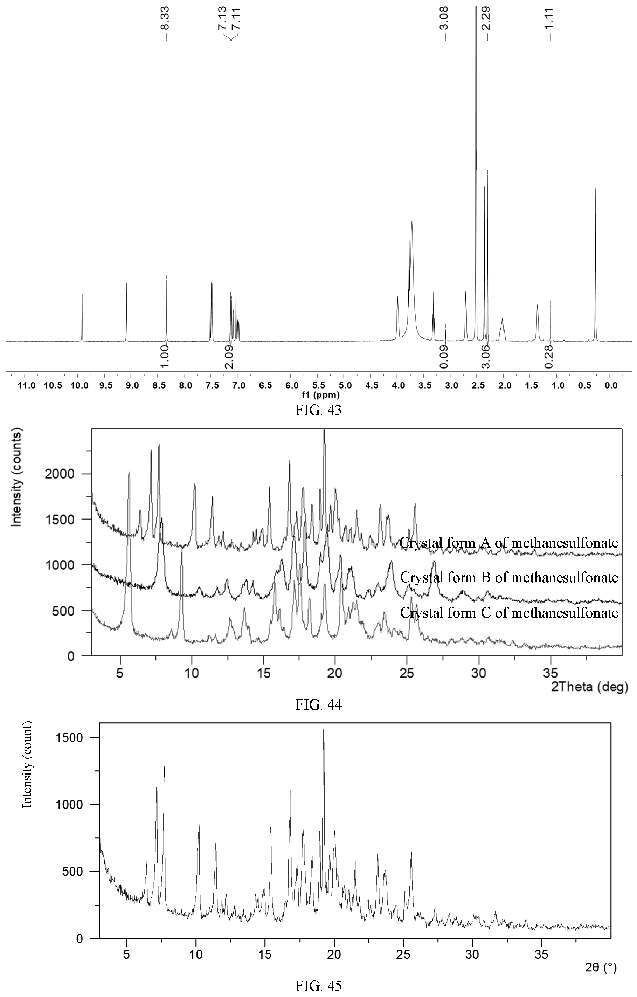

In some embodiments, a TGA/DSC profile of the crystal form B of the p-toluenesulfonate is substantially as shown in FIG. 42; a ¹H NMR spectrum of the crystal form B of the p-toluenesulfonate is substantially as shown in FIG. 43.

In some embodiments, the present disclosure provides crystal form A of the methanesulfonate of the compound of formula I, and the crystal form A of the methanesulfonate has an X-ray powder diffraction pattern comprising diffraction peaks at 2θ±0.2° diffraction angles of 7.1654, 7.7112, 10.2242, 15.4030, 16.8120, and 19.2492; further, the crystal form A of the methanesulfonate has an X-ray powder diffraction pattern comprising diffraction peaks at 2θ±0.2° diffraction angles of 7.1654, 7.7112, 10.2242, 11.4305, 15.4030, 16.8120, 17.7499, 18.3934, 18.9572, 19.2492, and 20.0359; furthermore, the crystal form A of the methanesulfonate has an X-ray powder diffraction pattern comprising diffraction peaks at 2θ±0.2° diffraction angles of 7.1654, 7.7112, 10.2242, 11.4305, 15.4030, 16.8120, 17.7499, 18.3934, 18.9572, 19.2492, 19.6796, 20.0359, 21.4943, 23.1420, and 25.5679; still further, the crystal form A of the methanesulfonate has an X-ray powder diffraction pattern comprising diffraction peaks at 2θ±0.2° diffraction angles of 6.4245, 7.1654, 7.7112, 10.2242, 11.4305, 12.1973, 14.3852, 14.9410, 15.4030, 16.8120, 17.3257, 17.7499, 18.3934, 18.9572, 19.2492, 19.6796, 20.0359, 20.7137, 21.0725, 21.4943, 22.4448, 23.1420, 23.6743, 24.4576, 25.1217, 25.5679, 27.3091, 28.2828, 30.2612, 31.6602, and 33.8876; still further, the crystal form A of the methanesulfonate has an XRPD pattern substantially as shown in FIG. 45.

In some embodiments, the crystal form A of the methanesulfonate has one or two of the following characteristics:
(1) a TGA curve of the crystal form A of the methanesulfonate showing a weight loss of 1-6%, preferably about 2-5% (e.g., 2.0%, 2.5%, 3.0%, 3.5%, 4.0%, 4.5%, or 5.0%) at 150.0±3 °C;
(2) a DSC curve of the crystal form A of the methanesulfonate having 1 endothermic peak at 153.8±3 °C.

In some embodiments, the crystal form A of the methanesulfonate has one or two of the following characteristics:
(1) a TGA curve of the crystal form A of the methanesulfonate showing a weight loss of 3.08% at 150.0±3 °C;
(2) a DSC curve of the crystal form A of the methanesulfonate having 1 endothermic peak at 153.8±3 °C.

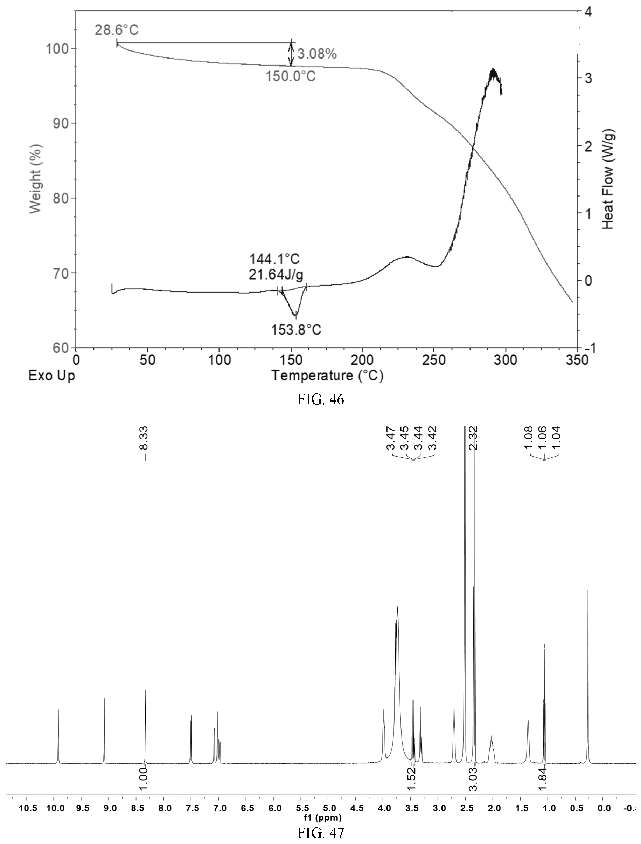

In some embodiments, a TGA/DSC profile of the crystal form A of the methanesulfonate is substantially as shown in FIG. 46; a ¹H NMR spectrum of the crystal form A of the methanesulfonate is as shown in FIG. 47.

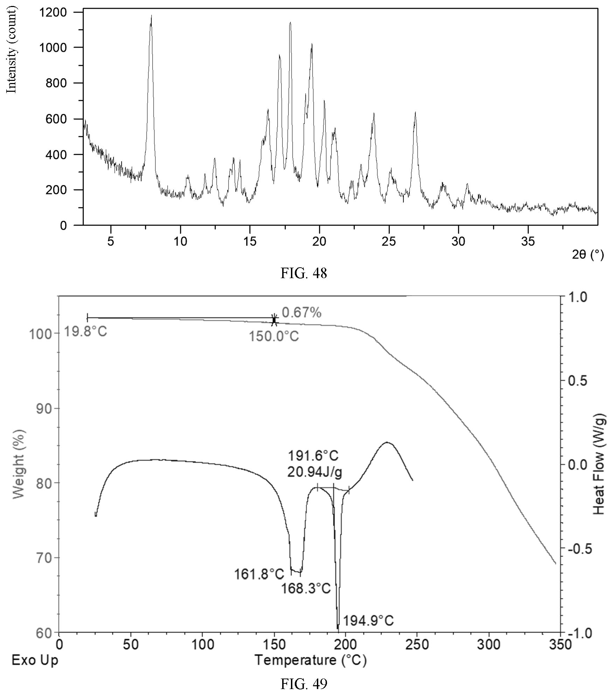

In some embodiments, the present disclosure provides crystal form B of the methanesulfonate of the compound of formula I, and the crystal form B of the methanesulfonate has an X-ray powder diffraction pattern comprising diffraction peaks at 2θ±0.2° diffraction angles of 7.9334, 17.1889, 17.9047, 19.4932, and 20.3844; further, the crystal form B of the methanesulfonate has an X-ray powder diffraction pattern comprising diffraction peaks at 2θ±0.2° diffraction angles of 7.9334, 16.2948, 17.1889, 17.9047, 18.9481, 19.4932, 20.3844, 21.2069, 23.9181, and 26.8731; furthermore, the crystal form B of the methanesulfonate has an X-ray powder diffraction pattern comprising diffraction peaks at 2θ±0.2° diffraction angles of 7.9334, 12.4699, 13.8134, 14.2662, 16.2948, 17.1889, 17.9047, 18.9481, 19.4932, 20.3844, 21.2069, 22.9645, 23.9181, and 26.8731; still further, the crystal form B of the methanesulfonate has an X-ray powder diffraction pattern comprising diffraction peaks at 2θ±0.2° diffraction angles of 7.9334, 10.5237, 11.7673, 12.4699, 13.8134, 14.2662, 16.2948, 17.1889, 17.9047, 18.9481, 19.4932, 20.3844, 21.2069, 22.3570, 22.9645, 23.9181, 25.0848, 26.8731, 28.8447, 30.5888, and 36.0120; still further, the crystal form B of the methanesulfonate has an XRPD pattern substantially as shown in FIG. 48.

In some embodiments, the crystal form B of the methanesulfonate has one or two of the following characteristics:
(1) a TGA curve of the crystal form B of the methanesulfonate showing a weight loss of 0-3%, preferably about 0-2% (e.g., 0.5%, 1.0%, 1.5%, or 2.0%) at 150.0±3 °C;
(2) a DSC curve of the crystal form B of the methanesulfonate having 3 endothermic peaks at 161.8±3 °C, 168.3±3 °C, and 194.9±3 °C.

In some embodiments, the crystal form B of the methanesulfonate has one or two of the following characteristics:
(1) a TGA curve of the crystal form B of the methanesulfonate showing a weight loss of 0.67% at 150.0±3 °C;
(2) a DSC curve of the crystal form B of the methanesulfonate having 3 endothermic peaks at 61.8±3 °C, 168.3±3 °C, and 194.9±3 °C.

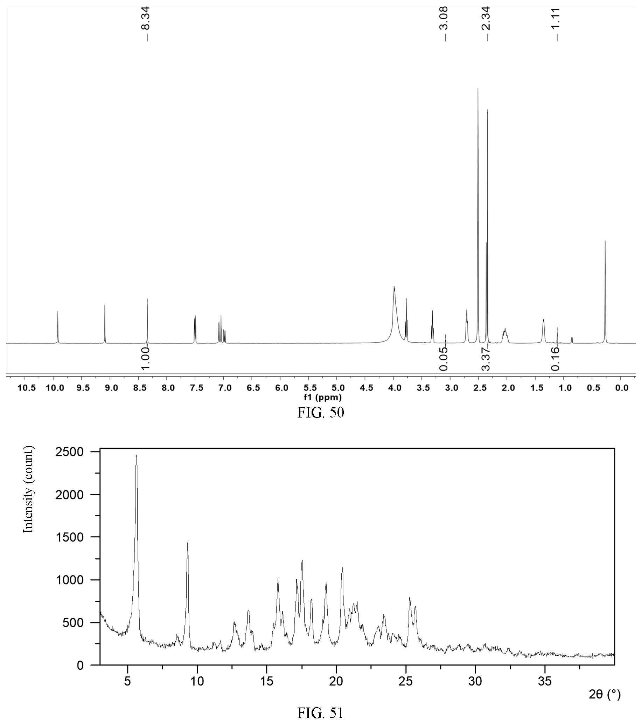

In some embodiments, a TGA/DSC profile of the crystal form B of the methanesulfonate is substantially as shown in FIG. 49; a ¹H NMR spectrum of the crystal form B of the methanesulfonate is substantially as shown in FIG. 50.

In some embodiments, the present disclosure provides crystal form C of the methanesulfonate of the compound of formula I, and the crystal form C of the methanesulfonate has an X-ray powder diffraction pattern comprising diffraction peaks at 2θ±0.2° diffraction angles of 5.6360, 9.3088, 15.7926, 17.1524, 17.5174, and 20.4316; further, the crystal form C of the methanesulfonate has an X-ray powder diffraction pattern comprising diffraction peaks at 2θ±0.2° diffraction angles of 5.6360, 9.3088, 13.6745, 15.7926, 17.1524, 17.5174, 18.1940, 19.2689, 20.4316, 21.5116, 25.2739, and 25.6773; furthermore, the crystal form C of the methanesulfonate has an X-ray powder diffraction pattern comprising diffraction peaks at 2θ±0.2° diffraction angles of 5.6360, 9.3088, 12.6477, 13.6745, 15.7926, 16.1257, 17.1524, 17.5174, 18.1940, 19.2689, 20.4316, 21.5116, 23.0206, 23.4450, 25.2739, and 25.6773; still further, the crystal form C of the methanesulfonate has an X-ray powder diffraction pattern comprising diffraction peaks at 2θ±0.2° diffraction angles of 5.6360, 8.5661, 9.3088, 11.6453, 12.6477, 13.6745, 15.7926, 16.1257, 17.1524, 17.5174, 18.1940, 19.2689, 20.4316, 21.5116, 23.0206, 23.4450, 25.2739, 25.6773, 29.4554, and 32.4112; still further, the crystal form C of the methanesulfonate has an XRPD pattern substantially as shown in FIG. 51.

In some embodiments, the crystal form C of the methanesulfonate has one or two of the following characteristics:
(1) a TGA curve of the crystal form C of the methanesulfonate showing a weight loss of 10-20%, preferably about 12-16% (e.g., 12.0%, 12.5%, 13.0%, 13.5%, 14.0%, 14.5%, 15.0%, 15.5%, or 16.0%) at 150.0±3 °C;
(2) a DSC curve of the crystal form C of the methanesulfonate having 3 endothermic peaks at 111.7±3 °C, 135.8±3 °C, and 139.6±3 °C.

In some embodiments, the crystal form C of the methanesulfonate has one or two of the following characteristics:
(1) a TGA curve of the crystal form C of the methanesulfonate showing a weight loss of 14.14% at 150.0±3 °C;
(2) a DSC curve of the crystal form C of the methanesulfonate having 3 endothermic peaks at 111.7±3 °C, 135.8±3 °C, and 139.6±3 °C.

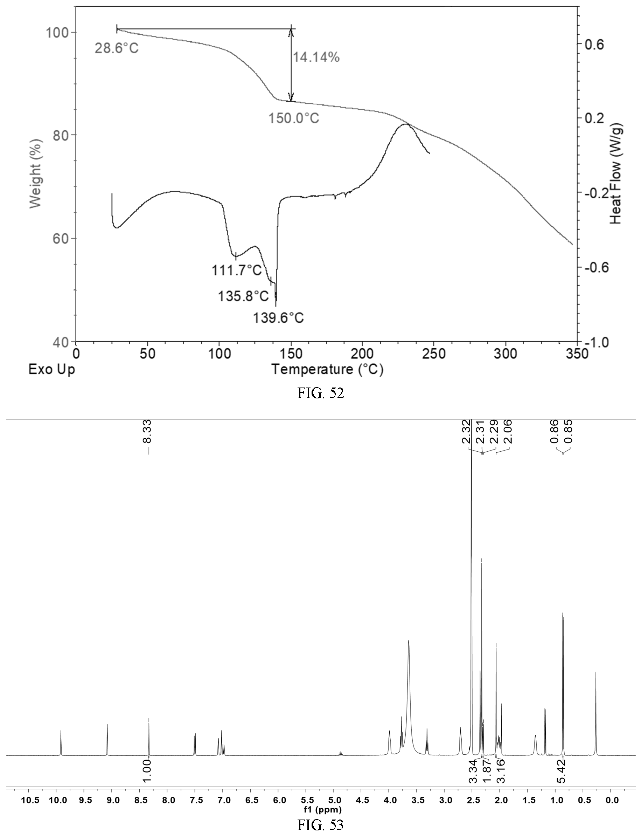

In some embodiments, a TGA/DSC profile of the crystal form C of the methanesulfonate is substantially as shown in FIG. 52; a ¹H NMR spectrum of the crystal form C of the methanesulfonate is substantially as shown in FIG. 53.

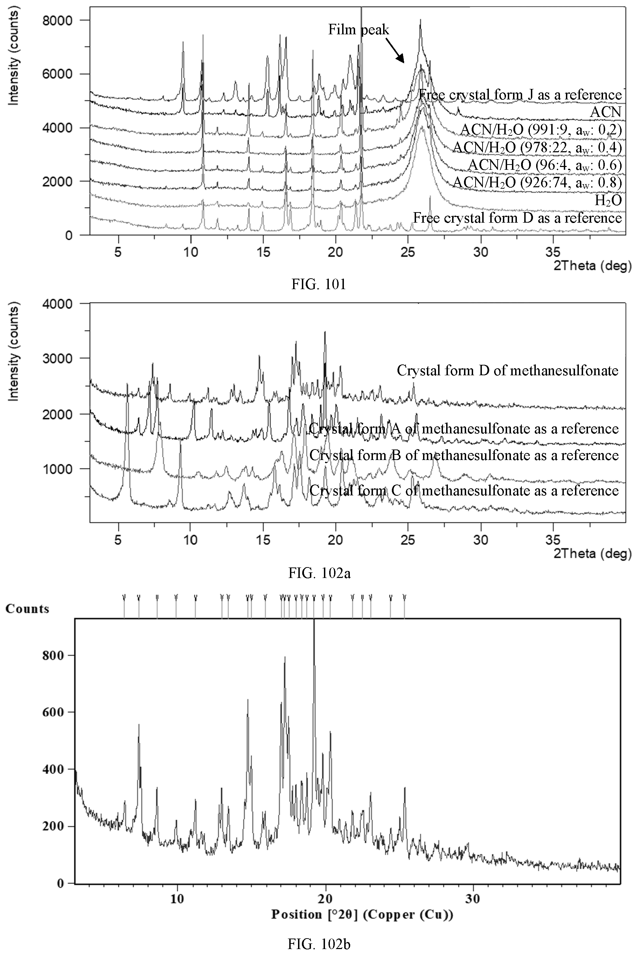

In some embodiments, the present disclosure provides crystal form D of the methanesulfonate of the compound of formula I, and the crystal form D of the methanesulfonate has an X-ray powder diffraction pattern comprising diffraction peaks at 2θ±0.2° diffraction angles of 7.3614, 14.7295, 17.0139, 17.2434, 17.5216, 19.2452, and 20.3331; further, the crystal form D of the methanesulfonate has an X-ray powder diffraction pattern comprising diffraction peaks at 2θ±0.2° diffraction angles of 7.3614, 14.7295, 14.9968, 17.0139, 17.2434, 17.5216, 19.2452, 19.8274, and 20.3331; furthermore, the crystal form D of the methanesulfonate has an X-ray powder diffraction pattern comprising diffraction peaks at 2θ±0.2° diffraction angles of 7.3614, 14.7295, 14.9968, 17.0139, 17.2434, 17.5216, 18.7631, 19.2452, 19.8274, 20.3331, and 25.3625; furthermore, the crystal form D of the methanesulfonate has an X-ray powder diffraction pattern comprising diffraction peaks at 2θ±0.2° diffraction angles of 7.3614, 8.5975, 11.1942, 12.9868, 13.4101, 14.7295, 14.9968, 17.0139, 17.2434, 17.5216, 18.0167, 18.3980, 18.7631, 19.2452, 19.8274, 20.3331, 21.8273, 22.4894, 23.0593, and 25.3625; still further, the crystal form D of the methanesulfonate has an X-ray powder diffraction pattern comprising diffraction peaks at 2θ±0.2° diffraction angles of 6.3734, 7.3614, 8.5975, 9.8950, 11.1942, 12.9868, 13.4101, 14.7295, 14.9968, 15.9124, 17.0139, 17.2434, 17.5216, 18.0167, 18.3980, 18.7631, 19.2452, 19.8274, 20.3331, 21.8273, 22.4894, 23.0593, 24.4152, and 25.3625; still further, the crystal form D of the methanesulfonate has an XRPD pattern substantially as shown in FIG. 102b.

In some embodiments, the crystal form D of the methanesulfonate has one or two or more of the following characteristics:
(1) a TGA curve showing a weight loss of 1-4%, preferably about 1.5-3% (e.g., 1.5%, 2.0%, 2.5%, or 3.0%), and more preferably about 2.62% at 150±3 °C;
(2) a DSC curve having 2 endothermic peaks at 40.3±3 °C and 156.6±3 °C.

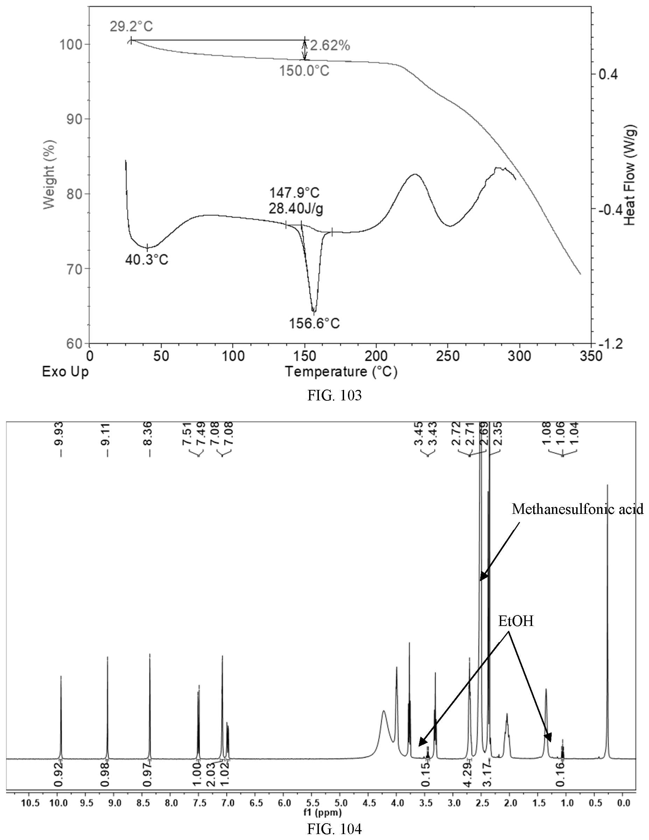

In some embodiments, a TGA/DSC profile of the crystal form D of the methanesulfonate is substantially as shown in FIG. 103; a ¹H NMR spectrum of the crystal form D of the methanesulfonate is substantially as shown in FIG. 104.

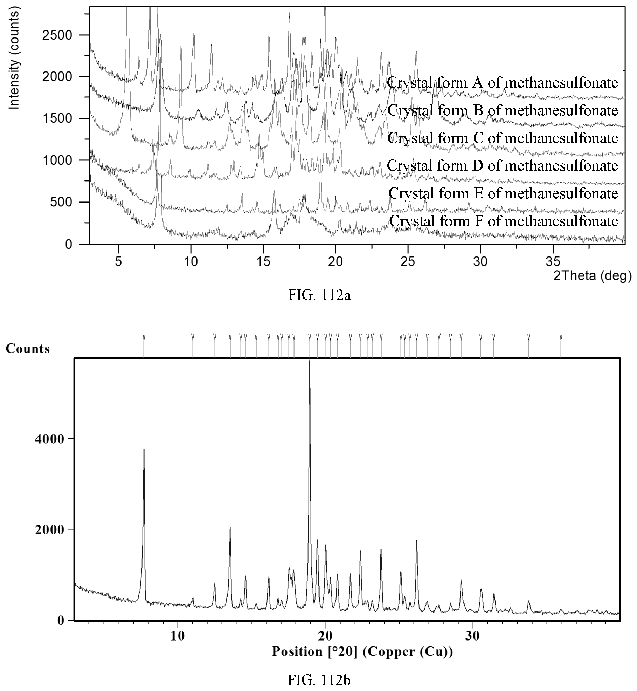

In some embodiments, the present disclosure provides crystal form E of the methanesulfonate of the compound of formula I, and the crystal form E of the methanesulfonate has an X-ray powder diffraction pattern comprising diffraction peaks at 2θ±0.2° diffraction angles of 7.6948, 13.5466, 18.9557, 19.4674, and 26.1850; further, the crystal form E of the methanesulfonate has an X-ray powder diffraction pattern comprising diffraction peaks at 2θ±0.2° diffraction angles of 7.6948, 13.5466, 18.9557, 19.4674, 20.0144, 23.7738, and 26.1850; furthermore, the crystal form E of the methanesulfonate has an X-ray powder diffraction pattern comprising diffraction peaks at 2θ±0.2° diffraction angles of 7.6948, 13.5466, 18.9557, 19.4674, 20.0144, 22.3698, 23.7738, and 26.1850; furthermore, the crystal form E of the methanesulfonate has an X-ray powder diffraction pattern comprising diffraction peaks at 2θ±0.2° diffraction angles of 7.6948, 13.5466, 14.5860, 16.1727, 17.5309, 17.8641, 18.9557, 19.4674, 20.0144, 20.3406, 20.8237, 21.6960, 22.3698, 23.7738, 25.1077, 26.1850, and 29.2032; still further, the crystal form E of the methanesulfonate has an X-ray powder diffraction pattern comprising diffraction peaks at 2θ±0.2° diffraction angles of 7.6948, 11.0258, 12.4963, 13.5466, 14.2591, 14.5860, 15.3154, 16.1727, 16.8137, 17.0486, 17.5309, 17.8641, 18.9557, 19.4674, 20.0144, 20.3406, 20.8237, 21.6960, 22.3698, 22.8750, 23.1733, 23.7738, 25.1077, 25.3713, 25.7306, 26.1850, 26.8900, 27.6964, 28.4667, 29.2032, 30.5281, 31.4140, 33.7897, and 35.9511; still further, the crystal form E of the methanesulfonate has an XRPD pattern substantially as shown in FIG. 112b.

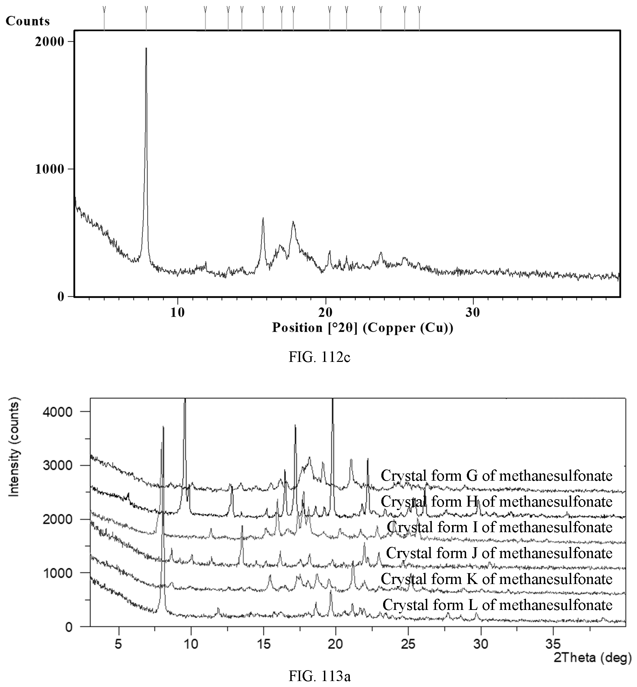

In some embodiments, the present disclosure provides crystal form F of the methanesulfonate of the compound of formula I, and the crystal form F of the methanesulfonate has an X-ray powder diffraction pattern comprising diffraction peaks at 2θ±0.2° diffraction angles of 7.8706, 15.7726, 17.0258, 17.8165, 20.2728, and 23.7446; further, the crystal form F of the methanesulfonate has an X-ray powder diffraction pattern comprising diffraction peaks at 2θ±0.2° diffraction angles of 7.8706, 15.7726, 17.0258, 17.8165, 20.2728, 23.7446, and 25.3530; furthermore, the crystal form F of the methanesulfonate has an X-ray powder diffraction pattern comprising diffraction peaks at 2θ±0.2° diffraction angles of 7.8706, 15.7726, 17.0258, 17.8165, 20.2728, 21.4207, 23.7446, and 25.3530; furthermore, the crystal form F of the methanesulfonate has an X-ray powder diffraction pattern comprising diffraction peaks at 2θ±0.2° diffraction angles of 7.8706, 11.8701, 15.7726, 17.0258, 17.8165, 20.2728, 21.4207, 23.7446, 25.3530, and 26.3257; still further, the crystal form F of the methanesulfonate has an X-ray powder diffraction pattern comprising diffraction peaks at 2θ±0.2° diffraction angles of 5.0171, 7.8706, 11.8701, 13.4221, 14.3209, 15.7726, 17.0258, 17.8165, 20.2728, 21.4207, 23.7446, 25.3530, and 26.3257; still further, the crystal form F of the methanesulfonate has an XRPD pattern substantially as shown in FIG. 112c.

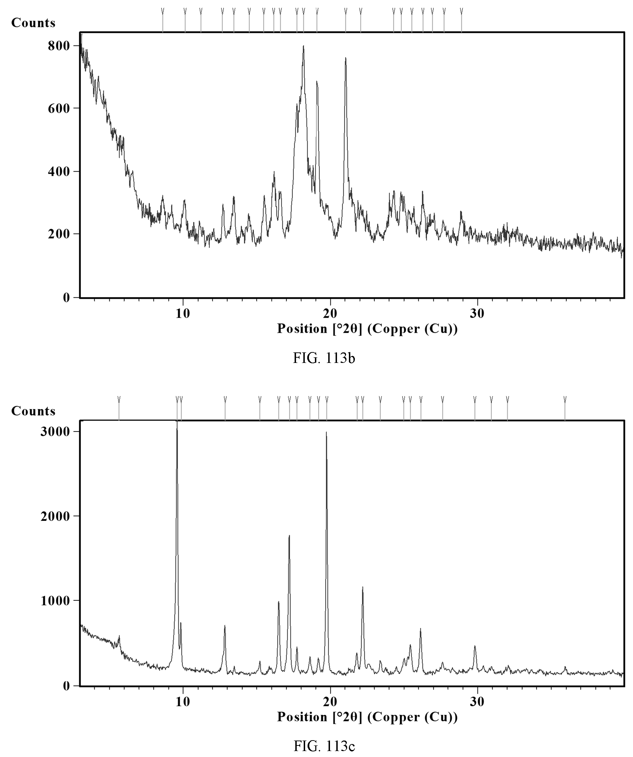

In some embodiments, the present disclosure provides crystal form G of the methanesulfonate of the compound of formula I, and the crystal form G of the methanesulfonate has an X-ray powder diffraction pattern comprising diffraction peaks at 2θ±0.2° diffraction angles of 16.1377, 17.7027, 18.1620, 19.1132, and 21.0530; further, the crystal form G of the methanesulfonate has an X-ray powder diffraction pattern comprising diffraction peaks at 2θ±0.2° diffraction angles of 15.4837, 16.1377, 16.5841, 17.7027, 18.1620, 19.1132, 21.0530, and 24.3157; furthermore, the crystal form G of the methanesulfonate has an X-ray powder diffraction pattern comprising diffraction peaks at 2θ±0.2° diffraction angles of 13.4333, 15.4837, 16.1377, 16.5841, 17.7027, 18.1620, 19.1132, 21.0530, 24.3157, 24.8063, and 26.2930; furthermore, the crystal form G of the methanesulfonate has an X-ray powder diffraction pattern comprising diffraction peaks at 2θ±0.2° diffraction angles of 8.6041, 10.1340, 12.6682, 13.4333, 15.4837, 16.1377, 16.5841, 17.7027, 18.1620, 19.1132, 21.0530, 22.0640, 24.3157, 24.8063, 25.5461, 26.2930, and 28.8940; still further, the crystal form G of the methanesulfonate has an X-ray powder diffraction pattern comprising diffraction peaks at 2θ±0.2° diffraction angles of 8.6041, 10.1340, 11.2069, 12.6682, 13.4333, 14.4922, 15.4837, 16.1377, 16.5841, 17.7027, 18.1620, 19.1132, 21.0530, 22.0640, 24.3157, 24.8063, 25.5461, 26.2930, 26.9311, 27.7204, and 28.8940; still further, the crystal form G of the methanesulfonate has an XRPD pattern substantially as shown in FIG. 113b.

In some embodiments, the present disclosure provides crystal form H of the methanesulfonate of the compound of formula I, and the crystal form of the methanesulfonate has an X-ray powder diffraction pattern comprising diffraction peaks at 2θ±0.2° diffraction angles of 9.5858, 16.4833, 17.2040, 19.7648, and 22.2051; further, the crystal form H of the methanesulfonate has an X-ray powder diffraction pattern comprising diffraction peaks at 2θ±0.2° diffraction angles of 9.5858, 9.8551, 12.8418, 16.4833, 17.2040, 19.7648, and 22.2051; furthermore, the crystal form H of the methanesulfonate has an X-ray powder diffraction pattern comprising diffraction peaks at 2θ±0.2° diffraction angles of 9.5858, 9.8551, 12.8418, 16.4833, 17.2040, 19.7648, 22.2051, 25.4281, and 26.1391; furthermore, the crystal form H of the methanesulfonate has an X-ray powder diffraction pattern comprising diffraction peaks at 2θ±0.2° diffraction angles of 9.5858, 9.8551, 12.8418, 16.4833, 17.2040, 17.7099, 18.5860, 19.7648, 21.8135, 22.2051, 25.4281, 26.1391, and 29.8198; still further, the crystal form H of the methanesulfonate has an X-ray powder diffraction pattern comprising diffraction peaks at 2θ±0.2° diffraction angles of 5.6472, 9.5858, 9.8551, 12.8418, 15.2067, 16.4833, 17.2040, 17.7099, 18.5860, 19.2029, 19.7648, 21.8135, 22.2051, 23.4137, 24.9851, 25.4281, 26.1391, 27.6138, 29.8198, 30.9209, 32.0327, and 35.9179; still further, the crystal form H of the methanesulfonate has an XRPD pattern substantially as shown in FIG. 113c.

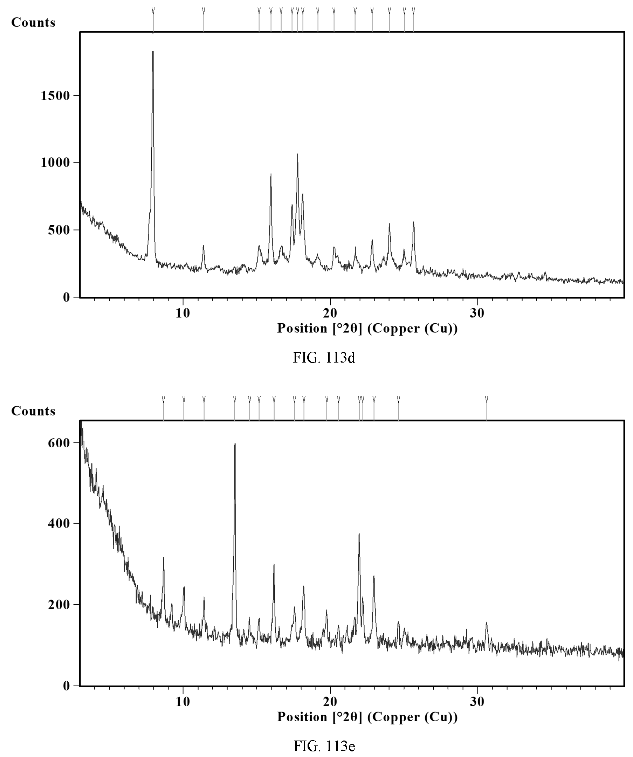

In some embodiments, the present disclosure provides crystal form I of the methanesulfonate of the compound of formula I, and the crystal form I of the methanesulfonate has an X-ray powder diffraction pattern comprising diffraction peaks at 2θ±0.2° diffraction angles of 7.9648, 15.9556, 17.3872, 17.7652, 18.1048, 24.0274, and 25.6562; further, the crystal form I of the methanesulfonate has an X-ray powder diffraction pattern comprising diffraction peaks at 2θ±0.2° diffraction angles of 7.9648, 15.9556, 17.3872, 17.7652, 18.1048, 22.8525, 24.0274, and 25.6562; furthermore, the crystal form I of the methanesulfonate has an X-ray powder diffraction pattern comprising diffraction peaks at 2θ±0.2° diffraction angles of 7.9648, 15.1578, 15.9556, 17.3872, 17.7652, 18.1048, 22.8525, 24.0274, and 25.6562; furthermore, the crystal form I of the methanesulfonate has an X-ray powder diffraction pattern comprising diffraction peaks at 2θ±0.2° diffraction angles of 7.9648, 11.3739, 15.1578, 15.9556, 16.6385, 17.3872, 17.7652, 18.1048, 20.2628, 22.8525, 24.0274, and 25.6562; still further, the crystal form I of the methanesulfonate has an X-ray powder diffraction pattern comprising diffraction peaks at 2θ±0.2° diffraction angles of 7.9648, 11.3739, 15.1578, 15.9556, 16.6385, 17.3872, 17.7652, 18.1048, 19.1430, 20.2628, 21.6954, 22.8525, 24.0274, 25.0167, and 25.6562; still further, the crystal form I of the methanesulfonate has an XRPD pattern substantially as shown in FIG. 113d.

In some embodiments, the present disclosure provides crystal form J of the methanesulfonate of the compound of formula I, and the crystal form J of the methanesulfonate has an X-ray powder diffraction pattern comprising diffraction peaks at 2θ±0.2° diffraction angles of 8.6620, 13.5049, 16.1555, 18.1900, 21.9852, 22.2107, and 22.9624; further, the crystal form J of the methanesulfonate has an X-ray powder diffraction pattern comprising diffraction peaks at 2θ±0.2° diffraction angles of 8.6620, 10.0632, 11.4255, 13.5049, 16.1555, 18.1900, 21.9852, 22.2107, and 22.9624; furthermore, the crystal form J of the methanesulfonate has an X-ray powder diffraction pattern comprising diffraction peaks at 2θ±0.2° diffraction angles of 8.6620, 10.0632, 11.4255, 13.5049, 16.1555, 17.5647, 18.1900, 19.7621, 21.9852, 22.2107, and 22.9624; furthermore, the crystal form J of the methanesulfonate has an X-ray powder diffraction pattern comprising diffraction peaks at 2θ±0.2° diffraction angles of 8.6620, 10.0632, 11.4255, 13.5049, 16.1555, 17.5647, 18.1900, 19.7621, 21.9852, 22.2107, 22.9624, and 24.6246; still further, the crystal form J of the methanesulfonate has an X-ray powder diffraction pattern comprising diffraction peaks at 2θ±0.2° diffraction angles of 8.6620, 10.0632, 11.4255, 13.5049, 14.5059, 15.1512, 16.1555, 17.5647, 18.1900, 19.7621, 20.5741, 21.9852, 22.2107, 22.9624, 24.6246, and 30.6181; still further, the crystal form J of the methanesulfonate has an XRPD pattern substantially as shown in FIG. 113e.

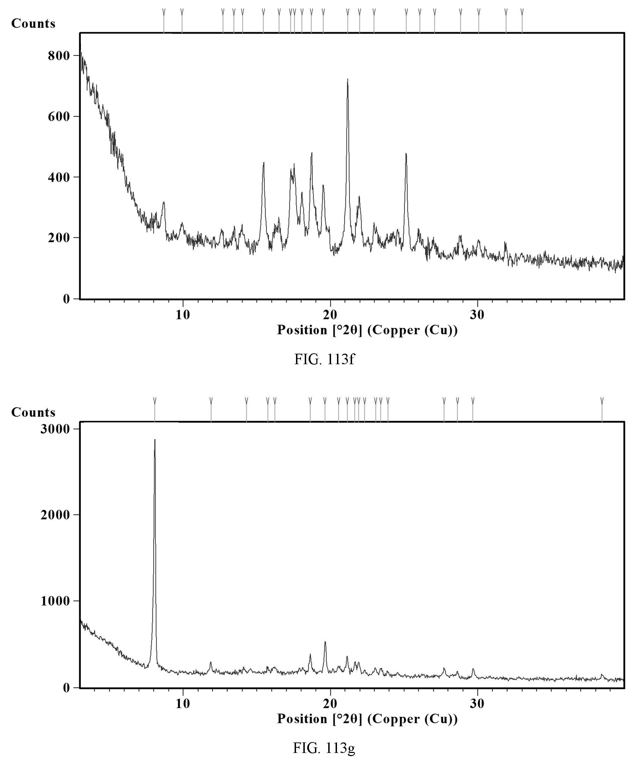

In some embodiments, the present disclosure provides crystal form K of the methanesulfonate of the compound of formula I, and the crystal form K of the methanesulfonate has an X-ray powder diffraction pattern comprising diffraction peaks at 2θ±0.2° diffraction angles of 15.4362, 17.2898, 17.5619, 18.6931, 21.1887, and 25.1553; further, the crystal form K of the methanesulfonate has an X-ray powder diffraction pattern comprising diffraction peaks at 2θ±0.2° diffraction angles of 15.4362, 17.2898, 17.5619, 18.6931, 19.5256, 21.1887, and 25.1553; furthermore, the crystal form K of the methanesulfonate has an X-ray powder diffraction pattern comprising diffraction peaks at 2θ±0.2° diffraction angles of 15.4362, 17.2898, 17.5619, 18.0642, 18.6931, 19.5256, 21.1887, 21.9878, and 25.1553; furthermore, the crystal form K of the methanesulfonate has an X-ray powder diffraction pattern comprising diffraction peaks at 2θ±0.2° diffraction angles of 8.6859, 15.4362, 16.5219, 17.2898, 17.5619, 18.0642, 18.6931, 19.5256, 21.1887, 21.9878, 22.9735, 25.1553, 26.0704, 28.8383, and 30.0689; still further, the crystal form K of the methanesulfonate has an X-ray powder diffraction pattern comprising diffraction peaks at 2θ±0.2° diffraction angles of 8.6859, 9.9129, 12.6815, 13.4500, 14.0343, 15.4362, 16.5219, 17.2898, 17.5619, 18.0642, 18.6931, 19.5256, 21.1887, 21.9878, 22.9735, 25.1553, 26.0704, 27.0875, 28.8383, 30.0689, 31.9147, and 33.0164; still further, the crystal form K of the methanesulfonate has an XRPD pattern substantially as shown in FIG. 113f.

In some embodiments, the present disclosure provides crystal form L of the methanesulfonate of the compound of formula I, and the crystal form L of the methanesulfonate has an X-ray powder diffraction pattern comprising diffraction peaks at 2θ±0.2° diffraction angles of 8.0867, 18.6275, 19.6478, and 21.1470; further, the crystal form L of the methanesulfonate has an X-ray powder diffraction pattern comprising diffraction peaks at 2θ±0.2° diffraction angles of 8.0867, 11.8848, 18.6275, 19.6478, 21.1470, 21.6729, and 21.9361; furthermore, the crystal form L of the methanesulfonate has an X-ray powder diffraction pattern comprising diffraction peaks at 2θ±0.2° diffraction angles of 8.0867, 11.8848, 18.6275, 19.6478, 21.1470, 21.6729, 21.9361, and 29.6831; furthermore, the crystal form L of the methanesulfonate has an X-ray powder diffraction pattern comprising diffraction peaks at 2θ±0.2° diffraction angles of 8.0867, 11.8848, 18.6275, 19.6478, 20.5586, 21.1470, 21.6729, 21.9361, 23.0719, 23.4214, 27.7310, and 29.6831; still further, the crystal form L of the methanesulfonate has an X-ray powder diffraction pattern comprising diffraction peaks at 2θ±0.2° diffraction angles of 8.0867, 11.8848, 14.2858, 15.7466, 16.2087, 18.6275, 19.6478, 20.5586, 21.1470, 21.6729, 21.9361, 22.3422, 23.0719, 23.4214, 23.9149, 27.7310, 28.6368, 29.6831, and 38.4394; still further, the crystal form L of the methanesulfonate has an XRPD pattern substantially as shown in FIG. 113g.

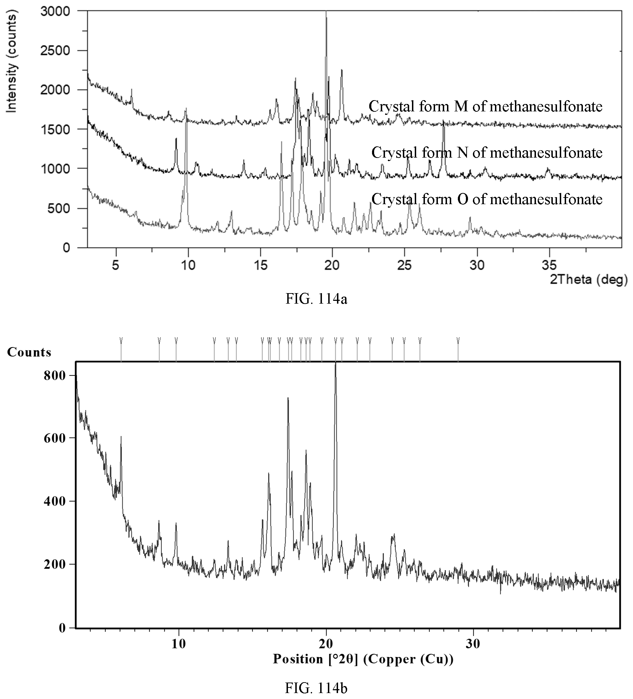

In some embodiments, the present disclosure provides crystal form M of the methanesulfonate of the compound of formula I, and the crystal form M of the methanesulfonate has an X-ray powder diffraction pattern comprising diffraction peaks at 2θ±0.2° diffraction angles of 16.0880, 17.4084, 17.6442, 18.6200, 18.8984, and 20.6457; further, the crystal form M of the methanesulfonate has an X-ray powder diffraction pattern comprising diffraction peaks at 2θ±0.2° diffraction angles of 6.0854, 16.0880, 16.2048, 17.4084, 17.6442, 18.6200, 18.8984, and 20.6457; furthermore, the crystal form M of the methanesulfonate has an X-ray powder diffraction pattern comprising diffraction peaks at 2θ±0.2° diffraction angles of 6.0854, 9.8088, 15.6545, 16.0880, 16.2048, 17.4084, 17.6442, 18.2844, 18.6200, 18.8984, and 20.6457; furthermore, the crystal form M of the methanesulfonate has an X-ray powder diffraction pattern comprising diffraction peaks at 2θ±0.2° diffraction angles of 6.0854, 8.6714, 9.8088, 13.3246, 15.6545, 16.0880, 16.2048, 17.4084, 17.6442, 18.2844, 18.6200, 18.8984, 19.7051, 20.6457, 21.0719, 22.1260, 24.4928, and 25.3051; still further, the crystal form M of the methanesulfonate has an X-ray powder diffraction pattern comprising diffraction peaks at 2θ±0.2° diffraction angles of 6.0854, 8.6714, 9.8088, 12.3906, 13.3246, 13.8974, 15.6545, 16.0880, 16.2048, 16.8014, 17.4084, 17.6442, 18.2844, 18.6200, 18.8984, 19.7051, 20.6457, 21.0719, 22.1260, 22.9780, 24.4928, 25.3051, 26.3685, and 28.9539; still further, the crystal form M of the methanesulfonate has an XRPD pattern substantially as shown in FIG. 114b.

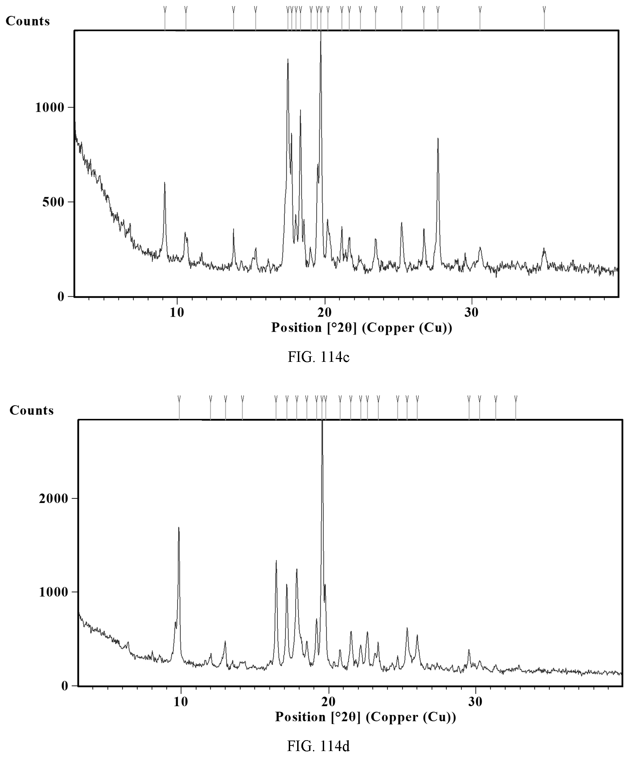

In some embodiments, the present disclosure provides crystal form N of the methanesulfonate of the compound of formula I, and the crystal form N of the methanesulfonate has an X-ray powder diffraction pattern comprising diffraction peaks at 2θ±0.2° diffraction angles of 17.4887, 17.7573, 18.3532, 19.4978, 19.7328, and 27.6786; further, the crystal form N of the methanesulfonate has an X-ray powder diffraction pattern comprising diffraction peaks at 2θ±0.2° diffraction angles of 9.1585, 17.4887, 17.7573, 18.3532, 19.4978, 19.7328, and 27.6786; furthermore, the crystal form N of the methanesulfonate has an X-ray powder diffraction pattern comprising diffraction peaks at 2θ±0.2° diffraction angles of 9.1585, 17.4887, 17.7573, 18.0588, 18.3532, 19.4978, 19.7328, 20.2234, 21.1657, 25.2039, and 27.6786; furthermore, the crystal form N of the methanesulfonate has an X-ray powder diffraction pattern comprising diffraction peaks at 2θ±0.2° diffraction angles of 9.1585, 10.5913, 13.8291, 15.3223, 17.4887, 17.7573, 18.0588, 18.3532, 19.4978, 19.7328, 20.2234, 21.1657, 21.6656, 23.4530, 25.2039, 26.7244, 27.6786, and 30.5646; still further, the crystal form N of the methanesulfonate has an X-ray powder diffraction pattern comprising diffraction peaks at 2θ±0.2° diffraction angles of 9.1585, 10.5913, 13.8291, 15.3223, 17.4887, 17.7573, 18.0588, 18.3532, 19.0631, 19.4978, 19.7328, 20.2234, 21.1657, 21.6656, 22.4118, 23.4530, 25.2039, 26.7244, 27.6786, 30.5646, and 34.8974; still further, the crystal form N of the methanesulfonate has an XRPD pattern substantially as shown in FIG. 114c.

In some embodiments, the present disclosure provides crystal form O of the methanesulfonate of the compound of formula I, and the crystal form O of the methanesulfonate has an X-ray powder diffraction pattern comprising diffraction peaks at 2θ±0.2° diffraction angles of 9.8632, 16.4454, 17.1724, 17.8526, 19.5652, and 19.7882; further, the crystal form O of the methanesulfonate has an X-ray powder diffraction pattern comprising diffraction peaks at 2θ±0.2° diffraction angles of 9.8632, 16.4454, 17.1724, 17.8526, 19.1922, 19.5652, and 19.7882; furthermore, the crystal form O of the methanesulfonate has an X-ray powder diffraction pattern comprising diffraction peaks at 2θ±0.2° diffraction angles of 9.8632, 16.4454, 17.1724, 17.8526, 19.1922, 19.5652, 19.7882, 21.5123, 22.6207, 25.3282, and 26.0079; furthermore, the crystal form O of the methanesulfonate has an X-ray powder diffraction pattern comprising diffraction peaks at 2θ±0.2° diffraction angles of 9.8632, 13.0102, 16.4454, 17.1724, 17.8526, 18.5219, 19.1922, 19.5652, 19.7882, 20.7837, 21.5123, 22.1595, 22.6207, 23.3625, 25.3282, 26.0079, and 29.5124; still further, the crystal form O of the methanesulfonate has an X-ray powder diffraction pattern comprising diffraction peaks at 2θ±0.2° diffraction angles of 9.8632, 11.9975, 13.0102, 14.1723, 16.4454, 17.1724, 17.8526, 18.5219, 19.1922, 19.5652, 19.7882, 20.7837, 21.5123, 22.1595, 22.6207, 23.3625, 24.6883, 25.3282, 26.0079, 29.5124, 30.2740, 31.3467, and 32.7120; still further, the crystal form O of the methanesulfonate has an XRPD pattern substantially as shown in FIG. 114d.

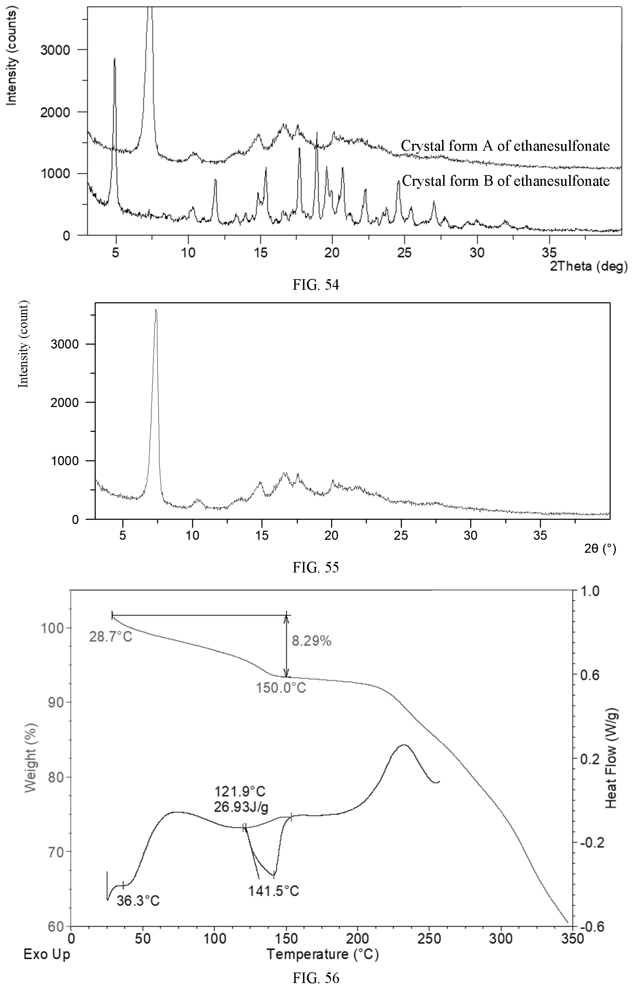

In some embodiments, the present disclosure provides crystal form A of the ethanesulfonate of the compound of formula I, and the crystal form A of the ethanesulfonate has an X-ray powder diffraction pattern comprising diffraction peaks at 2θ±0.2° diffraction angles of 7.4322, 14.8852, 16.6572, 17.5439, and 20.0726; further, the crystal form A of the ethanesulfonate has an X-ray powder diffraction pattern comprising diffraction peaks at 2θ±0.2° diffraction angles of 7.4322, 10.3920, 13.3770, 14.4406, 14.8852, 16.6572, 17.5439, 20.0726, and 22.0062; furthermore, the crystal form A of the ethanesulfonate has an XRPD pattern substantially as shown in FIG. 55.

In some embodiments, the crystal form A of the ethanesulfonate has one or two of the following characteristics:
(1) a TGA curve of the crystal form A of the ethanesulfonate showing a weight loss of 5-12%, preferably about 6-10% (e.g., 6.0%, 6.5%, 7.0%, 7.5%, 8.0%, 8.5%, 9.0%, 9.5%, or 10.0%) at 150.0±3 °C;
(2) a DSC curve of the crystal form A of the ethanesulfonate having 2 endothermic peaks at 36.3±3 °C and 141.5±3 °C.

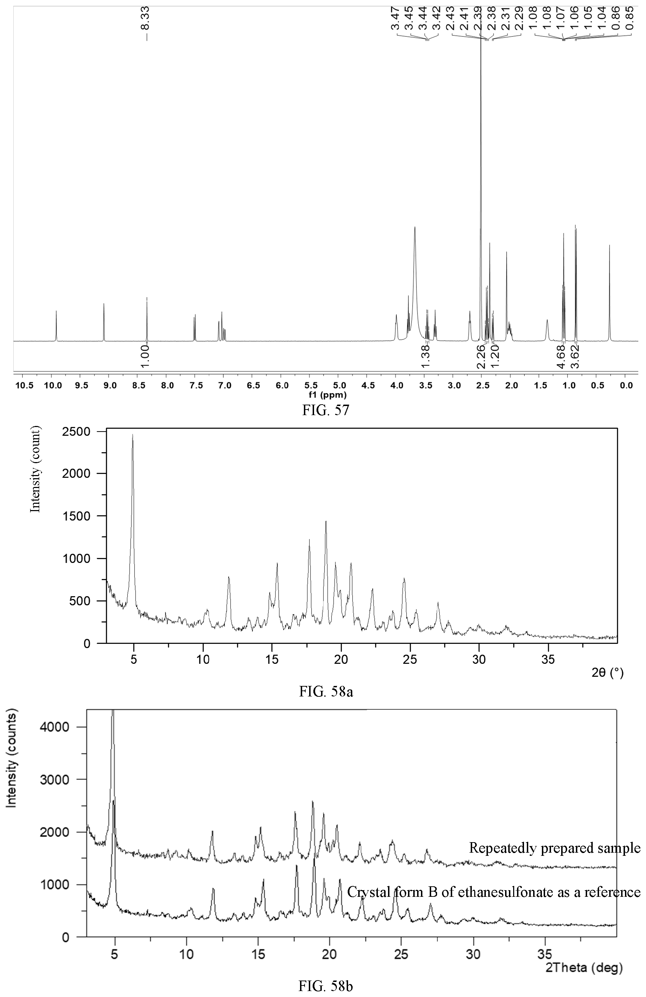

In some embodiments, the crystal form A of the ethanesulfonate has one or two of the following characteristics:
(1) a TGA curve of the crystal form A of the ethanesulfonate showing a weight loss of 8.29% at 150.0±3 °C;
(2) a DSC curve of the crystal form A of the ethanesulfonate having 2 endothermic peaks at 36.3±3 °C and 141.5±3 °C. In some embodiments, a TGA/DSC profile of the crystal form A of the ethanesulfonate is substantially as shown in FIG. 56; a ¹H NMR spectrum of the crystal form A of the ethanesulfonate is substantially as shown in FIG. 57.

In some embodiments, the present disclosure provides crystal form B of the ethanesulfonate of the compound of formula I, and the crystal form B of the ethanesulfonate has an X-ray powder diffraction pattern comprising diffraction peaks at 2θ±0.2° diffraction angles of 4.9173, 15.3679, 17.7077, 18.9091, 19.5992, and 20.7324; further, the crystal form B of the ethanesulfonate has an X-ray powder diffraction pattern comprising diffraction peaks at 2θ±0.2° diffraction angles of 4.9173, 11.9022, 15.3679, 17.7077, 18.9091, 19.5992, 19.9469, 20.7324, 22.2822, and 24.6012; furthermore, the crystal form B of the ethanesulfonate has an X-ray powder diffraction pattern comprising diffraction peaks at 2θ±0.2° diffraction angles of 4.9173, 11.9022, 14.8131, 15.3679, 17.7077, 18.9091, 19.5992, 19.9469, 20.7324, 22.2822, 23.7415, 24.6012, 25.4858, and 27.0042; still further, the crystal form B of the ethanesulfonate has an X-ray powder diffraction pattern comprising diffraction peaks at 2θ±0.2° diffraction angles of 4.9173, 10.3467, 11.9022, 13.3498, 13.9472, 14.8131, 15.3679, 16.5326, 17.7077, 18.9091, 19.5992, 19.9469, 20.7324, 21.2340, 22.2822, 23.7415, 24.6012, 25.4858, 27.0042, 27.7863, 29.2924, 29.9997, and 31.9631; still further, the crystal form B of the ethanesulfonate has an XRPD pattern substantially as shown in FIG. 58a or 58b. In some embodiments, the crystal form B of the ethanesulfonate has one or two of the following characteristics:
(1) a TGA curve of the crystal form B of the ethanesulfonate showing a weight loss of 3-10%, preferably about 4-8% (e.g., 4.0%, 4.5%, 5.0%, 5.5%, 6.0%, 6.5% 7.0%, 7.5%, or 8.0%) at 150.0±3 °C;
(2) a DSC curve of the crystal form B of the ethanesulfonate having 2 endothermic peaks at 107.1±3 °C and 145.3±3 °C.

In some embodiments, the crystal form B of the ethanesulfonate has one or two of the following characteristics:
(1) a TGA curve of the crystal form B of the ethanesulfonate showing a weight loss of 6.30% at 150.0±3 °C;
(2) a DSC curve of the crystal form B of the ethanesulfonate having 2 endothermic peaks at 107.1±3 °C and 145.3±3 °C.

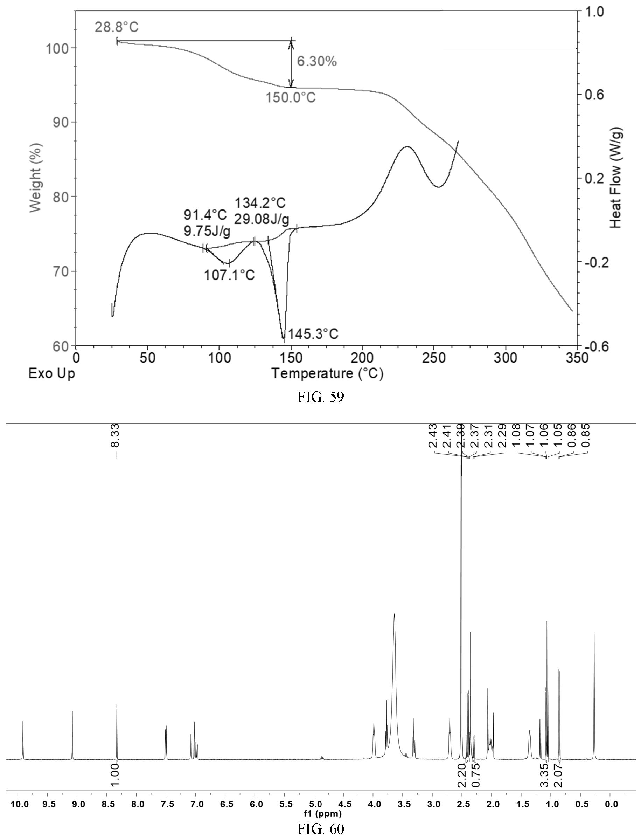

In some embodiments, a TGA/DSC profile of the crystal form B of the ethanesulfonate is substantially as shown in FIG. 59; a ¹H NMR spectrum of the crystal form B of the ethanesulfonate is substantially as shown in FIG. 60.

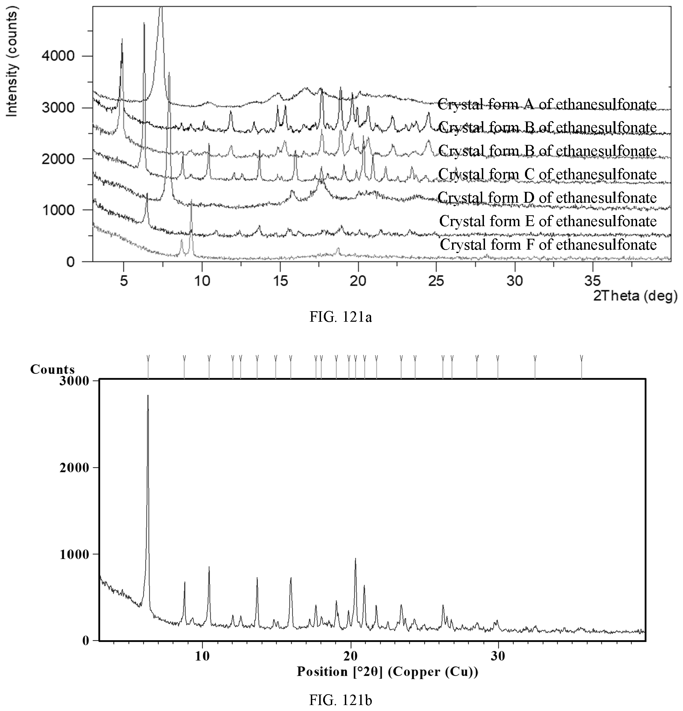

In some embodiments, the present disclosure provides crystal form C of the ethanesulfonate of the compound of formula I, and the crystal form C of the ethanesulfonate has an X-ray powder diffraction pattern comprising diffraction peaks at 2θ±0.2° diffraction angles of 6.3040, 10.4226, 13.6779, 15.9618, 20.3279, and 20.9348; further, the crystal form C of the ethanesulfonate has an X-ray powder diffraction pattern comprising diffraction peaks at 2θ±0.2° diffraction angles of 6.3040, 8.7771, 10.4226, 13.6779, 15.9618, 19.0549, 20.3279, and 20.9348; furthermore, the crystal form C of the ethanesulfonate has an X-ray powder diffraction pattern comprising diffraction peaks at 2θ±0.2° diffraction angles of 6.3040, 8.7771, 10.4226, 13.6779, 15.9618, 17.6528, 19.0549, 19.8646, 20.3279, 20.9348, 21.7435, 23.4169, and 26.2647; still further, the crystal form C of the ethanesulfonate has an X-ray powder diffraction pattern comprising diffraction peaks at 2θ±0.2° diffraction angles of 6.3040, 8.7771, 10.4226, 12.0274, 12.5635, 13.6779, 14.9315, 15.9618, 17.6528, 18.0467, 19.0549, 19.8646, 20.3279, 20.9348, 21.7435, 23.4169, 24.3601, 26.2647, 26.8633, 28.5527, 29.9377, 32.4837, and 35.6194; still further, the crystal form C of the ethanesulfonate has an XRPD pattern substantially as shown in FIG. 121b.

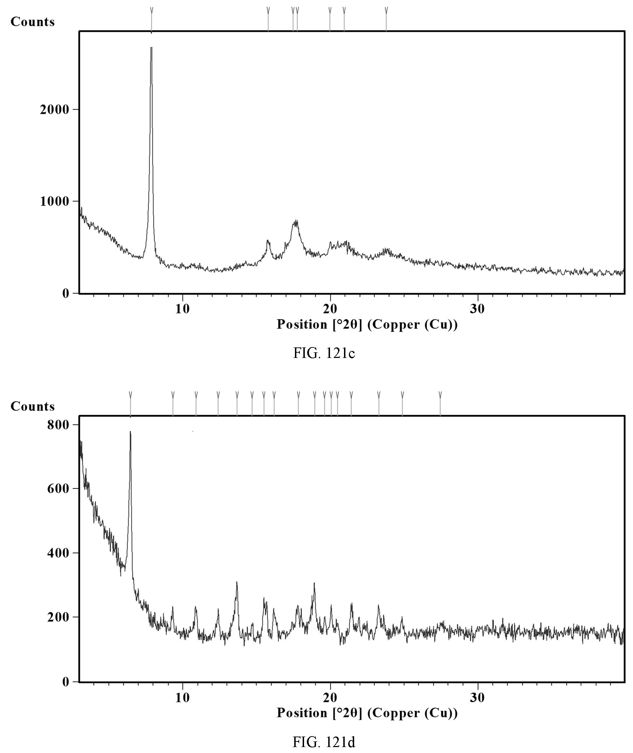

In some embodiments, the present disclosure provides crystal form D of the ethanesulfonate of the compound of formula I, and the crystal form D of the ethanesulfonate has an X-ray powder diffraction pattern comprising diffraction peaks at 2θ±0.2° diffraction angles of 7.9081, 15.7893, 17.4668, 17.7671, 19.9837, 20.9514, and 23.8046; further, the crystal form D of the ethanesulfonate has an XRPD pattern substantially as shown in FIG. 121c.

In some embodiments, the crystal form D of the ethanesulfonate has one or two or more of the following characteristics:
(1) a TGA curve showing a weight loss of 0.5-3%, preferably about 0.5-2% (e.g., 0.5%, 1.0%, 1.5%, or 2.0%), and more preferably about 1.70% at 150±3 °C;
(2) a DSC curve having 3 endothermic peaks at 55.5±3 °C, 142.4±3 °C, and 184.2±3 °C;
(3) a DSC curve having 1 exothermic peak at 91.6±3 °C;

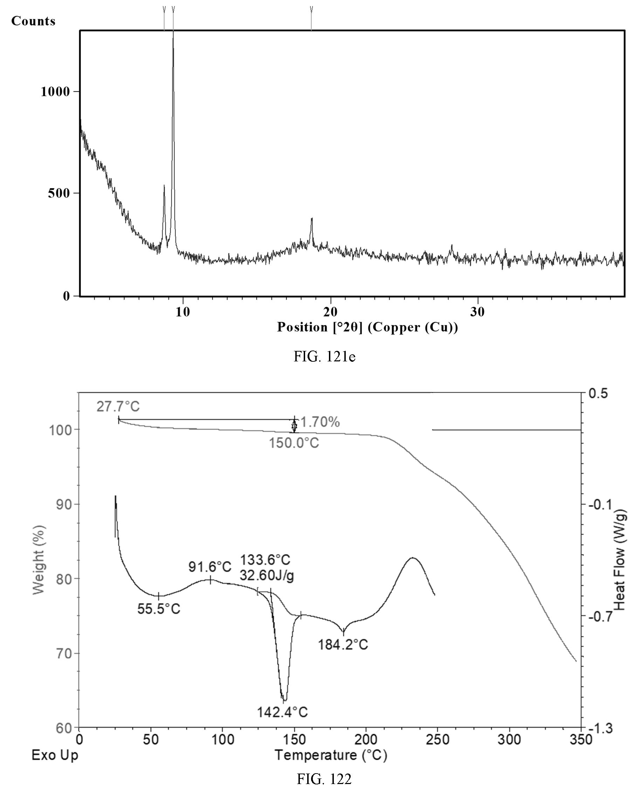

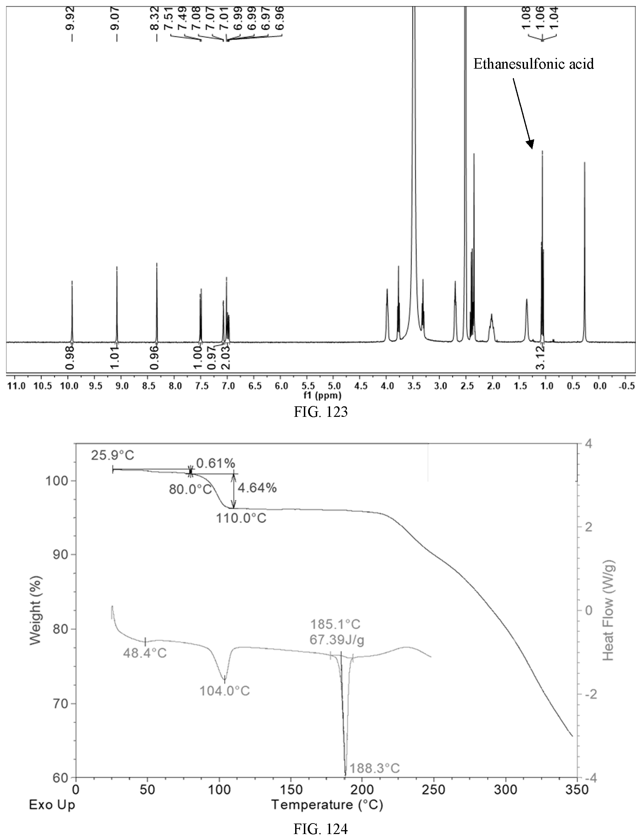

In some embodiments, a TGA/DSC profile of the crystal form D of the ethanesulfonate is substantially as shown in FIG. 122; a ¹H NMR spectrum of the crystal form D of the ethanesulfonate is substantially as shown in FIG. 123.

In some embodiments, the present disclosure provides crystal form E of the ethanesulfonate of the compound of formula I, and the crystal form E of the ethanesulfonate has an X-ray powder diffraction pattern comprising diffraction peaks at 2θ±0.2° diffraction angles of 6.4770, 13.6740, 15.4870, 18.9455, 20.0718, 21.4317, and 23.2933; further, the crystal form E of the ethanesulfonate has an X-ray powder diffraction pattern comprising diffraction peaks at 2θ±0.2° diffraction angles of 6.4770, 10.9010, 13.6740, 15.4870, 16.1908, 18.9455, 20.0718, 21.4317, and 23.2933; furthermore, the crystal form E of the ethanesulfonate has an X-ray powder diffraction pattern comprising diffraction peaks at 2θ±0.2° diffraction angles of 6.4770, 9.3320, 10.9010, 12.3962, 13.6740, 15.4870, 16.1908, 17.8603, 18.9455, 19.6221, 20.0718, 21.4317, and 23.2933; still further, the crystal form E of the ethanesulfonate has an X-ray powder diffraction pattern comprising diffraction peaks at 2θ±0.2° diffraction angles of 6.4770, 9.3320, 10.9010, 12.3962, 13.6740, 14.7088, 15.4870, 16.1908, 17.8603, 18.9455, 19.6221, 20.0718, 20.4917, 21.4317, 23.2933, 24.8830, and 27.4600; still further, the crystal form E of the ethanesulfonate has an XRPD pattern substantially as shown in FIG. 121d.

In some embodiments, the crystal form E of the ethanesulfonate has one or two or more of the following characteristics:
(1) a TGA curve showing a weight loss of 2-7%, preferably about 3-6% (e.g., 4.0%, 4.5%, 5.0%, or 5.5%), and more preferably about 4.64% at 110±3 °C;
(2) a DSC curve having 3 endothermic peaks at 48.4±3 °C, 104.0±3 °C, and 188.3±3 °C.

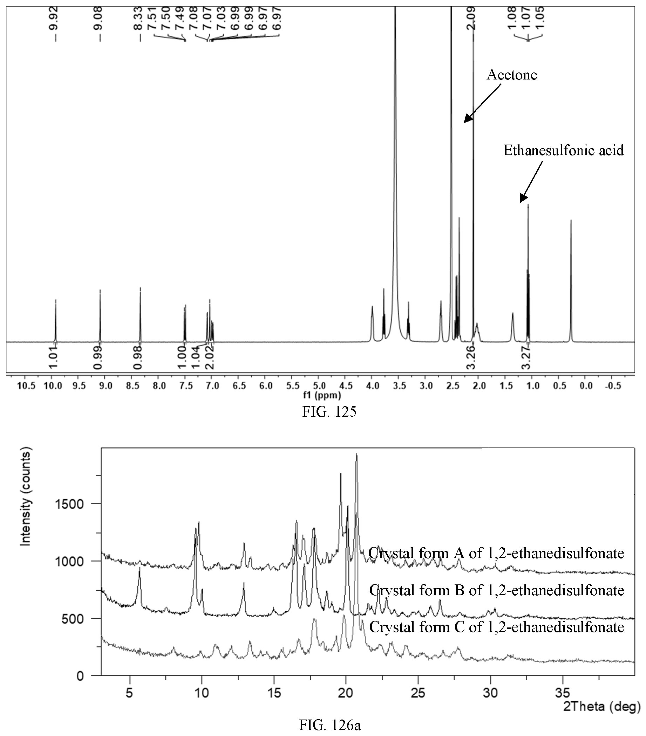

In some embodiments, a TGA/DSC profile of the crystal form E of the ethanesulfonate is substantially as shown in FIG. 124; a ¹H NMR spectrum of the crystal form E of the ethanesulfonate is substantially as shown in FIG. 125.

In some embodiments, the present disclosure provides crystal form F of the ethanesulfonate of the compound of formula I, and the crystal form F of the ethanesulfonate has an X-ray powder diffraction pattern comprising diffraction peaks at 2θ±0.2° diffraction angles of 8.7081, 9.3273, and 18.7028; further, the crystal form F of the ethanesulfonate has an XRPD pattern substantially as shown in FIG. 121e.

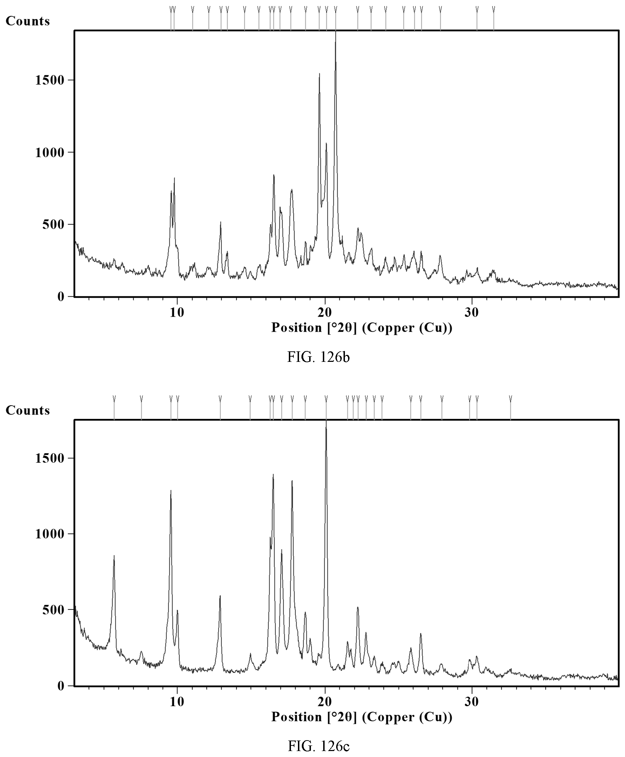

In some embodiments, the present disclosure provides crystal form A of the 1,2-ethanedisulfonate of the compound of formula I, and the crystal form A of the 1,2-ethanedisulfonate has an X-ray powder diffraction pattern comprising diffraction peaks at 2θ±0.2° diffraction angles of 9.5776, 9.7934, 16.5380, 17.6744, 19.6187, 20.1184, and 20.7238; further, the crystal form A of the 1,2-ethanedisulfonate has an X-ray powder diffraction pattern comprising diffraction peaks at 2θ±0.2° diffraction angles of 9.5776, 9.7934, 12.9489, 16.5380, 16.9718, 17.6744, 19.6187, 20.1184, and 20.7238; furthermore, the crystal form A of the 1,2-ethanedisulfonate has an X-ray powder diffraction pattern comprising diffraction peaks at 2θ±0.2° diffraction angles of 9.5776, 9.7934, 12.9489, 16.3117, 16.5380, 16.9718, 17.6744, 18.7068, 19.6187, 20.1184, 20.7238, and 22.2198; furthermore, the crystal form A of the 1,2-ethanedisulfonate has an X-ray powder diffraction pattern comprising diffraction peaks at 2θ±0.2° diffraction angles of 9.5776, 9.7934, 12.9489, 13.3845, 16.3117, 16.5380, 16.9718, 17.6744, 18.7068, 19.6187, 20.1184, 20.7238, 22.2198, 23.1294, 25.3534, 26.0706, 26.5387, and 27.8458; still further, the crystal form A of the 1,2-ethanedisulfonate has an X-ray powder diffraction pattern comprising diffraction peaks at 2θ±0.2° diffraction angles of 9.5776, 9.7934, 11.0447, 12.1309, 12.9489, 13.3845, 14.5687, 15.5203, 16.3117, 16.5380, 16.9718, 17.6744, 18.7068, 19.6187, 20.1184, 20.7238, 22.2198, 23.1294, 24.1339, 25.3534, 26.0706, 26.5387, 27.8458, 30.3527, and 31.4653; still further, the crystal form A of the 1,2-ethanedisulfonate has an XRPD pattern substantially as shown in FIG. 126b.

In some embodiments, the present disclosure provides crystal form B of the 1,2-ethanedisulfonate of the compound of formula I, and the crystal form B of the 1,2-ethanedisulfonate has an X-ray powder diffraction pattern comprising diffraction peaks at 2θ±0.2° diffraction angles of 9.5593, 16.2913, 16.5067, 17.0648, 17.7815, and 20.1011; further, the crystal form B of the 1,2-ethanedisulfonate has an X-ray powder diffraction pattern comprising diffraction peaks at 2θ±0.2° diffraction angles of 5.6887, 9.5593, 12.9139, 16.2913, 16.5067, 17.0648, 17.7815, and 20.1011; furthermore, the crystal form B of the 1,2-ethanedisulfonate has an X-ray powder diffraction pattern comprising diffraction peaks at 2θ±0.2° diffraction angles of 5.6887, 9.5593, 10.0235, 12.9139, 16.2913, 16.5067, 17.0648, 17.7815, 18.6648, 20.1011, and 22.2426; furthermore, the crystal form B of the 1,2-ethanedisulfonate has an X-ray powder diffraction pattern comprising diffraction peaks at 2θ±0.2° diffraction angles of 5.6887, 9.5593, 10.0235, 12.9139, 14.9480, 16.2913, 16.5067, 17.0648, 17.7815, 18.6648, 20.1011, 21.5441, 22.2426, 22.8020, 25.8424, 26.4853, 29.8463, and 30.3500; still further, the crystal form B of the 1,2-ethanedisulfonate has an X-ray powder diffraction pattern comprising diffraction peaks at 2θ±0.2° diffraction angles of 5.6887, 7.5361, 9.5593, 10.0235, 12.9139, 14.9480, 16.2913, 16.5067, 17.0648, 17.7815, 18.6648, 20.1011, 21.5441, 21.9422, 22.2426, 22.8020, 23.3535, 23.8750, 25.8424, 26.4853, 27.9265, 29.8463, 30.3500, and 32.6082; still further, the crystal form B of the 1,2-ethanedisulfonate has an XRPD pattern substantially as shown in FIG. 126c.

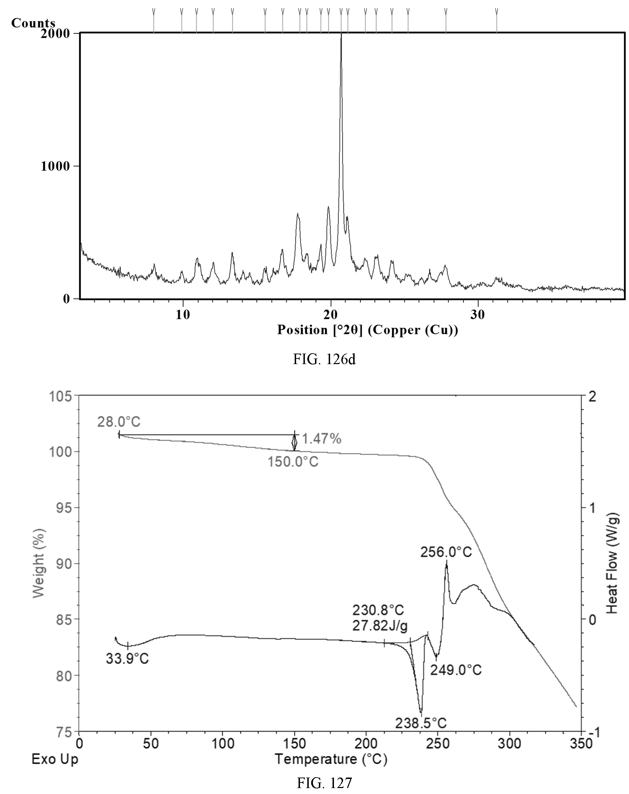

In some embodiments, the present disclosure provides crystal form C of the 1,2-ethanedisulfonate of the compound of formula I, and the crystal form C of the 1,2-ethanedisulfonate has an X-ray powder diffraction pattern comprising diffraction peaks at 2θ±0.2° diffraction angles of 17.8877, 19.3330, 19.8489, 20.7131, and 21.1631; further, the crystal form C of the 1,2-ethanedisulfonate has an X-ray powder diffraction pattern comprising diffraction peaks at 2θ±0.2° diffraction angles of 13.3305, 16.7417, 17.8877, 19.3330, 19.8489, 20.7131, and 21.1631; furthermore, the crystal form C of the 1,2-ethanedisulfonate has an X-ray powder diffraction pattern comprising diffraction peaks at 2θ±0.2° diffraction angles of 10.9129, 13.3305, 16.7417, 17.8877, 18.3757, 19.3330, 19.8489, 20.7131, 21.1631, and 23.0842; furthermore, the crystal form C of the 1,2-ethanedisulfonate has an X-ray powder diffraction pattern comprising diffraction peaks at 2θ±0.2° diffraction angles of 10.9129, 12.0262, 13.3305, 16.7417, 17.8877, 18.3757, 19.3330, 19.8489, 20.7131, 21.1631, 22.3700, 23.0842, 24.1491, and 27.8056; still further, the crystal form C of the 1,2-ethanedisulfonate has an X-ray powder diffraction pattern comprising diffraction peaks at 2θ±0.2° diffraction angles of 7.9970, 9.8985, 10.9129, 12.0262, 13.3305, 15.5409, 16.7417, 17.8877, 18.3757, 19.3330, 19.8489, 20.7131, 21.1631, 22.3700, 23.0842, 24.1491, 25.2521, 27.8056, and 31.2456; still further, the crystal form C of the 1,2-ethanedisulfonate has an XRPD pattern substantially as shown in FIG. 126d.

In another aspect, the present disclosure provides a preparation method for the free crystal form A of the compound of formula I, which comprises the following steps: placing the compound of formula I in an organic solvent, stirring, concentrating, drying, adding water, heating at reflux, stirring at room temperature overnight, filtering, and drying. The organic solvent is selected from acetonitrile.

In another aspect, the present disclosure provides a preparation method for the free crystal form B of the compound of formula I, which comprises the following steps: placing the compound of formula I in an organic solvent, slurrying for 1-5 days, centrifuging to separate the solid, and drying. The organic solvent is selected from an alcohol, preferably, the organic solvent is ethanol. The drying is selected from vacuum drying at a drying temperature of 40-60 °C, preferably 50 °C.

In another aspect, the present disclosure provides a preparation method for the free crystal form C of the compound of formula I, which comprises the following steps: placing the compound of formula I in an organic solvent, slurrying, centrifuging to separate the solid, and drying. The organic solvent is selected from MTBE. The drying is selected from vacuum drying at a drying temperature of 40-60 °C, preferably 50 °C. The slurrying is performed for 1-5 days, e.g., 1 day, 2 days, 3 days, 4 days, or 5 days.

According to an embodiment of the present disclosure, the compound of formula I may be prepared with reference to the synthesis method disclosed in Chinese Patent Application No. CN202310400928.8.

In another aspect, the present disclosure provides a preparation method for the free crystal form D of the compound of formula I, which comprises the following steps:
step 1. 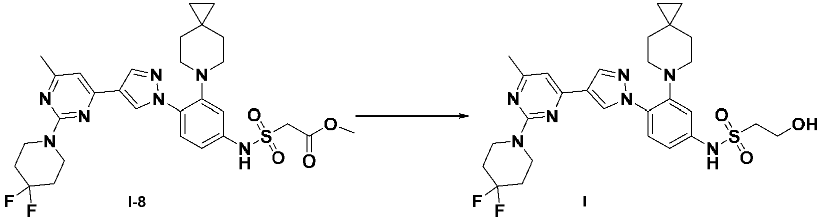 adding coompound I-8, THF, and absolute ethanol, stirring to dissolve completely, adding anhydrous calcium chloride, stirring to dissolve completely, cooling to 0 °C, adding sodium borohydride, allowing to react at room temperature overnight, pouring the reaction liquid into water, adding ethyl acetate, allowing to stand for phase separation, pouring out the supernatant, adding ethyl acetate to wash the bottom solid, concentrating the organic phase and purifying by silica gel column to obtain the compound of formula I, adding acetonitrile to dissolve completely, concentrating until no liquid drops fell, adding water and heating to 90-120 °C for slurrying for 4-6 h, filtering, washing the filter cake, then adding water again and heating to 90-120 °C for slurrying for 4-6 h, slurrying overnight at room temperature, filtering, and lyophilizing the solid to obtain a mixed crystal of the compound of formula I;
step 2. slurrying the mixed crystal of the compound of formula I in IPA/H₂O (volume ratio of 982:18) at room temperature for about 5 days, centrifuging to separate the solid, and air-drying in an open container at room temperature to obtain the crystal form D.

In another aspect, the present disclosure provides a preparation method for the free crystal form E of the compound of formula I, which comprises the following steps:
placing the mixed crystal of the compound of formula I under THF atmosphere for gas-solid diffusion, and air-drying in an open container at room temperature to obtain the target product.

In another aspect, the present disclosure provides a preparation method for the free crystal form F of the compound of formula I, which comprises the following steps:
heating the mixed crystal of the compound of formula I to 160-180 °C, and cooling to room temperature and room humidity to obtain the target product.

In another aspect, the present disclosure provides a preparation method for the free crystal form G of the compound of formula I, which comprises the following steps:
first dissolving the mixed crystal of the compound of formula I in 1,4-dioxane under H₂O atmosphere for gas-liquid diffusion to precipitate a solid, and drying in an open container in air to obtain the target product.

In another aspect, the present disclosure provides a preparation method for the free crystal form H of the compound of formula I, which comprises the following step:
placing the mixed crystal of the compound of formula I under DMSO atmosphere for gas-solid diffusion to obtain the target product.

In another aspect, the present disclosure provides a preparation method for the free crystal form I of the compound of formula I, which comprises the following steps:
placing the mixed crystal of the compound of formula I in MeOH, slurrying at room temperature for about 5 days, and air-drying in an open container at room temperature to obtain the target product.

In another aspect, the present disclosure provides a preparation method for the free crystal form J of the compound of formula I, which comprises the following steps:
heating the mixed crystal of the compound of formula I to 100 °C, and cooling to room temperature and room humidity to obtain the target product.

In another aspect, the present disclosure provides a preparation method for the free crystal form K of the compound of formula I, which comprises the following steps:
slurrying the mixed crystal of the compound of formula I in EtOH at room temperature for about 2 days, centrifuging to separate the solid, and drying under vacuum at room temperature to obtain the target product.

In another aspect, the present disclosure provides a preparation method for the free crystal form L of the compound of formula I, which comprises the following step:
slurrying the compound of formula I in MeOH at room temperature for about 3 days to obtain the target product.

In another aspect, the present disclosure further provides a preparation method for the pharmaceutically acceptable salt of the compound of formula I (including the crystal form of the pharmaceutically acceptable salt of the compound of formula I), which comprises the following methods:
Method I comprises the following steps: slurrying the compound of formula I or the mixed crystal of the compound of formula I with a salt-forming reagent (e.g., a corresponding acid) in solvent A, centrifuging to separate the solid, and drying. The solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water. The alcohol is selected from methanol and ethanol. The drying is selected from vacuum drying at a drying temperature of 40-60 °C, preferably 50 °C. The slurrying is performed for 1-5 days, e.g., 1 day, 2 days, 3 days, 4 days, or 5 days.
Method II comprises the following steps: slurrying the compound of formula I or the mixed crystal of the compound of formula I with a salt-forming reagent (e.g., a corresponding acid) in solvent A, adding solvent B for crystallization and gelling, performing temperature cycling between 50 °C and 5 °C to obtain a suspension, centrifuging to separate the solid, and drying. The solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water. The alcohol is selected from methanol and ethanol. The solvent B is selected from n-heptane. The drying is selected from vacuum drying at a drying temperature of 40-60 °C, preferably 50 °C. The slurrying is performed for 1-5 days, e.g., 1 day, 2 days, 3 days, 4 days, or 5 days.
Method III comprises the following steps: subjecting the compound of formula I or the mixed crystal of the compound of formula I and a salt-forming reagent (e.g., a corresponding acid) to temperature cycling between 50 °C and 5 °C in solvent A to obtain a solid, centrifuging to separate the solid, and drying. The solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water. The alcohol is selected from methanol and ethanol. The drying is selected from vacuum drying at a drying temperature of 40-60 °C, preferably 50 °C. The slurrying is performed for 1-5 days, e.g., 1 day, 2 days, 3 days, 4 days, or 5 days.

In yet another aspect, the present disclosure provides a pharmaceutical composition comprising one or more of the free crystal form (e.g., free crystal form A to free crystal form L) of the compound of formula I and the pharmaceutically acceptable salt (including the crystal form thereof) of the compound of formula I.

In yet another aspect, the present disclosure provides use of the free crystal form (e.g., free crystal form A to free crystal form L) of the compound of formula I, the pharmaceutically acceptable salt (including the crystal form thereof) of the compound of formula I, or the pharmaceutical composition for the manufacturing of a medicament, wherein the medicament may inhibit KIF18A; and/or prevent and/or treat a disease associated with KIF18A; the medicament is expected to be used for preventing and/or treating a proliferative disorder of a cancer, psoriasis, atopic dermatitis, an autoimmune disease, or an inflammatory bowel disease.

Cancers that should be mentioned include mesothelioma, neuroblastoma, rectal cancer, colon cancer, familiar adenomatous polyposis and hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid carcinoma, kidney cancer, renal parenchymal carcinoma, ovarian cancer, cervical cancer, corpus uteri cancer, endometrial cancer, choriocarcinoma, pancreatic cancer, prostate cancer, bladder cancer, testicular cancer, breast cancer, urinary cancer, melanoma, brain tumor, lymphoma, head and neck cancer, acute lymphoblastic leukemia, chronic lymphoblastic leukemia, acute myeloid leukemia, chronic myeloid leukemia, hepatocellular carcinoma, gallbladder cancer, bronchial cancer, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma.

Autoimmune diseases that should be mentioned include rheumatoid arthritis, systemic lupus erythematosus, Sjogren's syndrome, scleroderma, mixed connective tissue disease, dermatomyositis, polymyositis, Reiter's syndrome, autoimmune lymphoproliferative syndrome, multiple sclerosis, myasthenia gravis, and encephalomyelitis.

Inflammatory bowel diseases that should be mentioned include ulcerative colitis or Crohn's disease.

According to an embodiment of the present disclosure, the free crystal form (e.g., free crystal form A to free crystal form L) of the compound of formula I or the pharmaceutically acceptable salt (including the crystal form thereof) of the compound of formula I may be used in combination with a second active therapeutic drug.

The present disclosure further relates to a method of prevention and/or treatment of a disease associated with KIF18A, which comprises administering to a patient a therapeutically effective amount of the free crystal form (e.g., free crystal form A to free crystal form L) of the compound of formula I, the pharmaceutically acceptable salt (including the crystal form thereof) of the compound of formula I, or the pharmaceutical composition.

### Definitions and Explanations of Terms

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the specification of the present application. Unless otherwise stated, a numerical range set forth in the specification and claims shall be construed as at least including each specific integer value within the range. For example, when certain numerical ranges are defined or understood as "numbers", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

When "about" a certain numerical value is recited in the specification and claims, it shall be construed as including the numerical value itself, as well as numerical values within a range around the numerical value that is acceptable in the art, for example, numerical values within the range of ±15% of the numerical value, numerical values within the range of ±10% of the numerical value, numerical values within the range of ±5% of the numerical value, and the like. For example, about 10 represents that it includes a numerical value within the range of 10±1.5, i.e., within the range of 8.5 to 11.5; a numerical value within the range of 10±1.0, i.e., within the range of 9.0 to 11.0; and a numerical value within the range of 10±0.5, i.e., within the range of 9.5 to 10.5.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms).

The term "pharmaceutically acceptable" means that a formula component or an active ingredient does not unduly and adversely affect a general therapeutic target's health.

The term "crystal form" refers to crystal forms that have identical chemical composition but different spatial arrangements of crystal-forming molecules and/or ions.

The term "X-ray powder diffraction pattern substantially as shown in the figure" means that at least 50%, or at least 60%, or at least 70%, or at least 80%, or at least 90%, or at least 95%, or at least 99% of the main peaks shown in an X-ray powder diffraction pattern appear in the X-ray powder diffraction pattern. The main peak refers to a peak with a relative intensity greater than 10%, preferably greater than 20%, and more preferably greater than 30%, using the highest peak as a reference (the relative intensity of the highest peak is designated as 100%).

Those skilled in the art can determine appropriate amounts of any one of or a mixture (in any ratio) of any two of the salt, the crystal form of the salt and the free crystal form, and various pharmaceutically acceptable carriers and/or additional active ingredients in the pharmaceutical composition according to conventional methods.

### BRIEF DESCRIPTION OF THE DRAWINGS

FIG. 1 is an XRPD pattern of the free crystal form A.
FIG. 2 is a TGA/DSC profile of the free starting sample (crystal form A).
FIG. 3a is a ¹H NMR spectrum of the free starting sample (crystal form A).
FIG. 3b is an XRPD overlay of the free starting samples (crystal form A) before and after the heating experiment.
FIG. 4 is an XRPD overlay of samples of different free crystal forms.
FIG. 5 is an XRPD pattern of the free crystal form B.
FIG. 6 is a TGA/DSC profile of the free crystal form B.
FIG. 7a is a ¹H NMR spectrum of the free crystal form B.
FIG. 7b is a ¹H NMR spectrum of the free crystal form B after the heating experiment (60 °C).
FIG. 7c is a ¹H NMR spectrum of the free crystal form B after the heating experiment (120 °C).
FIG. 8 is an XRPD pattern of the free crystal form C.
FIG. 9 is a TGA/DSC profile of the free crystal form C.
FIG. 10 is a ¹H NMR spectrum of the free crystal form C.
FIG. 11 is an XRPD overlay of samples of different crystal forms of the hydrochloride.
FIG. 12a is an XRPD pattern of the crystal form A of the hydrochloride.
FIG. 12b is an XRPD pattern of the crystal form A of the hydrochloride prepared repeatedly.
FIG. 13 is a TGA/DSC profile of the crystal form A of the hydrochloride.
FIG. 14 is a ¹H NMR spectrum of the crystal form A of the hydrochloride.
FIG. 15 is an XRPD pattern of the crystal form B of the hydrochloride.
FIG. 16 is a TGA/DSC profile of the crystal form B of the hydrochloride.
FIG. 17 is a ¹H NMR spectrum of the crystal form B of the hydrochloride.
FIG. 18 is an XRPD pattern of the crystal form C of the hydrochloride.
FIG. 19 is a TGA/DSC profile of the crystal form C of the hydrochloride.
FIG. 20 is a ¹H NMR spectrum of the crystal form C of the hydrochloride.
FIG. 21 is an XRPD overlay of samples of different crystal forms of the sulfate.
FIG. 22 is an XRPD pattern of the crystal form A of the sulfate.
FIG. 23 is a TGA/DSC profile of the crystal form A of the sulfate.
FIG. 24 is a ¹H NMR spectrum of the crystal form A of the sulfate.
FIG. 25 is an XRPD pattern of the crystal form B of the sulfate.
FIG. 26 is a TGA/DSC profile of the crystal form B of the sulfate.
FIG. 27 is a ¹H NMR spectrum of the crystal form B of the sulfate.
FIG. 28 is an XRPD pattern of the crystal form C of the sulfate.
FIG. 29 is a TGA/DSC profile of the crystal form C of the sulfate.
FIG. 30 is a ¹H NMR spectrum of the crystal form C of the sulfate.
FIG. 31 is an XRPD pattern of the crystal form A of the maleate.
FIG. 32 is a TGA/DSC profile of the crystal form A of the maleate.
FIG. 33 is a ¹H NMR spectrum of the crystal form A of the maleate.
FIG. 34 is an XRPD pattern of the crystal form A of the phosphate.
FIG. 35 is a TGA/DSC profile of the crystal form A of the phosphate.
FIG. 36 is a ¹H NMR spectrum of the crystal form A of the phosphate.
FIG. 37 is an XRPD overlay of samples of different crystal forms of the p-toluenesulfonate.
FIG. 38 is an XRPD pattern of the crystal form A of the p-toluenesulfonate.
FIG. 39 is a TGA/DSC profile of the crystal form A of the p-toluenesulfonate.
FIG. 40 is a ¹H NMR spectrum of the crystal form A of the p-toluenesulfonate.
FIG. 41 is an XRPD pattern of the crystal form B of the p-toluenesulfonate.
FIG. 42 is a TGA/DSC profile of the crystal form B of the p-toluenesulfonate.
FIG. 43 is a ¹H NMR spectrum of the crystal form B of the p-toluenesulfonate.
FIG. 44 is an XRPD overlay of samples of different crystal forms of the methanesulfonate.
FIG. 45 is an XRPD pattern of the crystal form A of the methanesulfonate.
FIG. 46 is a TGA/DSC profile of the crystal form A of the methanesulfonate.
FIG. 47 is a ¹H NMR spectrum of the crystal form A of the methanesulfonate.
FIG. 48 is an XRPD pattern of the crystal form B of the methanesulfonate.
FIG. 49 is a TGA/DSC profile of the crystal form B of the methanesulfonate.
FIG. 50 is a ¹H NMR spectrum of the crystal form B of the methanesulfonate.
FIG. 51 is an XRPD pattern of the crystal form C of the methanesulfonate.
FIG. 52 is a TGA/DSC profile of the crystal form C of the methanesulfonate.
FIG. 53 is a ¹H NMR spectrum of the crystal form C of the methanesulfonate.
FIG. 54 is an XRPD overlay of samples of different crystal forms of the ethanesulfonate.
FIG. 55 is an XRPD pattern of the crystal form A of the ethanesulfonate.
FIG. 56 is a TGA/DSC profile of the crystal form A of the ethanesulfonate.
FIG. 57 is a ¹H NMR spectrum of the crystal form A of the ethanesulfonate.
FIG. 58a is an XRPD pattern of the crystal form B of the ethanesulfonate.
FIG. 58b is an XRPD overlay of the crystal form B of the ethanesulfonate prepared repeatedly.
FIG. 59 is a TGA/DSC profile of the crystal form B of the ethanesulfonate.
FIG. 60 is a ¹H NMR spectrum of the crystal form B of the ethanesulfonate.
FIG. 61 is a solubility curve graph of the crystal forms in water at room temperature.
FIG. 62 is a solubility curve graph of the crystal forms in SGF at room temperature.
FIG. 63 is a solubility curve graph of the crystal forms in FaSSIF at room temperature.
FIG. 64 is an XRPD pattern of free starting sample 2 (mixed crystal).
FIG. 65 is a TGA/DSC profile of free starting sample 2 (mixed crystal).
FIG. 66 is a ¹H NMR spectrum of the free starting sample 2 (mixed crystal).
FIG. 67 is an XRPD pattern of the free crystal form D.
FIG. 68 is a TGA/DSC profile of the free crystal form D.
FIG. 69 is a ¹H NMR spectrum of the free crystal form D.
FIG. 70 is a VT-XRPD overlay of the free crystal form D.
FIG. 71 is an XRPD pattern of the free crystal form E sample.
FIG. 72 is a TGA/DSC profile of the free crystal form E.
FIG. 73 is a ¹H NMR spectrum of the free crystal form E.
FIG. 74 is an XRPD overlay of the free crystal form E before and after the heating experiment.
FIG. 75 is a ¹H NMR spectrum of the free crystal form E after the heating experiment.
FIG. 76 is an XRPD pattern of the free crystal form F sample.
FIG. 77 is a TGA/DSC profile of the free crystal form F.
FIG. 78 is a ¹H NMR spectrum of the free crystal form F.
FIG. 79 is an XRPD pattern of the free crystal form G sample.
FIG. 80 is a TGA/DSC profile of the free crystal form G.
FIG. 81 is a ¹H NMR spectrum of the free crystal form G.
FIG. 82 is an XRPD overlay of the free crystal form G before and after the heating experiment.
FIG. 83 is a ¹H NMR spectrum of the free crystal form G after the heating experiment (120 °C).
FIG. 84 is a ¹H NMR spectrum of the free crystal form G after the heating experiment (160 °C).
FIG. 85a is an XRPD overlay of the free crystal form H samples before and after air-drying.
FIG. 85b is an XRPD pattern of the free crystal form H sample.
FIG. 86 is an XRPD pattern of the free crystal form I sample.
FIG. 87 is a TGA/DSC profile of the free crystal form I.
FIG. 88 is a ¹H NMR spectrum of the free crystal form I.
FIG. 89 is an XRPD overlay of the free crystal form I before and after the heating experiment.
FIG. 90 is an XRPD pattern of the free crystal form J sample.
FIG. 91 is a TGA/DSC profile of the free crystal form J.
FIG. 92 is a ¹H NMR spectrum of the free crystal form J.
FIG. 93 is a VH-XRPD overlay of the free crystal form J.
FIG. 94 is an XRPD pattern of the free crystal form K sample.
FIG. 95 is a TGA/DSC profile of the free crystal form K.
FIG. 96 is a 1H NMR spectrum of the free crystal form K.
FIG. 97 is an XRPD overlay of the free crystal form K before and after the heating experiment.
FIG. 98 is a ¹H NMR spectrum of the free crystal form K after the heating experiment.
FIG. 99a is an XRPD overlay of the free crystal form L samples.
FIG. 99b is an XRPD pattern of the free crystal form L sample.
FIG. 100 is an XRPD overlay of samples from the suspension competition experiment of free polymorphs (I/II).
FIG. 101 is an XRPD overlay of samples from the suspension competition experiment of free polymorphs (II/II).
FIG. 102a is an XRPD overlay of the sample of the crystal form D of the methanesulfonate.
FIG. 102b is an XRPD pattern of the sample of the crystal form D of the methanesulfonate.
FIG. 103 is a TGA/DSC profile of the crystal form D of the methanesulfonate.
FIG. 104 is a 1H NMR spectrum of the crystal form D of the methanesulfonate.
FIG. 105 is an XRPD overlay of samples of different crystal forms of the hydrochloride (I/II).
FIG. 106a is an XRPD overlay of samples of different crystal forms of the hydrochloride (II/II).
FIG. 106b is an XRPD pattern of the sample of the crystal form G of the hydrochloride.
FIG. 106c is an XRPD pattern of the sample of the crystal form H of the hydrochloride.
FIG. 106d is an XRPD pattern of the sample of the crystal form I of the hydrochloride.
FIG. 106e is an XRPD pattern of the sample of the crystal form J of the hydrochloride.
FIG. 106f is an XRPD pattern of the sample of the crystal form K of the hydrochloride.
FIG. 106g is an XRPD pattern of the sample of the crystal form L of the hydrochloride.
FIG. 106h is an XRPD pattern of the sample of the crystal form M of the hydrochloride.
FIG. 107 is an XRPD pattern of the sample of the crystal form D of the hydrochloride.
FIG. 108 is a TGA/DSC profile of the crystal form H of the hydrochloride.
FIG. 109 is a 1H NMR spectrum of the crystal form H of the hydrochloride.
FIG. 110 is an XRPD pattern of the sample of the crystal form E of the hydrochloride.
FIG. 111 is an XRPD pattern of the sample of the crystal form F of the hydrochloride.
FIG. 112a is an XRPD overlay of samples of different crystal forms of the methanesulfonate (I/III).
FIG. 112b is an XRPD pattern of the sample of the crystal form E of the methanesulfonate.
FIG. 112c is an XRPD pattern of the sample of the crystal form F of the methanesulfonate.
FIG. 113a is an XRPD overlay of samples of different crystal forms of the methanesulfonate (II/III).
FIG. 113b is an XRPD pattern of the sample of the crystal form G of the methanesulfonate.
FIG. 113c is an XRPD pattern of the sample of the crystal form H of the methanesulfonate.
FIG. 113d is an XRPD pattern of the sample of the crystal form I of the methanesulfonate.
FIG. 113e is an XRPD pattern of the sample of the crystal form J of the methanesulfonate.
FIG. 113f is an XRPD pattern of the sample of the crystal form K of the methanesulfonate.
FIG. 113g is an XRPD pattern of the sample of the crystal form L of the methanesulfonate.
FIG. 114a is an XRPD overlay of samples of different crystal forms of the methanesulfonate (III/III).
FIG. 114b is an XRPD pattern of the sample of the crystal form M of the methanesulfonate.
FIG. 114c is an XRPD pattern of the sample of the crystal form N of the methanesulfonate.
FIG. 114d is an XRPD pattern of the sample of the crystal form O of the methanesulfonate.
FIG. 115 is a TGA/DSC profile of the crystal form E of the methanesulfonate.
FIG. 116 is a 1H NMR spectrum of the crystal form E of the methanesulfonate.
FIG. 117 is a TGA/DSC profile of the crystal form F of the methanesulfonate.
FIG. 118 is a 1H NMR spectrum of the crystal form F of the methanesulfonate.
FIG. 119 is a TGA/DSC profile of the crystal form O of the methanesulfonate.
FIG. 120 is a 1H NMR spectrum of the crystal form O of the methanesulfonate.
FIG. 121a is an XRPD overlay of samples of different crystal forms of the ethanesulfonate.
FIG. 121b is an XRPD pattern of the sample of the crystal form C of the ethanesulfonate.
FIG. 121c is an XRPD pattern of the sample of the crystal form D of the ethanesulfonate.
FIG. 121d is an XRPD pattern of the sample of the crystal form E of the ethanesulfonate.
FIG. 121e is an XRPD pattern of the sample of the crystal form F of the ethanesulfonate.
FIG. 122 is a TGA/DSC profile of the crystal form D of the ethanesulfonate.
FIG. 123 is a 1H NMR spectrum of the crystal form D of the ethanesulfonate.
FIG. 124 is a TGA/DSC profile of the crystal form E of the ethanesulfonate.
FIG. 125 is a 1H NMR spectrum of the crystal form E of the ethanesulfonate.
FIG. 126a is an XRPD overlay of samples of different crystal forms of the 1,2-ethanedisulfonate.
FIG. 126b is an XRPD pattern of the sample of the crystal form A of the 1,2-ethanedisulfonate.
FIG. 126c is an XRPD pattern of the sample of the crystal form B of the 1,2-ethanedisulfonate.
FIG. 126d is an XRPD pattern of the sample of the crystal form C of the 1,2-ethanedisulfonate.
FIG. 127 is a TGA/DSC profile of the crystal form A of the 1,2-ethanedisulfonate.
FIG. 128 is a 1H NMR spectrum of the crystal form A of the 1,2-ethanedisulfonate.
FIG. 129 is a TGA/DSC profile of the crystal form B of the 1,2-ethanedisulfonate.
FIG. 130 is a 1H NMR spectrum of the crystal form B of the 1,2-ethanedisulfonate.
FIG. 131 is a TGA/DSC profile of the crystal form C of the 1,2-ethanedisulfonate.
FIG. 132 is a 1H NMR spectrum of the crystal form C of the 1,2-ethanedisulfonate.
FIG. 133 is simulated XRPD of a single crystal.
FIG. 134 is a molecular structure diagram of the thermal ellipsoid (50%) from single crystal diffraction.
FIG. 135 is a molecular structure packing diagram from single crystal diffraction, where the dashed lines represent hydrogen bond interactions.
FIG. 136 is a dynamic solubility curve graph at 37 °C.
FIG. 137 is an XRPD overlay of the solubility samples of the free crystal form D in H₂O.
FIG. 138 is an XRPD overlay of the solubility samples of the free crystal form D in SGF.
FIG. 139 is an XRPD overlay of the solubility samples of the free crystal form D in FaSSIF.
FIG. 140 is an XRPD overlay of the solubility samples of the free crystal form D in FeSSIF.
FIG. 141 is an XRPD overlay of the solubility samples of the crystal form B of the sulfate in H2O.
FIG. 142 is an XRPD overlay of the solubility samples of the crystal form B of the sulfate in SGF.
FIG. 143 is an XRPD overlay of the solubility samples of the crystal form B of the sulfate in FaSSIF.
FIG. 144 is an XRPD overlay of the solubility samples of the crystal form B of the sulfate in FeSSIF.
FIG. 145 is an XRPD overlay of the solubility samples of the crystal form B of the p-toluenesulfonate in H₂O.
FIG. 146 is an XRPD overlay of the solubility samples of the crystal form B of the p-toluenesulfonate in SGF.
FIG. 147 is an XRPD overlay of the solubility samples of the crystal form B of the p-toluenesulfonate in FaSSIF.
FIG. 148 is an XRPD overlay of the solubility samples of the crystal form B of the p-toluenesulfonate in FeSSIF.
FIG. 149 is an XRPD overlay of the solubility samples of the crystal form A of the maleate in H2O.
FIG. 150 is an XRPD overlay of the solubility samples of the crystal form A of the maleate in SGF.
FIG. 151 is an XRPD overlay of the solubility samples of the crystal form A of the maleate in FaSSIF.
FIG. 152 is an XRPD overlay of the solubility samples of the crystal form A of the maleate in FeSSIF.
FIG. 153 is an XRPD overlay of the solubility samples of the crystal form E of the methanesulfonate in H2O.
FIG. 154 is an XRPD overlay of the solubility samples of the crystal form E of the methanesulfonate in SGF.
FIG. 155 is an XRPD overlay of the solubility samples of the crystal form E of the methanesulfonate in FaSSIF.
FIG. 156 is an XRPD overlay of the solubility samples of the crystal form E of the methanesulfonate in FeSSIF.
FIG. 157 is a DVS profile of the free crystal form D.
FIG. 158 is an XRPD overlay of the free crystal form D before and after the DVS test.
FIG. 159 is a DVS profile of the crystal form B of the sulfate.
FIG. 160 is an XRPD overlay of the crystal form B of the sulfate before and after the DVS test.
FIG. 161 is a DVS profile of the crystal form B of the p-toluenesulfonate.
FIG. 162 is an XRPD overlay of the crystal form B of the p-toluenesulfonate before and after the DVS test.
FIG. 163 is a DVS profile of the crystal form A of the maleate.
FIG. 164 is an XRPD overlay of the crystal form A of the maleate before and after the DVS test.
FIG. 165 is a DVS profile of the crystal form E of the methanesulfonate.
FIG. 166 is an XRPD overlay of the crystal form E of the methanesulfonate before and after the DVS test.
FIG. 167 is an XRPD overlay of samples of the free crystal form D before and after the stability experiment.
FIG. 168 is an XRPD overlay of samples of the crystal form B of the sulfate before and after the stability experiment.
FIG. 169 is an XRPD overlay of samples of the crystal form B of the p-toluenesulfonate before and after the stability experiment.
FIG. 170 is an XRPD overlay of samples of the crystal form A of the maleate before and after the stability experiment.
FIG. 171 is an XRPD overlay of samples of the crystal form E of the methanesulfonate before and after the stability experiment.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods.

Unless otherwise stated, the "free starting sample" in the following examples refers to the compound of formula I prepared according to Example 1.

### 1. Instruments and Detection Methods Adopted in the Present Disclosure as Below:

### 1.1. X-ray powder diffraction (XRPD)

XRPD patterns were acquired on an X-ray powder diffraction analyzer manufactured by PANalytacal, and the scanning parameters are shown in Table A-1 below.

**Table A-1. XRPD test parameters**

| **Parameter** | **XRPD** | **VT-XRPD/VH-XRPD** |
|---|---|---|
| Model | Empyrean/X' Pert3 | Empyrean |
| X-ray | Cu, Kα, | Cu, Kα, |
| | Kα1 (Å):1.540598, | Kα1 (Å):1.540598, |
| | Kα2 (Å):1.544426 | Kα2 (Å):1.544426 |
| | Kα2/Kα1 intensity ratio:0.50 | Kα2/Kα1 intensity ratio:0.50 |
| X-ray light tube settings | 45 kV, 40 mA | 45 kV, 40 mA |
| Divergence slit | 1/8° | 1/8° |
| Scanning mode | Continuous | Continuous |
| Scanning range (°2θ) | 3-40 | 3-40 |
| Scanning time per step (s) | 46.7 | 33.0 |
| Scanning step length (°2θ) | 0.0263 | 0.0167 |
| Test time | About 5 min | About 10 min 13 s |

### 1.2. Thermogravimetric analysis (TGA) and differential scanning calorimetry (DSC)

TGA and DSC profiles were acquired on a TA 5500 thermogravimetric analyzer and a TA 2500 differential scanning calorimeter, respectively, and the test parameters are listed in Table A-2 below.

**Table A-2. TGA and DSC test parameters**

| **Parameter** | **TGA** | **DSC** |
|---|---|---|
| Method | Linear heating | Linear heating |
| Sample tray | Aluminum tray, uncovered | Aluminum tray, with/without cover |
| Temperature range | Room temperature-setting endpoint temperature | 25 °C-setting endpoint temperature |
| Purging rate (°C/min) | 10 | 10 |
| Protective gas | Nitrogen | Nitrogen |

### 1.3. Liquid-state nuclear magnetic resonance (¹H NMR)

The liquid-state nuclear magnetic resonance spectra were acquired on a Bruker 400M nuclear magnetic resonance spectrometer with DMSO-*d₆* as a solvent.

### 1.4. High performance liquid chromatography and ion chromatography (HPLC/IC)

In the test, the solubility results were collected by an Agilent 1260 high performance liquid chromatograph, and the salt-forming molar ratio results for ions were collected by an Agilent 1260 high performance liquid chromatograph and an ion chromatograph. The analysis conditions are shown in Tables A-3 and A-4.

**Table A-3. High performance liquid chromatography test conditions**

| | | | | |
|---|---|---|---|---|
| **(I) Liquid chromatograph** | **Agilent 1260 DAD detector** | | | |
| Chromatographic column | Xtimate C18, 3.0×150 mm, 3 µm | | | |
| Mobile phase | A: 0.1%MSA in H₂O | | | |
| | B: ACN | | | |
| | Molar ratio | | Solubility | |

| | Time (min) | %B | Time (min) | %B |
|---|---|---|---|---|
| | 0.0 | 15 | 0.0 | 15 |
| | 30.0 | 90 | 6.0 | 60 |
| Gradient | 40.0 | 90 | 11.0 | 70 |
| | 41.0 | 15 | 13.0 | 90 |
| | 45.0 | 15 | 15.0 | 90 |
| | -- | -- | 15.1 | 15 |
| | -- | -- | 18.0 | 15 |
| Run time | 45 min | | 18 min | |
| Flow rate of mobile phase | 0.5 mL/min | | | |
| Injection volume | 5 µL | | | |
| **(I) Liquid chromatograph** | **Agilent 1260 DAD detector** | | | |
| Detection wavelength | UV at 238 nm | | | |
| Column temperature | 25 °C | | | |
| Injector temperature | RT | | | |
| Diluent | ACN/H₂O (1:1, v/v) | | | |
| (II) Liquid chromatograph | Agilent 1260 chromatograph | | | |
| Chromatographic column | Ultimate AQ- C18, 250×4.6 mm, 5 µm | | | |
| Mobile phase | A: 0.1% MSA in H₂O | | | |
| | B: ACN | | | |

| | **Solubility** | | **Purity** | |
|---|---|---|---|---|
| | Time (min) | %B | Time (min) | %B |
| | 0.0 | 15 | 0.0 | 15 |
| Gradient | 15.0 | 90 | 30.0 | 90 |
| | 16.0 | 90 | 40.0 | 90 |
| | 16.1 | 15 | 41.0 | 15 |
| | 19.0 | 15 | 45.0 | 15 |
| Run time | 19 min | | 45 min | |
| Flow rate of mobile phase | | 1.0 mL/min | | |
| Injection volume | | 5 µL | | |
| Detection wavelength | | UV at 238 nm | | |
| Column temperature | | 25 °C | | |
| Injector temperature | | RT | | |
| Diluent | | ACN: H₂O=1/1 (v/v) | | |

**Table A-4. Ion chromatography test conditions**

| | |
|---|---|
| **(I) Ion chromatograph** | **ThermoFisher ICS-1100** |
| Chromatographic column | Dionex IonPac^{™} AS18 RFIC^{™} 4×250mm Analytical |
| Mobile phase | 25 mM NaOH |
| Injection volume | 25 µL |
| Flow rate | 1.0 mL/min |
| Temperature | 35 °C |
| Column temperature | 35 °C |
| Current | 80 mA |
| Run time | 20 min |
| **(II) Ion chromatograph** | **Thermo AQ RFIC** |
| **(I) Ion chromatograph** | **ThermoFisher ICS-1100** |
| Chromatographic column | Dionex IonPac^{™} AS11 HC Analytical(4×250mm) |
| Mobile phase | 35 Mm KOH |
| Injection volume | 25 µL |
| Flow rate | 1.5 mL/min |
| Temperature | 35 °C |
| Column temperature | 35 °C |
| Current | 87 mA |
| Run time | 12 min |

### 1.5. Dynamic vapor sorption (DVS)

Dynamic vapor sorption (DVS) curves were acquired on a DVS IntrInsic from Surface Measurement Systems (SMS). The relative humidity at 25 °C was corrected with the deliquescence points of LiCl, Mg(NO₃)₂, and KCl. The DVS test parameters are listed in Table A-5.

**Table A-5. DVS test parameters**

| **Parameter** | **Set value** |
|---|---|
| Temperature | 25 °C |
| Sample amount | 10-20 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0%RH-95%RH |
| RH gradient | 10%RH (0%RH ~ 90%RH & 90%RH ~ 0%RH) |
| | 5%RH (90%RH ~ 95%RH & 95%RH ~ 90%RH) |

### 1.6. Polarizing microscope (PLM)

The polarizing microscope data were acquired by a Carl Zeiss Axio Lab. A1 upright microscope at room temperature.

### 1.7. Karl Fischer moisture titrator (KF)

The KF test was performed using a Metrohm 870 or Metrohm 831 KF titrator from Metrohm.

### 1.8. Liquid-state nuclear magnetic resonance (¹H NMR)

The liquid-state nuclear magnetic resonance spectra were acquired on a Bruker 400M nuclear magnetic resonance spectrometer with DMSO-*d₆* as a solvent.

### 1.9. X-ray single crystal diffraction

Instrument model: D8 Venture
Determination basis: JY/T 0588-2020 General rules for crystal and molecular structure determination of small molecule compounds by single crystal X-ray diffractometer
Determination environment: test temperature: -173 °C; room temperature: 24 °C; relative humidity: 30%
X-ray tube: Ga target
Wavelength: 1.34139 Å
Tube voltage: 70 kV
Tube current: 3.57 mA
Scanning mode: ω-2θ scanning
Scanning speed: 0.5°/frame
Scanning range: 3.582-72.451°
Test temperature: 100(2) K

### 2. Reagents Used in the Present Disclosure as Shown in Table A-6 Below

**Table A-6. Names for abbreviations of solvents used in the tests**

| **Abbreviation** | **Name** | **Abbreviation** | **Name** |
|---|---|---|---|
| MeOH | Methanol | ACN | Acetonitrile |
| EtOH | Ethanol | DCM | Dichloromethane |
| IPA | Isopropanol | Ethyl formate | Ethyl formate |
| Acetone | Acetone | n-Heptane | n-Heptane |
| MIBK | Methyl isobutyl ketone | H₂O | Water |
| EtOAc | Ethyl acetate | DMSO | Dimethylsulfoxide |
| IPAc | Isopropyl acetate | THF | Tetrahydrofuran |
| MTBE | Methyl tert-butyl ether | 2-MeTHF | 2-Methyltetrahydrofuran |
| 2,2,2-Trifluoroethanol | Trifluoroethanol | 1,2-Dimethoxyethane | Ethylene glycol dimethyl ether |
| Toluene | Toluene | 1,4-Dioxane | 1,4-Dioxane |
| Cyclohexane | Cyclohexane | Isopropyl ether | Isopropyl ether |
| NMP | N-Methylpyrrolidone | Ether | Diethyl ether |
| CHCl₃ | Trichloromethane | MEK | Butanone |
| n-hexane | n-Hexane | Aniosle | Anisole |
| Xylene | Xylene | Cumene | Cumene |
| n-Pentane | n-Pentane | t-BuOH | tert-Butanol |
| n-PrOH | n-Propanol | Cyclohexone | Cyclohexanone |
| Methyl acetate | Methyl acetate | DMF | N,N-Dimethylformamide |
| DMAc | N,N-Dimethylacetamide | Chlorobenzene | Chlorobenzene |

### 3. Preparation of Biological Vehicles of the Present Disclosure

### 3.1. Preparation of simulated gastric fluid (SGF)

100 mg of NaCl and 50 mg of Triton X-100 were weighed into a 50 mL volumetric flask and then completely dissolved by addition of purified water. 816 µL of 1 M hydrochloric acid was added, and the pH was adjusted to 1.8 with 1 M hydrochloric acid or 1 M NaOH solution. The mixed solution was brought to the volume by addition of purified water.

### 3.2. Preparation of fasted state simulated intestinal fluid (FaSSIF)

340 mg of anhydrous NaH₂PO₄ and 620 mg of NaCl were weighed into a 100 mL volumetric flask, and then completely dissolved by addition of purified water. 55.44 µL of a 50% NaOH solution was then added, and the pH was adjusted to 6.5 with 1 M hydrochloric acid or 1 M NaOH solution. The mixed solution was brought to the volume by addition of purified water. Subsequently, 110 mg of SIF powder was weighed into a 50 mL volumetric flask and completely dissolved in the solution described above, and the mixed solution was brought to the volume.

### 3.3. Preparation of fed state simulated intestinal fluid (FeSSIF)

0.82 mL of glacial acetic acid and 1.18 g of NaCl were added to a 100 mL volumetric flask and completely dissolved by addition of purified water. 528 µL of a 50% NaOH solution was then added, the pH was adjusted to 5.0 with 1 M hydrochloric acid or 1 M NaOH solution, and the mixed solution was brought to the volume by addition of purified water. Subsequently, 560 mg of SIF powder was weighed into a 50 mL volumetric flask and completely dissolved in the solution described above, and the mixed solution was brought to the volume.

### Example 1. Preparation of Compound of Formula I

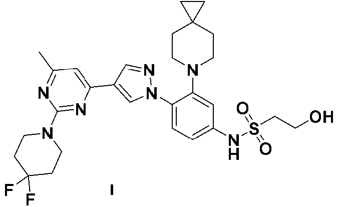

The compound of formula I can be prepared with reference to the synthesis method disclosed in Chinese Patent Application No. CN202310400928.8, and the synthetic route is as follows:

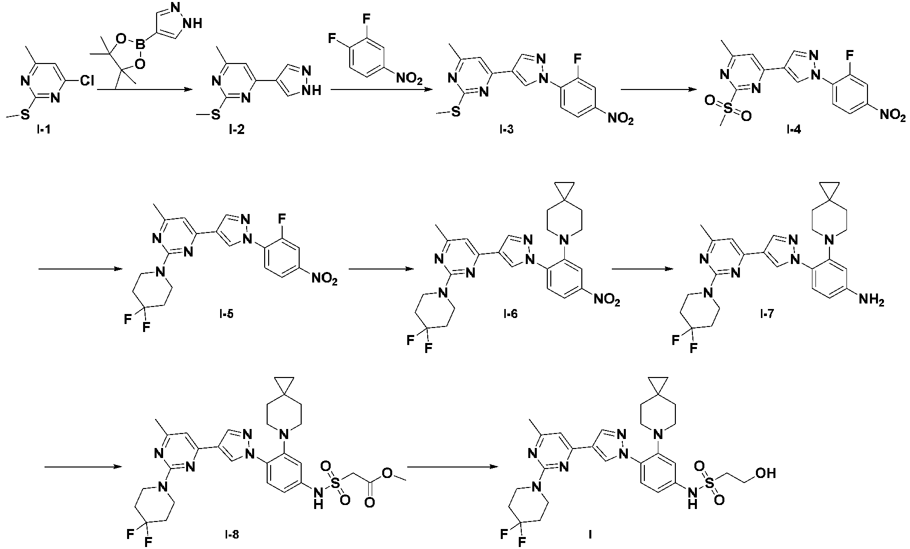

### Step 1: Synthesis of 4-methyl-2-(methylthio)-6-(1H-pyrazol-4-yl)pyrimidine

4-Chloro-6-methyl-2-(methylthio)pyrimidine (30 g, 172 mmol) was added to 300 mL of N,N-dimethylformamide, and then 4-(4,4,5,5-tetramethyl-1,3,2-dioxaborolan-2-yl)-1H-pyrazole (50 g, 258 mmol), potassium carbonate (47.5 g, 344 mmol), water (50 mL), and 1,1'-bis(diphenylphosphino)ferrocene dichloropalladium(II) (12.57 g, 17.18 mmol) were added. The mixture was heated to 105 °C and allowed to react for 16 h. LCMS showed that the starting materials were completely reacted. The reaction liquid was concentrated to dryness, and the residue was separated and purified by a silica gel column (PE:EA (V/V) = 1:1) to obtain 4-methyl-2-(methylthio)-6-(1H-pyrazol-4-yl)pyrimidine (20 g, yield: 56.4%). LC-MS, M/Z (ESI): 207.2 [M+H]⁺

### Step 2: Synthesis of 4-(1-(2-fluoro-4-nitrophenyl)-1H-pyrazol-4-yl)-6-methyl-2-(methylthio)pyrimidine

4-Methyl-2-(methylthio)-6-(1H-pyrazol-4-yl)pyrimidine (20 g, 97 mmol) was added to 20 mL of N,N-dimethylformamide, and then 1,2-difluoro-4-nitrobenzene (46.3 g, 291 mmol) and cesium carbonate (63.2 g, 194 mmol) were added. The mixture was allowed to react at room temperature for 16 h. LCMS showed that the starting materials were completely reacted. The reaction liquid was concentrated to dryness, and the residue was separated and purified by a silica gel column (volume ratio: PE:DCM:EA = 3:3:1) to obtain 4-(1-(2-fluoro-4-nitrophenyl)-1H-pyrazol-4-yl)-6-methyl-2-(methylthio)pyrimidine (23 g, yield: 68.7%). LC-MS, M/Z (ESI): 346.1 [M+H]⁺

### Step 3: Synthesis of 4-(1-(2-fluoro-4-nitrophenyl)-1H-pyrazol-4-yl)-6-methyl-2-(methylsulfonyl)pyrimidine

4-(1-(2-Fluoro-4-nitrophenyl)-1H-pyrazol-4-yl)-6-methyl-2-(methylthio)pyrimidine (23 g, 66.6 mmol) was added to 300 mL of dichloromethane, and then m-chloroperoxybenzoic acid (40.2 g, 233 mmol) was added. The mixture was allowed to react at room temperature for 1 h. LCMS showed that the starting materials were completely reacted. A saturated sodium sulfite solution (200 mL) was added, and the mixture was stirred for 30 min. A saturated sodium carbonate solution (100 mL) was added, and the mixture was stirred for 10 min and then extracted with dichloromethane (200 mL × 3), followed by liquid separation. The organic phase was concentrated to dryness, and the residue was separated and purified by a silica gel column (DCM:EA (V/V) = 3:1) to obtain 4-(1-(2-fluoro-4-nitrophenyl)-1H-pyrazol-4-yl)-6-methyl-2-(methylsulfonyl)pyrimidine (13 g, yield: 51.7%). LC-MS, M/Z (ESI): 378.1 [M+H]⁺

### Step 4: Synthesis of 2-(4,4-difluoropiperidin-1-yl)-4-(1-(2-fluoro-4-nitrophenyl)-1H-pyrazol-4-yl)-6-methylpyrimidine

4-(1-(2-Fluoro-4-nitrophenyl)-1H-pyrazol-4-yl)-6-methyl-2-(methylsulfonyl)pyrimidine (6 g, 15.9 mmol) was added to 60 mL of N,N-dimethylformamide, and then 4,4-difluoropiperidine hydrochloride (3.76 g, 23.85 mmol) and potassium carbonate (8.79 g, 63.6 mmol) were added. The mixture was heated to 105 °C and allowed to react overnight. LCMS showed that the starting materials were completely reacted. The reaction liquid was concentrated to dryness, and the residue was separated and purified by a silica gel column (volume ratio: PE:DCM:EA = 3:3:1) to obtain 2-(4,4-difluoropiperidin-1-yl)-4-(1-(2-fluoro-4-nitrophenyl)-1H-pyrazol-4-yl)-6-methylpyrimidine (6 g, yield: 90%). LC-MS, M/Z (ESI): 419.1 [M+H]⁺

### Step 5: Synthesis of 6-(2-(4-(2-(4,4-difluoropiperidin-1-yl)-6-methylpyrimidin-4-yl)-1H-pyrazol-1-yl)-5-nitrophenyl)-6-azaspiro[2.5]octane

2-(4,4-Difluoropiperidin-1-yl)-4-(1-(2-fluoro-4-nitrophenyl)-1H-pyrazol-4-yl)-6-methylpyrimidine (3 g, 7.17 mmol) was added to 30 mL of dimethylsulfoxide, and then 6-azaspiro[2.5]octane hydrochloride (1.588 g, 10.76 mmol) and potassium carbonate (3.96 g, 28.7 mmol) were added. The mixture was heated to 140 °C and allowed to react for 8 h. LCMS showed that the starting materials were completely reacted. The reaction liquid was poured into 200 mL of water, and the mixture was filtered to obtain 6-(2-(4-(2-(4,4-difluoropiperidin-1-yl)-6-methylpyrimidin-4-yl)-1H-pyrazol-1-yl)-5-nitrophenyl)-6-azaspiro[2.5]octane (2.6 g, yield: 71.2%). LC-MS, M/Z (ESI): 510.2 [M+H]⁺

### Step 6: Synthesis of 4-(4-(2-(4,4-difluoropiperidin-1-yl)-6-methylpyrimidin-4-yl)-1H-pyrazol-1-yl)-3-(6-azaspiro[2.5]octan-6-yl)aniline

6-(2-(4-(2-(4,4-Difluoropiperidin-1-yl)-6-methylpyrimidin-4-yl)-1H-pyrazol-1-yl)-5-nitrophenyl)-6-azaspiro[2.5]octane (2.6 g, 5.1 mmol) was added to 60 mL of ethanol, and then iron powder (2.85 g, 51 mmol) and ammonium chloride (2.73 g, 51 mmol) were added. The mixture was heated to 90 °C and allowed to react for 8 h. LCMS showed that the starting materials were completely reacted. The mixture was filtered and washed with 60 mL of ethanol. The filtrate was concentrated to dryness, and the residue was separated and purified by a silica gel column (PE:EA (V/V) = 3:1) to obtain 4-(4-(2-(4,4-difluoropiperidin-1-yl)-6-methylpyrimidin-4-yl)-1H-pyrazol-1-yl)-3-(6-azaspiro[2.5]octan-6-yl)aniline (2.4 g, yield: 98%). LC-MS, M/Z (ESI): 480.2 [M+H]⁺

### Step 7: Synthesis of methyl 2-(N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)sulfamoyl)acetate

4-(4-(2-(4,4-Difluoropiperidin-1-yl)-6-methylpyrimidin-4-yl)-1H-pyrazol-1-yl)-3-(6-azaspiro[2.5]octan-6-yl)aniline (2.4 g, 5 mmol) was added to 30 mL of dichloromethane, and then triethylamine (1.94 g, 15.01 mmol) and methyl 2-(chlorosulfonyl)acetate (1.036 g, 5 mmol) were added. The mixture was allowed to react at room temperature for 1 h. LCMS showed that the starting materials were completely reacted. The reaction liquid was concentrated to dryness, and the residue was separated and purified by a silica gel column (PE:EA (V/V) = 5:1) to obtain methyl 2-(N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)sulfamoyl)acetate (1.9 g, yield: 61.7%). LC-MS, M/Z (ESI): 616.2 [M+H]⁺

### Step 8: Synthesis of N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

Methyl 2-(N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)sulfamoyl)acetate (1.90 g, 3.1 mmol) was dissolved in tetrahydrofuran (20 mL), and then lithium borohydride (2 M, 1.85 mL) was added dropwise under nitrogen atmosphere at 0 °C. The mixture was allowed to react at 0 °C for 1 h. After the reaction was completed, the reaction liquid was quenched with a saturated ammonium chloride solution (50 mL) and extracted with ethyl acetate (200 mL × 3). The organic phase was washed with saturated brine (100 mL), dried over anhydrous sodium sulfate, filtered, and concentrated to obtain a crude product, which was then separated and purified by reverse phase Flash (0.1% HCl) to obtain N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide (1.31 g, 2.17 mmol, purity: 97.5%, yield: 71.0%).

The sample (1.31 g) obtained in this Preparation Example was added to acetonitrile (20 mL), and the mixture was stirred at room temperature for 2 h, concentrated, and dried. Then water (20 mL) was added, and the mixture was heated to 100 °C, refluxed for 3 h, stirred at room temperature overnight, and filtered. The solid was lyophilized to obtain a starting sample (or may be referred to as a "free starting sample") of the compound represented by formula I.

¹H NMR (400 MHz, CDCl₃) *δ* 8.92 (s, 1H), 8.12 (s, 1H), 7.56 (d, *J* = 8.4 Hz, 1H), 7.10 (s, 1H), 6.91-6.94 (m, 1H), 6.77 (s, 1H), 6.65 (s, 1H), 4.13-4.16 (m, 2H), 4.05-4.08 (m, 4H), 3.50 (s, 1H), 3.31-3.34 (m, 2H), 2.80-2.83 (m, 4H), 2.40 (s, 3H), 1.98-2.07 (m, 4H), 1.41-1.45 (m, 4H), 0.30 (s, 4H). LC-MS, M/Z (ESI): 588.3 [M+H]⁺.

### Example 2. Starting Sample 1 of Compound of Formula I (Free Crystal Form A)

The starting sample 1 of the compound of formula I prepared in Example 1 was identified and separately subjected to XRPD, TGA/DSC, and ¹H NMR characterization. The XRPD results (FIG. 1) show that the starting sample was a crystal form sample, which was designated as free crystal form A. The TGA/DSC results are shown in FIG. 2, and the TGA results show a weight loss of 0.68% when the sample was heated to 150 °C. The DSC results show that the sample had 2 endothermic peaks at 167.9 °C and 180.3 °C (peak temperatures) and 1 exothermic peak at 170.5 °C (peak temperature). ¹H NMR was tested with DMSO-*d6* as the solvent, and the specific results are shown in FIG. 3a. The XRPD analysis data of the free crystal form A of the compound of formula I obtained are shown in Table B1-1 below. The endothermic and exothermic signals of the sample of the free crystal form A before the melting signal at 180.3 °C on the DSC were investigated through a heating experiment. The results (FIG. ) show that when the sample of the free crystal form A was heated to 172 °C and then cooled to room temperature and room humidity, the sample was observed to convert to the free crystal form F. According to TGA/DSC and ¹H NMR data of the sample of the free crystal form A, the free crystal form A was presumed to be an anhydrous crystal form.

**Table B1-1. XRPD diffraction peak data of free crystal form A**

| Pos. [°2θ] | Height [cts] | FWHM Left [°2θ] | d-spacing [Å] | Rel. Int. [%] |
|---|---|---|---|---|
| 3.8074 | 1636.04 | 0.0768 | 23.21 | 25.59 |
| 9.8108 | 253.37 | 0.0768 | 9.02 | 3.96 |
| 10.3581 | 209.57 | 0.1535 | 8.54 | 3.28 |
| 11.3487 | 1943.66 | 0.0768 | 7.80 | 30.40 |
| 12.7984 | 172.95 | 0.1535 | 6.92 | 2.71 |
| 13.4688 | 308.62 | 0.1279 | 6.57 | 4.83 |
| 15.1458 | 621.11 | 0.0768 | 5.85 | 9.72 |
| 15.4196 | 415.53 | 0.1023 | 5.75 | 6.50 |
| 16.2456 | 365.10 | 0.1023 | 5.46 | 5.71 |
| 16.8635 | 254.27 | 0.1535 | 5.26 | 3.98 |
| 17.3003 | 295.83 | 0.1023 | 5.13 | 4.63 |
| 18.9612 | 6392.76 | 0.1279 | 4.68 | 100.00 |
| 20.7248 | 180.22 | 0.4093 | 4.29 | 2.82 |
| 22.6628 | 202.18 | 0.2558 | 3.92 | 3.16 |
| 23.6322 | 179.82 | 0.1535 | 3.76 | 2.81 |
| 24.5787 | 125.08 | 0.1535 | 3.62 | 1.96 |
| 25.7207 | 58.88 | 0.3070 | 3.46 | 0.92 |
| 26.6666 | 144.68 | 0.1535 | 3.34 | 2.26 |
| 27.4293 | 144.69 | 0.1535 | 3.25 | 2.26 |
| 28.2276 | 147.11 | 0.1535 | 3.16 | 2.30 |
| 30.5503 | 277.15 | 0.1791 | 2.93 | 4.34 |
| 35.7206 | 109.08 | 0.2047 | 2.51 | 1.71 |

### Example 3. Free Crystal Form B/C of Compound of Formula I

The free crystal form B and free crystal form C were obtained by separately slurrying the starting sample 1 of the compound of formula I in ethanol and MTBE at room temperature for about 3 days, followed by centrifuging to separate the solid, and then drying under vacuum at 50 °C. The XRPD overlay of the free sample is shown in FIG. 4. The XRPD results for the free crystal form B are shown in FIG. 5. The TGA/DSC results are listed in FIG. 6. The TGA results show a weight loss of 4.35% when the sample was heated to 120 °C; the DSC results show that the sample had 3 endothermic peaks at 108.5 °C, 165.8 °C, and 180.2 °C (peak temperatures) and 1 exothermic peak at 167.7 °C (peak temperature). The ¹H NMR results, as shown in FIG. 7a, show that the residual solvent EtOH and the free crystal form in the sample were in a molar ratio of 0.45 (about 3.4 wt%). The XRPD analysis data of the free crystal form B are shown in Table B1-2 below. The heating experimental results show that when the sample of the free crystal form B was heated to 60 °C and then cooled to room temperature and room humidity, the main component of the sample was still found to be the free crystal form B, but a multimodal pattern was observed; when the sample of the free crystal form B was heated to 120 °C and then cooled to room temperature and room humidity, the sample was observed to convert to free crystal form A+F. According to the characterization and ¹H NMR results of the samples after heating (FIG. and FIG. 7c), EtOH and the free crystal form in the sample heated at 60 °C were in a molar ratio of 0.10 (about 0.8 wt%); no EtOH residual was observed in the sample heated at 120 °C, and the free crystal form B was presumed to be an EtOH solvate or a co-solvate of EtOH and water.

**Table B 1-2. XRPD diffraction peak data of free crystal form B**

| Pos. [°2θ] | Height [cts] | FWHM Left [°2θ] | d-spacing [Å] | Rel. Int. [%] |
|---|---|---|---|---|
| 9.2508 | 270.43 | 0.1023 | 9.56 | 10.95 |
| 9.7812 | 2469.11 | 0.1279 | 9.04 | 100.00 |
| 9.9884 | 884.91 | 0.1023 | 8.86 | 35.84 |
| 10.8644 | 204.70 | 0.1023 | 8.14 | 8.29 |
| 13.3822 | 215.58 | 0.3070 | 6.62 | 8.73 |
| 14.5151 | 149.83 | 0.1535 | 6.10 | 6.07 |
| 15.0024 | 256.39 | 0.1023 | 5.91 | 10.38 |
| 15.2215 | 325.39 | 0.1023 | 5.82 | 13.18 |
| 16.3081 | 214.49 | 0.1023 | 5.44 | 8.69 |
| 16.7223 | 164.61 | 0.1535 | 5.30 | 6.67 |
| 17.1474 | 134.52 | 0.1535 | 5.17 | 5.45 |
| 18.1668 | 1494.35 | 0.1279 | 4.88 | 60.52 |
| 18.5510 | 1568.32 | 0.1791 | 4.78 | 63.52 |
| 19.0431 | 1773.96 | 0.1023 | 4.66 | 71.85 |
| 19.3191 | 978.13 | 0.1279 | 4.59 | 39.61 |
| 19.7143 | 1184.83 | 0.1023 | 4.50 | 47.99 |
| 20.2387 | 1688.10 | 0.1535 | 4.39 | 68.37 |
| 21.1776 | 1330.67 | 0.1023 | 4.20 | 53.89 |
| 22.0900 | 453.95 | 0.3070 | 4.02 | 18.39 |
| 23.0450 | 128.30 | 0.3582 | 3.86 | 5.20 |
| 23.7619 | 231.50 | 0.4093 | 3.74 | 9.38 |
| 25.7395 | 53.14 | 0.6140 | 3.46 | 2.15 |
| 27.6189 | 535.67 | 0.1791 | 3.23 | 21.69 |
| 28.2642 | 724.65 | 0.3070 | 3.16 | 29.35 |
| 30.0557 | 140.47 | 0.4093 | 2.97 | 5.69 |

The XRPD results for the free crystal form C are shown in FIG. 8. The TGA/DSC results for the free crystal form C are listed in FIG. 9. The TGA results show a weight loss of 2.77% when the sample was heated to 150 °C. The DSC results show that the sample had 2 endothermic peaks at 53.0 °C and 182.3 °C (peak temperatures). The 1H NMR results, as shown in FIG. 10, show that no MTBE solvent residue was found in the sample. The XRPD analysis data of the free crystal form C are shown in Table B1-3 below:

**Table B 1-3. XRPD diffraction peak data of free crystal form C**

| Pos. [°2θ] | Height [cts] | FWHM Left [°2θ] | d-spacing [Å] | Rel. Int. [%] |
|---|---|---|---|---|
| 3.1461 | 110.81 | 0.3070 | 28.08 | 3.42 |
| 8.1061 | 228.54 | 0.0768 | 10.91 | 7.05 |
| 9.1126 | 269.53 | 0.1023 | 9.70 | 8.32 |
| 9.5020 | 1823.52 | 0.1023 | 9.31 | 56.28 |
| 10.7612 | 1180.63 | 0.1023 | 8.22 | 36.44 |
| 11.2726 | 168.48 | 0.1279 | 7.85 | 5.20 |
| 12.2849 | 453.67 | 0.1023 | 7.20 | 14.00 |
| 13.1097 | 507.75 | 0.1023 | 6.75 | 15.67 |
| 14.2058 | 246.84 | 0.1279 | 6.23 | 7.62 |
| 14.7763 | 177.78 | 0.0768 | 6.00 | 5.49 |
| 15.3140 | 1334.07 | 0.1279 | 5.79 | 41.18 |
| 16.1724 | 2744.11 | 0.1023 | 5.48 | 84.70 |
| 16.5974 | 3239.82 | 0.1023 | 5.34 | 100.00 |
| 17.5352 | 323.99 | 0.1023 | 5.06 | 10.00 |
| 18.5313 | 1175.34 | 0.1279 | 4.79 | 36.28 |
| 18.8700 | 1523.11 | 0.1023 | 4.70 | 47.01 |
| 19.1799 | 867.42 | 0.1023 | 4.63 | 26.77 |
| 19.9398 | 1361.89 | 0.1023 | 4.45 | 42.04 |
| 20.5180 | 1586.78 | 0.1279 | 4.33 | 48.98 |
| 21.0065 | 1588.11 | 0.1023 | 4.23 | 49.02 |
| 21.2198 | 821.86 | 0.1023 | 4.19 | 25.37 |
| 21.5887 | 2440.01 | 0.1023 | 4.12 | 75.31 |
| 22.1453 | 370.61 | 0.1023 | 4.01 | 11.44 |
| 22.7421 | 186.48 | 0.1023 | 3.91 | 5.76 |
| 23.2908 | 295.33 | 0.1279 | 3.82 | 9.12 |
| 24.1476 | 365.15 | 0.1023 | 3.69 | 11.27 |
| 24.6794 | 186.67 | 0.1023 | 3.61 | 5.76 |
| 25.4796 | 254.65 | 0.1023 | 3.50 | 7.86 |
| 25.8930 | 1697.75 | 0.1535 | 3.44 | 52.40 |
| 27.4465 | 124.47 | 0.1535 | 3.25 | 3.84 |
| 28.4921 | 656.64 | 0.1279 | 3.13 | 20.27 |
| 29.8280 | 133.05 | 0.1535 | 3.00 | 4.11 |
| 30.7213 | 117.76 | 0.3582 | 2.91 | 3.63 |
| 31.4381 | 63.26 | 0.1535 | 2.85 | 1.95 |
| 32.6390 | 147.47 | 0.2047 | 2.74 | 4.55 |
| 35.3421 | 104.75 | 0.2047 | 2.54 | 3.23 |
| 36.5656 | 73.78 | 0.3070 | 2.46 | 2.28 |
| 38.4547 | 67.62 | 0.4093 | 2.34 | 2.09 |

### Example 4. Starting Sample 2 of Compound of Formula I

### 4.1. Preparation of starting sample 2 of compound of formula I

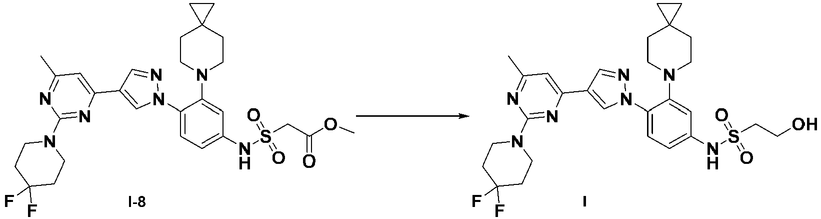

Compound I-8 (2.3 g, 3.74 mmol), 23 mL of THF, and 23 mL of absolute ethanol were added to a single-neck flask. The mixture was stirred until clear, and then anhydrous calcium chloride (1.03 g, 9.33 mmol) was added. The resulting mixture was stirred until completely dissolved. The mixture was cooled to 0 °C, and sodium borohydride (0.71 g, 18.68 mmol) was added. After the addition was completed, the reaction was performed at room temperature overnight. LCMS showed that the starting materials were completely reacted. The reaction liquid was poured into 46 mL of water, and 92 mL of ethyl acetate was added. The mixture was stirred for 5 min and then allowed to stand for phase separation. The supernatant was poured out, and 92 mL of ethyl acetate was added to wash the bottom solid. The organic phase was concentrated, mixed with silica gel, and subjected to column chromatography (PE:EA = 4:1-1:1-0:1) to obtain the target product. 30 mL of acetonitrile was added to dissolve the target product completely, and the mixture was concentrated until no liquid drops fell. 100 mL of water was added, and the mixture was heated to 100 °C for slurrying for 5 h and filtered. The filter cake was washed, and then 100 mL of water was added again. The mixture was heated to 100 °C for slurrying for 5 h, subjected to slurrying at room temperature overnight, and filtered. The solid was lyophilized to obtain starting sample 2 of the compound of formula I (1.8037 g).

### 4.2. Characterization of starting sample 2 of compound of formula I

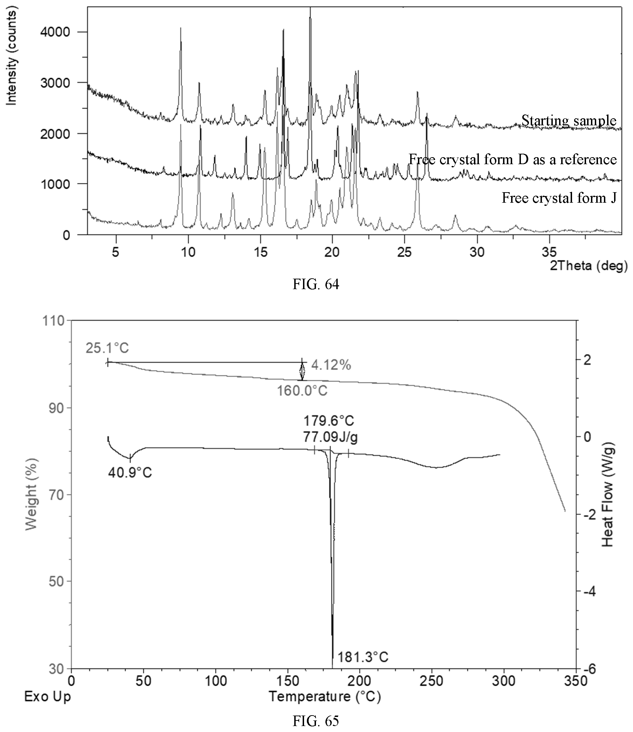

The XRPD and TGA/DSC characterization results for the starting sample 2 prepared in 4.1 described above are shown in FIGs. 64 and 65. The XRPD results show that the starting sample 2 was a crystal form sample, which was a mixed crystal of the free crystal form D and the free crystal form J. The TGA results show a weight loss of 4.12% when the sample was heated to 160 °C. The DSC results show that the sample had 2 endothermic peaks at 40.9 °C and 181.3 °C (peak temperatures). ¹H NMR was tested with DMSO-*d₆* as the solvent, and the specific results are shown in FIG. 66.

### Example 5. Free Crystal Form D of Compound of Formula I

The sample of the free crystal form D was obtained by slurrying the free starting sample 2 in IPA/H₂O (volume ratio of 982:18, a_{w} of about 0.2) at room temperature for about 5 days, centrifuging to separate the solid, and air-drying in an open container at room temperature. The XRPD and TGA/DSC results are shown in FIGs. 67 and 68, respectively. The TGA results show a weight loss of 5.79% when the sample was heated to 120 °C. The DSC results show that the sample had 2 endothermic peaks at 53.9 °C and 182.9 °C (peak temperatures). The ¹H NMR results (FIG. 69) show that IPA and the free crystal form in the sample were in a molar ratio of 0.01 (about 0.1 wt%). The free crystal form D was subjected to crystal form identification by VT-XRPD. The results (FIG. 70) show that: when the sample of the free crystal form D was purged under N₂ conditions for 20 min, heated to 120 °C under N₂ atmosphere, cooled to 30 °C, or exposed to room temperature and room humidity, the conversion to the free crystal form J was observed in all cases. According to the VT-XRPD and characterization results, the free crystal form D was presumed to be a hydrate, and after being purged with N₂ or heated, the free crystal form D was dehydrated and converted to the anhydrous crystal form J. The XRPD analysis data of the free crystal form D are shown in Table B1-4 below:

**Table B1-4. XRPD diffraction peak data of free crystal form D**

| **Pos. [°2θ]** | **Height [cts]** | **FWHM Left [°2θ]** | **d-spacing [Å]** | **Rel. Int. [%]** |
|---|---|---|---|---|
| 8.2985 | 129.26 | 0.1535 | 10.65 | 3.75 |
| 9.4241 | 132.13 | 0.1535 | 9.38 | 3.83 |
| 10.8461 | 1026.26 | 0.1023 | 8.16 | 29.75 |
| 11.8218 | 425.80 | 0.1023 | 7.49 | 12.35 |
| 12.5232 | 83.51 | 0.1535 | 7.07 | 2.42 |
| 13.2208 | 187.41 | 0.0768 | 6.70 | 5.43 |
| 13.9909 | 789.00 | 0.1023 | 6.33 | 22.88 |
| 14.9577 | 672.34 | 0.1023 | 5.92 | 19.49 |
| 16.5793 | 2551.91 | 0.1023 | 5.35 | 73.99 |
| 16.8857 | 963.53 | 0.0768 | 5.25 | 27.94 |
| 18.4363 | 3449.17 | 0.1023 | 4.81 | 100.00 |
| 18.7513 | 290.69 | 0.0768 | 4.73 | 8.43 |
| 18.9436 | 395.96 | 0.1023 | 4.68 | 11.48 |
| 20.1677 | 555.04 | 0.0768 | 4.40 | 16.09 |
| 20.3590 | 1025.48 | 0.0768 | 4.36 | 29.73 |
| 21.3940 | 1030.01 | 0.1279 | 4.15 | 29.86 |
| 21.7745 | 2050.47 | 0.1279 | 4.08 | 59.45 |
| 22.2999 | 202.84 | 0.2047 | 3.99 | 5.88 |
| 23.7694 | 244.81 | 0.1023 | 3.74 | 7.10 |
| 24.2605 | 319.07 | 0.0768 | 3.67 | 9.25 |
| 24.4938 | 309.75 | 0.0768 | 3.63 | 8.98 |
| 25.2628 | 319.51 | 0.1023 | 3.53 | 9.26 |
| 26.5122 | 1317.42 | 0.1023 | 3.36 | 38.20 |
| 29.2296 | 142.60 | 0.5117 | 3.06 | 4.13 |
| 29.7635 | 102.54 | 0.1535 | 3.00 | 2.97 |
| 30.8099 | 168.69 | 0.1023 | 2.90 | 4.89 |
| 38.8753 | 105.29 | 0.1535 | 2.32 | 3.05 |

### Example 6. Free Crystal Form E of Compound of Formula I

The sample of the free crystal form E was obtained by placing the free starting sample 2 under THF atmosphere for gas-solid diffusion, and air-drying in an open container at room temperature. The XRPD and TGA/DSC results are listed in FIGs. 71 and 72, respectively. The TGA results show a weight loss of 11.97% when heated to 140 °C. The DSC results show that the sample had 2 endothermic peaks at 123.0 °C and 182.9 °C (peak temperatures) and 1 exothermic peak at 148.0 °C (peak temperature). The ¹H NMR results, as shown in FIG. 73, show that THF and the free crystal form in the sample were in a molar ratio of 0.95 (about 10.4 wt%). The free crystal form E sample was subjected to crystal form identification through a heating experiment. The heating experiment results are shown in FIG. 74: when the sample of the free crystal form E was heated to 130 °C/160 °C and then cooled to room temperature and room humidity, the sample was observed to convert to the free crystal form J in both cases. According to the characterization and ¹H NMR results (FIG. 75) of the samples after the heating experiment, no solvent residue was observed in the sample of the free crystal form E after being heated to 160 °C, and the free crystal form E was presumed to be a THF solvate. The XRPD analysis data of the free crystal form E are shown in Table B1-5 below:

**Table B 1-5. XRPD diffraction peak data of free crystal form E**

| **Pos. [°2θ]** | **Height [cts]** | **FWHM Left [°2θ]** | **d-spacing [Å]** | **Rel. Int. [%]** |
|---|---|---|---|---|
| 8.6847 | 231.32 | 0.0768 | 10.18 | 9.31 |
| 8.9272 | 273.81 | 0.0768 | 9.91 | 11.03 |
| 9.1459 | 435.17 | 0.0768 | 9.67 | 17.52 |
| 9.4934 | 135.54 | 0.0768 | 9.32 | 5.46 |
| 12.1780 | 440.12 | 0.0768 | 7.27 | 17.72 |
| 12.7789 | 270.29 | 0.0768 | 6.93 | 10.88 |
| 15.3545 | 83.98 | 0.1535 | 5.77 | 3.38 |
| 16.7268 | 465.80 | 0.1535 | 5.30 | 18.76 |
| 17.2384 | 2483.30 | 0.1023 | 5.14 | 100.00 |
| 18.1543 | 286.43 | 0.0768 | 4.89 | 11.53 |
| 18.3327 | 586.99 | 0.0768 | 4.84 | 23.64 |
| 18.9674 | 344.13 | 0.0768 | 4.68 | 13.86 |
| 19.1326 | 604.89 | 0.1023 | 4.64 | 24.36 |
| 19.8795 | 216.65 | 0.1023 | 4.47 | 8.72 |
| 20.2931 | 296.13 | 0.0768 | 4.38 | 11.92 |
| 20.5505 | 600.01 | 0.1279 | 4.32 | 24.16 |
| 21.4769 | 1194.85 | 0.1023 | 4.14 | 48.12 |
| 21.8496 | 489.65 | 0.1023 | 4.07 | 19.72 |
| 22.1726 | 149.18 | 0.1023 | 4.01 | 6.01 |
| 23.0433 | 174.18 | 0.1023 | 3.86 | 7.01 |
| 24.8471 | 674.29 | 0.1023 | 3.58 | 27.15 |
| 25.6946 | 181.07 | 0.0768 | 3.47 | 7.29 |
| 26.8264 | 107.60 | 0.2047 | 3.32 | 4.33 |
| 28.5466 | 200.84 | 0.2047 | 3.13 | 8.09 |
| 29.5968 | 66.00 | 0.4093 | 3.02 | 2.66 |

### Example 7. Free Crystal Form F of Compound of Formula I

The sample of the free crystal form F was obtained by heating the free starting sample 2 to 172 °C and cooling to room temperature and room humidity. The XRPD and TGA/DSC results are listed in FIGs. 76 and 77, respectively. The TGA results show a weight loss of 1.19% when the sample was heated to 150 °C. The DSC results show that the sample had **1** endothermic peak at 181.1 °C (peak temperature). The ¹H NMR results, as shown in FIG. 78, indicate that no solvent residue was observed in the sample. According to the preparation method and characterization results for the free crystal form F, the free crystal form F was presumed to be an anhydrous crystal form. The XRPD analysis data of the free crystal form F are shown in Table B1-6 below:

**Table B1-6. XRPD diffraction peak data of free crystal form F**

| **Pos. [°2θ]** | **Height [cts]** | **FWHM Left [°2θ]** | **d-spacing [Å]** | **Rel. Int. [%]** |
|---|---|---|---|---|
| 7.2680 | 237.24 | 0.0768 | 12.16 | 5.32 |
| 9.7443 | 533.04 | 0.1023 | 9.08 | 11.95 |
| 10.9091 | 1531.48 | 0.0768 | 8.11 | 34.34 |
| 12.6503 | 152.73 | 0.1023 | 7.00 | 3.42 |
| 13.5597 | 284.93 | 0.1023 | 6.53 | 6.39 |
| 14.5589 | 732.22 | 0.1023 | 6.08 | 16.42 |
| 15.0095 | 232.32 | 0.1023 | 5.90 | 5.21 |
| 15.7854 | 1001.58 | 0.1023 | 5.61 | 22.46 |
| 17.1471 | 572.21 | 0.1279 | 5.17 | 12.83 |
| 17.7727 | 454.98 | 0.0768 | 4.99 | 10.20 |
| 18.2249 | 4460.10 | 0.1023 | 4.87 | 100.00 |
| 18.5345 | 870.97 | 0.1023 | 4.79 | 19.53 |
| 19.4299 | 177.57 | 0.1535 | 4.57 | 3.98 |
| 20.2138 | 489.58 | 0.1279 | 4.39 | 10.98 |
| 21.2140 | 191.59 | 0.1279 | 4.19 | 4.30 |
| 21.5620 | 94.04 | 0.1535 | 4.12 | 2.11 |
| 22.3451 | 197.25 | 0.1279 | 3.98 | 4.42 |
| 23.2974 | 201.26 | 0.1023 | 3.82 | 4.51 |
| 23.7177 | 111.39 | 0.1023 | 3.75 | 2.50 |
| 24.7687 | 284.71 | 0.1279 | 3.59 | 6.38 |
| 25.6111 | 219.63 | 0.1023 | 3.48 | 4.92 |
| 27.5720 | 133.74 | 0.1535 | 3.24 | 3.00 |
| 28.4674 | 73.03 | 0.1535 | 3.14 | 1.64 |
| 29.3309 | 59.99 | 0.2047 | 3.05 | 1.35 |
| 30.7039 | 67.96 | 0.1535 | 2.91 | 1.52 |
| 31.5840 | 172.91 | 0.1535 | 2.83 | 3.88 |
| 34.3936 | 65.71 | 0.1535 | 2.61 | 1.47 |
| 35.1042 | 33.89 | 0.4093 | 2.56 | 0.76 |
| 36.9389 | 58.66 | 0.2047 | 2.43 | 1.32 |
| 38.7357 | 115.27 | 0.1279 | 2.32 | 2.58 |

### Example 8. Free Crystal Form G of Compound of Formula I

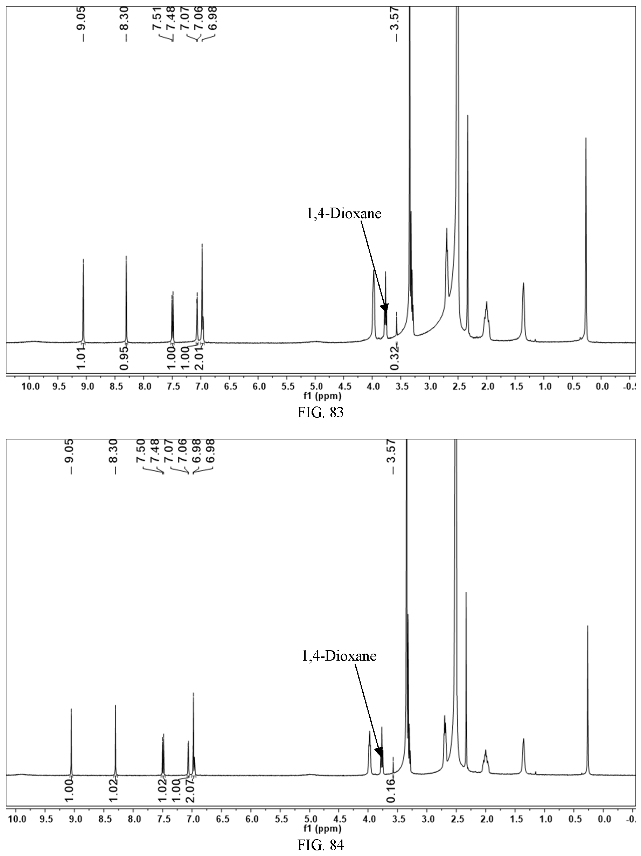

The sample of the free crystal form G was obtained by first dissolving the free starting sample 2 in 1,4-dioxane under H₂O atmosphere for gas-liquid diffusion to precipitate a solid, and drying in an open container in air. The XRPD and TGA/DSC results are listed in FIGs. 79 and 80, respectively. The TGA results show that the sample had a weight loss of 10.40% when heated to 110 °C, and the DSC results show that the sample had 3 endothermic peaks at 98.1 °C, 154.8 °C, and 180.8 °C (peak temperatures) and **1** exothermic peak at 157.8 °C (peak temperature). The ¹H NMR results, as shown in FIG. 81, show that 1,4-dioxane and the free crystal form in the sample were in a molar ratio of 0.72 (about 9.7 wt%). The free crystal form G was subjected to crystal form identification through a heating experiment. The results (FIG. 82) show that when the sample of the free crystal form G was heated to 120 °C and then cooled to room temperature and room humidity, the sample was observed to convert to the free crystal form F + multimodal; when the sample of the free crystal form G was heated to 160 °C and then cooled to room temperature and room humidity, the sample was observed to convert to the free crystal form F. The ¹H NMR results of the samples after heating (FIGs. 83 and 84) show that the molar ratios of the residual solvent 1,4-dioxane to the free crystal form in the samples of the free crystal form G after being heated to 120 °C and 160 °C were 0.04 (about 0.6 wt%) and 0.02 (about 0.3 wt%), respectively. According to the characterization and heating experiment results, the free crystal form G was presumed to be a 1,4-dioxane solvate. The XRPD analysis data of the free crystal form G are shown in Table B1-7 below:

**Table B1-7. XRPD diffraction peak data of free crystal form G**

| **Pos. [°2θ]** | **Height [cts]** | **FWHM Left [°2θ]** | **d-spacing [Å]** | **Rel. Int. [%]** |
|---|---|---|---|---|
| 6.1695 | 1781.96 | 0.0768 | 14.33 | 40.63 |
| 9.2517 | 896.59 | 0.0768 | 9.56 | 20.44 |
| 9.4965 | 1094.20 | 0.0768 | 9.31 | 24.95 |
| 10.2696 | 293.65 | 0.0768 | 8.61 | 6.70 |
| 10.5406 | 1600.25 | 0.0768 | 8.39 | 36.49 |
| 10.9989 | 303.71 | 0.2047 | 8.04 | 6.93 |
| 12.2893 | 2843.93 | 0.1023 | 7.20 | 64.85 |
| 14.5010 | 1394.58 | 0.1023 | 6.11 | 31.80 |
| 16.5003 | 264.97 | 0.0768 | 5.37 | 6.04 |
| 16.9986 | 750.64 | 0.1023 | 5.22 | 17.12 |
| 18.5441 | 4385.59 | 0.0768 | 4.78 | 100.00 |
| 18.8489 | 1081.83 | 0.0768 | 4.71 | 24.67 |
| 19.4906 | 2359.33 | 0.1023 | 4.55 | 53.80 |
| 20.6318 | 220.60 | 0.3582 | 4.31 | 5.03 |
| 21.1520 | 369.92 | 0.1023 | 4.20 | 8.43 |
| 21.6669 | 569.76 | 0.0768 | 4.10 | 12.99 |
| 22.4742 | 323.83 | 0.0768 | 3.96 | 7.38 |
| 22.7762 | 1882.30 | 0.1279 | 3.90 | 42.92 |
| 24.7980 | 420.59 | 0.2047 | 3.59 | 9.59 |
| 25.3519 | 275.52 | 0.1023 | 3.51 | 6.28 |
| 26.0012 | 60.35 | 0.3070 | 3.43 | 1.38 |
| 26.8697 | 112.11 | 0.1023 | 3.32 | 2.56 |
| 28.1782 | 256.46 | 0.1535 | 3.17 | 5.85 |

### Example 9. Free Crystal Form H of Compound of Formula I

The sample of the free crystal form H was obtained by placing the free starting sample 2 under DMSO atmosphere for gas-solid diffusion. After drying in air, the sample was observed to convert to the free crystal form D + multimodal. The XRPD overlay is shown in FIG. 85a. The XRPD of the free crystal form H is shown in FIG. 85b, and the analysis data are shown in Table B 1-8 below:

**Table B 1-8. XRPD diffraction peak data of free crystal form H**

| **Pos. [°2θ]** | **Height [cts]** | **FWHM Left [°2θ]** | **d-spacing [Å]** | **Rel. Int. [%]** |
|---|---|---|---|---|
| 5.1382 | 921.20 | 0.0768 | 17.20 | 74.39 |
| 8.6958 | 114.67 | 0.4093 | 10.17 | 9.26 |
| 10.2939 | 159.01 | 0.2047 | 8.59 | 12.84 |
| 11.0299 | 148.74 | 0.1535 | 8.02 | 12.01 |
| 11.5587 | 181.61 | 0.0768 | 7.66 | 14.67 |
| 12.1517 | 228.36 | 0.0768 | 7.28 | 18.44 |
| 13.0908 | 239.38 | 0.0768 | 6.76 | 19.33 |
| 14.5022 | 295.81 | 0.1023 | 6.11 | 23.89 |
| 14.9595 | 425.26 | 0.1023 | 5.92 | 34.34 |
| 15.2308 | 186.70 | 0.1279 | 5.82 | 15.08 |
| 16.5672 | 238.18 | 0.1535 | 5.35 | 19.23 |
| 17.0866 | 986.37 | 0.2047 | 5.19 | 79.65 |
| 17.5191 | 409.45 | 0.1279 | 5.06 | 33.06 |
| 17.7454 | 454.21 | 0.0768 | 5.00 | 36.68 |
| 18.4679 | 262.26 | 0.1023 | 4.80 | 21.18 |
| 18.9850 | 467.05 | 0.1279 | 4.67 | 37.72 |
| 19.4851 | 1238.36 | 0.1023 | 4.56 | 100.00 |
| 20.1891 | 697.07 | 0.1023 | 4.40 | 56.29 |
| 20.5085 | 318.05 | 0.2047 | 4.33 | 25.68 |
| 21.3352 | 557.62 | 0.1791 | 4.16 | 45.03 |
| 21.5416 | 389.92 | 0.0768 | 4.13 | 31.49 |
| 22.2233 | 241.72 | 0.3070 | 4.00 | 19.52 |
| 22.7317 | 122.84 | 0.2047 | 3.91 | 9.92 |
| 24.2236 | 206.75 | 0.1535 | 3.67 | 16.70 |
| 24.5735 | 324.30 | 0.0768 | 3.62 | 26.19 |
| 24.7905 | 406.97 | 0.1023 | 3.59 | 32.86 |
| 25.0488 | 248.59 | 0.1279 | 3.56 | 20.07 |
| 26.0331 | 156.59 | 0.3070 | 3.42 | 12.65 |
| 28.5683 | 168.99 | 0.2047 | 3.12 | 13.65 |

### Example 10. Free Crystal Form I of Compound of Formula I

The sample of the free crystal form I was obtained by slurrying the free starting sample 2 in MeOH at room temperature for about 5 days and then air-drying in an open container at room temperature. The XRPD and TGA/DSC results are listed in FIGs. 86 and 87, respectively. The TGA results show that the sample had a weight loss of 4.00% when heated to 120 °C, and the DSC results show that the sample had 3 endothermic peaks at 54.6 °C, 168.0 °C, and 181.2 °C (peak temperatures) and **1** exothermic peak at 170.5 °C (peak temperature). The ¹H NMR results (FIG. 88) show that no MeOH solvent residue was observed in the sample. The free crystal form I was subjected to crystal form identification through a heating experiment. The heating experiment results (FIG. 89) show that when the sample of the free crystal form I was heated to 100 °C and then cooled to room temperature and room humidity, the sample was observed to convert to the free crystal form A; when the sample of the free crystal form I was heated to 172 °C and then cooled to room temperature and room humidity, the sample was observed to convert to the free crystal form F. According to the characterization and heating experiment results, the free crystal form I was presumed to be a hydrate. The XRPD analysis data of the free crystal form I are shown in Table B1-9 below:

**Table B1-9. XRPD diffraction peak data of free crystal form I**

| **Pos. [°2θ]** | **Height [cts]** | **FWHM Left [°2θ]** | **d-spacing [Å]** | **Rel. Int. [%]** |
|---|---|---|---|---|
| 9.5592 | 1552.00 | 0.1023 | 9.25 | 84.98 |
| 12.8988 | 574.88 | 0.1023 | 6.86 | 31.48 |
| 14.7994 | 136.74 | 0.1535 | 5.99 | 7.49 |
| 15.8059 | 139.46 | 0.1279 | 5.61 | 7.64 |
| 17.8352 | 1826.24 | 0.1023 | 4.97 | 100.00 |
| 18.1790 | 451.34 | 0.1023 | 4.88 | 24.71 |
| 19.3336 | 1054.29 | 0.1279 | 4.59 | 57.73 |
| 19.8678 | 826.43 | 0.1023 | 4.47 | 45.25 |
| 20.1554 | 615.97 | 0.1279 | 4.41 | 33.73 |
| 20.7214 | 311.13 | 0.1535 | 4.29 | 17.04 |
| 20.9976 | 492.99 | 0.1023 | 4.23 | 26.99 |
| 23.2310 | 169.11 | 0.1535 | 3.83 | 9.26 |
| 27.1065 | 1105.51 | 0.1535 | 3.29 | 60.53 |
| 28.0012 | 408.97 | 0.1791 | 3.19 | 22.39 |

### Example 11. Free Crystal Form J of Compound of Formula I

The sample of the free crystal form J was obtained by heating the free starting sample 2 to 100 °C and cooling to room temperature and room humidity. The XRPD and TGA/DSC results are listed in FIGs. 90 and 91, respectively. The TGA results show that the sample had a weight loss of 1.09% when heated to 150 °C, and the DSC results show that the sample had **1** endothermic peak at 181.5 °C (peak temperature). The ¹H NMR results (FIG. 92) show that no solvent residue was observed in the sample. The variable-humidity XRPD (VH-XRPD) results for the sample of the free crystal form J (FIG. 93) show that no crystal form change was observed when the sample of the free crystal form J was placed at 30% RH, 10% RH, 20% RH, and 40% RH; when the humidity was increased to 60% RH, the sample was observed to convert to a mixed crystal of the crystal forms J and D; when the humidity was continued to be increased to 80% RH and 90% RH, the sample was observed to convert to the crystal form D; when the humidity was decreased to 30% RH again, the sample was still found to be the crystal form D. According to the preparation method and characterization data of the free crystal form J and the variable-temperature XRPD results of the crystal form D, the free crystal form J was presumed to be an anhydrous crystal form and tend to convert to the hydrate crystal form D under humidity conditions of > 40% RH. The XRPD analysis data of the free crystal form J are shown in Table B1-10 below:

**Table B1-10. XRPD diffraction peak data of free crystal form J**

| **Pos. [°2θ]** | **Height [cts]** | **FWHM Left [°2θ]** | **d-spacing [Å]** | **Rel. Int. [%]** |
|---|---|---|---|---|
| 9.4887 | 1642.04 | 0.1023 | 9.32 | 61.97 |
| 10.7503 | 1482.01 | 0.1023 | 8.23 | 55.93 |
| 12.2600 | 314.68 | 0.1279 | 7.22 | 11.88 |
| 13.0965 | 660.53 | 0.1535 | 6.76 | 24.93 |
| 14.1913 | 267.46 | 0.1023 | 6.24 | 10.09 |
| 15.3049 | 1964.93 | 0.1535 | 5.79 | 74.16 |
| 16.1644 | 2649.68 | 0.1279 | 5.48 | 100.00 |
| 16.5855 | 2648.72 | 0.1279 | 5.35 | 99.96 |
| 17.5096 | 203.45 | 0.1535 | 5.07 | 7.68 |
| 18.5225 | 695.19 | 0.1279 | 4.79 | 26.24 |
| 18.8695 | 1222.54 | 0.1279 | 4.70 | 46.14 |
| 19.1552 | 553.36 | 0.1023 | 4.63 | 20.88 |
| 19.9313 | 715.68 | 0.1279 | 4.45 | 27.01 |
| 20.5033 | 937.95 | 0.1535 | 4.33 | 35.40 |
| 20.9755 | 2140.03 | 0.2047 | 4.24 | 80.77 |
| 21.5744 | 2434.96 | 0.1535 | 4.12 | 91.90 |
| 22.1570 | 285.20 | 0.1535 | 4.01 | 10.76 |
| 23.2764 | 273.77 | 0.1535 | 3.82 | 10.33 |
| 24.1115 | 170.45 | 0.1535 | 3.69 | 6.43 |
| 24.6829 | 110.29 | 0.1535 | 3.61 | 4.16 |
| 25.8914 | 1814.27 | 0.1535 | 3.44 | 68.47 |
| 27.1763 | 140.85 | 0.4093 | 3.28 | 5.32 |
| 28.4642 | 417.21 | 0.1279 | 3.14 | 15.75 |
| 29.7254 | 55.96 | 0.4093 | 3.01 | 2.11 |
| 30.7261 | 141.85 | 0.3070 | 2.91 | 5.35 |
| 32.6167 | 180.30 | 0.1535 | 2.75 | 6.80 |

### Example 12. Free Crystal Form K of Compound of Formula I

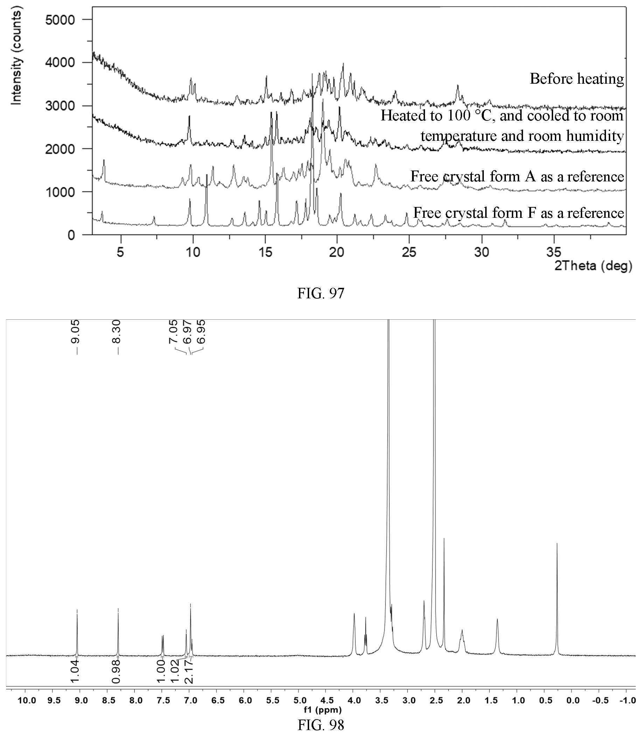

The sample of the free crystal form K was obtained by slurrying the free starting sample 2 in EtOH at room temperature for about 2 days, centrifuging to separate the solid, and then drying under vacuum at room temperature. The XRPD and TGA/DSC results are listed in FIGs. 94 and 95, respectively. The TGA results show that the sample had a weight loss of 8.24% when heated to 150 °C, and the DSC results show that the sample had 3 endothermic peaks at 72.0 °C, 165.2 °C, and 180.2 °C (peak temperatures) and **1** exothermic peak at 167.1 °C (peak temperature). The ¹H NMR results (FIG. 96) show that EtOH and the free crystal form in the sample were in a molar ratio of 0.27 (about 2.1 wt%). The free crystal form K was subjected to crystal form identification through a heating experiment. The results (FIG. 97) show that when the sample of the free crystal form K was heated to 100 °C and then cooled to room temperature and room humidity, the sample was observed to convert to the free crystal forms A + F. The ¹H NMR results (FIG. 98) of the sample of the free crystal form K after being heated to 100 °C show that no solvent residue was observed in the sample. According to the preparation conditions, characterization, and heating experiment results, the free crystal form K was presumed to be an EtOH solvate or a co-solvate of EtOH and water. The XRPD analysis data of the free crystal form K are shown in Table B1-11 below:

**Table B1-11. XRPD diffraction peak data of free crystal form K**

| **Pos. [°2θ]** | **Height [cps]** | **FWHM Left [°2θ]** | **d-spacing [Å]** | **Rel. Int. [%]** |
|---|---|---|---|---|
| 9.3256 | 52.90 | 0.1535 | 9.48 | 12.09 |
| 9.8469 | 263.49 | 0.1023 | 8.98 | 60.20 |
| 10.1208 | 202.11 | 0.1279 | 8.74 | 46.18 |
| 13.0145 | 83.72 | 0.1279 | 6.80 | 19.13 |
| 14.6920 | 104.21 | 0.1023 | 6.03 | 23.81 |
| 15.0722 | 324.48 | 0.0768 | 5.88 | 74.13 |
| 15.4155 | 126.22 | 0.1023 | 5.75 | 28.84 |
| 16.0745 | 116.24 | 0.1535 | 5.51 | 26.56 |
| 16.7829 | 171.66 | 0.1023 | 5.28 | 39.22 |
| 17.6356 | 145.14 | 0.1535 | 5.03 | 33.16 |
| 18.1386 | 150.91 | 0.1535 | 4.89 | 34.48 |
| 18.7484 | 363.39 | 0.1023 | 4.73 | 83.02 |
| 19.0653 | 364.37 | 0.0768 | 4.66 | 83.25 |
| 19.2235 | 288.28 | 0.2047 | 4.62 | 65.86 |
| 19.4283 | 298.52 | 0.1023 | 4.57 | 68.20 |
| 19.7672 | 310.76 | 0.0768 | 4.49 | 71.00 |
| 20.4112 | 437.69 | 0.1279 | 4.35 | 100.00 |
| 20.9312 | 366.26 | 0.1279 | 4.24 | 83.68 |
| 21.1881 | 281.40 | 0.1023 | 4.19 | 64.29 |
| 21.6963 | 206.87 | 0.2047 | 4.10 | 47.26 |
| 24.0591 | 170.46 | 0.1279 | 3.70 | 38.95 |
| 26.2506 | 60.01 | 0.2558 | 3.39 | 13.71 |
| 28.3413 | 240.92 | 0.1535 | 3.15 | 55.04 |
| 28.6573 | 134.70 | 0.0768 | 3.12 | 30.77 |
| 30.5394 | 89.15 | 0.1535 | 2.93 | 20.37 |
| 33.0658 | 21.37 | 0.6140 | 2.71 | 4.88 |

### Example 13. Free Crystal Form L of Compound of Formula I

The sample of the free crystal form L was obtained by slurrying the free starting sample 1 in MeOH at room temperature for about 3 days. The XRPD overlay is shown in FIG. 99a. The sample was converted to the free crystal form D after air drying. The XRPD of the free crystal form L is shown in FIG. 99b, and the analysis data are shown in Table B1-12 below:

**Table B1-12. XRPD diffraction peak data of free crystal form L**

| **Pos. [°2θ]** | **Height [cts]** | **FWHM Left [°2θ]** | **d-spacing [Å]** | **Rel. Int. [%]** |
|---|---|---|---|---|
| 8.8304 | 1059.06 | 0.0768 | 10.01 | 46.94 |
| 9.6805 | 176.85 | 0.1535 | 9.14 | 7.84 |
| 10.2446 | 435.82 | 0.0768 | 8.63 | 19.32 |
| 10.7612 | 433.78 | 0.1023 | 8.22 | 19.23 |
| 11.1727 | 567.96 | 0.1535 | 7.92 | 25.18 |
| 14.5973 | 449.14 | 0.0768 | 6.07 | 19.91 |
| 15.5971 | 299.58 | 0.1023 | 5.68 | 13.28 |
| 15.8341 | 911.54 | 0.1023 | 5.60 | 40.40 |
| 16.8550 | 542.52 | 0.0768 | 5.26 | 24.05 |
| 17.1190 | 475.22 | 0.0768 | 5.18 | 21.06 |
| 17.7145 | 882.52 | 0.1023 | 5.01 | 39.12 |
| 18.2884 | 117.25 | 0.1535 | 4.85 | 5.20 |
| 19.1795 | 1931.84 | 0.1023 | 4.63 | 85.63 |
| 19.7076 | 482.26 | 0.1023 | 4.50 | 21.38 |
| 20.0943 | 716.69 | 0.1023 | 4.42 | 31.77 |
| 20.5748 | 1492.42 | 0.1535 | 4.32 | 66.15 |
| 22.4409 | 668.91 | 0.1023 | 3.96 | 29.65 |
| 25.5115 | 2256.01 | 0.1535 | 3.49 | 100.00 |
| 28.7935 | 107.33 | 0.3070 | 3.10 | 4.76 |
| 29.8794 | 184.46 | 0.1535 | 2.99 | 8.18 |
| 31.2614 | 113.27 | 0.2047 | 2.86 | 5.02 |
| 32.0233 | 82.21 | 0.2047 | 2.79 | 3.64 |
| 35.9945 | 71.25 | 0.3070 | 2.50 | 3.16 |

### Example 14. Study on Conversion Relationship Between Free Crystal Forms of Compound of Formula I

In order to study the conversion relationship between the anhydrous crystal forms, suspension competition experiments were first performed on the anhydrous crystal forms A/F/J in different solvents at room temperature and 50 °C. The specific procedures were as follows: A certain amount of the sample of the free crystal form A was weighed out, and a corresponding solvent was added. After the mixture was suspended and stirred at a corresponding temperature for about 2 h, the mixture was filtered through a 0.45 µm PTFE filter membrane to obtain a saturated solution. 2-5 mg of each of the samples of the free anhydrous crystal forms A/F/J was weighed out and added to an HPLC vial, and the saturated solution was transferred to the vial. The vial was placed at a corresponding temperature. The mixture was suspended and stirred for about 4 days, and then the remaining solid was separated out and subjected to XRPD analysis. The experimental results are summarized in Table B1-13, and the XRPD results are shown in FIG. 100. The results show that the anhydrous crystal form J was obtained in the IPA system at room temperature and 50 °C and the CPME system at 50 °C; the hydrate crystal form D was obtained after suspension in the CPME system at room temperature.

The results of the suspension competition experiments among the anhydrous crystal forms show that the anhydrous crystal form J was a more stable crystal form at room temperature and 50 °C. Therefore, the suspension competition experiments of the anhydrous crystal form J and the hydrate crystal forms D/I under different water activities were further studied. The specific procedures were as follows: A certain amount of the free starting sample 2 (i.e., a mixed crystal of the free crystal forms D + J) was weighed out, and a corresponding solvent was added. After the mixture was suspended and stirred at a corresponding temperature for about 2 h, the mixture was filtered through a 0.45 µm PTFE filter membrane to obtain a saturated solution. 2-5 mg of each of the samples of the free crystal forms D/I/J was weighed out and added to an HPLC vial, and the saturated solution was transferred to the vial. The vial was placed at a corresponding temperature. The mixture was suspended and stirred for about 3 days, and then the solid was separated out and subjected to XRPD analysis. The experimental results are summarized in Table B1-14, and the XRPD results are shown in FIG. 101. The results show that the hydrate crystal form D was obtained under conditions of other water activities (0.2-1.0), except that the anhydrous crystal form J was obtained in the ACN system (a_{w}: 0).

**Table B1-13. Results of suspension competition experiments among anhydrous crystal forms**

| **Starting sample** | **Solvent** | **Temperature** | **XRPD result** |
|---|---|---|---|
| | IPA | RT | Free crystal form J |
| Free crystal forms A/F/J | CPME | | Free crystal form D |
| | IPA | 50 °C | Free crystal form J |
| | CPME | | Free crystal form J |

**Table B1-14. Results of suspension competition experiments between hydrate and anhydrous crystal forms**

| **Starting sample** | **Solvent** | **Temperature** | **XRPD result** |
|---|---|---|---|
| | ACN | | Free crystal form J |
| | ACN/H₂O (991:9, a_{w}~0.2) | | Free crystal form D |
| Free crystal forms D/I/J | ACN/H₂O (978:22, a_{w}~0.4) | RT | Free crystal form D |
| | ACN/H₂O (96:4, a_{w}~0.6) | | Free crystal form D |
| | ACN/H₂O (926:74, a_{w}~0.8) | | Free crystal form D |
| | H₂O | | Free crystal form D |

### Example 15. Preparation and Screening I of Salt Forms of Compound of Formula I

For the starting sample 1 (i.e., free crystal form A) of the compound of formula I prepared in Example 1, 10 acidic ligands (at a feeding molar ratio of free crystal form/acidic ligand of 1:1) and 3 solvent systems were selected according to the pKa (4.5, basic group) of the compound and the solubility of the starting material in different solvents at room temperature. The specific procedures for the screening test were as follows: About 30 mg of the sample of the starting free crystal form and equimolar amounts of different ligands were weighed out and added to HPLC vials, 0.6 mL of a solvent was added, and the mixtures were mixed to obtain suspensions. The liquid acid was first diluted with the corresponding solvent and then mixed with the starting sample. The mixtures were suspended and stirred at room temperature for about 3 days, centrifuged to separate the solids, and then dried under vacuum at 50 °C. The salt form screening test results are summarized in Table B2-1. The XRPD characterization results of the resulting solids show that a total of 15 crystal forms, including 2 free polymorphs and 7 salts, were obtained in the salt form screening test. The salt forms obtained by screening were subjected to TGA/DSC characterization, and the salt-forming molar ratios were determined by ¹H NMR and HPLC/IC. The salt form characterization results are summarized in Table B2-2.

**Table B2-1. Summary of salt form screening test results**

| **No.** | **Ligand acid** | **A** | **B** | **C** |
|---|---|---|---|---|
| | | **EtOH** | **MTBE** | **MIBK** |
| 0 | Blank | Free crystal form B | Free crystal form C | Free crystal form C |
| 1 | Hydrochloric acid | Crystal form A of hydrochloride | Free crystal form C + multimodal | Crystal form B of hydrochloride |
| 2 | Sulfuric acid | Crystal form A of sulfate | Crystal form B of sulfate | Crystal form C of sulfate |
| 3 | Maleic acid | Free crystal form B | Free crystal form C | Crystal form A of maleate^{#}* |
| 4 | L-aspartic acid | Free crystal form B | Free crystal form C | Free crystal form C |
| 5 | Phosphoric acid | Free crystal form B | Free crystal form C | Crystal form A of phosphate* |
| 6 | Fumaric acid | Free crystal form B | Free crystal form C | Free crystal form C |
| 7 | Tartaric acid | Free crystal form B | Free crystal form C | Free crystal form C |
| 8 | p-Toluenesulfonic acid | Crystal form A of p-toluenesulfonate | Crystal form B of p-toluenesulfonate | Crystal form B of p-toluenesulfonate |
| 9 | Methanesulfonic acid | Crystal form A of methanesulfonate | Crystal form B of methanesulfonate | Crystal form C of methanesulfonate* |
| 10 | Ethanesulfonic acid | Crystal form A of ethanesulfonate | Crystal form B of ethanesulfonate + free crystal form C | Crystal form B of ethanesulfonate |

| | | | | |
|---|---|---|---|---|
| *: The sample was observed to be gelled **and** then subjected to temperature cycling (50-5 °C). ^{#}: The sample was clear after being stirred at room temperature and 5 °C, and then n-heptane was added to induce crystallization. | | | | |

**Table B2-2. Summary of characterization results of salt forms obtained by screening**

| **Salt form** | | **TGA (weight loss, %)** | **DSC (endothermic peak, °C)** | **Molar ratio* (ligand: free crystal form)** | **Solvent residue* (wt%)** |
|---|---|---|---|---|---|
| Free | Crystal form A | 0.68 (150 °C) | 167.9, 170.5^, 180.3 | -- | ND |
| | Crystal form B | 4.35 (120 °C) | 108.5, 165.8, 167.7^, 180.2 | -- | 3.4 (EtOH) |
| | Crystal form C | 2.77 (150 °C) | 53.0, 182.3 | -- | ND |
| Hydrochloride | Crystal form A | 8.21 (160 °C) | 165.8 | 1.2 | 6.4 (EtOH) |
| | Crystal form B | 16.61 (180 °C) | 154.1, 171.0 | 1.1 | 13.8 (MIBK) |
| | Crystal form C | 4.67 (90 °C) | 77.8, 174.6 | 2.0 | ND |
| Sulfate | Crystal form A | 5.78 (150 °C) | 52.9, 167.1 | 1.1 | 5.8 (EtOH) |
| | Crystal form B | 1.16 (150 °C) | 69.7, 238.5 | 1.2 | ND |
| | Crystal form C | 2.83 (100 °C) | 92.4, 159.3, 184.8, 216.8, 224.0^ | 1.6 | ND |
| Crystal form A of maleate | | 0.41 (130 °C) | 141.1 | 1.0 | 0.4 (MIBK) |
| Crystal form A of phosphate | | 3.71 (150 °C) | 41.5, 91.8^, 169.6 | 4.3 | 1.6 (MIBK) |
| p-Toluenesulfonate | Crystal form A | 4.15 (150 °C) | 81.6, 156.0 | 1.0 | 2.7 (EtOH) 10.8 (MIBK) |
| | Crystal form B | 1.92 (150 °C) | 204.3 | 1.0 | 0.4 (MTBE) |
| Methanesulfonate | Crystal form A | 3.08 (150 °C) | 153.8 | 1.0 | 3.9 (EtOH) |
| | Crystal form B | 0.67 (150 °C) | 161.8, 168.3, 194.9 | 1.1 | 0.2 (MTBE) |
| | Crystal form C | 14.14 (150 °C) | 111.7, 135.8, 139.6 | 1.1 | 11.5 (MIBK) |
| Ethanesulfonate | Crystal form A | 8.29 (150 °C) | 36.3, 141.5 | 1.1 | 2.7 (EtOH) 7.6 (MIBK) |
| | Crystal form B | 6.30 (150 °C) | 107.1, 145.3 | 1.1 | 4.7 (MIBK) |

| | | | | | |
|---|---|---|---|---|---|
| *: The ratio or value was obtained by ¹H NMR or HPLC/IC calculation. ^: exothermic signal. ND: not detected. | | | | | |

### Example 16. Crystal Forms A/B/C of Hydrochloride

The crystal forms A and B of the hydrochloride were obtained by slurrying the starting samples 1 of the compound of formula I and an equimolar amount of hydrochloric acid in ethanol and MIBK, respectively, at room temperature for about 3 days, centrifuging to separate the solid samples, and then drying under vacuum at 50 °C. The crystal form C of the hydrochloride was obtained by slurrying the starting sample 1 of the compound of formula I and 0.3 V of concentrated hydrochloric acid in 15 V of acetone at room temperature for about 2 days, centrifuging to separate the solid, and then drying under vacuum at 50 °C. The XRPD overlay of the hydrochloride samples is shown in FIG. 11.

The XRPD results of the crystal form A of the hydrochloride are shown in FIG. 12a. The TGA/DSC results are listed in FIG. 13, where the TGA results show that the sample had a weight loss of 8.21% when heated to 160 °C; the DSC results show that the sample had **1** endothermic peak at 165.8 °C (peak temperature). The ¹H NMR results, as shown in FIG. 14, show that the residual solvent EtOH and the free crystal form in the sample were in a molar ratio of 0.93 (about 6.4 wt%). The HPLC/IC results indicate that hydrochloric acid and the free crystal form in the sample were in a molar ratio of 1.2:1. The XRPD analysis data of the crystal form A of the hydrochloride are shown in Table B2-3 below:

**Table B2-3. XRPD diffraction peak data of crystal form A of hydrochloride**

| Pos. [°2θ] | Height [cts] | FWHM Left [°2θ] | d-spacing [Å] | Rel. Int. [%] |
|---|---|---|---|---|
| 6.6910 | 293.89 | 0.1023 | 13.21 | 14.42 |
| 9.4043 | 81.79 | 0.3070 | 9.40 | 4.01 |
| 10.1755 | 569.49 | 0.1023 | 8.69 | 27.95 |
| 11.3549 | 297.61 | 0.1279 | 7.79 | 14.60 |
| 12.0715 | 189.74 | 0.0768 | 7.33 | 9.31 |
| 12.7466 | 157.63 | 0.1023 | 6.94 | 7.74 |
| 13.3874 | 200.83 | 0.0768 | 6.61 | 9.86 |
| 14.1825 | 141.44 | 0.1279 | 6.24 | 6.94 |
| 14.7835 | 758.37 | 0.0768 | 5.99 | 37.21 |
| 14.9893 | 784.37 | 0.1023 | 5.91 | 38.49 |
| 17.2365 | 76.72 | 0.1535 | 5.14 | 3.76 |
| 18.2960 | 781.70 | 0.1023 | 4.85 | 38.36 |
| 18.8832 | 1280.22 | 0.1279 | 4.70 | 62.82 |
| 19.3310 | 702.56 | 0.0768 | 4.59 | 34.48 |
| 19.5324 | 2037.82 | 0.1023 | 4.54 | 100.00 |
| 20.1382 | 1078.19 | 0.1279 | 4.41 | 52.91 |
| 20.9418 | 503.10 | 0.1279 | 4.24 | 24.69 |
| 21.3379 | 1394.17 | 0.1279 | 4.16 | 68.41 |
| 21.7432 | 1578.17 | 0.1279 | 4.09 | 77.44 |
| 22.5426 | 209.89 | 0.0768 | 3.94 | 10.30 |
| 23.5259 | 124.00 | 0.2558 | 3.78 | 6.08 |
| 24.0002 | 146.56 | 0.1535 | 3.71 | 7.19 |
| 25.3951 | 232.77 | 0.1535 | 3.51 | 11.42 |
| 25.7379 | 408.66 | 0.1023 | 3.46 | 20.05 |
| 26.6858 | 124.25 | 0.1535 | 3.34 | 6.10 |
| 27.2284 | 523.77 | 0.1535 | 3.28 | 25.70 |
| 27.7725 | 101.92 | 0.1535 | 3.21 | 5.00 |
| 28.5839 | 155.21 | 0.1279 | 3.12 | 7.62 |
| 29.1003 | 177.90 | 0.1279 | 3.07 | 8.73 |
| 29.8240 | 407.75 | 0.1535 | 3.00 | 20.01 |
| 30.7557 | 203.68 | 0.1279 | 2.91 | 9.99 |
| 31.8138 | 201.65 | 0.1535 | 2.81 | 9.90 |
| 33.7066 | 190.74 | 0.1535 | 2.66 | 9.36 |
| 34.5682 | 170.36 | 0.1535 | 2.59 | 8.36 |
| 35.3206 | 64.08 | 0.4093 | 2.54 | 3.14 |
| 36.1697 | 84.27 | 0.2047 | 2.48 | 4.14 |
| 37.1954 | 59.92 | 0.3070 | 2.42 | 2.94 |
| 38.5831 | 96.26 | 0.2047 | 2.33 | 4.72 |
| 39.2520 | 86.22 | 0.2047 | 2.30 | 4.23 |

The XRPD results of the crystal form B of the hydrochloride are shown in FIG. 15. The TGA/DSC results are listed in FIG. 16, where the TGA results show that the sample had a weight loss of 16.61% when heated to 180 °C; the DSC results show that the sample had 2 endothermic peaks at 154.1 °C and 171.0 °C (peak temperatures). The ¹H NMR results, as shown in FIG. 17, show that the residual solvent MIBK and the free crystal form in the sample were in a molar ratio of 1.00 (about 13.8 wt%). The HPLC/IC results indicate that hydrochloric acid and the free crystal form in the sample were in a molar ratio of 1.1:1. The XRPD analysis data of the crystal form B of the hydrochloride are shown in Table B2-4 below:

**Table B2-4. XRPD diffraction peak data of crystal form B of hydrochloride**

| Pos. [°2θ] | Height [cts] | FWHM Left [°2θ] | d-spacing [Å] | Rel. Int. [%] |
|---|---|---|---|---|
| 5.8284 | 4595.46 | 0.1023 | 15.16 | 100.00 |
| 10.2084 | 154.36 | 0.1535 | 8.67 | 3.36 |
| 12.0258 | 238.40 | 0.1279 | 7.36 | 5.19 |
| 12.9418 | 918.19 | 0.1279 | 6.84 | 19.98 |
| 14.5910 | 210.47 | 0.1279 | 6.07 | 4.58 |
| 15.6394 | 918.34 | 0.1023 | 5.67 | 19.98 |
| 15.8305 | 1440.45 | 0.1023 | 5.60 | 31.35 |
| 16.6807 | 492.27 | 0.1535 | 5.31 | 10.71 |
| 17.0761 | 411.51 | 0.1535 | 5.19 | 8.95 |
| 17.4248 | 941.63 | 0.1023 | 5.09 | 20.49 |
| 17.7208 | 1020.31 | 0.1279 | 5.01 | 22.20 |
| 18.9434 | 398.87 | 0.1279 | 4.68 | 8.68 |
| 19.3954 | 258.15 | 0.1791 | 4.58 | 5.62 |
| 19.8130 | 303.59 | 0.1023 | 4.48 | 6.61 |
| 20.3860 | 632.17 | 0.1279 | 4.36 | 13.76 |
| 21.1241 | 438.40 | 0.1791 | 4.21 | 9.54 |
| 23.0260 | 139.53 | 0.2047 | 3.86 | 3.04 |
| 23.5341 | 123.13 | 0.1535 | 3.78 | 2.68 |
| 24.6834 | 676.91 | 0.1279 | 3.61 | 14.73 |
| 25.1539 | 968.17 | 0.1535 | 3.54 | 21.07 |
| 26.7017 | 126.21 | 0.1535 | 3.34 | 2.75 |
| 27.1748 | 172.66 | 0.1535 | 3.28 | 3.76 |
| 28.3119 | 113.00 | 0.1535 | 3.15 | 2.46 |
| 29.2775 | 144.04 | 0.2558 | 3.05 | 3.13 |
| 30.8075 | 190.02 | 0.2047 | 2.90 | 4.13 |
| 34.0826 | 36.68 | 0.6140 | 2.63 | 0.80 |

The XRPD pattern of the crystal form C of the hydrochloride is shown in FIG. 18. The TGA/DSC results are listed in FIG. 19, where the TGA results show that the sample had a weight loss of 4.67% when heated to 90 °C and a weight loss of 6.00% when further heated to 170 °C; the DSC results show that the sample had 2 endothermic peaks at 77.8 °C and 174.6 °C (peak temperatures). The ¹H NMR results, as shown in FIG. 20, show that no acetone solvent residue was found in the sample. The HPLC/IC results indicate that hydrochloric acid and the free crystal form in the sample were in a molar ratio of 2.0:1. The XRPD analysis data of the crystal form C of the hydrochloride are shown in Table B2-5 below:

**Table B2-5. XRPD diffraction peak data of crystal form C of hydrochloride**

| Pos. [°2θ] | Height [cts] | FWHM Left [°2θ] | d-spacing [Å] | Rel. Int. [%] |
|---|---|---|---|---|
| 5.6027 | 889.42 | 0.0768 | 15.77 | 89.57 |
| 5.7758 | 893.34 | 0.0768 | 15.30 | 89.97 |
| 8.7367 | 364.89 | 0.0768 | 10.12 | 36.75 |
| 9.2140 | 160.24 | 0.1023 | 9.60 | 16.14 |
| 11.1522 | 258.60 | 0.1023 | 7.93 | 26.04 |
| 11.8436 | 145.92 | 0.1023 | 7.47 | 14.69 |
| 13.2815 | 417.22 | 0.0768 | 6.67 | 42.02 |
| 13.6699 | 992.96 | 0.0768 | 6.48 | 100.00 |
| 13.8622 | 464.47 | 0.0768 | 6.39 | 46.78 |
| 14.4660 | 582.64 | 0.0768 | 6.12 | 58.68 |
| 15.2083 | 142.65 | 0.1023 | 5.83 | 14.37 |
| 16.1990 | 209.95 | 0.1023 | 5.47 | 21.14 |
| 16.6600 | 276.84 | 0.1023 | 5.32 | 27.88 |
| 17.1465 | 482.51 | 0.1023 | 5.17 | 48.59 |
| 17.9792 | 240.83 | 0.1023 | 4.93 | 24.25 |
| 18.7028 | 442.51 | 0.1023 | 4.74 | 44.57 |
| 19.4828 | 325.25 | 0.1023 | 4.56 | 32.76 |
| 20.0737 | 189.90 | 0.1023 | 4.42 | 19.12 |
| 20.3774 | 140.77 | 0.1279 | 4.36 | 14.18 |
| 20.9473 | 289.17 | 0.1023 | 4.24 | 29.12 |
| 21.6765 | 558.87 | 0.1279 | 4.10 | 56.28 |
| 22.0633 | 212.66 | 0.1279 | 4.03 | 21.42 |
| 22.4844 | 273.11 | 0.2047 | 3.95 | 27.50 |
| 23.2062 | 555.21 | 0.1023 | 3.83 | 55.91 |
| 24.0140 | 196.24 | 0.1535 | 3.71 | 19.76 |
| 24.9698 | 119.40 | 0.2558 | 3.57 | 12.02 |
| 25.8392 | 154.27 | 0.2047 | 3.45 | 15.54 |
| 26.3673 | 250.12 | 0.2047 | 3.38 | 25.19 |
| 27.1991 | 553.74 | 0.1279 | 3.28 | 55.77 |
| 28.0148 | 125.25 | 0.6140 | 3.19 | 12.61 |
| 29.1691 | 165.13 | 0.1535 | 3.06 | 16.63 |
| 37.7780 | 45.14 | 0.6140 | 2.38 | 4.55 |

### Repeated preparation of crystal form A of hydrochloride:

A sample of the crystal form A of the hydrochloride prepared repeatedly was obtained by slurrying about 500 mg of the free starting sample 2 and an equimolar amount of hydrochloric acid in EtOH at room temperature for about 2 days, centrifuging to separate the solid sample, and then drying under vacuum at 50 °C. The XRPD results are listed in FIG. 12b.

### Example 17. Crystal forms A/B/C of Sulfate

The crystal forms A, B, and C of the sulfate were obtained by slurrying the starting samples 1 of the compound of formula I and an equimolar amount of sulfuric acid in ethanol, MTBE, and MIBK, respectively, at room temperature for about 3 days, centrifuging to separate the solid samples, and then drying under vacuum at 50 °C. The XRPD overlay of the sulfate samples is shown in FIG. 21.

The XRPD results of the crystal form A of the sulfate are shown in FIG. 22. The TGA/DSC results are listed in FIG. 23. The TGA results show that the sample had a weight loss of 5.78% when heated to 150 °C; the DSC results show that the sample had 2 endothermic peaks at 52.9 °C and 167.1 °C (peak temperatures). The ¹H NMR results, as shown in FIG. 24, show that the residual solvent ethanol and the free crystal form in the sample were in a molar ratio of 0.93 (about 5.8 wt%). The HPLC/IC results indicate that sulfuric acid and the free crystal form in the sample were in a molar ratio of 1.1:1. The XRPD analysis data of the crystal form A of the sulfate are shown in Table B2-6 below:

**Table B2-6. XRPD diffraction peak data of crystal form A of sulfate**

| Pos. [°2θ] | Height [cts] | FWHM Left [°2θ] | d-spacing [Å] | Rel. Int. [%] |
|---|---|---|---|---|
| 6.5297 | 575.74 | 0.1023 | 13.54 | 30.89 |
| 7.4156 | 930.73 | 0.0768 | 11.92 | 49.93 |
| 9.8272 | 161.20 | 0.2047 | 9.00 | 8.65 |
| 11.2590 | 767.10 | 0.1023 | 7.86 | 41.16 |
| 11.7996 | 273.75 | 0.1023 | 7.50 | 14.69 |
| 13.0075 | 76.04 | 0.1535 | 6.81 | 4.08 |
| 14.8207 | 428.75 | 0.1023 | 5.98 | 23.00 |
| 15.0692 | 402.19 | 0.1279 | 5.88 | 21.58 |
| 17.2920 | 961.99 | 0.1023 | 5.13 | 51.61 |
| 17.5479 | 455.02 | 0.1023 | 5.05 | 24.41 |
| 18.3831 | 574.84 | 0.1023 | 4.83 | 30.84 |
| 18.9056 | 594.31 | 0.1279 | 4.69 | 31.89 |
| 19.5870 | 1863.91 | 0.1279 | 4.53 | 100.00 |
| 19.8280 | 639.35 | 0.1279 | 4.48 | 34.30 |
| 20.1191 | 300.08 | 0.1023 | 4.41 | 16.10 |
| 21.1050 | 346.14 | 0.1279 | 4.21 | 18.57 |
| 21.9720 | 155.70 | 0.1535 | 4.05 | 8.35 |
| 23.1076 | 542.20 | 0.2303 | 3.85 | 29.09 |
| 23.6024 | 462.92 | 0.1279 | 3.77 | 24.84 |
| 25.1201 | 663.10 | 0.1535 | 3.55 | 35.58 |
| 27.2805 | 131.24 | 0.1535 | 3.27 | 7.04 |
| 28.7070 | 68.52 | 0.3070 | 3.11 | 3.68 |
| 29.8446 | 33.87 | 0.6140 | 2.99 | 1.82 |
| 32.6299 | 59.05 | 0.8187 | 2.74 | 3.17 |

The XRPD results of the crystal form B of the sulfate are shown in FIG. 25. The TGA/DSC results are listed in FIG. 26, where the TGA results show that the sample had a weight loss of 1.16% when heated to 150 °C; the DSC results show that the sample had 2 endothermic peaks at 69.7 °C and 238.5 °C (peak temperatures). The ¹H NMR results, as shown in FIG. 27, show that no MTBE solvent residue was found in the sample. The HPLC/IC results indicate that sulfuric acid and the free crystal form in the sample were in a molar ratio of 1.2:1. The XRPD analysis data of the crystal form B of the sulfate are shown in Table B2-7 below:

**Table B2-7. XRPD diffraction peak data of crystal form B of sulfate**

| Pos. [°2θ] | Height [cts] | FWHM Left [°2θ] | d-spacing [Å] | Rel. Int. [%] |
|---|---|---|---|---|
| 6.3375 | 324.34 | 0.1023 | 13.95 | 10.17 |
| 8.6916 | 156.71 | 0.1535 | 10.17 | 4.91 |
| 9.1864 | 213.75 | 0.1791 | 9.63 | 6.70 |
| 9.8491 | 1989.19 | 0.1535 | 8.98 | 62.35 |
| 11.7001 | 226.41 | 0.1279 | 7.56 | 7.10 |
| 12.1619 | 138.35 | 0.2047 | 7.28 | 4.34 |
| 13.0250 | 327.17 | 0.1023 | 6.80 | 10.26 |
| 14.4700 | 214.15 | 0.1535 | 6.12 | 6.71 |
| 15.1807 | 77.36 | 0.1535 | 5.84 | 2.42 |
| 16.5561 | 1562.55 | 0.1279 | 5.35 | 48.98 |
| 17.1270 | 1341.01 | 0.1279 | 5.18 | 42.03 |
| 17.8255 | 976.94 | 0.1279 | 4.98 | 30.62 |
| 18.1372 | 476.77 | 0.1279 | 4.89 | 14.94 |
| 19.0468 | 464.75 | 0.1279 | 4.66 | 14.57 |
| 19.6977 | 3190.28 | 0.1279 | 4.51 | 100.00 |
| 21.6775 | 235.43 | 0.1535 | 4.10 | 7.38 |
| 22.2378 | 400.35 | 0.1279 | 4.00 | 12.55 |
| 22.6654 | 563.77 | 0.1535 | 3.92 | 17.67 |
| 23.4512 | 262.97 | 0.1279 | 3.79 | 8.24 |
| 23.8100 | 231.84 | 0.1535 | 3.74 | 7.27 |
| 24.8186 | 146.61 | 0.1535 | 3.59 | 4.60 |
| 25.4720 | 246.71 | 0.1535 | 3.50 | 7.73 |
| 25.7586 | 377.25 | 0.1023 | 3.46 | 11.83 |
| 26.1622 | 500.88 | 0.1535 | 3.41 | 15.70 |
| 27.1217 | 79.68 | 0.4093 | 3.29 | 2.50 |
| 29.0464 | 113.29 | 0.2047 | 3.07 | 3.55 |
| 29.7168 | 366.36 | 0.1279 | 3.01 | 11.48 |
| 30.9706 | 180.12 | 0.1279 | 2.89 | 5.65 |
| 33.1687 | 62.95 | 0.3070 | 2.70 | 1.97 |
| 34.5562 | 63.94 | 0.3070 | 2.60 | 2.00 |

The XRPD results of the crystal form C of the sulfate are shown in FIG. 28. The TGA/DSC results are listed in FIG. 29, where the TGA results show that the sample had a weight loss of 2.83% when heated to 100 °C; the DSC results show that the sample had 4 endothermic peaks at 92.4 °C, 159.3 °C, 184.8 °C, and 216.8 °C (peak temperatures) and **1** exothermic peak at 224.0 °C (peak temperature). The ¹H NMR results, as shown in FIG. 30, show that no MIBK solvent residue was found in the sample. The HPLC/IC results indicate that sulfuric acid and the free crystal form in the sample were in a molar ratio of 1.6:1. The XRPD analysis data of the crystal form C of the sulfate are shown in Table B2-8 below:

**Table B2-8. XRPD diffraction peak data of crystal form C of sulfate**

| Pos. [°2θ] | Height [cts] | FWHM Left [°2θ] | d-spacing [Å] | Rel. Int. [%] |
|---|---|---|---|---|
| 7.4762 | 96.82 | 0.1535 | 11.82 | 2.82 |
| 8.0099 | 96.55 | 0.1535 | 11.04 | 2.81 |
| 8.9487 | 3027.54 | 0.1023 | 9.88 | 88.11 |
| 9.8314 | 583.94 | 0.1023 | 9.00 | 16.99 |
| 11.7697 | 87.51 | 0.1535 | 7.52 | 2.55 |
| 12.2693 | 300.90 | 0.1023 | 7.21 | 8.76 |
| 13.3788 | 244.43 | 0.1279 | 6.62 | 7.11 |
| 14.4549 | 607.80 | 0.1023 | 6.13 | 17.69 |
| 14.9774 | 160.85 | 0.1279 | 5.92 | 4.68 |
| 15.3199 | 204.35 | 0.0768 | 5.78 | 5.95 |
| 16.8293 | 244.01 | 0.1279 | 5.27 | 7.10 |
| 17.5523 | 1218.97 | 0.1279 | 5.05 | 35.48 |
| 17.9120 | 3436.13 | 0.1023 | 4.95 | 100.00 |
| 18.7584 | 708.83 | 0.1023 | 4.73 | 20.63 |
| 19.5850 | 584.52 | 0.1279 | 4.53 | 17.01 |
| 19.9597 | 1320.42 | 0.1535 | 4.45 | 38.43 |
| 20.6152 | 302.18 | 0.2047 | 4.31 | 8.79 |
| 20.9974 | 581.23 | 0.1535 | 4.23 | 16.92 |
| 21.9335 | 363.38 | 0.1279 | 4.05 | 10.58 |
| 22.4850 | 763.70 | 0.1023 | 3.95 | 22.23 |
| 22.9905 | 1248.46 | 0.1279 | 3.87 | 36.33 |
| 23.6939 | 324.67 | 0.0768 | 3.76 | 9.45 |
| 23.9937 | 316.54 | 0.1535 | 3.71 | 9.21 |
| 24.5952 | 551.20 | 0.1535 | 3.62 | 16.04 |
| 24.8904 | 231.19 | 0.0768 | 3.58 | 6.73 |
| 25.3759 | 260.70 | 0.1279 | 3.51 | 7.59 |
| 26.2847 | 250.45 | 0.1279 | 3.39 | 7.29 |
| 26.7497 | 387.53 | 0.1279 | 3.33 | 11.28 |
| 28.4461 | 71.62 | 0.4093 | 3.14 | 2.08 |
| 29.4160 | 559.22 | 0.1279 | 3.04 | 16.27 |
| 31.7091 | 70.43 | 0.1535 | 2.82 | 2.05 |
| 33.4595 | 103.06 | 0.2558 | 2.68 | 3.00 |
| 34.8478 | 84.06 | 0.2558 | 2.57 | 2.45 |
| 36.5099 | 60.45 | 0.6140 | 2.46 | 1.76 |
| 38.0959 | 62.09 | 0.3070 | 2.36 | 1.81 |

### Example 18. Crystal Form A of Maleate

Method I: The crystal form A of the maleate was obtained by slurrying the starting sample 1 of the compound of formula I and an equimolar amount of maleic acid in MIBK at 5 °C for about 3 days until the sample was clear, adding n-heptane to induce crystallization until the sample was gelled, performing temperature cycling on the sample between 50 °C and 5 °C to obtain a suspension, centrifuging to separate the solid sample, and drying under vacuum at 50 °C.

Method II: 1) About 50 mg of the starting sample of the compound of formula I and about 9.8 mg of maleic acid were weighed out and added to 500 µL of MIBK until the sample was dissolved completely; 2) a small amount of crystal seeds of the crystal form A of the maleate was added to the solution in step 1), and the mixture was magnetically stirred at room temperature to cause turbidity and then subjected to aging for about 30 min; 3) 500 µL of n-heptane was slowly added to the solution in step 2), no gelling was observed during the process, the mixture was successively magnetically stirred at room temperature for about 1 day, centrifuged to separate the solid, and then dried under vacuum at 50 °C overnight, and about 47.0 mg of the sample was collected.

The XRPD of the crystal form A of the maleate is shown in FIG. 31. The TGA/DSC results are detailed in FIG. 32, where the TGA results show that the sample had a weight loss of 0.42% when heated to 130 °C, and the DSC results show that the sample had 1 endothermic peak at 142.1 °C (peak temperature). The ¹H NMR results, as shown in FIG. 33, show that maleic acid and the free crystal form in the sample were in a molar ratio of 1.0, and the residual solvent MIBK and the free crystal form were in a molar ratio of 0.04 (about 0.4 wt%). The XRPD analysis data of the crystal form A of the maleate are shown in Table B2-9 below:

**Table B2-9. XRPD diffraction peak data of crystal form A of maleate**

| Pos. [°2θ] | Height [cts] | FWHM Left [°2θ] | d-spacing [Å] | Rel. Int. [%] |
|---|---|---|---|---|
| 7.4318 | 863.36 | 0.1023 | 11.90 | 13.39 |
| 8.1664 | 531.41 | 0.1023 | 10.83 | 8.24 |
| 10.4550 | 825.61 | 0.1023 | 8.46 | 12.80 |
| 11.4963 | 64.93 | 0.3070 | 7.70 | 1.01 |
| 13.7865 | 6449.49 | 0.1023 | 6.42 | 100.00 |
| 14.9748 | 594.52 | 0.1023 | 5.92 | 9.22 |
| 15.6098 | 336.11 | 0.1023 | 5.68 | 5.21 |
| 15.9646 | 885.25 | 0.1023 | 5.55 | 13.73 |
| 16.3294 | 3521.31 | 0.1023 | 5.43 | 54.60 |
| 17.2388 | 597.97 | 0.1023 | 5.14 | 9.27 |
| 17.7554 | 747.51 | 0.1023 | 5.00 | 11.59 |
| 18.2795 | 2952.42 | 0.1535 | 4.85 | 45.78 |
| 18.7232 | 546.10 | 0.1023 | 4.74 | 8.47 |
| 19.2252 | 346.06 | 0.1023 | 4.62 | 5.37 |
| 19.9626 | 366.14 | 0.1023 | 4.45 | 5.68 |
| 20.3336 | 2790.73 | 0.1279 | 4.37 | 43.27 |
| 20.8824 | 2299.94 | 0.1279 | 4.25 | 35.66 |
| 21.3104 | 1810.49 | 0.1023 | 4.17 | 28.07 |
| 21.5283 | 917.45 | 0.0768 | 4.13 | 14.23 |
| 21.8250 | 177.34 | 0.1023 | 4.07 | 2.75 |
| 22.5862 | 157.36 | 0.1023 | 3.94 | 2.44 |
| 23.1978 | 846.46 | 0.1023 | 3.83 | 13.12 |
| 23.7175 | 198.01 | 0.1279 | 3.75 | 3.07 |
| 24.1136 | 853.11 | 0.1279 | 3.69 | 13.23 |
| 24.7430 | 645.91 | 0.1023 | 3.60 | 10.01 |
| 25.0797 | 273.95 | 0.1279 | 3.55 | 4.25 |
| 26.1594 | 422.73 | 0.1535 | 3.41 | 6.55 |
| 27.1361 | 211.77 | 0.1279 | 3.29 | 3.28 |
| 27.5054 | 503.02 | 0.1791 | 3.24 | 7.80 |
| 28.4773 | 203.45 | 0.1279 | 3.13 | 3.15 |
| 29.4248 | 254.48 | 0.1279 | 3.04 | 3.95 |
| 30.0997 | 297.21 | 0.1535 | 2.97 | 4.61 |
| 30.4233 | 292.95 | 0.1279 | 2.94 | 4.54 |
| 31.3563 | 136.84 | 0.1535 | 2.85 | 2.12 |
| 32.1988 | 81.71 | 0.1535 | 2.78 | 1.27 |
| 32.8832 | 156.12 | 0.1791 | 2.72 | 2.42 |
| 34.8721 | 108.34 | 0.1791 | 2.57 | 1.68 |
| 36.2350 | 64.74 | 0.2047 | 2.48 | 1.00 |
| 37.8027 | 111.64 | 0.1791 | 2.38 | 1.73 |

### Example 19. Crystal Form A of Phosphate

The starting sample 1 of the compound of formula I and an equimolar amount of phosphoric acid were subjected to a temperature cycling between 50 °C and 5 °C in MIBK for about 6 days resulting in a colloidal solid, and the colloidal solid sample was then separated by centrifugation and dried under vacuum at 50 °C to obtain the crystal form A of the phosphate.The XRPD of the crystal form A of the phosphate is shown in FIG. 34. The TGA/DSC results are detailed in FIG. 35. The TGA results show that the sample had a weight loss of 3.71% when heated to 150 °C; the DSC results show that the sample had 2 endothermic peaks at 41.5 °C and 169.6 °C (peak temperatures) and 1 exothermic peak at 91.8 °C (peak temperature). The ¹H NMR results, as shown in FIG. 36, show that the residual solvent MIBK and the free crystal form in the sample were in a molar ratio of 0.16 (about 1.6 wt%). The HPLC/IC results indicate that phosphoric acid and the free crystal form in the sample were in a molar ratio of 4.3:1. The XRPD analysis data of the crystal form A of the phosphate are shown in Table B2-10 below:

**Table B2-10. XRPD diffraction peak data of crystal form A of phosphate**

| Pos. [°2θ] | Height [cts] | FWHM Left [°2θ] | d-spacing [Å] | Rel. Int. [%] |
|---|---|---|---|---|
| 3.2059 | 59.54 | 0.6140 | 27.56 | 9.20 |
| 8.8155 | 227.36 | 0.1023 | 10.03 | 35.12 |
| 9.3607 | 480.45 | 0.1023 | 9.45 | 74.20 |
| 11.1767 | 113.29 | 0.2558 | 7.92 | 17.50 |
| 13.9605 | 89.25 | 0.1535 | 6.34 | 13.78 |
| 16.4840 | 112.73 | 0.3070 | 5.38 | 17.41 |
| 17.8932 | 571.27 | 0.1023 | 4.96 | 88.23 |
| 18.4384 | 271.13 | 0.3582 | 4.81 | 41.88 |
| 19.3716 | 363.41 | 0.1791 | 4.58 | 56.13 |
| 19.8946 | 352.39 | 0.1023 | 4.46 | 54.43 |
| 20.5448 | 325.53 | 0.2047 | 4.32 | 50.28 |
| 21.1372 | 647.47 | 0.1023 | 4.20 | 100.00 |
| 22.4294 | 347.25 | 0.1023 | 3.96 | 53.63 |
| 23.1105 | 173.95 | 0.2558 | 3.85 | 26.87 |
| 23.9976 | 211.16 | 0.2047 | 3.71 | 32.61 |
| 24.5486 | 243.43 | 0.1279 | 3.63 | 37.60 |
| 25.7922 | 136.65 | 0.2047 | 3.45 | 21.11 |
| 26.3981 | 131.68 | 0.2558 | 3.38 | 20.34 |
| 26.9789 | 86.17 | 0.3070 | 3.30 | 13.31 |
| 28.2812 | 95.45 | 0.4093 | 3.16 | 14.74 |
| 30.2804 | 44.25 | 0.6140 | 2.95 | 6.83 |

### Example 20. Crystal Forms A/B of p-Toluenesulfonate

The crystal forms A/B of the p-toluenesulfonate were obtained by slurrying the starting samples 1 of the compound of formula I and an equimolar amount of p-toluenesulfonic acid in ethanol and MTBE, respectively, at room temperature for about 3 days, centrifuging to separate the solid samples, and then drying under vacuum at 50 °C. The XRPD overlay of the p-toluenesulfonate samples is shown in FIG. 37.

The XRPD results of the crystal form A of the p-toluenesulfonate are shown in FIG. 38. The TGA/DSC results are listed in FIG. 39, where the TGA results show that the sample had a weight loss of 4.15% when heated to 150 °C; the DSC results show that the sample had 2 endothermic peaks at 81.6 °C and 156.0 °C (peak temperatures). The ¹H NMR results, as shown in FIG. 40, show that p-toluenesulfonic acid and the free crystal form in the sample were in a molar ratio of 1.0, the residual solvent EtOH and the free crystal form were in a molar ratio of 0.52 (about 2.7 wt%), and MIBK and the free crystal form were in a molar ratio of 0.95 (about 10.8 wt%). The XRPD analysis data of the crystal form A of the p-toluenesulfonate are shown in Table B2-11 below:

**Table B2-11. XRPD diffraction peak data of crystal form A of p-toluenesulfonate**

| Pos. [°2θ] | Height [cts] | FWHM Left [°2θ] | d-spacing [Å] | Rel. Int. [%] |
|---|---|---|---|---|
| 5.6569 | 518.00 | 0.2303 | 15.62 | 74.33 |
| 6.5865 | 148.14 | 0.1535 | 13.42 | 21.26 |
| 9.0105 | 94.94 | 0.6140 | 9.81 | 13.62 |
| 14.4962 | 108.38 | 0.3070 | 6.11 | 15.55 |
| 17.1789 | 204.83 | 0.4093 | 5.16 | 29.39 |
| 19.7973 | 696.84 | 0.2047 | 4.48 | 100.00 |

The XRPD results of the crystal form B of the p-toluenesulfonate are shown in FIG. 41. The TGA/DSC results are listed in FIG. 42. The TGA results show that the sample had a weight loss of 1.92% when heated to 150 °C; the DSC results show that the sample had 1 endothermic peak at 204.3 °C (peak temperature). The ¹H NMR results, as shown in FIG. 43, show that p-toluenesulfonic acid and the free crystal form in the sample were in a molar ratio of 1.0, and the residual solvent MTBE and the free crystal form were in a molar ratio of 0.03 (about 0.4 wt%). The XRPD analysis data of the crystal form B of the p-toluenesulfonate are shown in Table B2-12 below:

**Table B2-12. XRPD diffraction peak data of crystal form B of p-toluenesulfonate**

| Pos. [°2θ] | Height [cts] | FWHM Left [°2θ] | d-spacing [Å] | Rel. Int. [%] |
|---|---|---|---|---|
| 6.8890 | 363.32 | 0.1023 | 12.83 | 20.72 |
| 7.6660 | 1439.97 | 0.1279 | 11.53 | 82.11 |
| 9.9665 | 282.18 | 0.1279 | 8.88 | 16.09 |
| 11.1056 | 798.59 | 0.1023 | 7.97 | 45.54 |
| 11.8448 | 277.37 | 0.1535 | 7.47 | 15.82 |
| 12.6617 | 218.72 | 0.1023 | 6.99 | 12.47 |
| 13.1706 | 210.20 | 0.1023 | 6.72 | 11.99 |
| 13.6098 | 483.01 | 0.0768 | 6.51 | 27.54 |
| 13.8415 | 464.72 | 0.1279 | 6.40 | 26.50 |
| 14.5769 | 821.65 | 0.1279 | 6.08 | 46.85 |
| 14.9845 | 615.32 | 0.1279 | 5.91 | 35.09 |
| 16.2695 | 1032.23 | 0.1535 | 5.45 | 58.86 |
| 17.6681 | 1753.76 | 0.1535 | 5.02 | 100.00 |
| 18.8718 | 402.93 | 0.1023 | 4.70 | 22.98 |
| 19.5433 | 1222.54 | 0.1279 | 4.54 | 69.71 |
| 19.9700 | 481.29 | 0.1023 | 4.45 | 27.44 |
| 20.3312 | 924.22 | 0.1279 | 4.37 | 52.70 |
| 20.7694 | 822.31 | 0.2558 | 4.28 | 46.89 |
| 21.3520 | 1492.62 | 0.1279 | 4.16 | 85.11 |
| 21.6902 | 278.02 | 0.1023 | 4.10 | 15.85 |
| 22.8795 | 680.09 | 0.2047 | 3.89 | 38.78 |
| 24.5060 | 659.00 | 0.1791 | 3.63 | 37.58 |
| 26.0500 | 705.60 | 0.1535 | 3.42 | 40.23 |
| 26.4583 | 447.33 | 0.1535 | 3.37 | 25.51 |
| 27.2822 | 201.07 | 0.2047 | 3.27 | 11.46 |
| 28.2460 | 583.08 | 0.1535 | 3.16 | 33.25 |
| 29.2107 | 79.94 | 0.3070 | 3.06 | 4.56 |
| 30.0879 | 72.92 | 0.6140 | 2.97 | 4.16 |
| 31.8132 | 81.43 | 0.4093 | 2.81 | 4.64 |
| 32.8460 | 191.14 | 0.2047 | 2.73 | 10.90 |
| 33.7653 | 83.68 | 0.1535 | 2.65 | 4.77 |
| 37.7617 | 46.38 | 0.4093 | 2.38 | 2.64 |

### Example 21. Crystal Forms A/B/C/D of Methanesulfonate

The crystal forms A/B of the methanesulfonate were obtained by slurrying the starting samples 1 of the compound of formula I and an equimolar amount of methanesulfonic acid in ethanol and MTBE, respectively, at room temperature for about 3 days, centrifuging to separate the solid samples, and then drying under vacuum at 50 °C. The crystal form C of the methanesulfonate was obtained by subjecting the starting sample of the compound of formula I and an equimolar amount of methanesulfonic acid to temperature cycling between 50 °C and 5 °C in MIBK for about 3 days, centrifuging to separate the solid sample, and then drying under vacuum at 50 °C. The XRPD overlay of the methanesulfonate samples is shown in FIG. 44.

The XRPD results of the crystal form A of the methanesulfonate are shown in FIG. 45. The TGA/DSC results are listed in FIG. 46, where the TGA results show that the sample had a weight loss of 3.08% when heated to 150 °C; the DSC results show that the sample had 1 endothermic peak at 153.8 °C (peak temperature). The ¹H NMR results, as shown in FIG. 47, show that methanesulfonic acid and the free crystal form in the sample were in a molar ratio of 1.0, and the residual solvent EtOH and the free crystal form were in a molar ratio of 0.60 (about 3.9 wt%). The XRPD analysis data of the crystal form A of the methanesulfonate are shown in Table B2-13 below:

**Table B2-13. XRPD diffraction peak data of crystal form A of methanesulfonate**

| Pos. [°2θ] | Height [cts] | FWHM Left [°2θ] | d-spacing [Å] | Rel. Int. [%] |
|---|---|---|---|---|
| 6.4245 | 282.67 | 0.1023 | 13.76 | 20.39 |
| 7.1654 | 967.47 | 0.1023 | 12.34 | 69.79 |
| 7.7112 | 1054.45 | 0.0768 | 11.47 | 76.07 |
| 10.2242 | 673.51 | 0.1791 | 8.65 | 48.59 |
| 11.4305 | 568.28 | 0.1535 | 7.74 | 40.99 |
| 12.1973 | 177.10 | 0.1023 | 7.26 | 12.78 |
| 14.3852 | 106.55 | 0.2558 | 6.16 | 7.69 |
| 14.9410 | 206.43 | 0.1791 | 5.93 | 14.89 |
| 15.4030 | 693.99 | 0.1279 | 5.75 | 50.06 |
| 16.8120 | 915.12 | 0.1279 | 5.27 | 66.02 |
| 17.3257 | 395.66 | 0.0768 | 5.12 | 28.54 |
| 17.7499 | 669.82 | 0.1791 | 5.00 | 48.32 |
| 18.3934 | 453.64 | 0.1279 | 4.82 | 32.73 |
| 18.9572 | 647.46 | 0.1023 | 4.68 | 46.71 |
| 19.2492 | 1386.21 | 0.1023 | 4.61 | 100.00 |
| 19.6796 | 455.29 | 0.1023 | 4.51 | 32.84 |
| 20.0359 | 646.84 | 0.1535 | 4.43 | 46.66 |
| 20.7137 | 229.25 | 0.1535 | 4.29 | 16.54 |
| 21.0725 | 225.04 | 0.1279 | 4.22 | 16.23 |
| 21.4943 | 427.97 | 0.1023 | 4.13 | 30.87 |
| 22.4448 | 150.84 | 0.1023 | 3.96 | 10.88 |
| 23.1420 | 493.13 | 0.1535 | 3.84 | 35.57 |
| 23.6743 | 358.44 | 0.2558 | 3.76 | 25.86 |
| 24.4576 | 119.30 | 0.2047 | 3.64 | 8.61 |
| 25.1217 | 234.41 | 0.1023 | 3.54 | 16.91 |
| 25.5679 | 516.48 | 0.1279 | 3.48 | 37.26 |
| 27.3091 | 120.89 | 0.2047 | 3.27 | 8.72 |
| 28.2828 | 72.70 | 0.2047 | 3.16 | 5.24 |
| 30.2612 | 60.76 | 0.3070 | 2.95 | 4.38 |
| 31.6602 | 103.53 | 0.2047 | 2.83 | 7.47 |
| 33.8876 | 64.24 | 0.1535 | 2.65 | 4.63 |

The XRPD results of the crystal form B of the methanesulfonate are shown in FIG. 48. The TGA/DSC results are listed in FIG. 49, where the TGA results show that the sample had a weight loss of 0.67% when heated to 150 °C; the DSC results show that the sample had 3 endothermic peaks at 161.8 °C, 168.3 °C, and 194.9 °C (peak temperatures). The ¹H NMR results, as shown in FIG. 50, show that methanesulfonic acid and the free crystal form in the sample were in a molar ratio of 1.1, and the residual solvent MTBE and the free crystal form were in a molar ratio of 0.02 (about 0.2 wt%). The XRPD analysis data of the crystal form B of the methanesulfonate are shown in Table B2-14 below:

**Table B2-14. XRPD diffraction peak data of crystal form B of methanesulfonate**

| Pos. [°2θ] | Height [cts] | FWHM Left [°2θ] | d-spacing [Å] | Rel. Int. [%] |
|---|---|---|---|---|
| 7.9334 | 893.25 | 0.2303 | 11.14 | 90.31 |
| 10.5237 | 112.85 | 0.2558 | 8.41 | 11.41 |
| 11.7673 | 122.98 | 0.2047 | 7.52 | 12.43 |
| 12.4699 | 226.58 | 0.1791 | 7.10 | 22.91 |
| 13.8134 | 228.81 | 0.1535 | 6.41 | 23.13 |
| 14.2662 | 221.77 | 0.1023 | 6.21 | 22.42 |
| 16.2948 | 488.08 | 0.2558 | 5.44 | 49.35 |
| 17.1889 | 767.08 | 0.2303 | 5.16 | 77.56 |
| 17.9047 | 989.06 | 0.1791 | 4.95 | 100.00 |
| 18.9481 | 528.44 | 0.1535 | 4.68 | 53.43 |
| 19.4932 | 793.46 | 0.2047 | 4.55 | 80.22 |
| 20.3844 | 533.63 | 0.1279 | 4.36 | 53.95 |
| 21.2069 | 316.08 | 0.4605 | 4.19 | 31.96 |
| 22.3570 | 88.43 | 0.3070 | 3.98 | 8.94 |
| 22.9645 | 197.80 | 0.2047 | 3.87 | 20.00 |
| 23.9181 | 478.14 | 0.4093 | 3.72 | 48.34 |
| 25.0848 | 174.89 | 0.2558 | 3.55 | 17.68 |
| 26.8731 | 499.75 | 0.3070 | 3.32 | 50.53 |
| 28.8447 | 117.65 | 0.7164 | 3.10 | 11.90 |
| 30.5888 | 127.30 | 0.2558 | 2.92 | 12.87 |
| 36.0120 | 34.91 | 0.6140 | 2.49 | 3.53 |

The XRPD results of the crystal form C of the methanesulfonate are shown in FIG. 51. The TGA/DSC results are listed in FIG. 52, where the TGA results show that the sample had a weight loss of 14.14% when heated to 150 °C; the DSC results show that the sample had 3 endothermic peaks at 111.7 °C, 135.8 °C, and 139.6 °C (peak temperatures). The ¹H NMR results, as shown in FIG. 53, show that methanesulfonic acid and the free crystal form in the sample were in a molar ratio of 1.1, and the residual solvent MIBK and the free crystal form were in a molar ratio of 0.90 (about 11.5 wt%). The XRPD analysis data of the crystal form C of the methanesulfonate are shown in Table B2-15 below:

**Table B2-15. XRPD diffraction peak data of crystal form C of methanesulfonate**

| Pos. [°2θ] | Height [cts] | FWHM Left [°2θ] | d-spacing [Å] | Rel. Int. [%] |
|---|---|---|---|---|
| 5.6360 | 2168.57 | 0.1535 | 15.68 | 100.00 |
| 8.5661 | 138.48 | 0.1535 | 10.32 | 6.39 |
| 9.3088 | 1217.60 | 0.1023 | 9.50 | 56.15 |
| 11.6453 | 82.24 | 0.2047 | 7.60 | 3.79 |
| 12.6477 | 316.59 | 0.1535 | 7.00 | 14.60 |
| 13.6745 | 441.85 | 0.1279 | 6.48 | 20.38 |
| 15.7926 | 786.49 | 0.1535 | 5.61 | 36.27 |
| 16.1257 | 425.87 | 0.1279 | 5.50 | 19.64 |
| 17.1524 | 763.78 | 0.2047 | 5.17 | 35.22 |
| 17.5174 | 1007.82 | 0.1535 | 5.06 | 46.47 |
| 18.1940 | 549.55 | 0.1791 | 4.88 | 25.34 |
| 19.2689 | 736.41 | 0.1535 | 4.61 | 33.96 |
| 20.4316 | 925.09 | 0.1535 | 4.35 | 42.66 |
| 21.5116 | 492.11 | 0.1535 | 4.13 | 22.69 |
| 23.0206 | 235.74 | 0.3582 | 3.86 | 10.87 |
| 23.4450 | 369.75 | 0.2047 | 3.79 | 17.05 |
| 25.2739 | 619.45 | 0.1535 | 3.52 | 28.56 |
| 25.6773 | 513.60 | 0.1535 | 3.47 | 23.68 |
| 29.4554 | 99.09 | 0.3070 | 3.03 | 4.57 |
| 32.4112 | 70.15 | 0.3070 | 2.76 | 3.23 |

The crystal form D of the methanesulfonate was obtained by slurrying about 500 mg of the free starting sample 2 and 1.5 molar amount of methanesulfonic acid in EtOH at room temperature for about 2 days, centrifuging to separate the solid sample, and then drying under vacuum at 50 °C. The XRPD overlay of the crystal forms A-D of the methanesulfonate is shown in FIG. 102a. The XRPD of the crystal form D of the methanesulfonate is shown in FIG. 102b. The TGA/DSC results of the crystal form D of the methanesulfonate (FIG. 103) show that the sample had a weight loss of 2.62% when heated to 150 °C, and 2 endothermic peaks at 40.3 °C and 156.6 °C (peak temperatures). The ¹H NMR results (FIG. 104) show that methanesulfonic acid and the free crystal form in the sample were in a molar ratio of 1.1, and EtOH and the free crystal form were in a molar ratio of 0.05 (about 0.3 wt%). The XRPD analysis data of the crystal form D of the methanesulfonate are shown in Table B2-16 below:

**Table B2-16. XRPD diffraction peak data of crystal form D of methanesulfonate**

| **Pos. [°2θ]** | **Height [cts]** | **FWHM Left [°2θ]** | **d-spacing [Å]** | **Rel. Int. [%]** |
|---|---|---|---|---|
| 6.3734 | 71.55 | 0.3070 | 13.87 | 8.89 |
| 7.3614 | 393.73 | 0.0768 | 12.01 | 48.89 |
| 8.5975 | 203.32 | 0.0768 | 10.29 | 25.25 |
| 9.8950 | 82.67 | 0.1535 | 8.94 | 10.27 |
| 11.1942 | 187.62 | 0.0768 | 7.90 | 23.30 |
| 12.9868 | 211.88 | 0.0768 | 6.82 | 26.31 |
| 13.4101 | 154.23 | 0.1279 | 6.60 | 19.15 |
| 14.7295 | 526.12 | 0.0768 | 6.01 | 65.34 |
| 14.9968 | 324.22 | 0.1023 | 5.91 | 40.26 |
| 15.9124 | 111.92 | 0.3070 | 5.57 | 13.90 |
| 17.0139 | 508.92 | 0.1023 | 5.21 | 63.20 |
| 17.2434 | 660.72 | 0.0768 | 5.14 | 82.05 |
| 17.5216 | 406.19 | 0.1023 | 5.06 | 50.44 |
| 18.0167 | 214.13 | 0.1023 | 4.92 | 26.59 |
| 18.3980 | 211.64 | 0.1279 | 4.82 | 26.28 |
| 18.7631 | 259.10 | 0.0768 | 4.73 | 32.18 |
| 19.2452 | 805.25 | 0.1279 | 4.61 | 100.00 |
| 19.8274 | 328.14 | 0.0768 | 4.48 | 40.75 |
| 20.3331 | 389.79 | 0.1279 | 4.37 | 48.41 |
| 21.8273 | 127.79 | 0.1535 | 4.07 | 15.87 |
| 22.4894 | 137.68 | 0.1535 | 3.95 | 17.10 |
| 23.0593 | 207.07 | 0.1279 | 3.86 | 25.72 |
| 24.4152 | 90.16 | 0.1535 | 3.65 | 11.20 |
| 25.3625 | 245.07 | 0.1023 | 3.51 | 30.43 |

### Example 22. Crystal forms A/B of Ethanesulfonate

The crystal forms A/B of the ethanesulfonate were obtained by slurrying the starting samples 1 of the compound of formula I and an equimolar amount of ethanesulfonic acid in ethanol and MIBK, respectively, at room temperature for about 3 days, centrifuging to separate the solid samples, and then drying under vacuum at 50 °C. The XRPD overlay of the ethanesulfonate samples is shown in FIG. 54.

The XRPD results of the crystal form A of the ethanesulfonate are shown in FIG. 55. The TGA/DSC results are listed in FIG. 56. The TGA results show that the sample had a weight loss of 8.29% when heated to 150 °C; the DSC results show that the sample had 2 endothermic peaks at 36.3 °C and 141.5 °C (peak temperatures). The ¹H NMR results, as shown in FIG. 57, show that ethanesulfonic acid and the free crystal form in the sample were in a molar ratio of 1.1, the residual solvent EtOH and the free crystal form were in a molar ratio of 0.46 (about 2.7 wt%), and MIBK and the free crystal form were in a molar ratio of 0.60 (about 7.6 wt%). The XRPD analysis data of the crystal form A of the ethanesulfonate are shown in Table B2-17 below:

**Table B2-17. XRPD diffraction peak data of crystal form A of ethanesulfonate**

| Pos. [°2θ] | Height [cts] | FWHM Left [°2θ] | d-spacing [Å] | Rel. Int. [%] |
|---|---|---|---|---|
| 7.4322 | 3230.01 | 0.3070 | 11.89 | 100.00 |
| 10.3920 | 136.71 | 0.5117 | 8.51 | 4.23 |
| 13.3770 | 107.66 | 0.8187 | 6.62 | 3.33 |
| 14.4406 | 202.95 | 0.8187 | 6.13 | 6.28 |
| 14.8852 | 345.99 | 0.2047 | 5.95 | 10.71 |
| 16.6572 | 443.19 | 0.5117 | 5.32 | 13.72 |
| 17.5439 | 424.23 | 0.1535 | 5.06 | 13.13 |
| 20.0726 | 304.16 | 0.1535 | 4.42 | 9.42 |
| 22.0062 | 207.95 | 0.8187 | 4.04 | 6.44 |

The XRPD results of the crystal form B of the ethanesulfonate are shown in FIG. 58a. The TGA/DSC results are listed in FIG. 59. The TGA results show that the sample had a weight loss of 6.30% when heated to 150 °C; the DSC results show that the sample had 2 endothermic peaks at 107.1 °C and 145.3 °C (peak temperatures). The ¹H NMR results, as shown in FIG. 60, show that ethanesulfonic acid and the free crystal form in the sample were in a molar ratio of 1.1, and the residual solvent MIBK and the free crystal form were in a molar ratio of 0.35 (about 4.7 wt%). The XRPD analysis data of the crystal form B of the ethanesulfonate are shown in Table B2-18 below:

**Table B2-18. XRPD diffraction peak data of crystal form B of ethanesulfonate**

| Pos. [°2θ] | Height [cts] | FWHM Left [°2θ] | d-spacing [Å] | Rel. Int. [%] |
|---|---|---|---|---|
| 4.9173 | 2061.73 | 0.1535 | 17.97 | 100.00 |
| 10.3467 | 189.55 | 0.3582 | 8.55 | 9.19 |
| 11.9022 | 592.20 | 0.1791 | 7.44 | 28.72 |
| 13.3498 | 116.40 | 0.2558 | 6.63 | 5.65 |
| 13.9472 | 141.22 | 0.1535 | 6.35 | 6.85 |
| 14.8131 | 409.36 | 0.1023 | 5.98 | 19.86 |
| 15.3679 | 744.24 | 0.1535 | 5.77 | 36.10 |
| 16.5326 | 162.41 | 0.2047 | 5.36 | 7.88 |
| 17.7077 | 1005.68 | 0.1791 | 5.01 | 48.78 |
| 18.9091 | 1268.33 | 0.1279 | 4.69 | 61.52 |
| 19.5992 | 752.75 | 0.1279 | 4.53 | 36.51 |
| 19.9469 | 438.39 | 0.1023 | 4.45 | 21.26 |
| 20.7324 | 724.55 | 0.1535 | 4.28 | 35.14 |
| 21.2340 | 119.32 | 0.2558 | 4.18 | 5.79 |
| 22.2822 | 468.52 | 0.1535 | 3.99 | 22.72 |
| 23.7415 | 229.80 | 0.1279 | 3.75 | 11.15 |
| 24.6012 | 603.74 | 0.2303 | 3.62 | 29.28 |
| 25.4858 | 214.80 | 0.2558 | 3.50 | 10.42 |
| 27.0042 | 352.62 | 0.1791 | 3.30 | 17.10 |
| 27.7863 | 127.51 | 0.3582 | 3.21 | 6.18 |
| 29.2924 | 81.16 | 0.3070 | 3.05 | 3.94 |
| 29.9997 | 108.87 | 0.3070 | 2.98 | 5.28 |
| 31.9631 | 108.67 | 0.3070 | 2.80 | 5.27 |

### Crystal form B of ethanesulfonate prepared repeatedly:

A sample of the crystal form B of the ethanesulfonate prepared repeatedly was obtained by slurrying about 500 mg of the free starting sample 2 and an equimolar amount of ethanesulfonic acid in MIBK at room temperature for about 4 days, centrifuging to separate the solid sample, and then drying under vacuum at 50 °C. The XRPD results (FIG. 58b) show that the sample prepared repeatedly was the crystal form B of the ethanesulfonate.

**Example 23. Preparation and Screening II of Salt Forms of Compound of Formula I**: Samples of the crystal form A of the hydrochloride prepared repeatedly (described in Example 16), the crystal form D of the methanesulfonate (described in Example 21), and the crystal form B of the ethanesulfonate prepared repeatedly (described in Example 22) were used as starting materials, and different solvent systems were selected for suspension stirring experiments at room temperature and 50 °C to select crystal form samples with relatively good solid state properties. The experimental procedures were as follows: About 20 mg of each sample of the corresponding strong acid salt was weighed out and added to an HPLC vial, and 0.5 mL of each of the solvents listed in Tables B2-19 to B2-21 were added. The resulting suspensions were placed at the corresponding temperature, magnetically stirred for about 3 days, and then centrifuged to separate the solids. The XRPD analysis was performed. The experimental results are summarized in Tables B2-19 to B2-21, and the results show that in the polymorph screening, a total of 12 crystal forms were observed for the hydrochloride, which were crystal forms A/B/D-M of the hydrochloride; a total of 12 crystal forms were observed for the methanesulfonate, which were crystal forms A/E-O of the methanesulfonate; and a total of 5 crystal forms were observed for the ethanesulfonate, which were crystal forms B-F of the ethanesulfonate. Representative crystal form samples with a relatively large number of times of obtaining in various solvent systems were selected for TGA/DSC characterization, and the salt-forming molar ratios were determined by ¹H NMR or HPLC/IC. The characterization results are summarized in Table B-22.

**Table B2-19. Summary of suspension stirring experiment results for hydrochloride**

| **Temperature** | **Solvent (v/v)** | **Result** |
|---|---|---|
| | MeOH | Crystal form J of hydrochloride |
| | EtOH | Crystal form A of hydrochloride |
| | Acetone | Crystal form K of hydrochloride |
| | MIBK | Crystal form B of hydrochloride |
| | MeOAc | Crystal form D of hydrochloride |
| RT | EtOAc | Crystal form H of hydrochloride |
| | ACN | Crystal form L of hydrochloride |
| | DCM | Crystal form I of hydrochloride |
| | MTBE | Low crystallinity |
| | THF | Crystal form M of hydrochloride |
| | Toluene | Crystal form H of hydrochloride |
| | H₂O | Free crystal form D |
| 50 °C | EtOH | Crystal form A of hydrochloride |
| | CHCl₃ | Crystal form D of hydrochloride (peak shift) |
| | MEK/H₂O (19:1) | Crystal form B of hydrochloride |
| | n-Heptane | Crystal form H of hydrochloride |
| | 1,4-Dioxane | Crystal form G of hydrochloride |
| | CPME | Crystal form H of hydrochloride |
| | 2-MeTHF | Crystal form E of hydrochloride |
| | DMSO/IPAc (1:9) | Crystal form F of hydrochloride |

**Table B2-20. Summary of suspension stirring experiment results for methanesulfonate**

| **Temperature** | **Solvent (v/v)** | **Result** |
|---|---|---|
| | MeOH/IPE (1:4) | Crystal form O of methanesulfonate |
| | EtOH | Crystal form H of methanesulfonate |
| | Acetone | Crystal form F of methanesulfonate |
| | MEK | Crystal form E of methanesulfonate |
| | Ethyl formate | Crystal form I of methanesulfonate |
| RT | ACN | Crystal form J of methanesulfonate |
| | DCM/MTBE (1:4) | Crystal form A of methanesulfonate |
| | THF | Low crystallinity |
| | Anisole | Crystal form K of methanesulfonate |
| | DMSO/EtOAc (1:9) | Crystal form L of methanesulfonate |
| | 1,4-Dioxane/H₂O (9:1) | Crystal form M of methanesulfonate |
| | t-BuOH | Crystal form N of methanesulfonate |
| | IPA/n-heptane (1:2) | Crystal form O of methanesulfonate |
| | Acetone/IPE (1:4) | Crystal form E of methanesulfonate |
| | CHCl₃/n-heptane (1:1) | Crystal form F of methanesulfonate |
| 50 °C | MIBK | Crystal form E of methanesulfonate |
| | CPME | Crystal form E of methanesulfonate |
| | 2-MeTHF/n-heptane (1:1) | Crystal form P of methanesulfonate* |
| | IPAc | Crystal form E of methanesulfonate |
| | Toluene | Crystal form G of methanesulfonate |

| | | |
|---|---|---|
| *: The gelled sample was observed and was dark brown. | | |

**Table B2-21. Summary of suspension stirring experiment results for ethanesulfonate**

| **Temperature** | **Solvent (v/v)** | **Result** |
|---|---|---|
| | MeOH/EtOAc (1:4) | Crystal form B of ethanesulfonate (peak shift) |
| | EtOH | Crystal form D of ethanesulfonate |
| | Acetone | Crystal form E of ethanesulfonate |
| | MIBK | Crystal form B of ethanesulfonate |
| | MeOAc | Crystal form B of ethanesulfonate |
| RT | IPAc | Crystal form B of ethanesulfonate |
| | ACN | Crystal form D of ethanesulfonate |
| | DCM | Crystal form D of ethanesulfonate (peak shift) |
| | THF/H₂O (9:1) | Crystal form F of ethanesulfonate |
| | 2-MeTHF | Crystal form B of ethanesulfonate |
| | MTBE | Crystal form B of ethanesulfonate (peak shift) |
| | DSMO/CHCl₃ (1:4) | Amorphous form |
| | n-Heptane | Crystal form B of ethanesulfonate |
| | IPA | Crystal form C of ethanesulfonate |
| | MEK | Crystal form B of ethanesulfonate |
| 50 °C | EtOAc | Crystal form B of ethanesulfonate (peak shift) |
| | ACN | Crystal form D of ethanesulfonate |
| | CPME | Crystal form B of ethanesulfonate |
| | Toluene | Crystal form B of ethanesulfonate |
| | 1,4-Dioxane/H₂O (9:1) | Black clear solution |

**Table B2-22. Summary of characterization results of salt forms obtained by screening**

| **Salt form** | **Crystal form** | **TGA weight loss (%)** | **DSC endothermic signal (°C, peak temperature)** | **Molar ratio^{#} (acid/base)** | **Solvent residue^{#} (wt%)** |
|---|---|---|---|---|---|
| Hydrochloride | Crystal form H | 7.61 (170 °C) | 155.5, 191.5 | 1.3 | ND |
| | Crystal form H | 4.50 (180 °C) | 99.3, 187.3 | 1.3 | ND |
| | Crystal form E | 0.85 (150 °C) | 198.2 | 1.0 | ND |
| Methanesulfonate | Crystal form F | 2.18 (160 °C) | 154.8, 195.6 | 1.0 | 0.8 (Acetone) |
| | Crystal form O | 0.17 (150 °C) | 185.9, 194.8 | 1.0 | ND |
| | | | | | 2.7 (MEK) |
| Ethanesulfonate | Crystal form B | 11.54 (120 °C) | 51.7, 102.4, 144.8 | 1.1 | 3.8 (MIBK) |
| | Crystal form B | 7.21 (150 °C) | 56.8, 102.6, 133.7, 145.6 | 1.1 | 4.1 (CPME) |
| | | | | | 2.5 (MIBK) |
| | Crystal form D | 1.70 (150 °C) | 55.5, 91.6^, 142.4, 184.2 | 1.0 | ND |
| | Crystal form E | 5.25 (110 °C) | 48.4, 104.0, 188.3 | 1.1 | 4.2 (Acetone) |

| | | | | | |
|---|---|---|---|---|---|
| ^{#}: The ratio or value was obtained by ¹H NMR or HPLC/IC calculation; ND: not detected; ^: exothermic peak. | | | | | |

### Example 24. Crystal Forms D-M of Hydrochloride

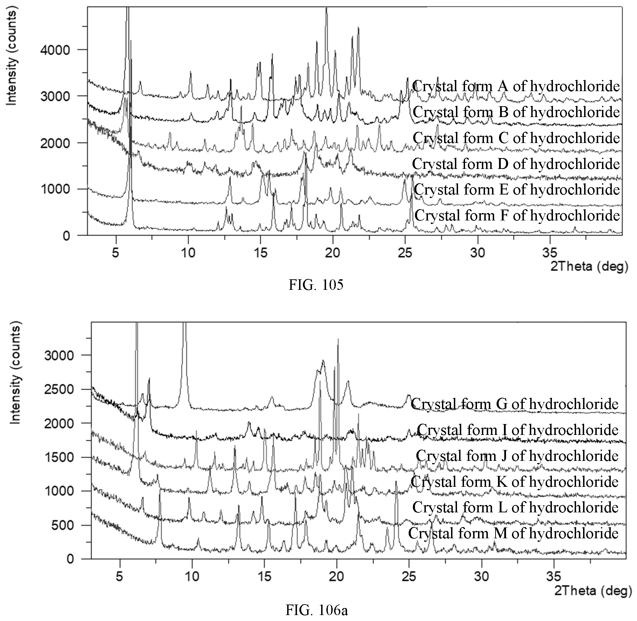

The crystal form D of the hydrochloride was obtained by slurrying the sample of the crystal form A of the hydrochloride prepared repeatedly in MeOAc at room temperature for about 6 days. The crystal forms E/F/G of the hydrochloride were obtained by slurrying the samples of the crystal form A of the hydrochloride prepared repeatedly in 2-MeTHF, DMSO/IPAc (1:9, v/v), and 1,4-dioxane, respectively, at 50 °C for about 3 days. The crystal form H of the hydrochloride was obtained by slurrying the sample of the crystal form A of the hydrochloride prepared repeatedly in toluene at room temperature for about 6 days, centrifuging to separate the solid, and then air-drying in an open container at room temperature. The crystal forms I/J/K/L/M of the hydrochloride were obtained by slurrying the samples of the crystal form A of the hydrochloride prepared repeatedly in DCM/MeOH/Acetone/ACN/THF, respectively, at room temperature for about 6 days. The XRPD overlays of the samples of the different crystal forms of the hydrochloride are shown in FIGs. 105 and 106a.

The TGA/DSC results of the sample of the crystal form H of the hydrochloride (obtained by slurrying in toluene) are shown in FIG. 108. The TGA results show that the sample had a weight loss of 7.61% when heated to 170 °C, and the DSC results show that the sample had 2 endothermic peaks at 155.5 °C and 191.5 °C (peak temperatures). The ¹H NMR results, as shown in FIG. 109, show that no toluene solvent residue was found in the sample. The HPLC/IC results show that in the sample of the crystal form H of the hydrochloride, hydrochloric acid and the free crystal form were in a molar ratio of 1.3.

The XRPD patterns of the crystal forms D, E, and F of the hydrochloride are shown in FIGs. 107, 110, and 111, respectively, and the XRPD analysis data are shown in Tables B2-23 to B2-25 below, respectively. The XRPD patterns of the crystal forms G, H, I, J, K, L, and M of the hydrochloride are shown in FIGs. 106b, 106c, 106d, 106e, 106f, 106g, and 106h, respectively, and the XRPD analysis data are shown in Tables B2-26 to B2-32 below, respectively.

**Table B2-23. XRPD diffraction peak data of crystal form D of hydrochloride**

| **Pos. [°2θ]** | **Height [cts]** | **FWHM Left [°2θ]** | **d-spacing [Å]** | **Rel. Int. [%]** |
|---|---|---|---|---|
| 6.5782 | 101.90 | 0.1535 | 13.44 | 35.49 |
| 9.9619 | 89.04 | 0.2047 | 8.88 | 31.01 |
| 11.1481 | 95.70 | 0.1535 | 7.94 | 33.33 |
| 11.8655 | 78.23 | 0.1535 | 7.46 | 27.25 |
| 12.8040 | 43.52 | 0.3070 | 6.91 | 15.16 |
| 14.4539 | 122.00 | 0.0768 | 6.13 | 42.49 |
| 14.6720 | 144.74 | 0.1279 | 6.04 | 50.41 |
| 18.0560 | 221.44 | 0.2047 | 4.91 | 77.12 |
| 18.7688 | 287.14 | 0.1535 | 4.73 | 100.00 |
| 20.3323 | 200.00 | 0.1791 | 4.37 | 69.65 |
| 21.2274 | 273.02 | 0.1791 | 4.19 | 95.08 |
| 26.6683 | 62.88 | 0.2047 | 3.34 | 21.90 |
| 29.0358 | 49.28 | 0.2047 | 3.08 | 17.16 |

**Table B2-24. XRPD diffraction peak data of crystal form E of hydrochloride**

| **Pos. [°2θ]** | **Height [cts]** | **FWHM Left [°2θ]** | **d-spacing [Å]** | **Rel. Int. [%]** |
|---|---|---|---|---|
| 5.9750 | 2071.71 | 0.1023 | 14.79 | 100.00 |
| 12.8980 | 570.07 | 0.1023 | 6.86 | 27.52 |
| 13.7925 | 136.53 | 0.0768 | 6.42 | 6.59 |
| 15.0507 | 514.26 | 0.1535 | 5.89 | 24.82 |
| 15.2093 | 632.05 | 0.1023 | 5.83 | 30.51 |
| 15.6063 | 741.42 | 0.1023 | 5.68 | 35.79 |
| 16.0284 | 301.55 | 0.1023 | 5.53 | 14.56 |
| 17.8054 | 509.52 | 0.1023 | 4.98 | 24.59 |
| 17.9714 | 621.82 | 0.1279 | 4.94 | 30.01 |
| 18.9298 | 135.56 | 0.1535 | 4.69 | 6.54 |
| 19.8405 | 356.32 | 0.1535 | 4.47 | 17.20 |
| 20.5435 | 339.78 | 0.1535 | 4.32 | 16.40 |
| 21.0218 | 109.37 | 0.2558 | 4.23 | 5.28 |
| 21.9935 | 92.72 | 0.2047 | 4.04 | 4.48 |
| 22.5488 | 164.64 | 0.3070 | 3.94 | 7.95 |
| 24.9674 | 530.20 | 0.2558 | 3.57 | 25.59 |
| 25.4838 | 649.32 | 0.1279 | 3.50 | 31.34 |
| 26.1257 | 188.94 | 0.1535 | 3.41 | 9.12 |
| 27.4268 | 139.63 | 0.2047 | 3.25 | 6.74 |
| 29.8322 | 83.38 | 0.3070 | 3.00 | 4.02 |
| 30.4635 | 86.32 | 0.1535 | 2.93 | 4.17 |
| 31.7533 | 50.22 | 0.2558 | 2.82 | 2.42 |

**Table B2-25. XRPD diffraction peak data of crystal form F of hydrochloride**

| **Pos. [°2θ]** | **Height [cts]** | **FWHM Left [°2θ]** | **d-spacing [Å]** | **Rel. Int. [%]** |
|---|---|---|---|---|
| 6.0385 | 3842.91 | 0.0768 | 14.64 | 100.00 |
| 12.0772 | 175.24 | 0.0768 | 7.33 | 4.56 |
| 12.6362 | 520.65 | 0.1023 | 7.01 | 13.55 |
| 13.0399 | 370.21 | 0.0768 | 6.79 | 9.63 |
| 13.5971 | 99.67 | 0.0768 | 6.51 | 2.59 |
| 14.9525 | 160.18 | 0.1023 | 5.93 | 4.17 |
| 15.9019 | 834.30 | 0.1023 | 5.57 | 21.71 |
| 16.8142 | 230.07 | 0.0768 | 5.27 | 5.99 |
| 17.1363 | 565.88 | 0.1023 | 5.17 | 14.73 |
| 18.1478 | 1636.88 | 0.1023 | 4.89 | 42.59 |
| 18.4776 | 213.50 | 0.0768 | 4.80 | 5.56 |
| 18.8427 | 374.21 | 0.1023 | 4.71 | 9.74 |
| 19.3354 | 197.55 | 0.1279 | 4.59 | 5.14 |
| 20.5749 | 603.22 | 0.1023 | 4.32 | 15.70 |
| 21.3694 | 211.34 | 0.1023 | 4.16 | 5.50 |
| 21.8092 | 358.65 | 0.1023 | 4.08 | 9.33 |
| 23.2223 | 88.64 | 0.2047 | 3.83 | 2.31 |
| 25.1076 | 243.47 | 0.1279 | 3.55 | 6.34 |
| 25.4190 | 771.77 | 0.1535 | 3.50 | 20.08 |
| 27.1737 | 88.10 | 0.2047 | 3.28 | 2.29 |
| 27.8200 | 149.15 | 0.1791 | 3.21 | 3.88 |
| 28.2136 | 173.43 | 0.1535 | 3.16 | 4.51 |
| 28.9299 | 50.24 | 0.5117 | 3.09 | 1.31 |
| 29.9049 | 148.44 | 0.1023 | 2.99 | 3.86 |
| 31.9291 | 37.37 | 0.3070 | 2.80 | 0.97 |
| 34.1705 | 40.31 | 0.3070 | 2.62 | 1.05 |
| 35.2232 | 33.01 | 0.3070 | 2.55 | 0.86 |
| 36.7164 | 125.68 | 0.0768 | 2.45 | 3.27 |
| 39.1368 | 50.23 | 0.1535 | 2.30 | 1.31 |

**Table B2-26. XRPD diffraction peak data of crystal form G of hydrochloride**

| **Pos. [°2θ]** | **Height [cts]** | **FWHM Left [°2θ]** | **d-spacing [Å]** | **Rel. Int. [%]** |
|---|---|---|---|---|
| 6.5684 | 280.97 | 0.1535 | 13.46 | 11.31 |
| 7.0798 | 247.13 | 0.1023 | 12.49 | 9.95 |
| 9.4719 | 2483.94 | 0.2303 | 9.34 | 100.00 |
| 13.6772 | 54.16 | 0.3070 | 6.47 | 2.18 |
| 14.4725 | 100.12 | 0.1535 | 6.12 | 4.03 |
| 15.5605 | 273.80 | 0.2047 | 5.69 | 11.02 |
| 18.6112 | 782.03 | 0.2558 | 4.77 | 31.48 |
| 19.0898 | 1054.92 | 0.2558 | 4.65 | 42.47 |
| 20.7520 | 606.13 | 0.1791 | 4.28 | 24.40 |
| 22.1933 | 151.33 | 0.8187 | 4.01 | 6.09 |
| 24.9637 | 335.34 | 0.2558 | 3.57 | 13.50 |
| 28.7471 | 139.19 | 0.4093 | 3.11 | 5.60 |

**Table B2-27. XRPD diffraction peak data of crystal form H of hydrochloride**

| **Pos. [°2θ]** | **Height [cts]** | **FWHM Left [°2θ]** | **d-spacing [Å]** | **Rel. Int. [%]** |
|---|---|---|---|---|
| 7.5428 | 192.21 | 0.1023 | 11.72 | 29.62 |
| 8.4236 | 164.35 | 0.1023 | 10.50 | 25.33 |
| 10.0644 | 99.44 | 0.0768 | 8.79 | 15.33 |
| 10.4677 | 93.88 | 0.1023 | 8.45 | 14.47 |
| 12.0841 | 143.57 | 0.0768 | 7.32 | 22.13 |
| 12.4381 | 184.11 | 0.1023 | 7.12 | 28.38 |
| 12.9273 | 299.35 | 0.1023 | 6.85 | 46.14 |
| 13.5430 | 107.13 | 0.1023 | 6.54 | 16.51 |
| 15.1559 | 286.19 | 0.1023 | 5.85 | 44.11 |
| 15.5659 | 292.13 | 0.1023 | 5.69 | 45.03 |
| 16.2484 | 65.99 | 0.1535 | 5.46 | 10.17 |
| 16.8381 | 210.84 | 0.0768 | 5.27 | 32.50 |
| 17.6473 | 434.93 | 0.1023 | 5.03 | 67.03 |
| 18.2972 | 648.82 | 0.1279 | 4.85 | 100.00 |
| 18.7344 | 159.14 | 0.1023 | 4.74 | 24.53 |
| 19.1721 | 194.29 | 0.0768 | 4.63 | 29.95 |
| 19.4651 | 563.49 | 0.1023 | 4.56 | 86.85 |
| 19.7972 | 643.40 | 0.1535 | 4.48 | 99.16 |
| 20.4603 | 539.63 | 0.1279 | 4.34 | 83.17 |
| 20.7147 | 235.69 | 0.0768 | 4.29 | 36.33 |
| 20.9217 | 170.95 | 0.0768 | 4.25 | 26.35 |
| 21.5046 | 176.46 | 0.0768 | 4.13 | 27.20 |
| 21.7941 | 103.56 | 0.1023 | 4.08 | 15.96 |
| 22.4391 | 117.38 | 0.0768 | 3.96 | 18.09 |
| 22.7460 | 85.75 | 0.1791 | 3.91 | 13.22 |
| 23.8221 | 100.31 | 0.0768 | 3.74 | 15.46 |
| 25.3590 | 123.34 | 0.0768 | 3.51 | 19.01 |
| 25.9795 | 80.65 | 0.1535 | 3.43 | 12.43 |
| 26.4453 | 54.89 | 0.2047 | 3.37 | 8.46 |
| 27.1720 | 46.81 | 0.3070 | 3.28 | 7.21 |
| 27.6629 | 92.62 | 0.1279 | 3.22 | 14.27 |
| 28.6111 | 70.74 | 0.2047 | 3.12 | 10.90 |
| 29.0095 | 79.79 | 0.1791 | 3.08 | 12.30 |
| 30.6017 | 65.36 | 0.2558 | 2.92 | 10.07 |

**Table B2-28. XRPD diffraction peak data of crystal form I of hydrochloride**

| **Pos. [°2θ]** | **Height [cts]** | **FWHM Left [°2θ]** | **d-spacing [Å]** | **Rel. Int. [%]** |
|---|---|---|---|---|
| 6.2065 | 142.14 | 0.1535 | 14.24 | 22.44 |
| 7.0236 | 633.51 | 0.1023 | 12.59 | 100.00 |
| 11.6337 | 75.55 | 0.1535 | 7.61 | 11.93 |
| 13.8915 | 207.31 | 0.1535 | 6.38 | 32.72 |
| 14.6038 | 148.71 | 0.1791 | 6.07 | 23.47 |
| 15.6403 | 86.85 | 0.1535 | 5.67 | 13.71 |
| 17.0255 | 53.22 | 0.3070 | 5.21 | 8.40 |
| 17.7770 | 99.81 | 0.2047 | 4.99 | 15.76 |
| 19.2571 | 65.26 | 0.3070 | 4.61 | 10.30 |
| 20.8063 | 110.61 | 0.2558 | 4.27 | 17.46 |
| 21.1874 | 112.54 | 0.1535 | 4.19 | 17.76 |
| 23.6537 | 50.74 | 0.2558 | 3.76 | 8.01 |
| 24.9901 | 150.65 | 0.1791 | 3.56 | 23.78 |
| 25.4883 | 82.62 | 0.8187 | 3.49 | 13.04 |

**Table B2-29. XRPD diffraction peak data of crystal form J of hydrochloride**

| **Pos. [°2θ]** | **Height [cts]** | **FWHM Left [°2θ]** | **d-spacing [Å]** | **Rel. Int. [%]** |
|---|---|---|---|---|
| 6.7191 | 116.82 | 0.1535 | 13.16 | 6.23 |
| 9.4898 | 153.41 | 0.1023 | 9.32 | 8.18 |
| 10.3031 | 559.65 | 0.0768 | 8.59 | 29.84 |
| 11.5598 | 260.07 | 0.0768 | 7.66 | 13.87 |
| 12.9670 | 359.62 | 0.0768 | 6.83 | 19.17 |
| 13.7721 | 205.03 | 0.1023 | 6.43 | 10.93 |
| 14.2248 | 129.28 | 0.1535 | 6.23 | 6.89 |
| 15.0739 | 581.66 | 0.1535 | 5.88 | 31.01 |
| 17.8940 | 128.92 | 0.1535 | 4.96 | 6.87 |
| 18.5121 | 520.97 | 0.1023 | 4.79 | 27.77 |
| 18.8389 | 1247.88 | 0.0768 | 4.71 | 66.53 |
| 19.5450 | 190.15 | 0.0768 | 4.54 | 10.14 |
| 19.8236 | 1431.70 | 0.1023 | 4.48 | 76.33 |
| 20.0800 | 1875.71 | 0.1023 | 4.42 | 100.00 |
| 21.0834 | 212.39 | 0.1279 | 4.21 | 11.32 |
| 21.4914 | 851.13 | 0.1023 | 4.13 | 45.38 |
| 21.7526 | 334.95 | 0.0768 | 4.09 | 17.86 |
| 22.0336 | 511.13 | 0.0768 | 4.03 | 27.25 |
| 22.2164 | 371.22 | 0.1023 | 4.00 | 19.79 |
| 22.5454 | 266.03 | 0.1023 | 3.94 | 14.18 |
| 24.4676 | 64.17 | 0.3070 | 3.64 | 3.42 |
| 25.6500 | 186.69 | 0.1023 | 3.47 | 9.95 |
| 26.1804 | 139.00 | 0.1535 | 3.40 | 7.41 |
| 27.4986 | 174.02 | 0.2047 | 3.24 | 9.28 |
| 29.5851 | 63.41 | 0.3070 | 3.02 | 3.38 |
| 30.2463 | 235.84 | 0.1023 | 2.95 | 12.57 |
| 31.2643 | 59.40 | 0.3070 | 2.86 | 3.17 |
| 32.4691 | 74.12 | 0.1535 | 2.76 | 3.95 |
| 34.1280 | 81.01 | 0.3070 | 2.63 | 4.32 |

**Table B2-30. XRPD diffraction peak data of crystal form K of hydrochloride**

| **Pos. [°2θ]** | **Height [cts]** | **FWHM Left [°2θ]** | **d-spacing [Å]** | **Rel. Int. [%]** |
|---|---|---|---|---|
| 6.1719 | 3015.30 | 0.1279 | 14.32 | 100.00 |
| 7.6126 | 195.59 | 0.1023 | 11.61 | 6.49 |
| 11.2365 | 386.39 | 0.1279 | 7.87 | 12.81 |
| 12.9493 | 658.60 | 0.1023 | 6.84 | 21.84 |
| 13.9646 | 193.54 | 0.1023 | 6.34 | 6.42 |
| 15.6381 | 816.29 | 0.1023 | 5.67 | 27.07 |
| 16.6189 | 137.38 | 0.2047 | 5.33 | 4.56 |
| 17.1911 | 133.63 | 0.1535 | 5.16 | 4.43 |
| 17.8017 | 264.20 | 0.1279 | 4.98 | 8.76 |
| 18.5078 | 309.13 | 0.1535 | 4.79 | 10.25 |
| 19.8165 | 190.78 | 0.1535 | 4.48 | 6.33 |
| 20.5819 | 410.43 | 0.1791 | 4.32 | 13.61 |
| 21.7916 | 108.42 | 0.6140 | 4.08 | 3.60 |
| 22.5977 | 108.98 | 0.2047 | 3.93 | 3.61 |
| 25.3451 | 350.60 | 0.2047 | 3.51 | 11.63 |
| 26.0590 | 263.14 | 0.1535 | 3.42 | 8.73 |
| 26.3085 | 285.58 | 0.1535 | 3.39 | 9.47 |
| 30.7181 | 103.69 | 0.6140 | 2.91 | 3.44 |

**Table B2-31. XRPD diffraction peak data of crystal form L of hydrochloride**

| **Pos. [°2θ]** | **Height [cts]** | **FWHM Left [°2θ]** | **d-spacing [Å]** | **Rel. Int. [%]** |
|---|---|---|---|---|
| 7.7752 | 796.05 | 0.1279 | 11.37 | 75.37 |
| 10.4411 | 144.33 | 0.1535 | 8.47 | 13.66 |
| 13.2333 | 670.68 | 0.1279 | 6.69 | 63.50 |
| 13.8951 | 99.27 | 0.2047 | 6.37 | 9.40 |
| 15.3025 | 422.76 | 0.1535 | 5.79 | 40.02 |
| 16.3403 | 157.80 | 0.2047 | 5.42 | 14.94 |
| 17.1547 | 812.06 | 0.1023 | 5.17 | 76.88 |
| 17.8681 | 446.90 | 0.1535 | 4.96 | 42.31 |
| 19.2621 | 154.04 | 0.1535 | 4.61 | 14.58 |
| 19.9563 | 81.41 | 0.2558 | 4.45 | 7.71 |
| 21.4837 | 586.66 | 0.1023 | 4.14 | 55.54 |
| 22.4156 | 109.93 | 0.2558 | 3.97 | 10.41 |
| 23.4738 | 355.46 | 0.1279 | 3.79 | 33.65 |
| 24.0925 | 1056.24 | 0.1791 | 3.69 | 100.00 |
| 25.5726 | 158.58 | 0.1535 | 3.48 | 15.01 |
| 26.5641 | 435.40 | 0.1791 | 3.36 | 41.22 |
| 28.1186 | 130.20 | 0.2047 | 3.17 | 12.33 |
| 30.8899 | 150.82 | 0.1535 | 2.89 | 14.28 |
| 38.4894 | 45.34 | 0.3070 | 2.34 | 4.29 |

**Table B2-32. XRPD diffraction peak data of crystal form M of hydrochloride**

| **Pos. [°2θ]** | **Height [cts]** | **FWHM Left [°2θ]** | **d-spacing [Å]** | **Rel. Int. [%]** |
|---|---|---|---|---|
| 6.5546 | 229.91 | 0.1023 | 13.49 | 27.35 |
| 9.8040 | 353.16 | 0.1023 | 9.02 | 42.01 |
| 10.8235 | 154.43 | 0.1535 | 8.17 | 18.37 |
| 12.0038 | 180.99 | 0.1023 | 7.37 | 21.53 |
| 13.1333 | 147.76 | 0.1279 | 6.74 | 17.58 |
| 14.2374 | 186.49 | 0.1279 | 6.22 | 22.18 |
| 14.8344 | 407.52 | 0.1279 | 5.97 | 48.47 |
| 17.8676 | 181.94 | 0.2047 | 4.96 | 21.64 |
| 18.8157 | 729.86 | 0.1791 | 4.72 | 86.81 |
| 19.2503 | 357.36 | 0.1023 | 4.61 | 42.51 |
| 20.6275 | 597.95 | 0.1535 | 4.31 | 71.12 |
| 21.0461 | 840.72 | 0.1535 | 4.22 | 100.00 |
| 22.8294 | 95.10 | 0.3070 | 3.90 | 11.31 |
| 24.8213 | 64.99 | 0.4093 | 3.59 | 7.73 |
| 26.8393 | 123.14 | 0.1535 | 3.32 | 14.65 |
| 28.6992 | 127.39 | 0.2047 | 3.11 | 15.15 |
| 29.5703 | 92.79 | 0.5117 | 3.02 | 11.04 |
| 33.9685 | 59.15 | 0.3070 | 2.64 | 7.04 |

### Example 25. Crystal Forms E-O of Methanesulfonate

The crystal form E of the methanesulfonate was obtained by slurrying the sample of the crystal form D of the methanesulfonate in IPAc at 50 °C for about 3 days, centrifuging to separate the solid, and then air-drying in an open container at room temperature. The crystal form F of the methanesulfonate was obtained by slurrying the sample of the crystal form D of the methanesulfonate in acetone at room temperature for about 6 days, centrifuging to separate the solid, and then air-drying in an open container at room temperature. The crystal form G of the methanesulfonate was obtained by slurrying the sample of the crystal form D of the methanesulfonate in toluene at 50 °C for about 3 days. The crystal forms H/I/J/K/L of the methanesulfonate were obtained by slurrying the samples of the crystal form D of the methanesulfonate in EtOH/ethyl formate/ACN/anisole/DMSO/EtOAc (1:9, v/v), respectively, at room temperature for about 6 days. The crystal forms M/N of the methanesulfonate were obtained by slurrying the samples of the crystal form D of the methanesulfonate in 1,4-dioxane/H₂O (9:1, v/v) and t-BuOH, respectively, at room temperature for about 6 days. The crystal form O of the methanesulfonate was obtained by slurrying the sample of the crystal form D of the methanesulfonate in MeOH/IPE (1:4, v/v) at room temperature for about 6 days, centrifuging to separate the solid, and then air-drying in an open container at room temperature. The XRPD overlays of the samples of the different crystal forms of the methanesulfonate are shown in FIGs. 112a, 113a, and 114a.

The XRPD patterns of the crystal forms E and F of the methanesulfonate are shown in FIGs. 112b and 112c, respectively; the XRPD patterns of the crystal forms G, H, I, J, K, and L of the methanesulfonate are shown in FIGs. 113b to 113g, respectively; the XRPD patterns of the crystal forms M, N, and O of the methanesulfonate are shown in FIGs. 114b, 114c, and 114d, respectively. The XRPD analysis data of the crystal forms described above are shown in Tables B2-33 to B2-43 below, respectively.

**Table B2-33. XRPD diffraction peak data of crystal form E of methanesulfonate**

| **Pos. [°2θ]** | **Height [cts]** | **FWHM Left [°2θ]** | **d-spacing [Å]** | **Rel. Int. [%]** |
|---|---|---|---|---|
| 7.6948 | 3403.95 | 0.1023 | 11.49 | 61.18 |
| 11.0258 | 178.15 | 0.0768 | 8.02 | 3.20 |
| 12.4963 | 550.64 | 0.0768 | 7.08 | 9.90 |
| 13.5466 | 1776.38 | 0.1023 | 6.54 | 31.93 |
| 14.2591 | 207.52 | 0.0768 | 6.21 | 3.73 |
| 14.5860 | 726.93 | 0.1023 | 6.07 | 13.07 |
| 15.3154 | 106.38 | 0.1535 | 5.79 | 1.91 |
| 16.1727 | 714.93 | 0.1023 | 5.48 | 12.85 |
| 16.8137 | 251.14 | 0.0768 | 5.27 | 4.51 |
| 17.0486 | 212.60 | 0.1023 | 5.20 | 3.82 |
| 17.5309 | 894.35 | 0.1279 | 5.06 | 16.08 |
| 17.8641 | 870.18 | 0.1535 | 4.97 | 15.64 |
| 18.9557 | 5563.61 | 0.1023 | 4.68 | 100.00 |
| 19.4674 | 1554.67 | 0.1279 | 4.56 | 27.94 |
| 20.0144 | 1445.47 | 0.1023 | 4.44 | 25.98 |
| 20.3406 | 705.75 | 0.0768 | 4.37 | 12.69 |
| 20.8237 | 805.79 | 0.1023 | 4.27 | 14.48 |
| 21.6960 | 857.99 | 0.1279 | 4.10 | 15.42 |
| 22.3698 | 1337.52 | 0.1023 | 3.97 | 24.04 |
| 22.8750 | 243.85 | 0.1023 | 3.89 | 4.38 |
| 23.1733 | 248.95 | 0.1279 | 3.84 | 4.47 |
| 23.7738 | 1418.92 | 0.1279 | 3.74 | 25.50 |
| 25.1077 | 898.03 | 0.1279 | 3.55 | 16.14 |
| 25.3713 | 331.45 | 0.1023 | 3.51 | 5.96 |
| 25.7306 | 211.58 | 0.1023 | 3.46 | 3.80 |
| 26.1850 | 1586.72 | 0.1279 | 3.40 | 28.52 |
| 26.8900 | 224.44 | 0.1791 | 3.32 | 4.03 |
| 27.6964 | 176.18 | 0.1023 | 3.22 | 3.17 |
| 28.4667 | 200.78 | 0.1023 | 3.14 | 3.61 |
| 29.2032 | 723.02 | 0.1279 | 3.06 | 13.00 |
| 30.5281 | 520.33 | 0.1791 | 2.93 | 9.35 |
| 31.4140 | 440.01 | 0.1279 | 2.85 | 7.91 |
| 33.7897 | 278.52 | 0.1535 | 2.65 | 5.01 |
| 35.9511 | 83.79 | 0.3070 | 2.50 | 1.51 |

**Table B2-34. XRPD diffraction peak data of crystal form F of methanesulfonate**

| **Pos. [°2θ]** | **Height [cts]** | **FWHM Left [°2θ]** | **d-spacing [Å]** | **Rel. Int. [%]** |
|---|---|---|---|---|
| 5.0171 | 24.16 | 0.8187 | 17.61 | 1.30 |
| 7.8706 | 1864.80 | 0.1023 | 11.23 | 100.00 |
| 11.8701 | 56.31 | 0.1535 | 7.46 | 3.02 |
| 13.4221 | 48.03 | 0.1535 | 6.60 | 2.58 |
| 14.3209 | 40.77 | 0.3070 | 6.18 | 2.19 |
| 15.7726 | 446.88 | 0.1791 | 5.62 | 23.96 |
| 17.0258 | 210.24 | 0.6140 | 5.21 | 11.27 |
| 17.8165 | 399.38 | 0.3070 | 4.98 | 21.42 |
| 20.2728 | 173.07 | 0.1279 | 4.38 | 9.28 |
| 21.4207 | 111.14 | 0.1535 | 4.15 | 5.96 |
| 23.7446 | 162.10 | 0.2047 | 3.75 | 8.69 |
| 25.3530 | 117.37 | 0.3070 | 3.51 | 6.29 |
| 26.3257 | 62.59 | 0.1535 | 3.39 | 3.36 |

**Table B2-35. XRPD diffraction peak data of crystal form G of methanesulfonate**

| **Pos. [°2θ]** | **Height [cts]** | **FWHM Left [°2θ]** | **d-spacing [Å]** | **Rel. Int. [%]** |
|---|---|---|---|---|
| 8.6041 | 74.82 | 0.2047 | 10.28 | 12.14 |
| 10.1340 | 71.65 | 0.1535 | 8.73 | 11.63 |
| 11.2069 | 17.25 | 0.2047 | 7.90 | 2.80 |
| 12.6682 | 87.58 | 0.1535 | 6.99 | 14.21 |
| 13.4333 | 111.66 | 0.1535 | 6.59 | 18.12 |
| 14.4922 | 59.04 | 0.2047 | 6.11 | 9.58 |
| 15.4837 | 136.14 | 0.1279 | 5.72 | 22.09 |
| 16.1377 | 186.02 | 0.2047 | 5.49 | 30.19 |
| 16.5841 | 144.91 | 0.1535 | 5.35 | 23.52 |
| 17.7027 | 427.33 | 0.0768 | 5.01 | 69.35 |
| 18.1620 | 616.22 | 0.0768 | 4.88 | 100.00 |
| 19.1132 | 492.98 | 0.1535 | 4.64 | 80.00 |
| 21.0530 | 575.18 | 0.1535 | 4.22 | 93.34 |
| 22.0640 | 86.11 | 0.3070 | 4.03 | 13.97 |
| 24.3157 | 153.99 | 0.1535 | 3.66 | 24.99 |
| 24.8063 | 134.11 | 0.2047 | 3.59 | 21.76 |
| 25.5461 | 69.05 | 0.3070 | 3.49 | 11.21 |
| 26.2930 | 120.98 | 0.1535 | 3.39 | 19.63 |
| 26.9311 | 50.76 | 0.3070 | 3.31 | 8.24 |
| 27.7204 | 38.94 | 0.3070 | 3.22 | 6.32 |
| 28.8940 | 73.70 | 0.2047 | 3.09 | 11.96 |

**Table B2-36. XRPD diffraction peak data of crystal form H of methanesulfonate**

| **Pos. [°2θ]** | **Height [cts]** | **FWHM Left [°2θ]** | **d-spacing [Å]** | **Rel. Int. [%]** |
|---|---|---|---|---|
| 5.6472 | 172.00 | 0.1535 | 15.65 | 5.77 |
| 9.5858 | 2980.29 | 0.1023 | 9.23 | 100.00 |
| 9.8551 | 554.79 | 0.1023 | 8.98 | 18.62 |
| 12.8418 | 558.96 | 0.1023 | 6.89 | 18.76 |
| 15.2067 | 151.97 | 0.1023 | 5.83 | 5.10 |
| 16.4833 | 849.42 | 0.1023 | 5.38 | 28.50 |
| 17.2040 | 1650.86 | 0.1279 | 5.15 | 55.39 |
| 17.7099 | 323.41 | 0.1023 | 5.01 | 10.85 |
| 18.5860 | 203.24 | 0.1023 | 4.77 | 6.82 |
| 19.2029 | 177.05 | 0.1279 | 4.62 | 5.94 |
| 19.7648 | 2854.41 | 0.1279 | 4.49 | 95.78 |
| 21.8135 | 249.97 | 0.1023 | 4.07 | 8.39 |
| 22.2051 | 1030.25 | 0.1279 | 4.00 | 34.57 |
| 23.4137 | 144.55 | 0.1279 | 3.80 | 4.85 |
| 24.9851 | 159.46 | 0.1535 | 3.56 | 5.35 |
| 25.4281 | 338.74 | 0.1023 | 3.50 | 11.37 |
| 26.1391 | 493.61 | 0.1279 | 3.41 | 16.56 |
| 27.6138 | 101.55 | 0.3070 | 3.23 | 3.41 |
| 29.8198 | 323.38 | 0.1535 | 3.00 | 10.85 |
| 30.9209 | 60.63 | 0.3070 | 2.89 | 2.03 |
| 32.0327 | 62.10 | 0.3070 | 2.79 | 2.08 |
| 35.9179 | 55.67 | 0.3070 | 2.50 | 1.87 |

**Table B2-37. XRPD diffraction peak data of crystal form I of methanesulfonate**

| **Pos. [°2θ]** | **Height [cts]** | **FWHM Left [°2θ]** | **d-spacing [Å]** | **Rel. Int. [%]** |
|---|---|---|---|---|
| 7.9648 | 1728.01 | 0.1023 | 11.10 | 100.00 |
| 11.3739 | 170.04 | 0.1535 | 7.78 | 9.84 |
| 15.1578 | 174.15 | 0.2047 | 5.85 | 10.08 |
| 15.9556 | 713.07 | 0.1023 | 5.55 | 41.27 |
| 16.6385 | 166.43 | 0.2047 | 5.33 | 9.63 |
| 17.3872 | 478.16 | 0.1279 | 5.10 | 27.67 |
| 17.7652 | 862.27 | 0.1279 | 4.99 | 49.90 |
| 18.1048 | 558.28 | 0.1023 | 4.90 | 32.31 |
| 19.1430 | 88.48 | 0.3070 | 4.64 | 5.12 |
| 20.2628 | 167.08 | 0.1535 | 4.38 | 9.67 |
| 21.6954 | 132.29 | 0.1535 | 4.10 | 7.66 |
| 22.8525 | 223.51 | 0.1023 | 3.89 | 12.93 |
| 24.0274 | 350.90 | 0.1023 | 3.70 | 20.31 |
| 25.0167 | 148.97 | 0.1535 | 3.56 | 8.62 |
| 25.6562 | 361.00 | 0.1279 | 3.47 | 20.89 |

**Table B2-38. XRPD diffraction peak data of crystal form J of methanesulfonate**

| **Pos. [°2θ]** | **Height [cts]** | **FWHM Left [°2θ]** | **d-spacing [Å]** | **Rel. Int. [%]** |
|---|---|---|---|---|
| 8.6620 | 155.60 | 0.0768 | 10.21 | 30.90 |
| 10.0632 | 100.21 | 0.1023 | 8.79 | 19.90 |
| 11.4255 | 88.39 | 0.0768 | 7.74 | 17.55 |
| 13.5049 | 503.55 | 0.0768 | 6.56 | 100.00 |
| 14.5059 | 40.15 | 0.1535 | 6.11 | 7.97 |
| 15.1512 | 52.44 | 0.1023 | 5.85 | 10.41 |
| 16.1555 | 196.67 | 0.0768 | 5.49 | 39.06 |
| 17.5647 | 79.27 | 0.1023 | 5.05 | 15.74 |
| 18.1900 | 141.47 | 0.1279 | 4.88 | 28.09 |
| 19.7621 | 78.71 | 0.1023 | 4.49 | 15.63 |
| 20.5741 | 41.90 | 0.1023 | 4.32 | 8.32 |
| 21.9852 | 255.84 | 0.1023 | 4.04 | 50.81 |
| 22.2107 | 114.46 | 0.0768 | 4.00 | 22.73 |
| 22.9624 | 172.64 | 0.1023 | 3.87 | 34.29 |
| 24.6246 | 56.49 | 0.1023 | 3.62 | 11.22 |
| 30.6181 | 51.28 | 0.1535 | 2.92 | 10.18 |

**Table B2-39. XRPD diffraction peak data of crystal form K of methanesulfonate**

| **Pos. [°2θ]** | **Height [cts]** | **FWHM Left [°2θ]** | **d-spacing [Å]** | **Rel. Int. [%]** |
|---|---|---|---|---|
| 8.6859 | 95.89 | 0.1535 | 10.18 | 16.94 |
| 9.9129 | 45.27 | 0.3070 | 8.92 | 8.00 |
| 12.6815 | 25.30 | 0.1791 | 6.98 | 4.47 |
| 13.4500 | 47.73 | 0.1535 | 6.58 | 8.43 |
| 14.0343 | 46.22 | 0.3070 | 6.31 | 8.16 |
| 15.4362 | 265.57 | 0.1535 | 5.74 | 46.91 |
| 16.5219 | 79.74 | 0.1535 | 5.37 | 14.08 |
| 17.2898 | 251.09 | 0.1279 | 5.13 | 44.35 |
| 17.5619 | 244.88 | 0.1535 | 5.05 | 43.25 |
| 18.0642 | 187.07 | 0.1791 | 4.91 | 33.04 |
| 18.6931 | 307.17 | 0.1535 | 4.75 | 54.26 |
| 19.5256 | 212.95 | 0.1535 | 4.55 | 37.61 |
| 21.1887 | 566.17 | 0.1279 | 4.19 | 100.00 |
| 21.9878 | 171.27 | 0.3070 | 4.04 | 30.25 |
| 22.9735 | 91.91 | 0.2047 | 3.87 | 16.23 |
| 25.1553 | 337.38 | 0.1279 | 3.54 | 59.59 |
| 26.0704 | 59.83 | 0.3070 | 3.42 | 10.57 |
| 27.0875 | 39.37 | 0.3070 | 3.29 | 6.95 |
| 28.8383 | 67.75 | 0.1535 | 3.10 | 11.97 |
| 30.0689 | 61.62 | 0.1535 | 2.97 | 10.88 |
| 31.9147 | 42.71 | 0.1535 | 2.80 | 7.54 |
| 33.0164 | 23.42 | 0.3070 | 2.71 | 4.14 |

**Table B2-40. XRPD diffraction peak data of crystal form L of methanesulfonate**

| **Pos. [°2θ]** | **Height [cts]** | **FWHM Left [°2θ]** | **d-spacing [Å]** | **Rel. Int. [%]** |
|---|---|---|---|---|
| 8.0867 | 2881.27 | 0.1023 | 10.93 | 100.00 |
| 11.8848 | 144.08 | 0.0768 | 7.45 | 5.00 |
| 14.2858 | 34.86 | 0.8187 | 6.20 | 1.21 |
| 15.7466 | 63.87 | 0.1535 | 5.63 | 2.22 |
| 16.2087 | 71.20 | 0.2558 | 5.47 | 2.47 |
| 18.6275 | 215.59 | 0.1279 | 4.76 | 7.48 |
| 19.6478 | 383.55 | 0.1023 | 4.52 | 13.31 |
| 20.5586 | 93.64 | 0.2047 | 4.32 | 3.25 |
| 21.1470 | 221.06 | 0.0768 | 4.20 | 7.67 |
| 21.6729 | 149.83 | 0.0768 | 4.10 | 5.20 |
| 21.9361 | 152.18 | 0.1279 | 4.05 | 5.28 |
| 22.3422 | 59.56 | 0.1535 | 3.98 | 2.07 |
| 23.0719 | 89.31 | 0.1535 | 3.86 | 3.10 |
| 23.4214 | 89.49 | 0.1279 | 3.80 | 3.11 |
| 23.9149 | 56.12 | 0.1535 | 3.72 | 1.95 |
| 27.7310 | 113.48 | 0.1279 | 3.22 | 3.94 |
| 28.6368 | 69.98 | 0.1535 | 3.12 | 2.43 |
| 29.6831 | 121.52 | 0.1279 | 3.01 | 4.22 |
| 38.4394 | 53.87 | 0.1535 | 2.34 | 1.87 |

**Table B2-41. XRPD diffraction peak data of crystal form M of methanesulfonate**

| **Pos. [°2θ]** | **Height [cts]** | **FWHM Left [°2θ]** | **d-spacing [Å]** | **Rel. Int. [%]** |
|---|---|---|---|---|
| 6.0854 | 244.54 | 0.0768 | 14.52 | 37.13 |
| 8.6714 | 90.84 | 0.2047 | 10.20 | 13.79 |
| 9.8088 | 140.80 | 0.1023 | 9.02 | 21.38 |
| 12.3906 | 37.84 | 0.1535 | 7.14 | 5.75 |
| 13.3246 | 102.08 | 0.1023 | 6.65 | 15.50 |
| 13.8974 | 39.51 | 0.1535 | 6.37 | 6.00 |
| 15.6545 | 171.61 | 0.1023 | 5.66 | 26.06 |
| 16.0880 | 315.25 | 0.1023 | 5.51 | 47.86 |
| 16.2048 | 236.29 | 0.0768 | 5.47 | 35.87 |
| 16.8014 | 57.89 | 0.1279 | 5.28 | 8.79 |
| 17.4084 | 552.93 | 0.1023 | 5.09 | 83.95 |
| 17.6442 | 318.15 | 0.1023 | 5.03 | 48.30 |
| 18.2844 | 176.75 | 0.0768 | 4.85 | 26.83 |
| 18.6200 | 378.39 | 0.1023 | 4.77 | 57.45 |
| 18.8984 | 264.64 | 0.1023 | 4.70 | 40.18 |
| 19.7051 | 112.10 | 0.0768 | 4.51 | 17.02 |
| 20.6457 | 658.65 | 0.1535 | 4.30 | 100.00 |
| 21.0719 | 85.35 | 0.1535 | 4.22 | 12.96 |
| 22.1260 | 75.30 | 0.3070 | 4.02 | 11.43 |
| 22.9780 | 39.65 | 0.1535 | 3.87 | 6.02 |
| 24.4928 | 115.06 | 0.3582 | 3.63 | 17.47 |
| 25.3051 | 83.25 | 0.1535 | 3.52 | 12.64 |
| 26.3685 | 49.08 | 0.1535 | 3.38 | 7.45 |
| 28.9539 | 29.06 | 0.6140 | 3.08 | 4.41 |

**Table B2-42. XRPD diffraction peak data of crystal form N of methanesulfonate**

| **Pos. [°2θ]** | **Height [cts]** | **FWHM Left [°2θ]** | **d-spacing [Å]** | **Rel. Int. [%]** |
|---|---|---|---|---|
| 9.1585 | 406.65 | 0.0768 | 9.66 | 32.13 |
| 10.5913 | 125.25 | 0.2558 | 8.35 | 9.90 |
| 13.8291 | 212.69 | 0.1023 | 6.40 | 16.81 |
| 15.3223 | 114.14 | 0.3070 | 5.78 | 9.02 |
| 17.4887 | 1112.08 | 0.1023 | 5.07 | 87.87 |
| 17.7573 | 699.16 | 0.1023 | 4.99 | 55.24 |
| 18.0588 | 254.08 | 0.1023 | 4.91 | 20.08 |
| 18.3532 | 843.89 | 0.1023 | 4.83 | 66.68 |
| 19.0631 | 93.53 | 0.1535 | 4.66 | 7.39 |
| 19.4978 | 534.95 | 0.0768 | 4.55 | 42.27 |
| 19.7328 | 1265.63 | 0.1279 | 4.50 | 100.00 |
| 20.2234 | 240.67 | 0.2047 | 4.39 | 19.02 |
| 21.1657 | 227.99 | 0.1023 | 4.20 | 18.01 |
| 21.6656 | 167.05 | 0.2047 | 4.10 | 13.20 |
| 22.4118 | 50.06 | 0.3070 | 3.97 | 3.96 |
| 23.4530 | 162.39 | 0.1023 | 3.79 | 12.83 |
| 25.2039 | 254.89 | 0.1535 | 3.53 | 20.14 |
| 26.7244 | 193.81 | 0.1023 | 3.34 | 15.31 |
| 27.6786 | 702.52 | 0.1535 | 3.22 | 55.51 |
| 30.5646 | 118.62 | 0.2047 | 2.92 | 9.37 |
| 34.8974 | 111.64 | 0.2558 | 2.57 | 8.82 |

**Table B2-43. XRPD diffraction peak data of crystal form O of methanesulfonate**

| **Pos. [°2θ]** | **Height [cts]** | **FWHM Left [°2θ]** | **d-spacing [Å]** | **Rel. Int. [%]** |
|---|---|---|---|---|
| 9.8632 | 1483.56 | 0.1023 | 8.97 | 54.48 |
| 11.9975 | 116.95 | 0.1535 | 7.38 | 4.29 |
| 13.0102 | 274.36 | 0.1023 | 6.80 | 10.07 |
| 14.1723 | 59.91 | 0.3070 | 6.25 | 2.20 |
| 16.4454 | 1174.55 | 0.1279 | 5.39 | 43.13 |
| 17.1724 | 896.12 | 0.1279 | 5.16 | 32.91 |
| 17.8526 | 1068.28 | 0.1023 | 4.97 | 39.23 |
| 18.5219 | 296.29 | 0.1279 | 4.79 | 10.88 |
| 19.1922 | 531.50 | 0.1023 | 4.62 | 19.52 |
| 19.5652 | 2723.30 | 0.1279 | 4.54 | 100.00 |
| 19.7882 | 884.76 | 0.0768 | 4.49 | 32.49 |
| 20.7837 | 202.62 | 0.1535 | 4.27 | 7.44 |
| 21.5123 | 403.38 | 0.1023 | 4.13 | 14.81 |
| 22.1595 | 241.92 | 0.1535 | 4.01 | 8.88 |
| 22.6207 | 401.09 | 0.1535 | 3.93 | 14.73 |
| 23.3625 | 287.92 | 0.0768 | 3.81 | 10.57 |
| 24.6883 | 116.66 | 0.1279 | 3.61 | 4.28 |
| 25.3282 | 456.56 | 0.1279 | 3.52 | 16.76 |
| 26.0079 | 373.81 | 0.1023 | 3.43 | 13.73 |
| 29.5124 | 238.89 | 0.1023 | 3.03 | 8.77 |
| 30.2740 | 119.05 | 0.1535 | 2.95 | 4.37 |
| 31.3467 | 69.12 | 0.3070 | 2.85 | 2.54 |
| 32.7120 | 46.70 | 0.8187 | 2.74 | 1.71 |

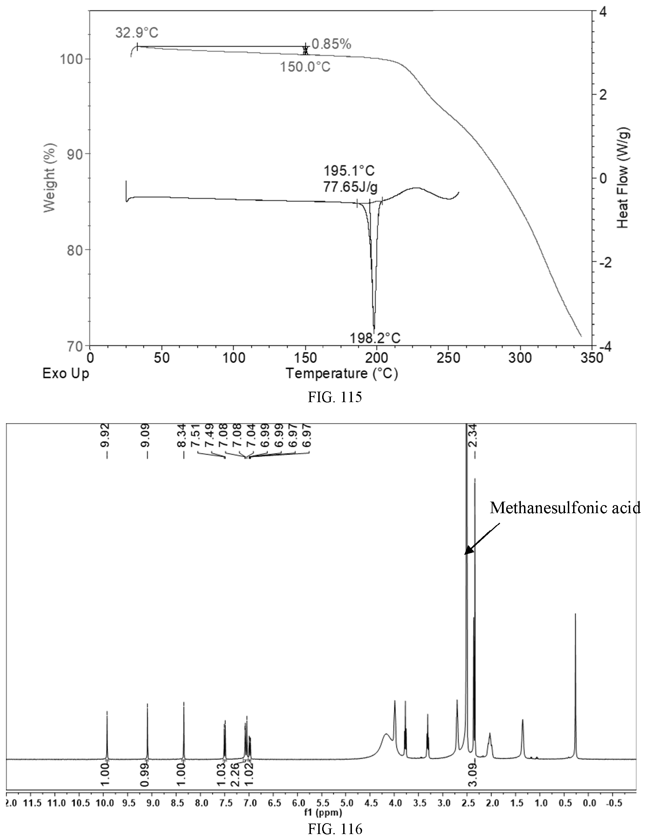

The TGA/DSC results of the sample of the crystal form E of the methanesulfonate are shown in FIG. 115. The TGA results show that the sample had a weight loss of 0.85% when heated to 150 °C, and the DSC results show that the sample had 1 endothermic peak at 198.2 °C (peak temperature). The ¹H NMR results, as shown in FIG. 116, show that methanesulfonic acid and the free crystal form in the sample were in a molar ratio of 1.0, and no IPAc solvent residue was found.

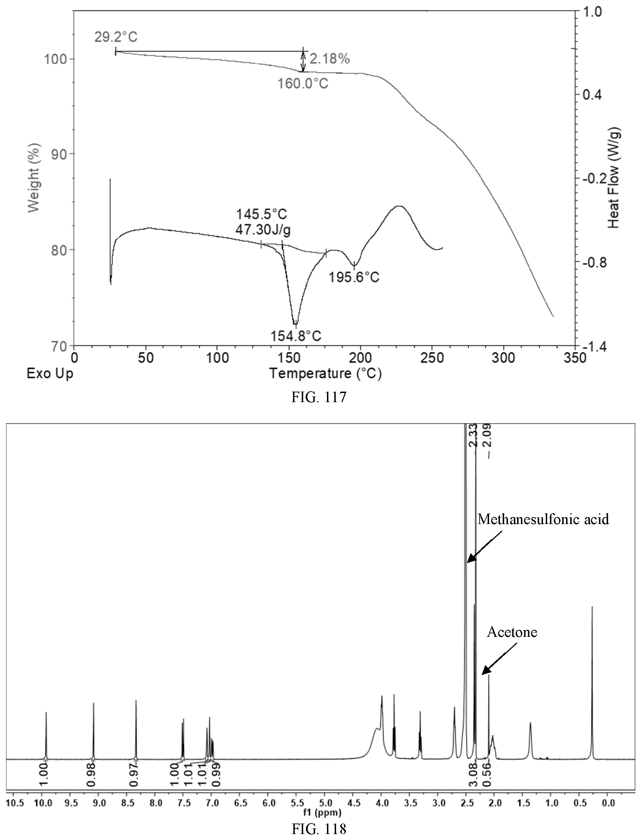

The TGA/DSC results of the sample of the crystal form F of the methanesulfonate are shown in FIG. 117. The TGA results show that the sample had a weight loss of 2.18% when heated to 160 °C, and the DSC results show that the sample had 2 endothermic peaks at 154.8 °C and 195.6 °C (peak temperatures). The ¹H NMR results, as shown in FIG. 118, show that methanesulfonic acid and the free crystal form in the sample were in a molar ratio of 1.0, and acetone and the free crystal form were in a molar ratio of 0.09 (about 0.8 wt%).

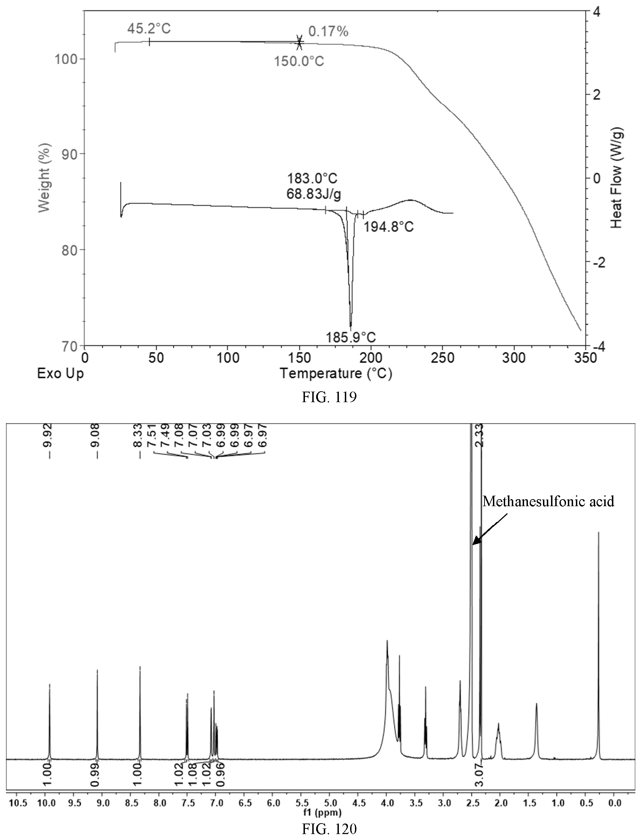

The TGA/DSC results of the sample of the crystal form O of the methanesulfonate are shown in FIG. 119. The TGA results show that the sample had a weight loss of 0.17% when heated to 150 °C, and the DSC results show that the sample had 2 endothermic peaks at 185.9 °C and 194.8 °C (peak temperatures). The ¹H NMR results, as shown in FIG. 120, show that methanesulfonic acid and the free crystal form in the sample were in a molar ratio of 1.0, and no MeOH and IPE solvent residues were found.

### Example 26. C/D/E/F of Ethanesulfonate

The crystal form C of the ethanesulfonate was obtained by slurrying the sample of the crystal form B of the ethanesulfonate prepared repeatedly in IPA at 50 °C for about 3 days. The crystal forms D/E of the ethanesulfonate were obtained by slurrying the samples of the crystal form B of the ethanesulfonate prepared repeatedly in EtOH and acetone, respectively, at room temperature for about 5 days, centrifuging to separate the solids, and then air-drying in an open container at room temperature. The crystal form F of the ethanesulfonate was obtained by slurrying the sample of the crystal form B of the ethanesulfonate prepared repeatedly in THF/H₂O (9:1, v/v) at room temperature for about 5 days. The XRPD overlay of the samples of the different crystal forms of the ethanesulfonate is listed in FIG. 121a.

The TGA/DSC results of the crystal form D of the ethanesulfonate are listed in FIG. 122. The TGA results show that the sample had a weight loss of 1.70% when heated to 150 °C, and the DSC results show that the sample had 3 endothermic peaks at 55.5 °C, 142.4 °C, and 184.2 °C (peak temperatures) and 1 exothermic peak at 91.6 °C (peak temperature). The ¹H NMR results, as shown in FIG. 123, show that ethanesulfonic acid and the free crystal form in the sample were in a molar ratio of 1.0, and no EtOH solvent residue was observed.

The TGA/DSC results of the crystal form E of the ethanesulfonate are shown in FIG. 124. The TGA results show that the sample had a weight loss of 0.61% when heated to 80 °C and a weight loss of 4.64% when further heated to 110 °C, and the DSC results show that the sample had 3 endothermic peaks at 48.4 °C, 104.0 °C, and 188.3 °C (peak temperatures). The ¹H NMR results, as shown in FIG. 125, show that ethanesulfonic acid and the free crystal form in the sample were in a molar ratio of 1.1, and the solvent acetone and the free crystal form were in a molar ratio of 0.54 (about 4.2 wt%).

The XRPD patterns of the crystal forms C, D, E, and F of the ethanesulfonate are shown in FIGs. 121b, 121c, 121d, and 121e, respectively, and the XRPD analysis data are shown in Tables B2-44 to B2-47 below, respectively.

**Table B2-44. XRPD diffraction peak data of crystal form C of ethanesulfonate**

| **Pos. [°2θ]** | **Height [cts]** | **FWHM Left [°2θ]** | **d-spacing [Å]** | **Rel. Int. [%]** |
|---|---|---|---|---|
| 6.3040 | 2839.06 | 0.0768 | 14.02 | 100.00 |
| 8.7771 | 495.39 | 0.1023 | 10.07 | 17.45 |
| 10.4226 | 692.10 | 0.1023 | 8.49 | 24.38 |
| 12.0274 | 121.48 | 0.1535 | 7.36 | 4.28 |
| 12.5635 | 118.14 | 0.1535 | 7.05 | 4.16 |
| 13.6779 | 582.76 | 0.1023 | 6.47 | 20.53 |
| 14.9315 | 49.70 | 0.3070 | 5.93 | 1.75 |
| 15.9618 | 572.17 | 0.1279 | 5.55 | 20.15 |
| 17.6528 | 275.37 | 0.1023 | 5.02 | 9.70 |
| 18.0467 | 135.43 | 0.1535 | 4.92 | 4.77 |
| 19.0549 | 327.17 | 0.0768 | 4.66 | 11.52 |
| 19.8646 | 219.23 | 0.1023 | 4.47 | 7.72 |
| 20.3279 | 826.80 | 0.1023 | 4.37 | 29.12 |
| 20.9348 | 513.75 | 0.1023 | 4.24 | 18.10 |
| 21.7435 | 282.81 | 0.1023 | 4.09 | 9.96 |
| 23.4169 | 292.83 | 0.1279 | 3.80 | 10.31 |
| 24.3601 | 104.02 | 0.2047 | 3.65 | 3.66 |
| 26.2647 | 293.26 | 0.1279 | 3.39 | 10.33 |
| 26.8633 | 103.40 | 0.1535 | 3.32 | 3.64 |
| 28.5527 | 90.41 | 0.1535 | 3.13 | 3.18 |
| 29.9377 | 110.88 | 0.3070 | 2.98 | 3.91 |
| 32.4837 | 59.96 | 0.3070 | 2.76 | 2.11 |
| 35.6194 | 44.31 | 0.4093 | 2.52 | 1.56 |

**Table B2-45. XRPD diffraction peak data of crystal form D of ethanesulfonate**

| **Pos. [°2θ]** | **Height [cts]** | **FWHM Left [°2θ]** | **d-spacing [Å]** | **Rel. Int. [%]** |
|---|---|---|---|---|
| 7.9081 | 2499.03 | 0.1535 | 11.18 | 100.00 |
| 15.7893 | 254.15 | 0.2047 | 5.61 | 10.17 |
| 17.4668 | 419.01 | 0.1535 | 5.08 | 16.77 |
| 17.7671 | 452.74 | 0.2047 | 4.99 | 18.12 |
| 19.9837 | 193.60 | 0.1535 | 4.44 | 7.75 |
| 20.9514 | 199.51 | 0.4093 | 4.24 | 7.98 |
| 23.8046 | 106.87 | 0.5117 | 3.74 | 4.28 |

**Table B2-46. XRPD diffraction peak data of crystal form E of ethanesulfonate**

| **Pos. [°2θ]** | **Height [cts]** | **FWHM Left [°2θ]** | **d-spacing [Å]** | **Rel. Int. [%]** |
|---|---|---|---|---|
| 6.4770 | 514.13 | 0.1023 | 13.65 | 100.00 |
| 9.3320 | 65.92 | 0.1023 | 9.48 | 12.82 |
| 10.9010 | 82.45 | 0.1535 | 8.12 | 16.04 |
| 12.3962 | 71.56 | 0.1535 | 7.14 | 13.92 |
| 13.6740 | 164.68 | 0.1279 | 6.48 | 32.03 |
| 14.7088 | 41.15 | 0.1535 | 6.02 | 8.00 |
| 15.4870 | 96.19 | 0.3070 | 5.72 | 18.71 |
| 16.1908 | 80.26 | 0.2047 | 5.47 | 15.61 |
| 17.8603 | 73.00 | 0.4093 | 4.97 | 14.20 |
| 18.9455 | 153.47 | 0.1023 | 4.68 | 29.85 |
| 19.6221 | 59.75 | 0.1535 | 4.52 | 11.62 |
| 20.0718 | 100.45 | 0.1023 | 4.42 | 19.54 |
| 20.4917 | 39.95 | 0.1535 | 4.33 | 7.77 |
| 21.4317 | 95.71 | 0.1535 | 4.15 | 18.62 |
| 23.2933 | 88.12 | 0.1279 | 3.82 | 17.14 |
| 24.8830 | 44.72 | 0.1535 | 3.58 | 8.70 |
| 27.4600 | 25.88 | 0.4093 | 3.25 | 5.03 |

**Table B2-47. XRPD diffraction peak data of crystal form F of ethanesulfonate**

| **Pos. [°2θ]** | **Height [cts]** | **FWHM Left [°2θ]** | **d-spacing [Å]** | **Rel. Int. [%]** |
|---|---|---|---|---|
| 8.7081 | 324.49 | 0.1023 | 10.15 | 29.42 |
| 9.3273 | 1103.04 | 0.1023 | 9.48 | 100.00 |
| 18.7028 | 199.35 | 0.1535 | 4.74 | 18.07 |

### Example 27. Crystal Forms A/B/C of 1,2-Ethanedisulfonate

The crystal forms A/B of the 1,2-ethanedisulfonate were obtained by slurrying the free starting samples 2 and an equimolar amount of 1,2-ethanedisulfonic acid in EtOH and EtOAc, respectively, at room temperature for about 2 days, centrifuging to separate the solid samples, and then drying under vacuum at 50 °C. The crystal form C of the 1,2-ethanedisulfonate was obtained by slurrying the free starting sample 2 and an equimolar amount of 1,2-ethanedisulfonic acid in 2-MeTHF at room temperature overnight to obtain a gel, then performing temperature cycling between 50 °C and 5 °C, slurrying for about 1 day, remaining a small amount of the gel sample, followed by discarding the gel sample, centrifuging to separate the solid sample, and then drying under vacuum at 50 °C. The XRPD overlay is shown in FIG. 126a. The XRPD patterns of the crystal forms A, B, and C of the 1,2-ethanedisulfonate are shown in FIGs. 126b, 126c, and 126d, and the XRPD analysis data are shown in Tables B2-48 to B2-50 below.

**Table B2-48. XRPD diffraction peak data of crystal form A of 1,2-ethanedisulfonate**

| **Pos. [°2θ]** | **Height [cts]** | **FWHM Left [°2θ]** | **d-spacing [Å]** | **Rel. Int. [%]** |
|---|---|---|---|---|
| 9.5776 | 593.37 | 0.0768 | 9.23 | 35.04 |
| 9.7934 | 681.24 | 0.0768 | 9.03 | 40.23 |
| 11.0447 | 66.40 | 0.4093 | 8.01 | 3.92 |
| 12.1309 | 66.00 | 0.3070 | 7.30 | 3.90 |
| 12.9489 | 385.56 | 0.1023 | 6.84 | 22.77 |
| 13.3845 | 181.72 | 0.2047 | 6.62 | 10.73 |
| 14.5687 | 58.40 | 0.2047 | 6.08 | 3.45 |
| 15.5203 | 49.70 | 0.2047 | 5.71 | 2.94 |
| 16.3117 | 332.70 | 0.0768 | 5.43 | 19.65 |
| 16.5380 | 685.13 | 0.1279 | 5.36 | 40.46 |
| 16.9718 | 449.35 | 0.1279 | 5.22 | 26.54 |
| 17.6744 | 521.91 | 0.1279 | 5.02 | 30.82 |
| 18.7068 | 197.97 | 0.1279 | 4.74 | 11.69 |
| 19.6187 | 1377.27 | 0.1023 | 4.53 | 81.34 |
| 20.1184 | 883.97 | 0.1023 | 4.41 | 52.20 |
| 20.7238 | 1693.28 | 0.1535 | 4.29 | 100.00 |
| 22.2198 | 288.90 | 0.1535 | 4.00 | 17.06 |
| 23.1294 | 177.09 | 0.2047 | 3.85 | 10.46 |
| 24.1339 | 112.18 | 0.2047 | 3.69 | 6.62 |
| 25.3534 | 153.55 | 0.1535 | 3.51 | 9.07 |
| 26.0706 | 166.55 | 0.2558 | 3.42 | 9.84 |
| 26.5387 | 180.81 | 0.1023 | 3.36 | 10.68 |
| 27.8458 | 175.54 | 0.1535 | 3.20 | 10.37 |
| 30.3527 | 88.61 | 0.3070 | 2.94 | 5.23 |
| 31.4653 | 89.20 | 0.4093 | 2.84 | 5.27 |

**Table B2-49. XRPD diffraction peak data of crystal form B of 1,2-ethanedisulfonate**

| **Pos. [°2θ]** | **Height [cts]** | **FWHM Left [°2θ]** | **d-spacing [Å]** | **Rel. Int. [%]** |
|---|---|---|---|---|
| 5.6887 | 655.87 | 0.1023 | 15.54 | 39.90 |
| 7.5361 | 65.88 | 0.3070 | 11.73 | 4.01 |
| 9.5593 | 1185.89 | 0.1279 | 9.25 | 72.14 |
| 10.0235 | 396.89 | 0.1023 | 8.82 | 24.14 |
| 12.9139 | 509.38 | 0.1023 | 6.86 | 30.99 |
| 14.9480 | 100.32 | 0.2047 | 5.93 | 6.10 |
| 16.2913 | 854.79 | 0.0768 | 5.44 | 52.00 |
| 16.5067 | 1288.66 | 0.1023 | 5.37 | 78.39 |
| 17.0648 | 768.72 | 0.2047 | 5.20 | 46.76 |
| 17.7815 | 1245.46 | 0.1535 | 4.99 | 75.76 |
| 18.6648 | 379.07 | 0.1535 | 4.75 | 23.06 |
| 20.1011 | 1643.95 | 0.1535 | 4.42 | 100.00 |
| 21.5441 | 182.51 | 0.1023 | 4.12 | 11.10 |
| 21.9422 | 61.29 | 0.4093 | 4.05 | 3.73 |
| 22.2426 | 417.67 | 0.1535 | 4.00 | 25.41 |
| 22.8020 | 252.34 | 0.1279 | 3.90 | 15.35 |
| 23.3535 | 92.19 | 0.1535 | 3.81 | 5.61 |
| 23.8750 | 48.64 | 0.2047 | 3.73 | 2.96 |
| 25.8424 | 168.15 | 0.1791 | 3.45 | 10.23 |
| 26.4853 | 264.38 | 0.1535 | 3.37 | 16.08 |
| 27.9265 | 63.81 | 0.3070 | 3.19 | 3.88 |
| 29.8463 | 106.97 | 0.1535 | 2.99 | 6.51 |
| 30.3500 | 118.77 | 0.2047 | 2.95 | 7.22 |
| 32.6082 | 41.44 | 0.6140 | 2.75 | 2.52 |

**Table B2-50. XRPD diffraction peak data of crystal form C of 1,2-ethanedisulfonate**

| **Pos. [°2θ]** | **Height [cts]** | **FWHM Left [°2θ]** | **d-spacing [Å]** | **Rel. Int. [%]** |
|---|---|---|---|---|
| 7.9970 | 98.56 | 0.3070 | 11.06 | 5.27 |
| 9.8985 | 69.05 | 0.3070 | 8.94 | 3.69 |
| 10.9129 | 171.45 | 0.2558 | 8.11 | 9.17 |
| 12.0262 | 136.78 | 0.2047 | 7.36 | 7.32 |
| 13.3305 | 215.89 | 0.2558 | 6.64 | 11.55 |
| 15.5409 | 69.67 | 0.2558 | 5.70 | 3.73 |
| 16.7417 | 222.07 | 0.2303 | 5.30 | 11.88 |
| 17.8877 | 452.58 | 0.3070 | 4.96 | 24.21 |
| 18.3757 | 180.57 | 0.1535 | 4.83 | 9.66 |
| 19.3330 | 256.85 | 0.1279 | 4.59 | 13.74 |
| 19.8489 | 540.02 | 0.2047 | 4.47 | 28.88 |
| 20.7131 | 1869.70 | 0.1791 | 4.29 | 100.00 |
| 21.1631 | 441.99 | 0.1535 | 4.20 | 23.64 |
| 22.3700 | 149.60 | 0.2558 | 3.97 | 8.00 |
| 23.0842 | 169.67 | 0.3070 | 3.85 | 9.07 |
| 24.1491 | 150.46 | 0.2558 | 3.69 | 8.05 |
| 25.2521 | 55.19 | 0.4093 | 3.53 | 2.95 |
| 27.8056 | 147.59 | 0.2047 | 3.21 | 7.89 |
| 31.2456 | 77.04 | 0.4093 | 2.86 | 4.12 |

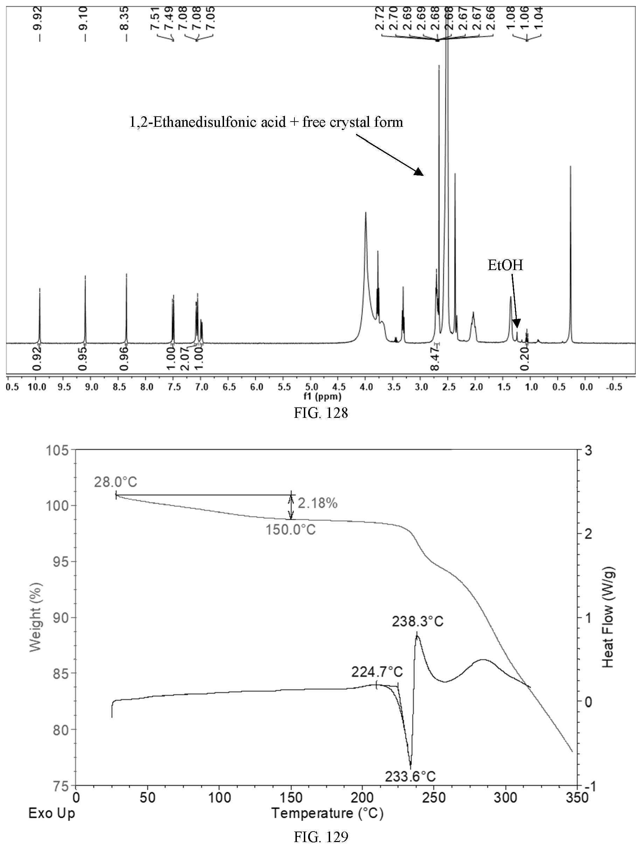

The TGA/DSC results of the crystal form A of the 1,2-ethanedisulfonate are listed in FIG. 127. The TGA results show that the sample had a weight loss of 1.47% when heated to 150 °C, and the DSC results show that the sample had 3 endothermic peaks at 33.9 °C, 238.5 °C, and 249.0 °C (peak temperatures) and 1 exothermic peak at 256.0 °C (peak temperature). The ¹H NMR results, as shown in FIG. 128, show that 1,2-ethanedisulfonic acid and the free crystal form in the sample were in a molar ratio of 1.1, and EtOH and the free crystal form were in a molar ratio of 0.07 (about 0.4 wt%).

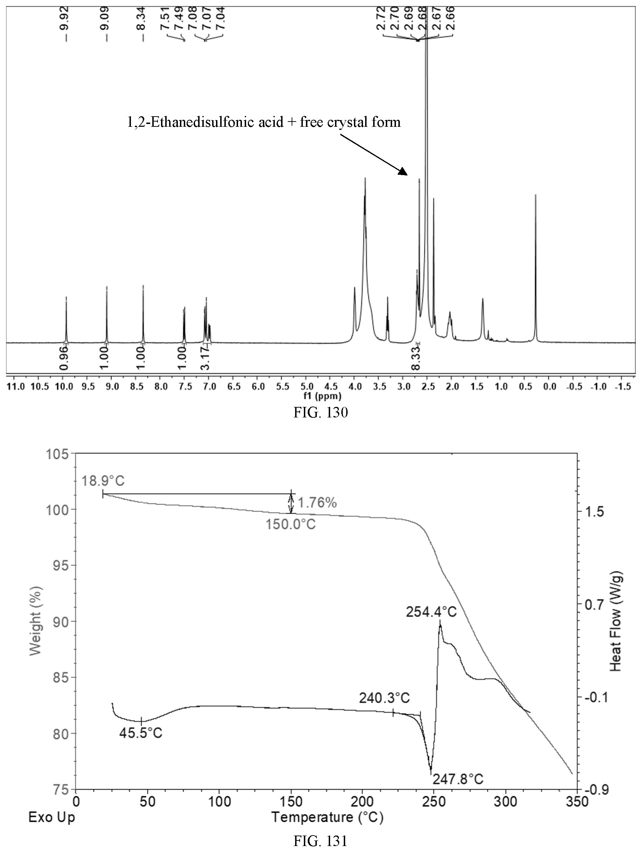

The TGA/DSC results of the crystal form B of the 1,2-ethanedisulfonate are listed in FIG. 129. The TGA results show that the sample had a weight loss of 2.18% when heated to 150 °C, and the DSC results show that the sample had 1 endothermic peak at 233.6 °C (peak temperature) and 1 exothermic peak at 238.3 °C (peak temperature). The ¹H NMR results, as shown in FIG. 130, show that 1,2-ethanedisulfonic acid and the free crystal form in the sample were in a molar ratio of 1.1, and no EtOAc solvent residue was observed.

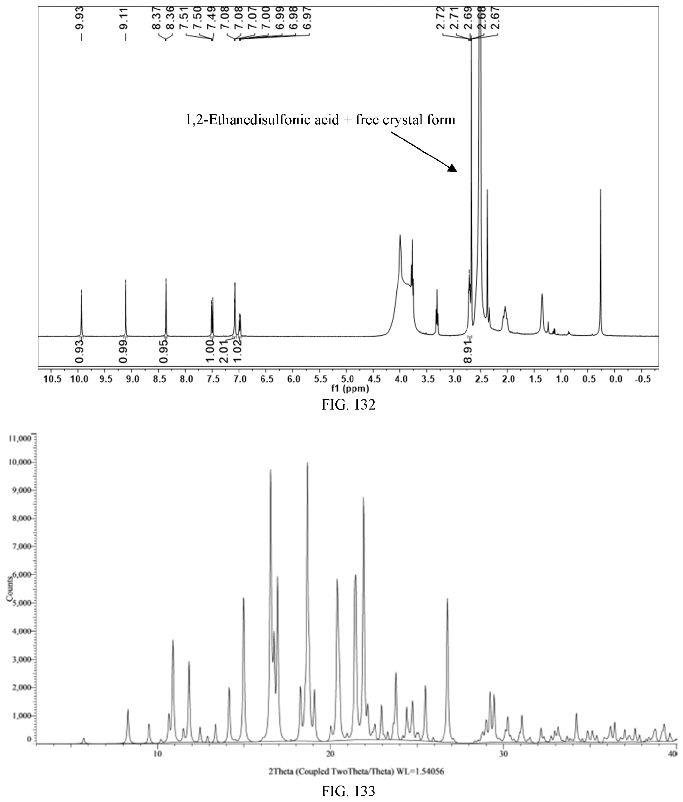

The TGA/DSC results of the crystal form C of the 1,2-ethanedisulfonate are listed in FIG. 131. The TGA results show that the sample had a weight loss of 1.76% when heated to 150 °C, and the DSC results show that the sample had 2 endothermic peaks at 45.5 °C and 247.8 °C (peak temperatures) and 1 exothermic peak at 254.4 °C (peak temperature). The ¹H NMR results, as shown in FIG. 132, show that 1,2-ethanedisulfonic acid and the free crystal form in the sample were in a molar ratio of 1.2, and no 2-MeTHF solvent residue was observed.

### Example 28

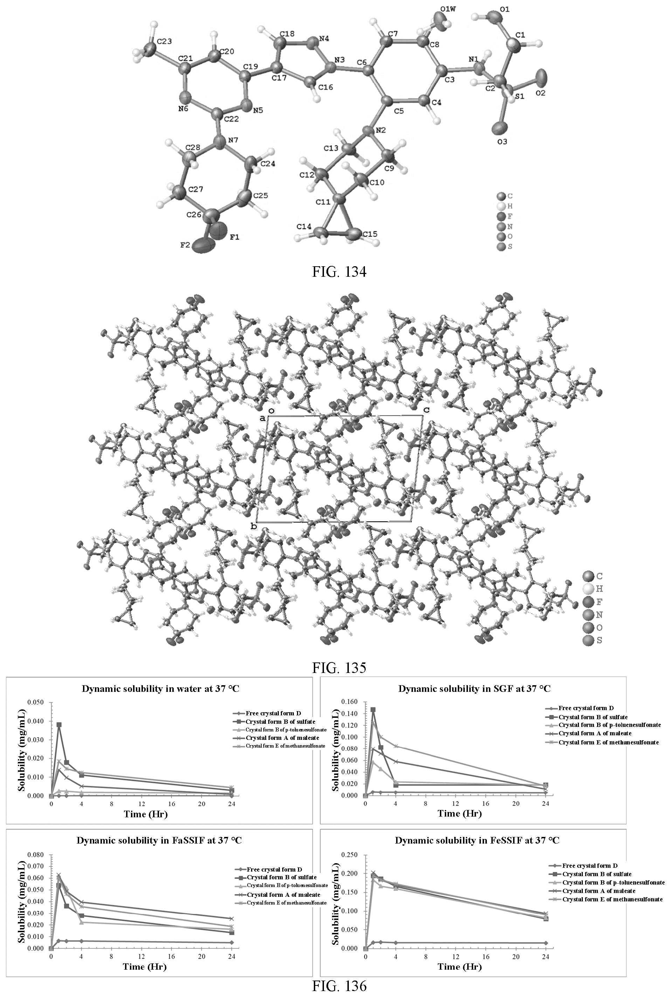

20 mg of the compound of formula I (which could be prepared according to Example 1) was weighed out and added to an EP tube. Methanol (3 mL) was added, and the mixture was shaken until the sample was completely dissolved. Purified water (0.5 mL) was added, and the solution was filtered through a microporous filter membrane into a 4 mL glass bottle. The bottle was sealed with a sealing film, and three small holes were made on the film. The solution was left to stand for volatilization. After 5 days, the precipitation of a transparent crystal was observed, and the crystal was subjected to single crystal detection. The single crystal simulated XRPD is shown in FIG. 133, which is consistent with the XRPD of the powder sample of the crystal form D. The single crystal diffraction thermal ellipsoid (50%) molecular structure is shown in FIG. 134, which proves that the single crystal sample contains one water of crystallization, indicating that the crystal form D is a monohydrate. The molecular structure packing diagram from single crystal diffraction is shown in FIG. 135. The X-ray single crystal diffraction results are shown in Table B3-1 below.

| Table B3-1. Crystal parameters | | |
|---|---|---|
| Molecular formula | C₂₈H₃₇F₂N₇O₄S | |
| Molecular weight | 605.70 | |
| Temperature | 200(2) K | |
| Wavelength | 1.34139 Å | |
| Crystal system | triclinic | |
| space group | P-1 | |
| Unit cell parameters | a = 8.9217(3) Å | α= 95.3240(10)°. |
| | b = 10.9062(5) Å | β = 97.7830(10)°. |
| | c = 15.6399(5) Å | γ = 100.4940(10)°. |
| Volume | 1471.74(10) Å3 | |
| Z | 2 | |
| Density (calculated) | 1.367 Mg/m3 | |
| Absorption coefficient | 0.964 mm-1 | |
| F(000) | 640 | |
| Crystal size | 0.300 × 0.100 × 0.100 mm3 | |

### Test Example 1

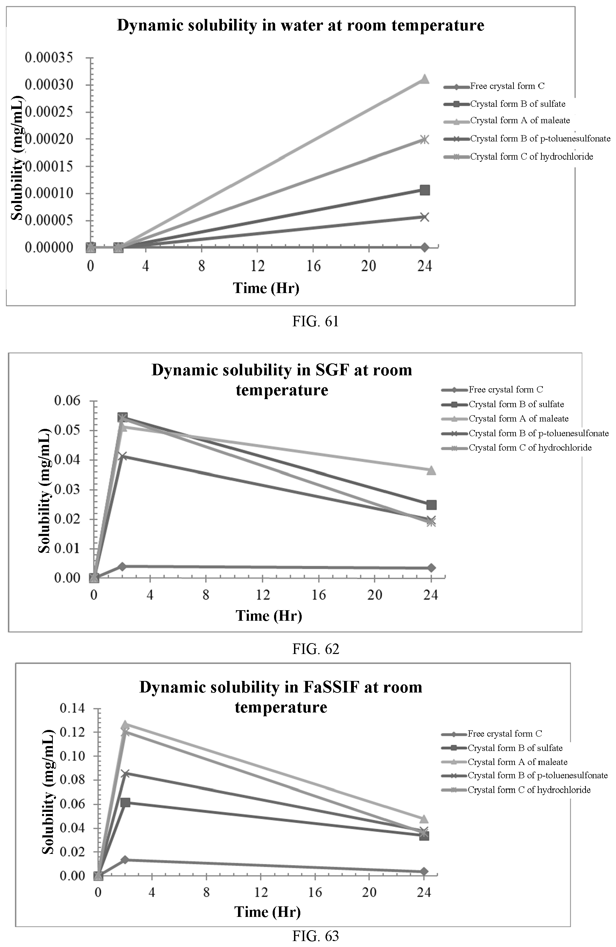

Solids at a feeding concentration of 1 mg/mL (calculated based on free crystal form) were rotationally mixed with corresponding vehicles at room temperature. The solubility of each sample in three systems of water, SGF, and FaSSIF was determined at different time points (2 h and 24 h). After sampling at each time point, the samples were centrifuged (at 10000 rpm) and filtered (through a 0.22 µm PTFE filter head), the HPLC concentrations and pH values of the filtrates were determined, and the centrifuged solid samples were subjected to XRPD analysis. The solubility test results are summarized in Table C-1. The solubility curves of the free crystal form C, the crystal form B of the sulfate, the crystal form A of the maleate, the crystal form B of the p-toluenesulfonate, and the crystal form C of the hydrochloride in H₂O, SGF, and FaSSIF at room temperature are shown in FIGs. 61-63.

**Table C-1. Summary of solubility test results at room temperature**

| **Starting material** | **Solvent** | **2 h** | | **24 h** | |
|---|---|---|---|---|---|
| | | S | pH | S | pH |
| Free crystal form C | H₂O | <LOQ | 8.7 | <LOQ | 7.2 |
| | SGF | 0.0040 | 1.9 | 0.0034 | 1.9 |
| | FaSSIF | 0.0136 | 6.5 | 0.0038 | 6.5 |
| Crystal form B of sulfate | H₂O | <LOQ | 2.7 | 0.0001 | 2.5 |
| | SGF | 0.0545 | 1.8 | 0.0250 | 1.8 |
| | FaSSIF | 0.0616 | 6.3 | 0.0338 | 6.2 |
| Crystal form A of maleate | H₂O | <LOQ | 6.5 | 0.0003 | 2.7 |
| | SGF | 0.0512 | 1.9 | 0.0366 | 1.9 |
| | FaSSIF | 0.1268 | 6.4 | 0.0479 | 6.2 |
| Crystal form B of p-toluenesulfonate | H₂O | <LOQ | 3.4 | 0.0001 | 2.8 |
| | SGF | 0.0413 | 1.9 | 0.0197 | 1.8 |
| | FaSSIF | 0.0858 | 6.5 | 0.0378 | 6.4 |
| Crystal form C of hydrochloride | H₂O | <LOQ | 2.7 | 0.0002 | 2.7 |
| | SGF | 0.0541 | 1.8 | 0.0188 | 1.8 |
| | FaSSIF | 0.1204 | 6.3 | 0.0363 | 6.3 |

### S: solubility (mg/mL).

LOQ: limit of quantitation, which refers to the lowest amount of an analyte in a sample that can be quantitatively determined.

### Test Example 2

The dynamic solubility, hygroscopicity, and solid state stability of the crystal forms of the present disclosure were evaluated.

### (I) Dynamic solubility

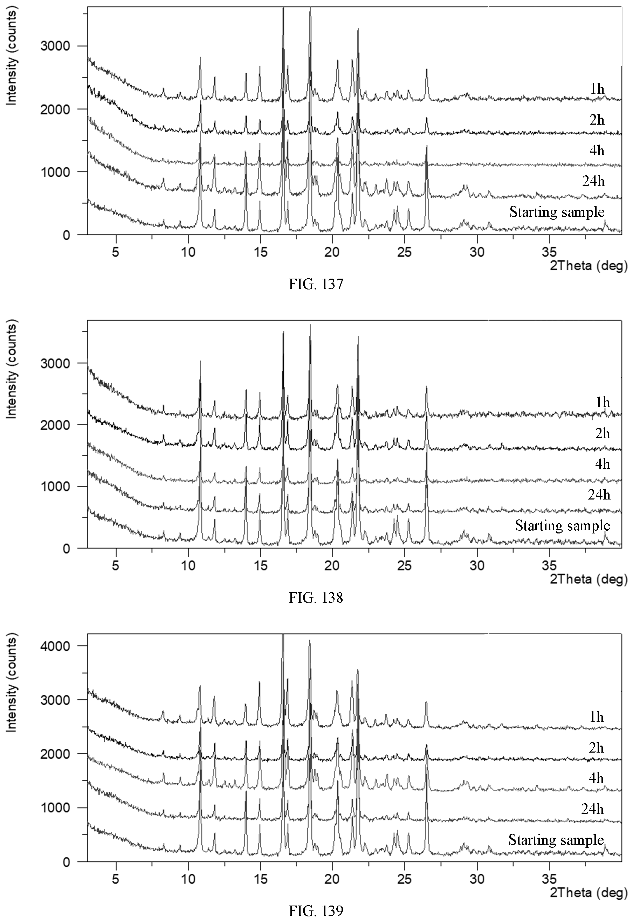

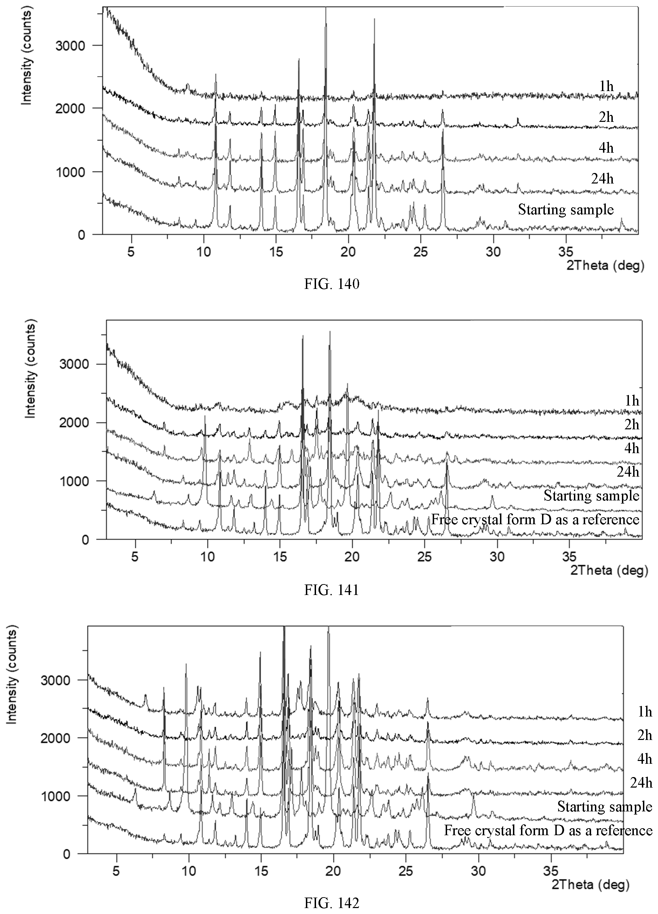

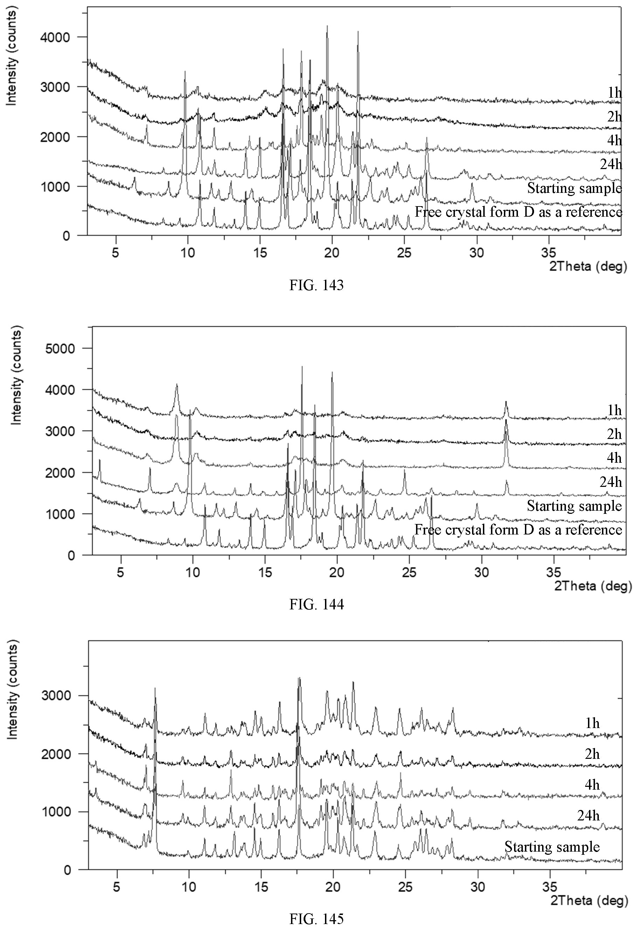

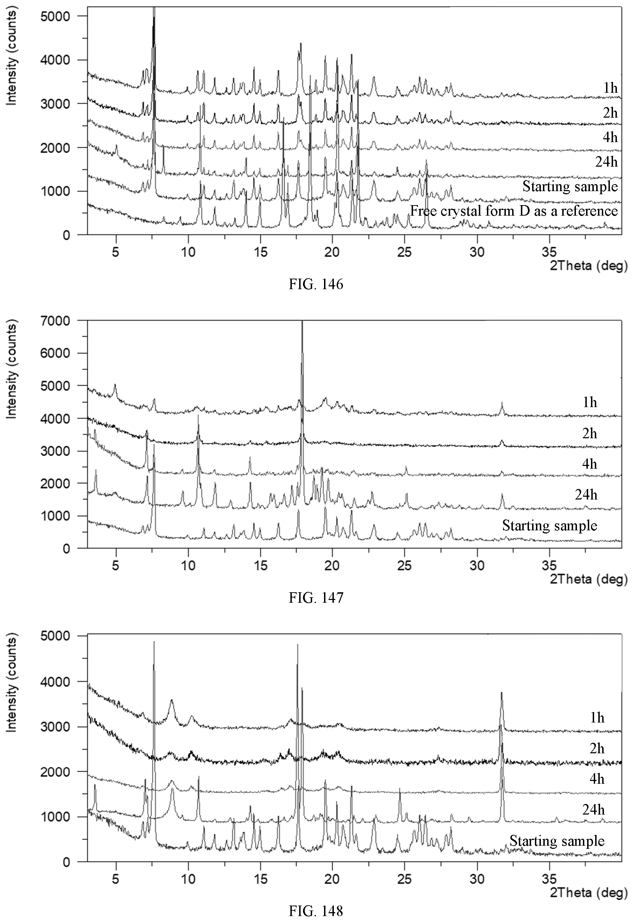

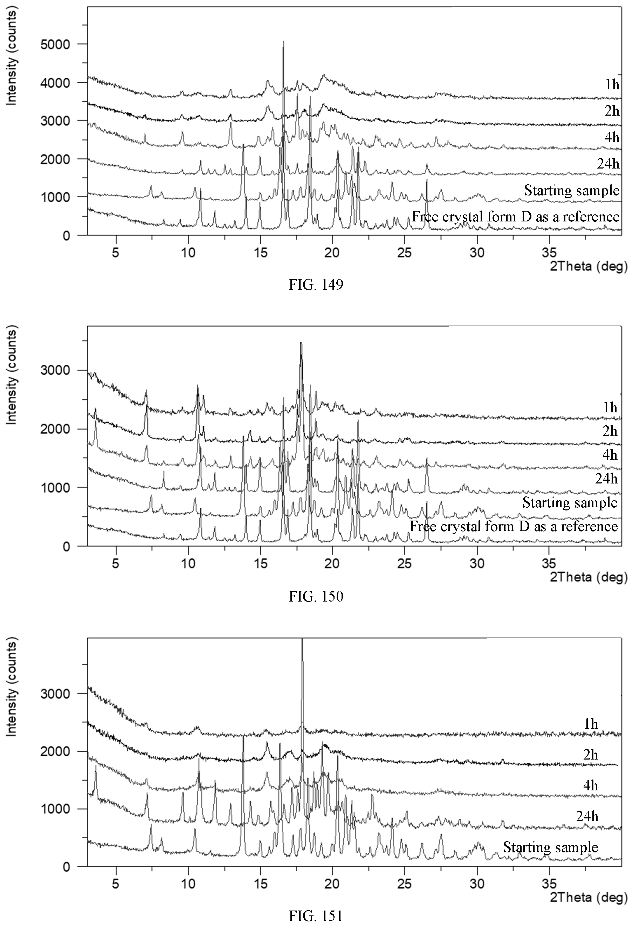

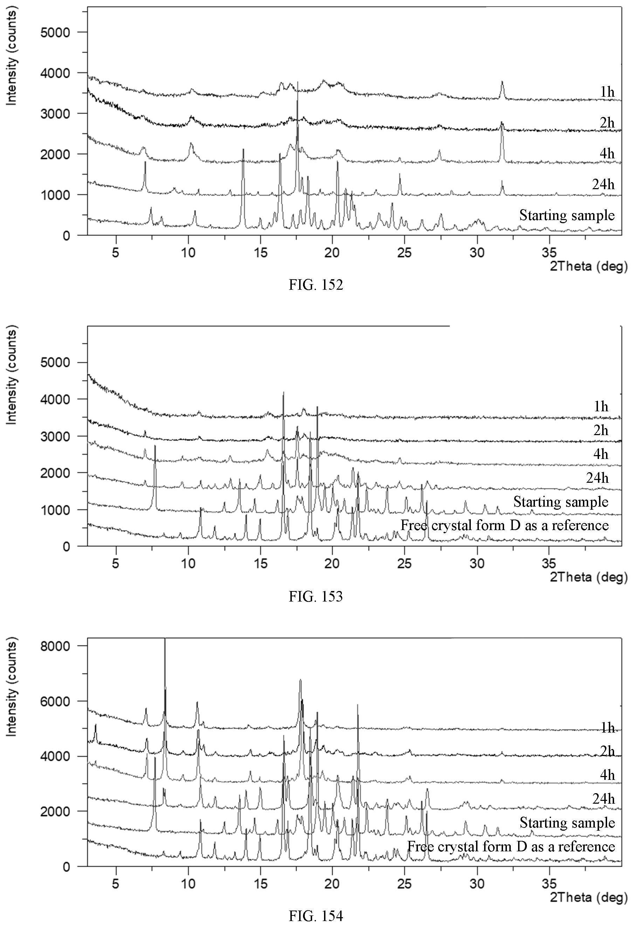

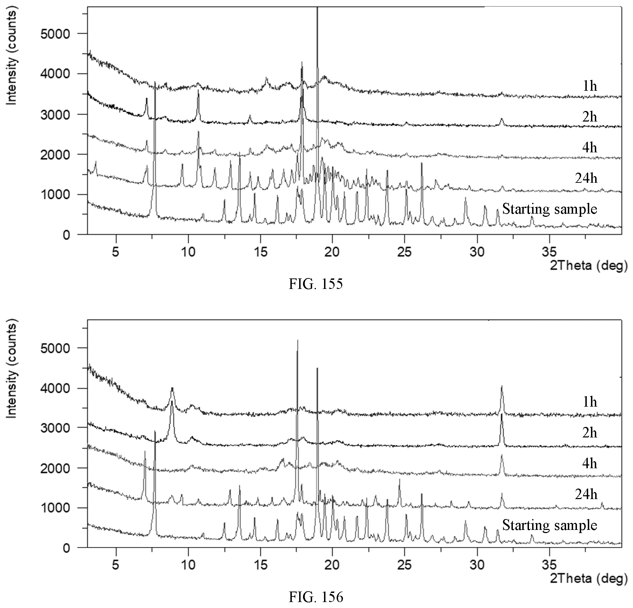

Solids at a feeding concentration of 10 mg/mL (calculated based on free crystal form) were rotationally mixed with corresponding vehicles at 37 °C. The solubility of each sample in H₂O, SGF, FaSSIF, and FeSSIF was determined at different time points (1 h, 2 h, 4 h, and 24 h). After sampling at each time point, the samples were centrifuged (at 10000 rpm) and filtered (through 0.45 µm PTFE), the HPLC concentrations and pH values of the filtrates were determined, and the centrifuged solid samples were subjected to XRPD analysis. The solubility experiment results are summarized in Table C-2. The solubility curves are shown in FIG. 136. The XRPD results of the samples after the solubility tests are shown in FIGs. 137-156. The experimental results show that the solubility of all candidate salt forms in H₂O, SGF, FaSSIF, and FeSSIF at 37 °C at 1/2/4/24 h was increased to different degrees as compared to the free crystal form. The XRPD results show that the samples of all salt forms had different degrees of crystal form changes after the dynamic solubility test, and the samples of the free crystal forms had no crystal form changes after the dynamic solubility test.

**Table C-2. Summary of dynamic solubility test results at 37 °C**

| **Starting material** | **Vehicle** | **1 h** | | | **2 h** | | | **4 h** | | | **24 h** | | |
|---|---|---|---|---|---|---|---|---|---|---|---|---|---|
| | | S | pH | FC | S | pH | FC | S | pH | FC | S | pH | FC |
| Free crystal form D | H₂O | 0.0001 | 6.91 | No | 0.0001 | 7.64 | No | 0.0002 | 7.88 | No | <LOQ | 7.65 | No |
| | SGF | 0.0062 | 1.26 | No | 0.0057 | 1.38 | No | 0.0059 | 1.29 | No | 0.0051 | 1.32 | No |
| | FaSSIF | 0.0065 | 6.51 | No | 0.0065 | 6.53 | No | 0.0063 | 6.55 | No | 0.0050 | 6.49 | No |
| | FeSSIF | 0.0162 | 4.97 | No | 0.0172 | 5.01 | No | 0.0154 | 4.97 | No | 0.0150 | 4.96 | No |
| Crystal form B of sulfate | H₂O | 0.0382 | 1.50 | Yes | 0.0180 | 1.63 | Yes | 0.0111 | 1.55 | Yes | 0.0030 | 1.56 | Yes |
| | SGF | 0.1469 | 1.26 | Yes | 0.0824 | 1.38 | Yes | 0.0181 | 1.29 | Yes | 0.0185 | 1.32 | Yes |
| | FaSSIF | 0.0539 | 2.06 | Yes | 0.0364 | 2.13 | Yes | 0.0281 | 2.07 | Yes | 0.0136 | 2.08 | Yes |
| | FeSSIF | 0.1941 | 4.50 | Yes | 0.1862 | 4.49 | Yes | 0.1657 | 4.48 | Yes | 0.0790 | 4.48 | Yes |
| Crystal form B of p-toluenesulfonate | H₂O | 0.0027 | 2.17 | Yes | 0.0025 | 2.17 | Yes | 0.0019 | 2.09 | Yes | 0.0015 | 1.95 | Yes |
| | SGF | 0.0568 | 1.64 | Yes | 0.0456 | 1.63 | Yes | 0.0233 | 1.60 | Yes | 0.0181 | 1.59 | Yes |
| | FaSSIF | 0.0614 | 2.92 | Yes | 0.0517 | 2.84 | Yes | 0.0222 | 2.74 | Yes | 0.0163 | 2.68 | Yes |
| | FeSSIF | 0.1828 | 4.68 | Yes | 0.1665 | 4.66 | Yes | 0.1599 | 4.68 | Yes | 0.0814 | 4.74 | Yes |
| Crystal form A of maleate | H₂O | 0.0139 | 2.00 | Yes | 0.0098 | 2.05 | Yes | 0.0052 | 1.98 | Yes | 0.0009 | 2.03 | Yes |
| | SGF | 0.0795 | 1.55 | Yes | 0.0718 | 1.57 | Yes | 0.0579 | 1.54 | Yes | 0.0111 | 1.58 | Yes |
| | FaSSIF | 0.0627 | 2.94 | Yes | 0.0489 | 2.90 | Yes | 0.0397 | 2.90 | Yes | 0.0253 | 2.90 | Yes |
| | FeSSIF | 0.2025 | 4.66 | Yes | 0.1824 | 4.68 | Yes | 0.1695 | 4.67 | Yes | 0.0940 | 4.70 | Yes |
| Crystal form E of methanesulfonate | H₂O | 0.0185 | 1.79 | Yes | 0.0145 | 1.76 | Yes | 0.0124 | 1.80 | Yes | 0.0045 | 1.76 | Yes |
| | SGF | 0.1251 | 1.49 | Yes | 0.1002 | 1.46 | Yes | 0.0846 | 1.48 | Yes | 0.0164 | 1.54 | Yes |
| | FaSSIF | 0.0604 | 2.78 | Yes | 0.0466 | 2.76 | Yes | 0.0359 | 2.77 | Yes | 0.0187 | 2.84 | Yes |
| | FeSSIF | 0.1944 | 4.69 | Yes | 0.1834 | 4.68 | Yes | 0.1725 | 4.70 | Yes | 0.0899 | 4.70 | Yes |

| | | | | | | | | | | | | | |
|---|---|---|---|---|---|---|---|---|---|---|---|---|---|
| S: solubility (mg/mL); FC: crystal form change; | | | | | | | | | | | | | |

### (II) Hygroscopicity

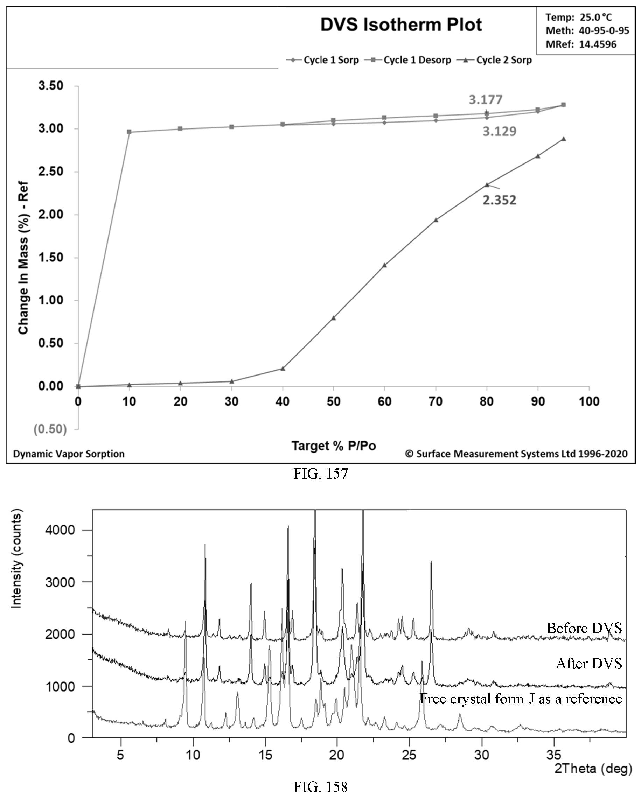

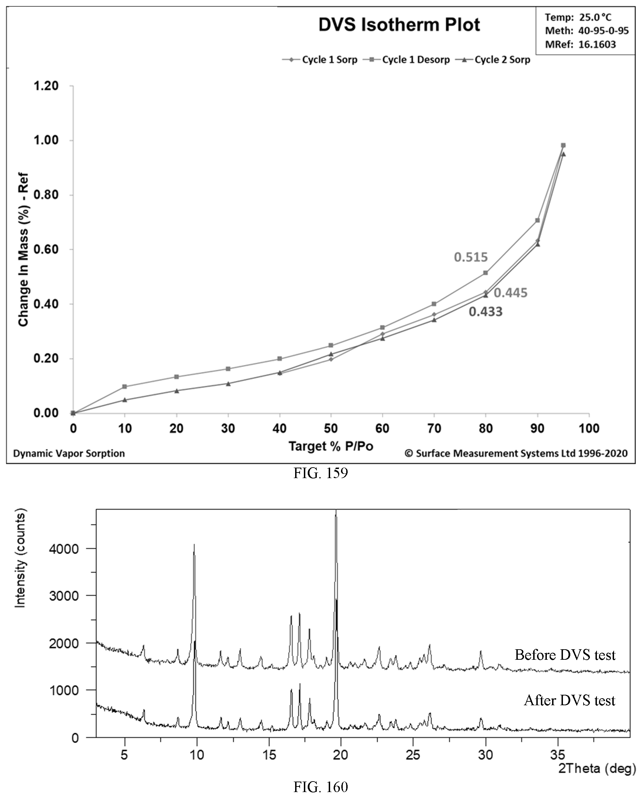

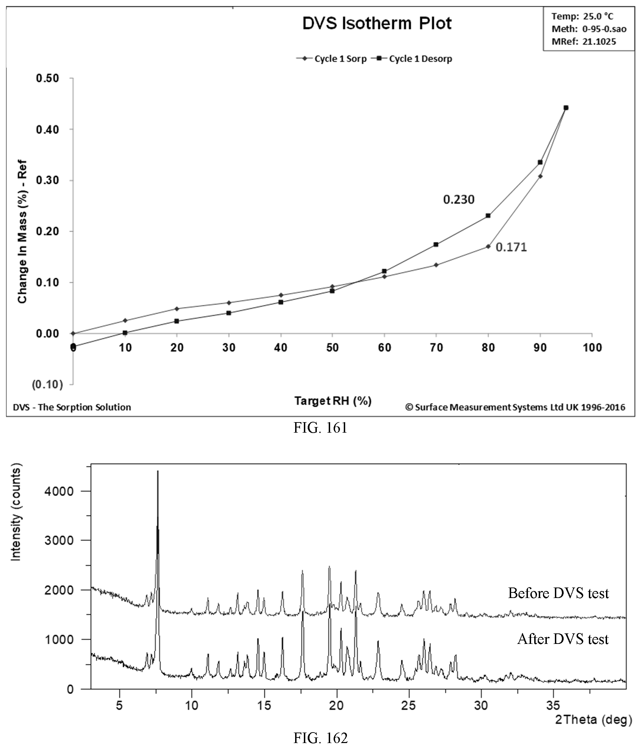

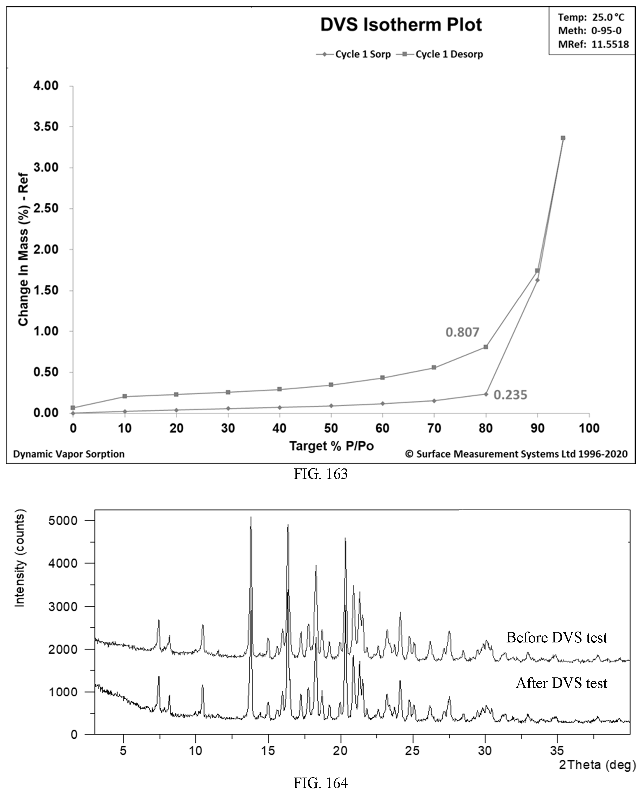

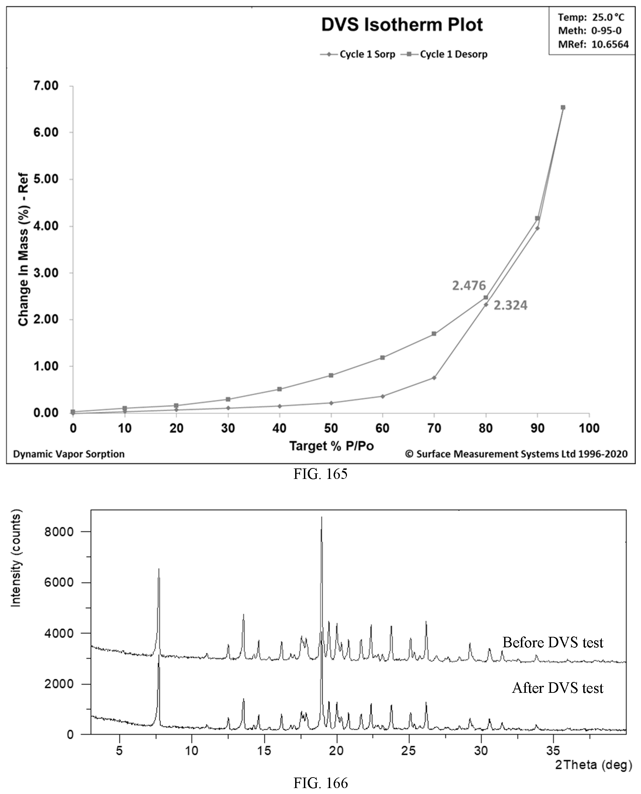

The hygroscopicity of the crystal form B of the sulfate, the crystal form B of the p-toluenesulfonate, the crystal form A of the maleate, the crystal form E of the methanesulfonate, and the free crystal form D was evaluated using a dynamic vapor sorption (DVS) instrument. The percentage changes in mass of the samples when the humidity changed (0% RH to 95% RH) at a constant temperature of 25 °C were collected. The DVS test results and XRPD results for the samples before and after the DVS test are shown in FIGs. 157-166. The results show that the vapor sorption values of the free crystal form D, the crystal form B of the sulfate, the crystal form B of the p-toluenesulfonate, the crystal form A of the maleate, and the crystal form E of the methanesulfonate at 25 °C/80% RH were 3.129%, 0.445%, 0.171%, 0.235%, and 2.324%, respectively. Except that the free crystal form D was converted to the free crystal forms D + J after the DVS test, the crystal forms of the other samples remained unchanged before and after the DVS test.

### (III) Solid state stability

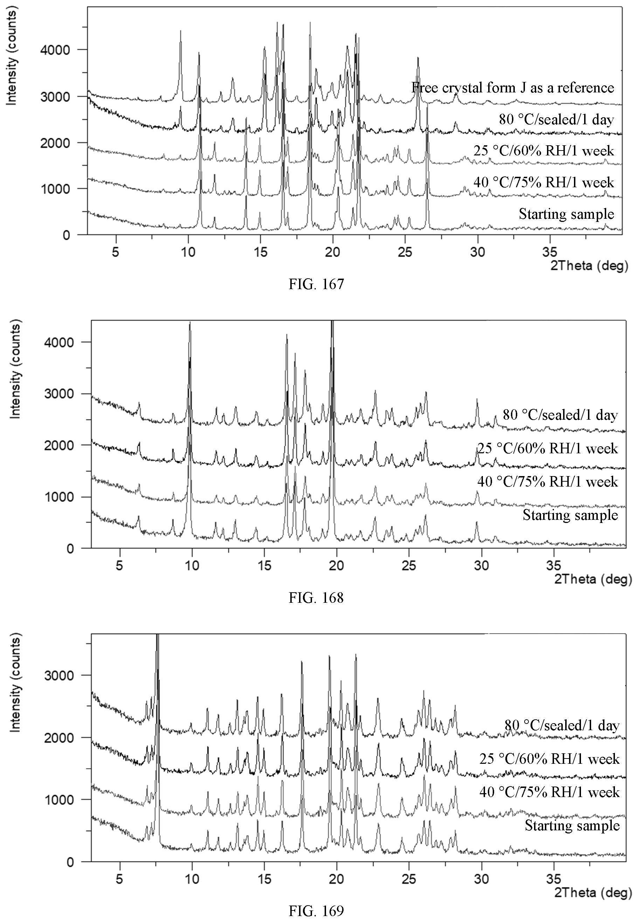

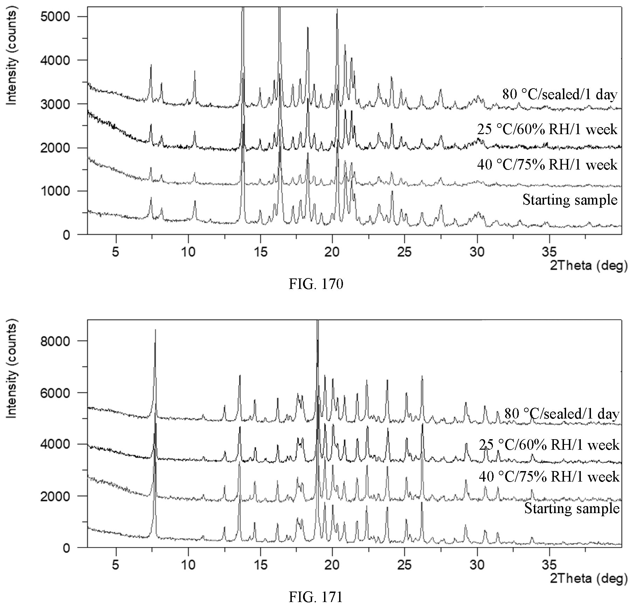

The crystal form B of the sulfate, the crystal form B of the p-toluenesulfonate, the crystal form A of the maleate, the crystal form E of the methanesulfonate, and the free crystal form D were placed in a sealed container at 80 °C for 1 day and in an open container at 25 °C/60% RH and 40 °C/75% RH for 1 week, and then the physical and chemical stability of the samples was tested by XRPD and HPLC. The purity data are shown in Table C-3, and the XRPD results are shown in FIGs. 167-171. The stability evaluation results show that no significant change in purity was observed in all samples after the test. The XRPD results show that, except that the free crystal form D was converted to the free crystal forms J + D after being placed under the 80 °C/sealed condition for 1 day, the crystal forms of the other samples remained unchanged.

**Table C-3. Summary of solid state stability evaluation**

| **Crystal form (Batch No.)** | **Conditions** | | **HPLC results** | | |
|---|---|---|---|---|---|
| | | | Purity (area%) | Relative to starting (%) | Crystal form |
| | Starting | | 98.49 | -- | -- |
| Free cryst al form D | 80 °C/sealed | 1 day | 98.51 | 100.0 | Free crystal forms J + D |
| | 25 °C/60% RH/open | 1 week | 98.52 | 100.0 | Free crystal form D |
| | 40 °C/75% RH/open | 1 week | 98.46 | 100.0 | |
| Crystal form B of sulfate | Starting | | 98.00 | -- | -- |
| | 80 °C/sealed | 1 day | 97.98 | 100.0 | Crystal form B of sulfate |
| | 25 °C/60% RH/open | 1 week | 97.97 | 100.0 | |
| | 40 °C/75% RH/open | 1 week | 97.98 | 100.0 | |
| Crystal form B of p-toluenesulfonate | Starting | | 98.28 | -- | -- |
| | 80 °C/sealed | 1 day | 98.28 | 100.0 | Crystal form B of p-toluenesulfonate |
| | 25 °C/60% RH/open | 1 week | 98.27 | 100.0 | |
| | 40 °C/75% RH/open | 1 week | 98.25 | 100.0 | |
| Crystal form A of maleate | Starting | | 98.29 | -- | -- |
| | 80 °C/sealed | 1 day | 98.08 | 99.8 | Crystal form A of maleate |
| | 25 °C/60% RH/open | 1 week | 98.26 | 100.0 | |
| | 40 °C/75% RH/open | 1 week | 98.30 | 100.0 | |
| Crystal form E of methanesulfonate | Starting | | 98.36 | -- | -- |
| | 80 °C/sealed | 1 day | 98.37 | 100.0 | Crystal form E of methanesulfonate |
| | 25 °C/60% RH/open | 1 week | 98.39 | 100.0 | |
| | 40 °C/75% RH/open | 1 week | 98.39 | 100.0 | |

### Test Example 3. Assay on Inhibition of KIF18A Enzymatic Activity by Compound of Formula I

The recombinant protein KIF18A (kinesin domain: aa 1-467) used in this assay was expressed by the Bac-to-Bac baculovirus expression system from Invitrogen, and the resulting protein was purified for use in this assay (Seki, M. et al. 2003 Nucleic Acids Res.). Quantitative detection of KIF18A enzymatic activity was completed using an ADP-Glo^{™} kinase assay kit (Promega Inc). Related procedures strictly followed the product instructions, which are briefly described as follows:
The reaction system consisted of a test compound, the recombinant protein KIF18A (aa 1-467), ATP (Promega Inc), and an assay buffer. The test compound was prepared into a 0.5 mM stock solution using DMSO (Sigma Inc) and then serially diluted in DMSO. The assay buffer consisted of an aqueous solution of 15 mM Tris, 10 mM MgCl₂ (Sigma Inc), 0.01% Pluronic F-68 (Life Technologies Inc), 1 µM paclitaxel (Cytoskeleton Inc), 30 µg/mL porcine tubulin (Cytoskeleton Inc), and 2% DMSO. The KIF18A protein (final concentration: 80 nM) and the compound at different concentrations (1 µL) were added to the prepared assay buffer (50 µL), and the mixture was incubated at room temperature for 15 min. Then, ATP (final concentration: 80 µM) was added to the reaction mixture, and the resulting mixture was incubated at room temperature for 3 h. After the reaction was completed, 5 µL of the ADP-Glo^{™} reagent and 2.5 µL of the reaction mixture were added to a 384-well plate (Grenier Inc). The resulting mixture was mixed homogeneously, then sealed by an aluminum foil sealing film, and incubated at room temperature in the dark for 40 min. Finally, 10 µL of the ADP-Glo^{™} assay reagent was added to each reaction well, and the mixture was incubated at room temperature in the dark for 40 min. After all reactions were completed, the luminescence values of the wells were read on a microplate reader (Molecular Device_SpectraMax Id5), and the inhibition ratios were calculated. The calculation formula for the inhibition rates was as follows: inhibition rate = (1 - (compound group - blank group) / (negative control group - blank group)) × 100%. Then, based on the inhibition rates at different compound concentrations, the IC₅₀ value of the test compound was calculated using the GraphPad Prism 8 software. The IC₅₀ value of the compound of formula I was 0.011 µM.

### Test Example 4. Assay on Inhibition of OVCAR3 Tumor Cell Proliferation by Compound of Formula I

NIH:OVCAR-3 cells (ATCC, Cat No. HTB-161, purchased from Co-Bier) were seeded into a T75 culture flask and cultured in an RPMI 1640 medium containing 20% FBS for 3 days in preparation for subsequent culturing and seeding into a 96-well plate for the cell proliferation assay. The OVCAR3 cells were seeded at a density of 3000 cells/100 µL/well into the 96-well cell plate, and then the plate was incubated in an incubator (37 °C, 5% CO₂) for 18 h. On the second day, drug treatment was started. Serially diluted test compound solutions (each drug starting at a concentration of 10 µM, diluted in DMSO, with a dilution ratio of 1:3, and nine gradient points for each drug) or DMSO (negative control) was added to the medium in the plate at 100 µL/well, and a blank group was set up with only medium, but no seeded cell strains. After the drug addition, the plate was incubated in the incubator for another 3 days (37 °C, 5% CO₂). On the fourth day, plate detection was performed. To each well, 100 µL of CellTiter-Glo reagent (Promega G9243) was added. The plate was shaken for 5 min and then left to stand at room temperature for 5 min. The chemical luminescence signal value of each well was determined using a microplate reader (PerkinElmer EnVision^{®}2104). Cell proliferation inhibition rate = (1 - (compound group - blank group) / (DMSO group - blank group)) × 100%. Then, the IC₅₀ value of the test compound was calculated using the GraphPad Prism 8 software. The IC₅₀ value of the compound of formula I was 0.096 µM.

The embodiments of the present disclosure have been described above. However, the embodiments described above are not intended to limit the present disclosure. Any modification, equivalent replacement, improvement, and the like made without departing from the spirit and principle of the present disclosure shall fall within the protection scope of the present disclosure.

## Claims

1. A crystal form of a compound of formula I, wherein the compound of formula I has a structure shown below:

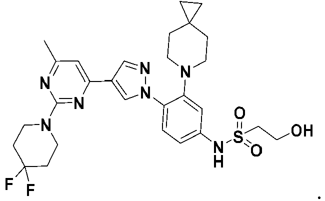

2. The crystal form of the compound of formula I according to claim 1, wherein the crystal form is free crystal form A of the compound of formula I, and the free crystal form A has an X-ray powder diffraction pattern comprising diffraction peaks at 2θ±0.2° diffraction angles of 3.8074, 11.3487, 15.1458, 15.4196, 16.2456, and 18.9612; further, the free crystal form A has an X-ray powder diffraction pattern comprising diffraction peaks at 2θ±0.2° diffraction angles of 3.8074, 9.8108, 10.3581, 11.3487, 13.4688, 15.1458, 15.4196, 16.2456, 16.8635, 17.3003, 18.9612, 22.6628, and 30.5503; furthermore, the free crystal form A has an X-ray powder diffraction pattern comprising diffraction peaks at 2θ±0.2° diffraction angles of 3.8074, 9.8108, 10.3581, 11.3487, 12.7984, 13.4688, 15.1458, 15.4196, 16.2456, 16.8635, 17.3003, 18.9612, 20.7248, 22.6628, 23.6322, 30.5503, 26.6666, 27.4293, and 28.2276; still further, the free crystal form A has an X-ray powder diffraction pattern comprising diffraction peaks at 2θ±0.2° diffraction angles of 3.8074, 9.8108, 10.3581, 11.3487, 12.7984, 13.4688, 15.1458, 15.4196, 16.2456, 16.8635, 17.3003, 18.9612, 20.7248, 22.6628, 23.6322, 24.5787, 25.7207, 26.6666, 27.4293, 28.2276, 30.5503, and 35.7206; still further, the free crystal form A has an XRPD pattern substantially as shown in FIG. 1;
preferably, the free crystal form A has one, two, or three of the following characteristics:
(1) a TGA curve of the free crystal form A showing a weight loss of 0-2%, preferably about 0-1% (e.g., 0.1%, 0.2%, 0.3%, 0.4%, 0.5%, 0.6%, 0.7%, or 0.8%) at 150.0±3 °C;
(2) a DSC curve of the free crystal form A having 2 endothermic peaks at 167.9±3 °C and 180.3±3 °C;
(3) a DSC curve of the free crystal form A having 1 exothermic peak at 170.5 °C±3 °C;
preferably, the free crystal form A has one, two, or three of the following characteristics:
(1) a TGA curve of the free crystal form A showing a weight loss of about 0.68% at 150.0±3 °C;
(2) a DSC curve of the free crystal form A having 2 endothermic peaks at 167.9±3 °C and 180.3±3 °C;
(3) a DSC curve of the free crystal form A having 1 exothermic peak at 170.5 °C±3 °C;
preferably, a TGA/DSC profile of the free crystal form A is substantially as shown in FIG. 2; a ¹H NMR spectrum of the free crystal form A is substantially as shown in FIG. 3a;
preferably, the free crystal form is an anhydrate;
or the crystal form is free crystal form B of the compound of formula I, and the free crystal form B has an X-ray powder diffraction pattern comprising diffraction peaks at 2θ±0.2° diffraction angles of 9.7812, 18.1668, 18.5510, 19.0431, 20.2387, and 21.1776; further, the free crystal form B has an X-ray powder diffraction pattern comprising diffraction peaks at 2θ±0.2° diffraction angles of 9.7812, 18.1668, 18.5510, 19.0431, 19.3191, 19.7143, 20.2387, and 21.1776; furthermore, the free crystal form B has an X-ray powder diffraction pattern comprising diffraction peaks at 2θ±0.2° diffraction angles of 9.7812, 9.9884, 18.1668, 18.5510, 19.0431, 19.3191, 19.7143, 20.2387, and 21.1776; furthermore, the free crystal form B has an X-ray powder diffraction pattern comprising diffraction peaks at 2θ±0.2° diffraction angles of 9.2508, 9.7812, 9.9884, 15.0024, 15.2215, 18.1668, 18.5510, 19.0431, 19.3191, 19.7143, 20.2387, 21.1776, 22.0900, 27.6189, and 28.2642; still further, the free crystal form B has an X-ray powder diffraction pattern comprising diffraction peaks at 2θ±0.2° diffraction angles of 9.2508, 9.7812, 9.9884, 10.8644, 13.3822, 14.5151, 15.0024, 15.2215, 16.3081, 16.7223, 17.1474, 18.1668, 18.5510, 19.0431, 19.3191, 19.7143, 20.2387, 21.1776, 22.0900, 23.0450, 23.7619, 25.7395, 27.6189, 28.2642, and 30.0557; still further, the free crystal form B has an XRPD pattern substantially as shown in FIG. 5;
preferably, the free crystal form B has one, two, or three of the following characteristics:
(1) a TGA curve of the free crystal form B showing a weight loss of 2-6%, preferably about 3-5% (e.g., 3.0%, 3.5%, 4.0%, or 4.5%) at 120.0±3 °C;
(2) a DSC curve of the free crystal form B having 3 endothermic peaks at 108.5±3 °C, 165.8±3 °C, and 180.2±3 °C;
(3) a DSC curve of the free crystal form B having 1 exothermic peak at 167.7 °C±3 °C;
preferably, the free crystal form B has one, two, or three of the following characteristics:
(1) a TGA curve of the free crystal form B showing a weight loss of about 4.35% at 120.0±3 °C;
(2) a DSC curve of the free crystal form B having 3 endothermic peaks at 108.5±3 °C, 165.8±3 °C, and 180.2±3 °C;
(3) a DSC curve of the free crystal form B having 1 exothermic peak at 167.7 °C±3 °C;
preferably, a TGA/DSC profile of the free crystal form B is substantially as shown in FIG. 6; a ¹H NMR spectrum of the free crystal form B is substantially as shown in FIG. 7a;
or the crystal form is free crystal form C of the compound of formula I, and the free crystal form C has an X-ray powder diffraction pattern comprising diffraction peaks at 2θ±0.2° diffraction angles of 9.5020, 16.1724, 16.5974, 21.5887, and 25.8930; further, the free crystal form C has an X-ray powder diffraction pattern comprising diffraction peaks at 2θ±0.2° diffraction angles of 9.5020, 15.3140, 16.1724, 16.5974, 18.8700, 19.9398, 20.5180, 21.0065, 21.5887, and 25.8930; furthermore, the free crystal form C has an X-ray powder diffraction pattern comprising diffraction peaks at 2θ±0.2° diffraction angles of 9.5020, 10.7612, 15.3140, 16.1724, 16.5974, 18.5313, 18.8700, 19.1799, 19.9398, 20.5180, 21.0065, 21.5887, 25.8930, and 28.4921; still further, the free crystal form C has an X-ray powder diffraction pattern comprising diffraction peaks at 2θ±0.2° diffraction angles of 3.1461, 8.1061, 9.1126, 9.5020, 10.7612, 11.2726, 12.2849, 13.1097, 14.2058, 14.7763, 15.3140, 16.1724, 16.5974, 17.5352, 18.5313, 18.8700, 19.1799, 19.9398, 20.5180, 21.0065, 21.2198, 21.5887, 22.1453, 22.7421, 23.2908, 24.1476, 24.6794, 25.4796, 25.8930, 27.4465, 28.4921, 29.8280, 30.7213, 31.4381, 32.6390, 35.3421, 36.5656, and 38.4547; still further, the free crystal form C has an XRPD pattern substantially as shown in FIG. 8;
preferably, the free crystal form C has one or two of the following characteristics:
(1) a TGA curve of the free crystal form C showing a weight loss of 1-6%, preferably about 1-4% (e.g., 1.0%, 1.5%, 2.0%, 2.5%, 3.0%, 3.5%, or 4.0%) at 150.0±3 °C;
(2) a DSC curve of the free crystal form C having 2 endothermic peaks at 53.0±3 °C and 182.3±3 °C;
preferably, the free crystal form C has one or two of the following characteristics:
(1) a TGA curve of the free crystal form C showing a weight loss of about 2.77% at 150.0±3 °C;
(2) a DSC curve of the free crystal form C having 2 endothermic peaks at 53.0±3 °C and 182.3±3 °C;
preferably, a TGA/DSC profile of the free crystal form C is substantially as shown in FIG. 9; a ¹H NMR spectrum of the free crystal form C is substantially as shown in FIG. 10;
or the crystal form is free crystal form D of the compound of formula I, and the free crystal form D has an X-ray powder diffraction pattern comprising diffraction peaks at 2θ±0.2° diffraction angles of 10.8461, 16.5793, 18.4363, 20.3590, 21.3940, 21.7745, and 26.5122; further, the free crystal form D has an X-ray powder diffraction pattern comprising diffraction peaks at 2θ±0.2° diffraction angles of 10.8461, 13.9909, 16.5793, 16.8857, 18.4363, 20.3590, 21.3940, 21.7745, and 26.5122; furthermore, the free crystal form D has an X-ray powder diffraction pattern comprising diffraction peaks at 2θ±0.2° diffraction angles of 10.8461, 13.9909, 14.9577, 16.5793, 16.8857, 18.4363, 20.1677, 20.3590, 21.3940, 21.7745, and 26.5122; furthermore, the free crystal form D has an X-ray powder diffraction pattern comprising diffraction peaks at 2θ±0.2° diffraction angles of 10.8461, 11.8218, 13.9909, 14.9577, 16.5793, 16.8857, 18.4363, 18.7513, 18.9436, 20.1677, 20.3590, 21.3940, 21.7745, 22.2999, 23.7694, 24.2605, 24.4938, 25.2628, 26.5122, 29.7635, and 30.8099; still further, the free crystal form D has an X-ray powder diffraction pattern comprising diffraction peaks at 2θ±0.2° diffraction angles of 8.2985, 9.4241, 10.8461, 11.8218, 12.5232, 13.2208, 13.9909, 14.9577, 16.5793, 16.8857, 18.4363, 18.7513, 18.9436, 20.1677, 20.3590, 21.3940, 21.7745, 22.2999, 23.7694, 24.2605, 24.4938, 25.2628, 26.5122, 29.2296, 29.7635, 30.8099, and 38.8753; still further, the free crystal form D has an XRPD pattern substantially as shown in FIG. 67;
preferably, the free crystal form D has one or two or more of the following characteristics:
(1) a TGA curve showing a weight loss of 3.0-8.0%, preferably about 4.0-7.0% (e.g., 5.0%, 5.5%, 6.0%, or 6.5%), and more preferably about 5.79% at 120+3 °C;
(2) a DSC curve having 2 endothermic peaks at 53.9±3 °C and 182.9±3 °C;
preferably, a TGA/DSC profile of the free crystal form D is substantially as shown in FIG. 68; a ¹H NMR spectrum of the free crystal form D is substantially as shown in FIG. 69;
preferably, the free crystal form D is a hydrate;
preferably, the free crystal form D has a water content of 2.0-3.5%, preferably 2.0-3.0%;
preferably, the free crystal form D is a monohydrate;
preferably, the free crystal form D has the following crystal parameters:
crystal system triclinic
space group P-1
unit cell parameters
a = 8.9217(3) Å, b = 10.9062(5) Å, c = 15.6399(5) Å
α = 95.3240(10)°, β = 97.7830(10)°, γ = 100.4940(10)°;
preferably, the free crystal form D has the parameters listed in Table B3-1;
or the crystal form is free crystal form E of the compound of formula I, and the free crystal form E has an X-ray powder diffraction pattern comprising diffraction peaks at 2θ±0.2° diffraction angles of 17.2384, 18.3327, 19.1326, 20.5505, 21.4769, and 24.8471; further, the free crystal form E has an X-ray powder diffraction pattern comprising diffraction peaks at 2θ±0.2° diffraction angles of 16.7268, 17.2384, 18.3327, 19.1326, 20.5505, 21.4769, 21.8496, and 24.8471; furthermore, the free crystal form E has an X-ray powder diffraction pattern comprising diffraction peaks at 2θ±0.2° diffraction angles of 9.1459, 12.1780, 16.7268, 17.2384, 18.3327, 18.9674, 19.1326, 20.5505, 21.4769, 21.8496, and 24.8471; furthermore, the free crystal form E has an X-ray powder diffraction pattern comprising diffraction peaks at 2θ±0.2° diffraction angles of 8.6847, 8.9272, 9.1459, 12.1780, 12.7789, 16.7268, 17.2384, 18.1543, 18.3327, 18.9674, 19.1326, 19.8795, 20.2931, 20.5505, 21.4769, 21.8496, 24.8471, and 28.5466; still further, the free crystal form E has an X-ray powder diffraction pattern comprising diffraction peaks at 2θ±0.2° diffraction angles of 8.6847, 8.9272, 9.1459, 9.4934, 12.1780, 12.7789, 15.3545, 16.7268, 17.2384, 18.1543, 18.3327, 18.9674, 19.1326, 19.8795, 20.2931, 20.5505, 21.4769, 21.8496, 22.1726, 23.0433, 24.8471, 25.6946, 26.8264, 28.5466, and 29.5968; still further, the free crystal form E has an XRPD pattern substantially as shown in FIG. 71;
preferably, the free crystal form E has one or two or more of the following characteristics:
(1) a TGA curve showing a weight loss of 9-14%, preferably about 10-13% (e.g., 10.5%, 11.0%, 11.5%, 12.0%, or 12.5%), and more preferably about 11.97% at 140±3 °C;
(2) a DSC curve having 2 endothermic peaks at 123.0±3 °C and 182.9±3 °C;
(3) a DSC curve having 1 exothermic peak at 148.0±3 °C;
preferably, a TGA/DSC profile of the free crystal form E is substantially as shown in FIG. 72; a ¹H NMR spectrum of the free crystal form E is substantially as shown in FIG. 73;
preferably, the free crystal form E is a solvate;
preferably, the free crystal form E is a THF solvate;
preferably, in the THF solvate of the free crystal form E, THF and the free crystal form are in a molar ratio of 0.95:1; or the crystal form is free crystal form F of the compound of formula I, and the free crystal form F has an X-ray powder diffraction pattern comprising diffraction peaks at 2θ±0.2° diffraction angles of 10.9091, 14.5589, 15.7854, 18.2249, and 18.5345; further, the free crystal form F has an X-ray powder diffraction pattern comprising diffraction peaks at 2θ±0.2° diffraction angles of 9.7443, 10.9091, 14.5589, 15.7854, 17.1471, 18.2249, and 18.5345; furthermore, the free crystal form F has an X-ray powder diffraction pattern comprising diffraction peaks at 2θ±0.2° diffraction angles of 9.7443, 10.9091, 14.5589, 15.7854, 17.1471, 17.7727, 18.2249, 18.5345, and 20.2138; furthermore, the free crystal form F has an X-ray powder diffraction pattern comprising diffraction peaks at 2θ±0.2° diffraction angles of 7.2680, 9.7443, 10.9091, 13.5597, 14.5589, 15.7854, 17.1471, 17.7727, 18.2249, 18.5345, 20.2138, and 24.7687; still further, the free crystal form F has an X-ray powder diffraction pattern comprising diffraction peaks at 2θ±0.2° diffraction angles of 7.2680, 9.7443, 10.9091, 12.6503, 13.5597, 14.5589, 15.0095, 15.7854, 17.1471, 17.7727, 18.2249, 18.5345, 19.4299, 20.2138, 21.2140, 21.5620, 22.3451, 23.2974, 23.7177, 24.7687, 25.6111, 27.5720, 28.4674, 29.3309, 30.7039, 31.5840, 34.3936, 35.1042, 36.9389, and 38.7357; still further, the free crystal form F has an XRPD pattern substantially as shown in FIG. 76;
preferably, the free crystal form F has one or two or more of the following characteristics:
(1) a TGA curve showing a weight loss of 0.5-2.5%, preferably about 0.5-2.0% (e.g., 0.5%, 1.0%, or 1.5%), and more preferably about 1.19% at 150±3 °C;
(2) a DSC curve having 1 endothermic peak at 181.1±3 °C (peak temperature);
preferably, a TGA/DSC profile of the free crystal form F is substantially as shown in FIG. 77; a ¹H NMR spectrum of the free crystal form F is substantially as shown in FIG. 78;
preferably, the free crystal form F is an anhydrate;
or the crystal form is free crystal form G of the compound of formula I, and the free crystal form G has an X-ray powder diffraction pattern comprising diffraction peaks at 2θ±0.2° diffraction angles of 6.1695, 12.2893, 18.5441, 19.4906, and 22.7762; further, the free crystal form G has an X-ray powder diffraction pattern comprising diffraction peaks at 2θ±0.2° diffraction angles of 6.1695, 10.5406, 12.2893, 14.5010, 18.5441, 19.4906, and 22.7762; furthermore, the free crystal form G has an X-ray powder diffraction pattern comprising diffraction peaks at 2θ±0.2° diffraction angles of 6.1695, 9.2517, 9.4965, 10.5406, 12.2893, 14.5010, 18.5441, 18.8489, 19.4906, and 22.7762; furthermore, the free crystal form G has an X-ray powder diffraction pattern comprising diffraction peaks at 2θ±0.2° diffraction angles of 6.1695, 9.2517, 9.4965, 10.5406, 12.2893, 14.5010, 16.9986, 18.5441, 18.8489, 19.4906, 21.1520, 21.6669, 22.4742, 22.7762, and 24.7980; still further, the free crystal form G has an X-ray powder diffraction pattern comprising diffraction peaks at 2θ±0.2° diffraction angles of 6.1695, 9.2517, 9.4965, 10.2696, 10.5406, 10.9989, 12.2893, 14.5010, 16.5003, 16.9986, 18.5441, 18.8489, 19.4906, 20.6318, 21.1520, 21.6669, 22.4742, 22.7762, 24.7980, 25.3519, 26.0012, 26.8697, and 28.1782; still further, the free crystal form G has an XRPD pattern substantially as shown in FIG. 79;
preferably, the free crystal form G has one or two or more of the following characteristics:
(1) a TGA curve showing a weight loss of 8-13%, preferably about 9-11% (e.g., 9.5%, 10.0%, 10.5%, or 11.0%), and more preferably about 10.40% at 110±3 °C;
(2) a DSC curve having 3 endothermic peaks at 98.1±3 °C, 154.8±3 °C, and 180.8±3 °C;
(3) a DSC curve having 1 exothermic peak at 157.8±3 °C;
preferably, a TGA/DSC profile of the free crystal form G is substantially as shown in FIG. 80; a ¹H NMR spectrum of the free crystal form G is substantially as shown in FIG. 81;
preferably, the free crystal form G is a solvate;
preferably, the free crystal form G is a 1,4-dioxane solvate; in some embodiments, in the solvate, 1,4-dioxane and the free crystal form are in a molar ratio of 0.04 (about 0.6 wt%) or 0.02 (about 0.3 wt%);
or the crystal form is free crystal form H of the compound of formula I, and the free crystal form H has an X-ray powder diffraction pattern comprising diffraction peaks at 2θ±0.2° diffraction angles of 5.1382, 17.0866, 18.9850, 19.4851, 20.1891, and 21.3352; further, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, and 21.3352; furthermore, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, 21.3352, 21.5416, and 24.7905; furthermore, the free crystal form H has an X-ray powder diffraction pattern comprising diffraction peaks at 2θ±0.2° diffraction angles of 5.1382, 14.9595, 17.0866, 17.5191, 17.7454, 18.9850, 19.4851, 20.1891, 20.5085, 21.3352, 21.5416, 24.5735, and 24.7905; still further, the free crystal form H has an X-ray powder diffraction pattern comprising diffraction peaks at 2θ±0.2° diffraction angles of 5.1382, 8.6958, 10.2939, 11.0299, 11.5587, 12.1517, 13.0908, 14.5022, 14.9595, 15.2308, 16.5672, 17.0866, 17.5191, 17.7454, 18.4679, 18.9850, 19.4851, 20.1891, 20.5085, 21.3352, 21.5416, 22.2233, 22.7317, 24.2236, 24.5735, 24.7905, 25.0488, 26.0331, and 28.5683; still further, the free crystal form H has an XRPD pattern substantially as shown in FIG. 85b;
or the crystal form is free crystal form I of the compound of formula I, and the free crystal form I has an X-ray powder diffraction pattern comprising diffraction peaks at 2θ±0.2° diffraction angles of 9.5592, 17.8352, 19.3336, 19.8678, 20.1554, and 27.1065; further, the free crystal form I has an X-ray powder diffraction pattern comprising diffraction peaks at 2θ±0.2° diffraction angles of 9.5592, 12.8988, 17.8352, 19.3336, 19.8678, 20.1554, and 27.1065; furthermore, the free crystal form I has an X-ray powder diffraction pattern comprising diffraction peaks at 2θ±0.2° diffraction angles of 9.5592, 12.8988, 17.8352, 19.3336, 19.8678, 20.1554, 20.9976, and 27.1065; furthermore, the free crystal form I has an X-ray powder diffraction pattern comprising diffraction peaks at 2θ±0.2° diffraction angles of 9.5592, 12.8988, 17.8352, 18.1790, 19.3336, 19.8678, 20.1554, 20.7214, 20.9976, 27.1065, and 28.0012; still further, the free crystal form I has an X-ray powder diffraction pattern comprising diffraction peaks at 2θ±0.2° diffraction angles of 9.5592, 12.8988, 14.7994, 15.8059, 17.8352, 18.1790, 19.3336, 19.8678, 20.1554, 20.7214, 20.9976, 23.2310, 27.1065, and 28.0012; still further, the free crystal form I has an XRPD pattern substantially as shown in FIG. 86;
preferably, the free crystal form I has one or two or more of the following characteristics:
(1) a TGA curve showing a weight loss of 2-7%, preferably about 3-6% (e.g., 3.5%, 4.0%, or 4.5%) at 120±3 °C;
(2) a DSC curve having 3 endothermic peaks at 54.6±3 °C, 168.0±3 °C, and 181.2±3 °C;
(3) a DSC curve having 1 exothermic peak at 170.5±3 °C;
preferably, a TGA/DSC profile of the free crystal form I is substantially as shown in FIG. 87; a ¹H NMR spectrum of the free crystal form I is substantially as shown in FIG. 88;
preferably, the free crystal form I is a hydrate;
or the crystal form is free crystal form J of the compound of formula I, and the free crystal form J has an X-ray powder diffraction pattern comprising diffraction peaks at 2θ±0.2° diffraction angles of 9.4887, 10.7503, 15.3049, 16.1644, 16.5855, 20.9755, 21.5744, and 25.8914; further, the free crystal form J has an X-ray powder diffraction pattern comprising diffraction peaks at 2θ±0.2° diffraction angles of 9.4887, 10.7503, 15.3049, 16.1644, 16.5855, 18.8695, 20.5033, 20.9755, 21.5744, and 25.8914; furthermore, the free crystal form J has an X-ray powder diffraction pattern comprising diffraction peaks at 2θ±0.2° diffraction angles of 9.4887, 10.7503, 15.3049, 16.1644, 16.5855, 18.5225, 18.8695, 19.9313, 20.5033, 20.9755, 21.5744, and 25.8914; furthermore, the free crystal form J has an X-ray powder diffraction pattern comprising diffraction peaks at 2θ±0.2° diffraction angles of 9.4887, 10.7503, 12.2600, 13.0965, 14.1913, 15.3049, 16.1644, 16.5855, 18.5225, 18.8695, 19.1552, 19.9313, 20.5033, 20.9755, 21.5744, 22.1570, 23.2764, 25.8914, and 28.4642; still further, the free crystal form J has an X-ray powder diffraction pattern comprising diffraction peaks at 2θ±0.2° diffraction angles of 9.4887, 10.7503, 12.2600, 13.0965, 14.1913, 15.3049, 16.1644, 16.5855, 17.5096, 18.5225, 18.8695, 19.1552, 19.9313, 20.5033, 20.9755, 21.5744, 22.1570, 23.2764, 24.1115, 24.6829, 25.8914, 27.1763, 28.4642, 29.7254, 30.7261, and 32.6167; still further, the free crystal form J has an XRPD pattern substantially as shown in FIG. 90;
preferably, the free crystal form J has one or two or more of the following characteristics:
(1) a TGA curve showing a weight loss of 0.5-2.5%, preferably about 0.5-1.5% (e.g., 0.5%, 1.0%, or 1.5%), and more preferably about 1.09% at 150±3 °C;
(2) a DSC curve having 1 endothermic peak at 181.5±3 °C;
preferably, a TGA/DSC profile of the free crystal form J is substantially as shown in FIG. 91; a ¹H NMR spectrum of the free crystal form J is substantially as shown in FIG. 92;
preferably, the free crystal form J is an anhydrate;
or the crystal form is free crystal form K of the compound of formula I, and the free crystal form K has an X-ray powder diffraction pattern comprising diffraction peaks at 2θ±0.2° diffraction angles of 15.0722, 18.7484, 19.0653, 19.7672, 20.4112, and 20.9312; further, the free crystal form K has an X-ray powder diffraction pattern comprising diffraction peaks at 2θ±0.2° diffraction angles of 9.8469, 15.0722, 18.7484, 19.0653, 19.2235, 19.4283, 19.7672, 20.4112, 20.9312, and 21.1881; furthermore, the free crystal form K has an X-ray powder diffraction pattern comprising diffraction peaks at 2θ±0.2° diffraction angles of 9.8469, 10.1208, 15.0722, 18.7484, 19.0653, 19.2235, 19.4283, 19.7672, 20.4112, 20.9312, 21.1881, 21.6963, and 28.3413; furthermore, the free crystal form K has an X-ray powder diffraction pattern comprising diffraction peaks at 2θ±0.2° diffraction angles of 9.8469, 10.1208, 14.6920, 15.0722, 15.4155, 16.0745, 16.7829, 17.6356, 18.1386, 18.7484, 19.0653, 19.2235, 19.4283, 19.7672, 20.4112, 20.9312, 21.1881, 21.6963, 24.0591, 28.3413, 28.6573, and 30.5394; still further, the free crystal form K has an X-ray powder diffraction pattern comprising diffraction peaks at 2θ±0.2° diffraction angles of 9.3256, 9.8469, 10.1208, 13.0145, 14.6920, 15.0722, 15.4155, 16.0745, 16.7829, 17.6356, 18.1386, 18.7484, 19.0653, 19.2235, 19.4283, 19.7672, 20.4112, 20.9312, 21.1881, 21.6963, 24.0591, 26.2506, 28.3413, 28.6573, 30.5394, and 33.0658; still further, the free crystal form K has an XRPD pattern substantially as shown in FIG. 94;
preferably, the free crystal form K has one or two or more of the following characteristics:
(1) a TGA curve showing a weight loss of 6-11%, preferably about 7-10% (e.g., 7.0%, 7.5%, 8.0%, 8.5%, or 9.0%), and more preferably about 8.24% at 150±3 °C;
(2) a DSC curve having 3 endothermic peaks at 72.0±3 °C, 165.2±3 °C, and 180.2±3 °C;
(3) a DSC curve having 1 exothermic peak at 167.1±3 °.
preferably, a TGA/DSC profile of the free crystal form K is substantially as shown in FIG. 95; a ¹H NMR spectrum of the free crystal form K is substantially as shown in FIG. 96;
preferably, the free crystal form K is a solvate;
preferably, the free crystal form K is an EtOH solvate or a co-solvate of EtOH and water;
preferably, in the solvate, EtOH and the free crystal form are in a molar ratio of 0.27;
or the crystal form is free crystal form L of the compound of formula I, and the free crystal form L has an X-ray powder diffraction pattern comprising diffraction peaks at 2θ±0.2° diffraction angles of 8.8304, 15.8341, 17.7145, 19.1795, 20.5748, and 25.5115; further, the free crystal form L has an X-ray powder diffraction pattern comprising diffraction peaks at 2θ±0.2° diffraction angles of 8.8304, 15.8341, 17.7145, 19.1795, 20.0943, 20.5748, and 25.5115; furthermore, the free crystal form L has an X-ray powder diffraction pattern comprising diffraction peaks at 2θ±0.2° diffraction angles of 8.8304, 15.8341, 17.7145, 19.1795, 20.0943, 20.5748, 22.4409, and 25.5115; furthermore, the free crystal form L has an X-ray powder diffraction pattern comprising diffraction peaks at 2θ±0.2° diffraction angles of 8.8304, 11.1727, 15.8341, 16.8550, 17.1190, 17.7145, 19.1795, 19.7076, 20.0943, 20.5748, 22.4409, and 25.5115; still further, the free crystal form L has an X-ray powder diffraction pattern comprising diffraction peaks at 2θ±0.2° diffraction angles of 8.8304, 9.6805, 10.2446, 10.7612, 11.1727, 14.5973, 15.5971, 15.8341, 16.8550, 17.1190, 17.7145, 18.2884, 19.1795, 19.7076, 20.0943, 20.5748, 22.4409, 25.5115, 28.7935, 29.8794, 31.2614, 32.0233, and 35.9945; still further, the free crystal form L has an XRPD pattern substantially as shown in FIG. 99b.

3. A pharmaceutically acceptable salt of a compound of formula I or a crystal form thereof, wherein the pharmaceutically acceptable salt is selected from a salt formed by the compound of formula I with an acid, and the acid may be selected from an inorganic acid and an organic acid;
wherein the compound of formula I has a structure shown below: 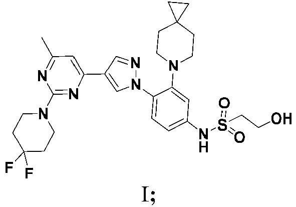
preferably, the pharmaceutically acceptable salt of the compound of formula I is selected from hydrochloride, sulfate, maleate, phosphate, p-toluenesulfonate, methanesulfonate, ethanesulfonate, L-aspartate, fumarate, and tartrate of the compound of formula I;
preferably, the pharmaceutically acceptable salt of the compound of formula I is hydrochloride, sulfate, maleate, phosphate, p-toluenesulfonate, methanesulfonate, and ethanesulfonate of the compound of formula I;
preferably, when the compound of formula I forms a salt with an acid, the acid and the compound of formula I may be in a molar ratio of 5:1 to 1:5, e.g., 5:1, 4.5:1, 4:1, 3.5:1, 3:1, 2.5:1, 2:1, 1.5:1, 1:1, 1:1.5, 1:2, 1:2.5, 1:3, 1:3.5, 1:4, 1:4.5, or 1:5.

4. The pharmaceutically acceptable salt of the compound of formula I or the crystal form thereof according to claim 3, wherein the crystal form is a crystal form of the hydrochloride of the compound of formula I; preferably, the crystal form is crystal form A of the hydrochloride of the compound of formula I, and the crystal form A of the hydrochloride has an X-ray powder diffraction pattern comprising diffraction peaks at 2θ±0.2° diffraction angles of 18.8832, 19.5324, 20.1382, 21.3379, and 21.7432; further, the crystal form A of the hydrochloride has an X-ray powder diffraction pattern comprising diffraction peaks at 2θ±0.2° diffraction angles of 14.7835, 14.9893, 18.2960, 18.8832, 19.3310, 19.5324, 20.1382, 21.3379, and 21.7432; furthermore, the crystal form A of the hydrochloride has an X-ray powder diffraction pattern comprising diffraction peaks at 2θ±0.2° diffraction angles of 10.1755, 14.7835, 14.9893, 18.2960, 18.8832, 19.3310, 19.5324, 20.1382, 20.9418, 21.3379, 21.7432, 25.7379, 27.2284, and 29.8240; still further, the crystal form A of the hydrochloride has an X-ray powder diffraction pattern comprising diffraction peaks at 2θ±0.2° diffraction angles of 6.6910, 9.4043, 10.1755, 11.3549, 12.0715, 12.7466, 13.3874, 14.1825, 14.7835, 14.9893, 17.2365, 18.2960, 18.8832, 19.3310, 19.5324, 20.1382, 20.9418, 21.3379, 21.7432, 22.5426, 23.5259, 24.0002, 25.3951, 25.7379, 26.6858, 27.2284, 27.7725, 28.5839, 29.1003, 29.8240, 30.7557, 31.8138, 33.7066, 34.5682, 35.3206, 36.1697, 37.1954, 38.5831, and 39.2520; still further, the crystal form A of the hydrochloride has an XRPD pattern substantially as shown in FIG. 12a or 12b;
preferably, the crystal form A of the hydrochloride has one or two of the following characteristics:
(1) a TGA curve of the crystal form A of the hydrochloride showing a weight loss of 4-12%, preferably about 6-10% (e.g., 6.0%, 6.5%, 7.0%, 7.5%, 8.0%, 8.5%, 9.0%, or 9.5%) at 160.0±3 °C;
(2) a DSC curve of the crystal form A of the hydrochloride having an endothermic peak at 165.8±3 °C;
in some embodiments, the crystal form A of the hydrochloride has one or two of the following characteristics:
(1) a TGA curve of the crystal form A of the hydrochloride showing a weight loss of 8.21% at 160.0±3 °C;
(2) a DSC curve of the crystal form A of the hydrochloride having an endothermic peak at 165.8±3 °C;
preferably, a TGA/DSC profile of the crystal form A of the hydrochloride is substantially as shown in FIG. 13; a ¹H NMR spectrum of the crystal form A of the hydrochloride is substantially as shown in FIG. 14;
preferably, the crystal form is crystal form B of the hydrochloride of the compound of formula I, and the crystal form B of the hydrochloride has an X-ray powder diffraction pattern comprising diffraction peaks at 2θ±0.2° diffraction angles of 5.8284, 12.9418, 15.8305, 17.4248, 17.7208, and 25.1539; further, the crystal form B of the hydrochloride has an X-ray powder diffraction pattern comprising diffraction peaks at 2θ±0.2° diffraction angles of 5.8284, 12.9418, 15.6394, 15.8305, 16.6807, 17.4248, 17.7208, 20.3860, 21.1241, 24.6834, and 25.1539; furthermore, the crystal form B of the hydrochloride has an X-ray powder diffraction pattern comprising diffraction peaks at 2θ±0.2° diffraction angles of 5.8284, 12.9418, 15.6394, 15.8305, 16.6807, 17.4248, 17.7208, 20.3860, 21.1241, 24.6834, and 25.1539; furthermore, the crystal form B of the hydrochloride has an X-ray powder diffraction pattern comprising diffraction peaks at 2θ±0.2° diffraction angles of 5.8284, 12.0258, 12.9418, 14.5910, 15.6394, 15.8305, 16.6807, 17.4248, 17.7208, 18.9434, 19.3954, 19.8130, 20.3860, 21.1241, 24.6834, and 25.1539; still further, the crystal form B of the hydrochloride has an X-ray powder diffraction pattern comprising diffraction peaks at 2θ±0.2° diffraction angles of 5.8284, 10.2084, 12.0258, 12.9418, 14.5910, 15.6394, 15.8305, 16.6807, 17.0761, 17.4248, 17.7208, 18.9434, 19.3954, 19.8130, 20.3860, 21.1241, 23.0260, 23.5341, 24.6834, 25.1539, 26.7017, 27.1748, 28.3119, 29.2775, 30.8075, and 34.0826; still further, the crystal form B of the hydrochloride has an XRPD pattern substantially as shown in FIG. 15;
preferably, the crystal form B of the hydrochloride has one or two of the following characteristics:
(1) a TGA curve of the crystal form B of the hydrochloride showing a weight loss of 10-20%, preferably about 12-18% (e.g., 12.0%, 12.5%, 13.0%, 13.5%, 14.0%, 14.5%, 15.0%, 15.5%, 16.0%, 16.5%, 17.0%, 17.5%, or 18.0%) at 180.0±3 °C;
(2) a DSC curve of the crystal form B of the hydrochloride having 2 endothermic peaks at 154.1±3 °C and 171.0±3 °C;
preferably, the crystal form B of the hydrochloride has one or two of the following characteristics:
(1) a TGA curve of the crystal form B of the hydrochloride showing a weight loss of 16.61% at 180.0±3 °C;
(2) a DSC curve of the crystal form B of the hydrochloride having 2 endothermic peaks at 154.1±3 °C and 171.0±3 °C; preferably, a TGA/DSC profile of the crystal form B of the hydrochloride is substantially as shown in FIG. 16; a ¹H NMR spectrum of the crystal form B of the hydrochloride is substantially as shown in FIG. 17;
preferably, the crystal form is crystal form C of the hydrochloride of the compound of formula I, and the crystal form C of the hydrochloride has an X-ray powder diffraction pattern comprising diffraction peaks at 2θ±0.2° diffraction angles of 5.6027, 5.7758, 13.6699, 14.4660, 21.6765, 23.2062, and 27.1991; further, the crystal form C of the hydrochloride has an X-ray powder diffraction pattern comprising diffraction peaks at 2θ±0.2° diffraction angles of 5.6027, 5.7758, 13.6699, 14.4660, 17.1465, 18.7028, 21.6765, 23.2062, and 27.1991; furthermore, the crystal form C of the hydrochloride has an X-ray powder diffraction pattern comprising diffraction peaks at 2θ±0.2° diffraction angles of 5.6027, 5.7758, 13.2815, 13.6699, 13.8622, 14.4660, 17.1465, 18.7028, 21.6765, 23.2062, and 27.1991; furthermore, the crystal form C of the hydrochloride has an X-ray powder diffraction pattern comprising diffraction peaks at 2θ±0.2° diffraction angles of 5.6027, 5.7758, 8.7367, 11.1522, 13.2815, 13.6699, 13.8622, 14.4660, 16.6600, 17.1465, 18.7028, 19.4828, 20.9473, 21.6765, 22.4844, 23.2062, 26.3673, and 27.1991; still further, the crystal form C of the hydrochloride has an X-ray powder diffraction pattern comprising diffraction peaks at 2θ±0.2° diffraction angles of 5.6027, 5.7758, 8.7367, 9.2140, 11.1522, 11.8436, 13.2815, 13.6699, 13.8622, 14.4660, 15.2083, 16.1990, 16.6600, 17.1465, 17.9792, 18.7028, 19.4828, 20.0737, 20.3774, 20.9473, 21.6765, 22.0633, 22.4844, 23.2062, 24.0140, 24.9698, 25.8392, 26.3673, 27.1991, 28.0148, 29.1691, and 37.7780; still further, the crystal form C of the hydrochloride has an XRPD pattern substantially as shown in FIG. 18;
preferably, the crystal form C of the hydrochloride has one, two, or three of the following characteristics:
(1) a TGA curve of the crystal form C of the hydrochloride showing a weight loss of 2-10%, preferably about 3-6% (e.g., 3.0%, 3.5%, 4.0%, 4.5%, 5.0%, 5.5%, or 6.0%) at 90.0±3 °C;
(2) a TGA curve of the crystal form C of the hydrochloride showing a weight loss of 2-10%, preferably about 5-8% (e.g., 5.0%, 5.5%, 6.0%, 6.5%, 7.0%, 7.5%, or 8.0%) at 170.0±3 °C;
(3) a DSC curve of the crystal form C of the hydrochloride having 2 endothermic peaks at 77.8±3 °C and 174.6±3 °C;
preferably, the crystal form C of the hydrochloride has one, two, or three of the following characteristics:
(1) a TGA curve of the crystal form C of the hydrochloride showing a weight loss of 4.67% at 90.0±3 °C;
(2) a TGA curve of the crystal form C of the hydrochloride showing a weight loss of 6.00% at 170.0±3 °C;
(3) a DSC curve of the crystal form C of the hydrochloride having 2 endothermic peaks at 77.8±3 °C and 174.6±3 °C;
preferably, a TGA/DSC profile of the crystal form C of the hydrochloride is substantially as shown in FIG. 19; a ¹H NMR spectrum of the crystal form B of the hydrochloride is substantially as shown in FIG. 20;
preferably, the crystal form is crystal form D of the hydrochloride of the compound of formula I, and the crystal form D of the hydrochloride has an X-ray powder diffraction pattern comprising diffraction peaks at 2θ±0.2° diffraction angles of 14.4539, 14.6720, 18.0560, 18.7688, 20.3323, and 21.2274; further, the crystal form D of the hydrochloride has an X-ray powder diffraction pattern comprising diffraction peaks at 2θ±0.2° diffraction angles of 6.5782, 11.8655, 14.4539, 14.6720, 18.0560, 18.7688, 20.3323, and 21.2274; furthermore, the crystal form D of the hydrochloride has an X-ray powder diffraction pattern comprising diffraction peaks at 2θ±0.2° diffraction angles of 6.5782, 9.9619, 11.8655, 14.4539, 14.6720, 18.0560, 18.7688, 20.3323, and 21.2274; furthermore, the crystal form D of the hydrochloride has an X-ray powder diffraction pattern comprising diffraction peaks at 2θ±0.2° diffraction angles of 6.5782, 9.9619, 11.1481, 11.8655, 14.4539, 14.6720, 18.0560, 18.7688, 20.3323, 21.2274, and 26.6683; still further, the crystal form D of the hydrochloride has an X-ray powder diffraction pattern comprising diffraction peaks at 2θ±0.2° diffraction angles of 6.5782, 9.9619, 11.1481, 11.8655, 12.8040, 14.4539, 14.6720, 18.0560, 18.7688, 20.3323, 21.2274, 26.6683, and 29.0358; still further, the crystal form D of the hydrochloride has an XRPD pattern substantially as shown in FIG. 107;
preferably, the crystal form is crystal form E of the hydrochloride of the compound of formula I, and the crystal form E of the hydrochloride has an X-ray powder diffraction pattern comprising diffraction peaks at 2θ±0.2° diffraction angles of 5.9750, 12.8980, 15.2093, 15.6063, 17.9714, and 25.4838; further, the crystal form E of the hydrochloride has an X-ray powder diffraction pattern comprising diffraction peaks at 2θ±0.2° diffraction angles of 5.9750, 12.8980, 15.2093, 15.6063, 17.9714, 24.9674, and 25.4838; furthermore, the crystal form E of the hydrochloride has an X-ray powder diffraction pattern comprising diffraction peaks at 2θ±0.2° diffraction angles of 5.9750, 12.8980, 15.0507, 15.2093, 15.6063, 17.8054, 17.9714, 24.9674, and 25.4838; furthermore, the crystal form E of the hydrochloride has an X-ray powder diffraction pattern comprising diffraction peaks at 2θ±0.2° diffraction angles of 5.9750, 12.8980, 15.0507, 15.2093, 15.6063, 16.0284, 17.8054, 17.9714, 19.8405, 20.5435, 22.5488, 24.9674, 25.4838, and 26.1257; still further, the crystal form E of the hydrochloride has an X-ray powder diffraction pattern comprising diffraction peaks at 2θ±0.2° diffraction angles of 5.9750, 12.8980, 13.7925, 15.0507, 15.2093, 15.6063, 16.0284, 17.8054, 17.9714, 18.9298, 19.8405, 20.5435, 21.0218, 21.9935, 22.5488, 24.9674, 25.4838, 26.1257, 27.4268, 29.8322, 30.4635, and 31.7533; still further, the crystal form E of the hydrochloride has an XRPD pattern substantially as shown in FIG. 110;
preferably, the crystal form is crystal form F of the hydrochloride of the compound of formula I, and the crystal form F of the hydrochloride has an X-ray powder diffraction pattern comprising diffraction peaks at 2θ±0.2° diffraction angles of 6.0385, 15.9019, 18.1478, 20.5749, and 25.4190; further, the crystal form F of the hydrochloride has an X-ray powder diffraction pattern comprising diffraction peaks at 2θ±0.2° diffraction angles of 6.0385, 12.6362, 15.9019, 17.1363, 18.1478, 20.5749, and 25.4190; furthermore, the crystal form F of the hydrochloride has an X-ray powder diffraction pattern comprising diffraction peaks at 2θ±0.2° diffraction angles of 6.0385, 12.6362, 13.0399, 15.9019, 17.1363, 18.1478, 18.8427, 20.5749, 21.8092, and 25.4190; furthermore, the crystal form F of the hydrochloride has an X-ray powder diffraction pattern comprising diffraction peaks at 2θ±0.2° diffraction angles of 6.0385, 12.6362, 13.0399, 15.9019, 16.8142, 17.1363, 18.1478, 18.4776, 18.8427, 19.3354, 20.5749, 21.3694, 21.8092, 25.1076, and 25.4190; still further, the crystal form F of the hydrochloride has an X-ray powder diffraction pattern comprising diffraction peaks at 2θ±0.2° diffraction angles of 6.0385, 12.0772, 12.6362, 13.0399, 13.5971, 14.9525, 15.9019, 16.8142, 17.1363, 18.1478, 18.4776, 18.8427, 19.3354, 20.5749, 21.3694, 21.8092, 23.2223, 25.1076, 25.4190, 27.1737, 27.8200, 28.2136, 28.9299, 29.9049, 31.9291, 34.1705, 35.2232, 36.7164, and 39.1368; still further, the crystal form F of the hydrochloride has an XRPD pattern substantially as shown in FIG. 111;
preferably, the crystal form is crystal form G of the hydrochloride of the compound of formula I, and the crystal form G of the hydrochloride has an X-ray powder diffraction pattern comprising diffraction peaks at 2θ±0.2° diffraction angles of 9.4719, 18.6112, 19.0898, 20.7520, and 24.9637; further, the crystal form G of the hydrochloride has an X-ray powder diffraction pattern comprising diffraction peaks at 2θ±0.2° diffraction angles of 6.5684, 9.4719, 15.5605, 18.6112, 19.0898, 20.7520, and 24.9637; furthermore, the crystal form G of the hydrochloride has an X-ray powder diffraction pattern comprising diffraction peaks at 2θ±0.2° diffraction angles of 6.5684, 7.0798, 9.4719, 15.5605, 18.6112, 19.0898, 20.7520, and 24.9637; furthermore, the crystal form G of the hydrochloride has an X-ray powder diffraction pattern comprising diffraction peaks at 2θ±0.2° diffraction angles of 6.5684, 7.0798, 9.4719, 15.5605, 18.6112, 19.0898, 20.7520, 22.1933, 24.9637, and 28.7471; still further, the crystal form G of the hydrochloride has an X-ray powder diffraction pattern comprising diffraction peaks at 2θ±0.2° diffraction angles of 6.5684, 7.0798, 9.4719, 13.6772, 14.4725, 15.5605, 18.6112, 19.0898, 20.7520, 22.1933, 24.9637, and 28.7471; still further, the crystal form G of the hydrochloride has an XRPD pattern substantially as shown in FIG. 106b;
preferably, the crystal form is crystal form H of the hydrochloride of the compound of formula I, and the crystal form H of the hydrochloride has an X-ray powder diffraction pattern comprising diffraction peaks at 2θ±0.2° diffraction angles of 12.9273, 15.1559, 15.5659, 17.6473, 18.2972, 19.4651, 19.7972, and 20.4603; further, the crystal form H of the hydrochloride has an X-ray powder diffraction pattern comprising diffraction peaks at 2θ±0.2° diffraction angles of 12.9273, 15.1559, 15.5659, 16.8381, 17.6473, 18.2972, 19.4651, 19.7972, 20.4603, and 20.7147; furthermore, the crystal form H of the hydrochloride has an X-ray powder diffraction pattern comprising diffraction peaks at 2θ±0.2° diffraction angles of 7.5428, 12.4381, 12.9273, 15.1559, 15.5659, 16.8381, 17.6473, 18.2972, 19.1721, 19.4651, 19.7972, 20.4603, and 20.7147; furthermore, the crystal form H of the hydrochloride has an X-ray powder diffraction pattern comprising diffraction peaks at 2θ±0.2° diffraction angles of 7.5428, 8.4236, 10.0644, 10.4677, 12.0841, 12.4381, 12.9273, 13.5430, 15.1559, 15.5659, 16.8381, 17.6473, 18.2972, 18.7344, 19.1721, 19.4651, 19.7972, 20.4603, 20.7147, 20.9217, 21.5046, 21.7941, 22.4391, 22.7460, 23.8221, 25.3590, and 27.6629; still further, the crystal form H of the hydrochloride has an X-ray powder diffraction pattern comprising diffraction peaks at 2θ±0.2° diffraction angles of 7.5428, 8.4236, 10.0644, 10.4677, 12.0841, 12.4381, 12.9273, 13.5430, 15.1559, 15.5659, 16.2484, 16.8381, 17.6473, 18.2972, 18.7344, 19.1721, 19.4651, 19.7972, 20.4603, 20.7147, 20.9217, 21.5046, 21.7941, 22.4391, 22.7460, 23.8221, 25.3590, 25.9795, 26.4453, 27.1720, 27.6629, 28.6111, 29.0095, and 30.6017; still further, the crystal form H of the hydrochloride has an XRPD pattern substantially as shown in FIG. 106c;
preferably, the crystal form H of the hydrochloride has one or two or more of the following characteristics:
(1) a TGA curve showing a weight loss of 5-10%, preferably about 6-9% (e.g., 7.0%, 7.5%, or 8.0%), and more preferably about 7.61% at 170±3 °C;
(2) a DSC curve having 2 endothermic peaks at 155.5 °C and 191.5 °C;
preferably, a TGA/DSC profile of the crystal form H of the hydrochloride is substantially as shown in FIG. 108; a ¹H NMR spectrum of the crystal form H of the hydrochloride is substantially as shown in FIG. 109;
preferably, the crystal form is crystal form I of the hydrochloride of the compound of formula I, and the crystal form I of the hydrochloride has an X-ray powder diffraction pattern comprising diffraction peaks at 2θ±0.2° diffraction angles of 6.2065, 7.0236, 13.8915, 14.6038, 20.8063, 21.1874, and 24.9901; further, the crystal form I of the hydrochloride has an X-ray powder diffraction pattern comprising diffraction peaks at 2θ±0.2° diffraction angles of 6.2065, 7.0236, 13.8915, 14.6038, 17.7770, 20.8063, 21.1874, and 24.9901; furthermore, the crystal form I of the hydrochloride has an X-ray powder diffraction pattern comprising diffraction peaks at 2θ±0.2° diffraction angles of 6.2065, 7.0236, 13.8915, 14.6038, 15.6403, 17.7770, 20.8063, 21.1874, 24.9901, and 25.4883; furthermore, the crystal form I of the hydrochloride has an X-ray powder diffraction pattern comprising diffraction peaks at 2θ±0.2° diffraction angles of 6.2065, 7.0236, 11.6337, 13.8915, 14.6038, 15.6403, 17.7770, 19.2571, 20.8063, 21.1874, 24.9901, and 25.4883; still further, the crystal form I of the hydrochloride has an X-ray powder diffraction pattern comprising diffraction peaks at 2θ±0.2° diffraction angles of 6.2065, 7.0236, 11.6337, 13.8915, 14.6038, 15.6403, 17.0255, 17.7770, 19.2571, 20.8063, 21.1874, 23.6537, 24.9901, and 25.4883; still further, the crystal form I of the hydrochloride has an XRPD pattern substantially as shown in FIG. 106d;
preferably, the crystal form is crystal form J of the hydrochloride of the compound of formula I, and the crystal form J of the hydrochloride has an X-ray powder diffraction pattern comprising diffraction peaks at 2θ±0.2° diffraction angles of 10.3031, 15.0739, 18.8389, 19.8236, 20.0800, and 21.4914; further, the crystal form J of the hydrochloride has an X-ray powder diffraction pattern comprising diffraction peaks at 2θ±0.2° diffraction angles of 10.3031, 15.0739, 18.5121, 18.8389, 19.8236, 20.0800, 21.4914, and 22.0336; furthermore, the crystal form J of the hydrochloride has an X-ray powder diffraction pattern comprising diffraction peaks at 2θ±0.2° diffraction angles of 10.3031, 12.9670, 15.0739, 18.5121, 18.8389, 19.8236, 20.0800, 21.4914, 21.7526, 22.0336, and 22.2164;
furthermore, the crystal form J of the hydrochloride has an X-ray powder diffraction pattern comprising diffraction peaks at 2θ±0.2° diffraction angles of 6.7191, 9.4898, 10.3031, 11.5598, 12.9670, 13.7721, 14.2248, 15.0739, 17.8940, 18.5121, 18.8389, 19.5450, 19.8236, 20.0800, 21.0834, 21.4914, 21.7526, 22.0336, 22.2164, 22.5454, 25.6500, 26.1804, 27.4986, and 30.2463; still further, the crystal form J of the hydrochloride has an X-ray powder diffraction pattern comprising diffraction peaks at 2θ±0.2° diffraction angles of 6.7191, 9.4898, 10.3031, 11.5598, 12.9670, 13.7721, 14.2248, 15.0739, 17.8940, 18.5121, 18.8389, 19.5450, 19.8236, 20.0800, 21.0834, 21.4914, 21.7526, 22.0336, 22.2164, 22.5454, 24.4676, 25.6500, 26.1804, 27.4986, 29.5851, 30.2463, 31.2643, 32.4691, and 34.1280; still further, the crystal form J of the hydrochloride has an XRPD pattern substantially as shown in FIG. 106e;
preferably, the crystal form is crystal form K of the hydrochloride of the compound of formula I, and the crystal form K of the hydrochloride has an X-ray powder diffraction pattern comprising diffraction peaks at 2θ±0.2° diffraction angles of 6.1719, 11.2365, 12.9493, 15.6381, 20.5819, and 25.3451; further, the crystal form K of the hydrochloride has an X-ray powder diffraction pattern comprising diffraction peaks at 2θ±0.2° diffraction angles of 6.1719, 11.2365, 12.9493, 15.6381, 18.5078, 20.5819, and 25.3451; furthermore, the crystal form K of the hydrochloride has an X-ray powder diffraction pattern comprising diffraction peaks at 2θ±0.2° diffraction angles of 6.1719, 11.2365, 12.9493, 15.6381, 17.8017, 18.5078, 20.5819, 25.3451, 26.0590, and 26.3085; furthermore, the crystal form K of the hydrochloride has an X-ray powder diffraction pattern comprising diffraction peaks at 2θ±0.2° diffraction angles of 6.1719, 7.6126, 11.2365, 12.9493, 13.9646, 15.6381, 17.8017, 18.5078, 19.8165, 20.5819, 25.3451, 26.0590, and 26.3085; still further, the crystal form K of the hydrochloride has an X-ray powder diffraction pattern comprising diffraction peaks at 2θ±0.2° diffraction angles of 6.1719, 7.6126, 11.2365, 12.9493, 13.9646, 15.6381, 16.6189, 17.1911, 17.8017, 18.5078, 19.8165, 20.5819, 21.7916, 22.5977, 25.3451, 26.0590, 26.3085, and 30.7181; still further, the crystal form K of the hydrochloride has an XRPD pattern substantially as shown in FIG. 106f;
preferably, the crystal form is crystal form L of the hydrochloride of the compound of formula I, and the crystal form L of the hydrochloride has an X-ray powder diffraction pattern comprising diffraction peaks at 2θ±0.2° diffraction angles of 7.7752, 13.8951, 17.1547, 21.4837, and 24.0925; further, the crystal form L of the hydrochloride has an X-ray powder diffraction pattern comprising diffraction peaks at 2θ±0.2° diffraction angles of 7.7752, 13.8951, 15.3025, 17.1547, 17.8681, 21.4837, 24.0925, and 26.5641; furthermore, the crystal form L of the hydrochloride has an X-ray powder diffraction pattern comprising diffraction peaks at 2θ±0.2° diffraction angles of 7.7752, 13.8951, 15.3025, 17.1547, 17.8681, 21.4837, 23.4738, 24.0925, and 26.5641; furthermore, the crystal form L of the hydrochloride has an X-ray powder diffraction pattern comprising diffraction peaks at 2θ±0.2° diffraction angles of 7.7752, 10.4411, 13.8951, 15.3025, 16.3403, 17.1547, 17.8681, 19.2621, 21.4837, 23.4738, 24.0925, 25.5726, 26.5641, 28.1186, and 30.8899; still further, the crystal form L of the hydrochloride has an X-ray powder diffraction pattern comprising diffraction peaks at 2θ±0.2° diffraction angles of 7.7752, 10.4411, 13.2333, 13.8951, 15.3025, 16.3403, 17.1547, 17.8681, 19.2621, 19.9563, 21.4837, 22.4156, 23.4738, 24.0925, 25.5726, 26.5641, 28.1186, 30.8899, and 38.4894; still further, the crystal form L of the hydrochloride has an XRPD pattern substantially as shown in FIG. 106g;
preferably, the crystal form is crystal form M of the hydrochloride of the compound of formula I, and the crystal form M of the hydrochloride has an X-ray powder diffraction pattern comprising diffraction peaks at 2θ±0.2° diffraction angles of 9.8040, 14.8344, 18.8157, 19.2503, 20.6275, and 21.0461; further, the crystal form M of the hydrochloride has an X-ray powder diffraction pattern comprising diffraction peaks at 2θ±0.2° diffraction angles of 6.5546, 9.8040, 14.8344, 18.8157, 19.2503, 20.6275, and 21.0461; furthermore, the crystal form M of the hydrochloride has an X-ray powder diffraction pattern comprising diffraction peaks at 2θ±0.2° diffraction angles of 6.5546, 9.8040, 12.0038, 14.2374, 14.8344, 17.8676, 18.8157, 19.2503, 20.6275, and 21.0461; furthermore, the crystal form M of the hydrochloride has an X-ray powder diffraction pattern comprising diffraction peaks at 2θ±0.2° diffraction angles of 6.5546, 9.8040, 10.8235, 12.0038, 13.1333, 14.2374, 14.8344, 17.8676, 18.8157, 19.2503, 20.6275, 21.0461, 26.8393, and 28.6992; still further, the crystal form M of the hydrochloride has an X-ray powder diffraction pattern comprising diffraction peaks at 2θ±0.2° diffraction angles of 6.5546, 9.8040, 10.8235, 12.0038, 13.1333, 14.2374, 14.8344, 17.8676, 18.8157, 19.2503, 20.6275, 21.0461, 22.8294, 24.8213, 26.8393, 28.6992, 29.5703, and 33.9685; still further, the crystal form M of the hydrochloride has an XRPD pattern substantially as shown in FIG. 106h.

5. The pharmaceutically acceptable salt of the compound of formula I or the crystal form thereof according to claim 3, wherein the crystal form is a crystal form of the sulfate of the compound of formula I; preferably, the crystal form is crystal form A of the sulfate of the compound of formula I, and the crystal form A of the sulfate has an X-ray powder diffraction pattern comprising diffraction peaks at 2θ±0.2° diffraction angles of 6.5297, 7.4156, 11.2590, 17.2920, and 19.5870; further, the crystal form A of the sulfate has an X-ray powder diffraction pattern comprising diffraction peaks at 2θ±0.2° diffraction angles of 6.5297, 7.4156, 11.2590, 17.2920, 18.3831, 18.9056, 19.5870, 23.1076, and 25.1201; furthermore, the crystal form A of the sulfate has an X-ray powder diffraction pattern comprising diffraction peaks at 2θ±0.2° diffraction angles of 6.5297, 7.4156, 11.2590, 17.2920, 18.3831, 18.9056, 19.5870, 19.8280, 23.1076, and 25.1201; furthermore, the crystal form A of the sulfate has an X-ray powder diffraction pattern comprising diffraction peaks at 2θ±0.2° diffraction angles of 6.5297, 7.4156, 11.2590, 14.8207, 15.0692, 17.2920, 17.5479, 18.3831, 18.9056, 19.5870, 19.8280, 21.1050, 23.1076, 23.6024, and 25.1201; still further, the crystal form A of the sulfate has an X-ray powder diffraction pattern comprising diffraction peaks at 2θ±0.2° diffraction angles of 6.5297, 7.4156, 9.8272, 11.2590, 11.7996, 13.0075, 14.8207, 15.0692, 17.2920, 17.5479, 18.3831, 18.9056, 19.5870, 19.8280, 20.1191, 21.1050, 21.9720, 23.1076, 23.6024, 25.1201, 27.2805, 28.7070, 29.8446, and 32.6299; still further, the crystal form A of the sulfate has an XRPD pattern substantially as shown in FIG. 22;
preferably, the crystal form A of the sulfate has one or two of the following characteristics:
(1) a TGA curve of the crystal form A of the sulfate showing a weight loss of 2-10%, preferably about 4-7% (e.g., 4.0%, 4.5%, 5.0%, 5.5%, 6.0%, 6.5%, or 7.0%) at 150.0±3 °C;
(2) a DSC curve of the crystal form A of the sulfate having 2 endothermic peaks at 52.9±3 °C and 167.1±3 °C;
preferably, the crystal form A of the sulfate has one or two of the following characteristics:
(1) a TGA curve of the crystal form A of the sulfate showing a weight loss of 5.78% at 150.0±3 °C;
(2) a DSC curve of the crystal form A of the sulfate having 2 endothermic peaks at 52.9±3 °C and 167.1±3 °C;
preferably, a TGA/DSC profile of the crystal form A of the sulfate is substantially as shown in FIG. 23; a ¹H NMR spectrum of the crystal form A of the sulfate is substantially as shown in FIG. 24;
preferably, the crystal form is crystal form B of the sulfate of the compound of formula I, and the crystal form B of the sulfate has an X-ray powder diffraction pattern comprising diffraction peaks at 2θ±0.2° diffraction angles of 9.8491, 16.5561, 17.1270, 17.8255, and 19.6977; further, the crystal form B of the sulfate has an X-ray powder diffraction pattern comprising diffraction peaks at 2θ±0.2° diffraction angles of 9.8491, 16.5561, 17.1270, 17.8255, 18.1372, 19.6977, 19.0468, 22.6654, and 26.1622; furthermore, the crystal form B of the sulfate has an X-ray powder diffraction pattern comprising diffraction peaks at 2θ±0.2° diffraction angles of 6.3375, 9.8491, 13.0250, 16.5561, 17.1270, 17.8255, 18.1372, 19.6977, 19.0468, 22.2378, 22.6654, 25.7586, 26.1622, and 29.7168; still further, the crystal form B of the sulfate has an X-ray powder diffraction pattern comprising diffraction peaks at 2θ±0.2° diffraction angles of 6.3375, 8.6916, 9.1864, 9.8491, 11.7001, 12.1619, 13.0250, 14.4700, 15.1807, 16.5561, 17.1270, 17.8255, 18.1372, 19.0468, 19.6977, 21.6775, 22.2378, 22.6654, 23.4512, 23.8100, 24.8186, 25.4720, 25.7586, 26.1622, 27.1217, 29.0464, 29.7168, 30.9706, 33.1687, and 34.5562; still further, the crystal form B of the sulfate has an XRPD pattern substantially as shown in FIG. 25;
preferably, the crystal form B of the sulfate has one or two of the following characteristics:
(1) a TGA curve of the crystal form B of the sulfate showing a weight loss of 0-5%, preferably about 1-3% (e.g., 1.0%, 1.5%, 2.0%, 2.5%, or 3.0%) at 150.0±3 °C;
(2) a DSC curve of the crystal form B of the sulfate having 2 endothermic peaks at 69.7±3 °C and 238.5±3 °C;
preferably, the crystal form B of the sulfate has one or two of the following characteristics:
(1) a TGA curve of the crystal form B of the sulfate showing a weight loss of 1.16% at 150.0±3 °C;
(2) a DSC curve of the crystal form B of the sulfate having 2 endothermic peaks at 69.7±3 °C and 238.5±3 °C;
preferably, a TGA/DSC profile of the crystal form B of the sulfate is substantially as shown in FIG. 26; a ¹H NMR spectrum of the crystal form B of the sulfate is substantially as shown in FIG. 27;
preferably, the crystal form is crystal form C of the sulfate of the compound of formula I, and the crystal form C of the sulfate has an X-ray powder diffraction pattern comprising diffraction peaks at 2θ±0.2° diffraction angles of 8.9487, 17.5523, 17.9120, 19.9597, and 22.9905; further, the crystal form C of the sulfate has an X-ray powder diffraction pattern comprising diffraction peaks at 2θ±0.2° diffraction angles of 8.9487, 9.8314, 14.4549, 17.5523, 17.9120, 18.7584, 19.5850, 19.9597, 20.9974, 22.4850, 22.9905, 24.5952, and 29.4160; furthermore, the crystal form C of the sulfate has an X-ray powder diffraction pattern comprising diffraction peaks at 2θ±0.2° diffraction angles of 8.9487, 9.8314, 12.2693, 14.4549, 17.5523, 17.9120, 18.7584, 19.5850, 19.9597, 20.6152, 20.9974, 21.9335, 22.4850, 22.9905, 23.6939, 23.9937, 24.5952, 26.7497, and 29.4160; still further, the crystal form C of the sulfate has an X-ray powder diffraction pattern comprising diffraction peaks at 2θ±0.2° diffraction angles of 7.4762, 8.0099, 8.9487, 9.8314, 11.7697, 12.2693, 13.3788, 14.4549, 14.9774, 15.3199, 16.8293, 17.5523, 17.9120, 18.7584, 19.5850, 19.9597, 20.6152, 20.9974, 21.9335, 22.4850, 22.9905, 23.6939, 23.9937, 24.5952, 24.8904, 25.3759, 26.2847, 26.7497, 28.4461, 29.4160, 31.7091, 33.4595, 34.8478, 36.5099, and 38.0959; still further, the crystal form C of the sulfate has an XRPD pattern substantially as shown in FIG. 28;
preferably, the crystal form C of the sulfate has one, two, or three of the following characteristics:
(1) a TGA curve of the crystal form C of the sulfate showing a weight loss of 1-5%, preferably about 1-4% (e.g., 1.0%, 1.5%, 2.0%, 2.5%, 3.0%, 3.5%, or 4.0%) at 100.0±3 °C;
(2) a DSC curve of the crystal form C of the sulfate having 4 endothermic peaks at 92.4±3 °C, 159.3±3 °C, 184.8±3 °C, and 216.8±3 °C;
(3) a DSC curve of the crystal form C of the sulfate having 1 exothermic peak at 224.0±3 °C;
in some embodiments, the crystal form C of the sulfate has one, two, or three of the following characteristics:
(1) a TGA curve of the crystal form C of the sulfate showing a weight loss of 2.83% at 100.0±3 °C;
(2) a DSC curve of the crystal form C of the sulfate having 4 endothermic peaks at 92.4±3 °C, 159.3±3 °C, 184.8±3 °C, and 216.8±3 °C;
(3) a DSC curve of the crystal form C of the sulfate having 1 exothermic peak at 224.0±3 °C;
in some embodiments, a TGA/DSC profile of the crystal form C of the sulfate is substantially as shown in FIG. 29; a ¹H NMR spectrum of the crystal form C of the sulfate is as shown in FIG. 30.

6. The pharmaceutically acceptable salt of the compound of formula I or the crystal form thereof according to claim 3, wherein the crystal form is crystal form A of the maleate of the compound of formula I, and the crystal form A of the maleate has an X-ray powder diffraction pattern comprising diffraction peaks at 2θ±0.2° diffraction angles of 13.7865, 16.3294, 18.2795, 20.3336, and 20.8824; further, the crystal form A of the maleate has an X-ray powder diffraction pattern comprising diffraction peaks at 2θ±0.2° diffraction angles of 7.4318, 13.7865, 15.9646, 16.3294, 18.2795, 20.3336, 20.8824, 21.3104, 21.5283, 23.1978, and 24.1136; furthermore, the crystal form A of the maleate has an X-ray powder diffraction pattern comprising diffraction peaks at 2θ±0.2° diffraction angles of 7.4318, 10.4550, 13.7865, 15.9646, 16.3294, 17.7554, 18.2795, 20.3336, 20.8824, 21.3104, 21.5283, 23.1978, 24.1136, and 24.7430; still further, the crystal form A of the maleate has an X-ray powder diffraction pattern comprising diffraction peaks at 2θ±0.2° diffraction angles of 7.4318, 8.1664, 10.4550, 11.4963, 13.7865, 14.9748, 15.6098, 15.9646, 16.3294, 17.2388, 17.7554, 18.2795, 18.7232, 19.2252, 19.9626, 20.3336, 20.8824, 21.3104, 21.5283, 21.8250, 22.5862, 23.1978, 23.7175, 24.1136, 24.7430, 25.0797, 26.1594, 27.1361, 27.5054, 28.4773, 29.4248, 30.0997, 30.4233, 31.3563, 32.1988, 32.8832, 34.8721, 36.2350, and 37.8027; still further, the crystal form A of the maleate has an XRPD pattern substantially as shown in FIG. 31;
preferably, the crystal form A of the maleate has one or two of the following characteristics:
(1) a TGA curve of the crystal form A of the maleate showing a weight loss of 0-4%, preferably about 0-3% (e.g., 0.5%, 1.0%, 1.5%, 2.0%, 2.5%, or 3.0%) at 130.0±3 °C;
(2) a DSC curve of the crystal form A of the maleate having 1 endothermic peak at 142.1±3 °C;
preferably, the crystal form A of the maleate has one or two of the following characteristics:
(1) a TGA curve of the crystal form A of the maleate showing a weight loss of 0.42% at 130.0±3 °C;
(2) a DSC curve of the crystal form A of the maleate having 1 endothermic peak at 142.1±3 °C;
preferably, a TGA/DSC profile of the crystal form A of the maleate is substantially as shown in FIG. 32; a ¹H NMR spectrum of the crystal form A of the maleate is as shown in FIG. 33.

7. The pharmaceutically acceptable salt of the compound of formula I or the crystal form thereof according to claim 3, wherein the crystal form is crystal form A of the phosphate of the compound of formula I, and the crystal form A of the phosphate has an X-ray powder diffraction pattern comprising diffraction peaks at 2θ±0.2° diffraction angles of 9.3607, 17.8932, 19.3716, 19.8946, 20.5448, 21.1372, and 22.4294; further, the crystal form A of the phosphate has an X-ray powder diffraction pattern comprising diffraction peaks at 2θ±0.2° diffraction angles of 8.8155, 9.3607, 17.8932, 18.4384, 19.3716, 19.8946, 20.5448, 21.1372, 22.4294, and 24.5486; furthermore, the crystal form A of the phosphate has an X-ray powder diffraction pattern comprising diffraction peaks at 2θ±0.2° diffraction angles of 8.8155, 9.3607, 11.1767, 16.4840, 17.8932, 18.4384, 19.3716, 19.8946, 20.5448, 21.1372, 22.4294, 23.1105, 23.9976, 24.5486, 24.5486, 25.7922, and 26.3981; still further, the crystal form A of the phosphate has an X-ray powder diffraction pattern comprising diffraction peaks at 2θ±0.2° diffraction angles of 3.2059, 8.8155, 9.3607, 11.1767, 13.9605, 16.4840, 17.8932, 18.4384, 19.3716, 19.8946, 20.5448, 21.1372, 22.4294, 23.1105, 23.9976, 24.5486, 25.7922, 26.3981, 26.9789, 28.2812, and 30.2804; still further, the crystal form A of the phosphate has an XRPD pattern substantially as shown in FIG. 34;
preferably, the crystal form A of the phosphate has one, two, or three of the following characteristics:
(1) a TGA curve of the crystal form A of the phosphate showing a weight loss of 1-6%, preferably about 2-5% (e.g., 2.0%, 2.5%, 3.0%, 3.5%, 4.0%, 4.5%, or 5.0%) at 150.0±3 °C;
(2) a DSC curve of the crystal form A of the phosphate having 2 endothermic peaks at 41.5±3 °C and 169.6±3 °C;
(3) a DSC curve of the crystal form A of the phosphate having 1 exothermic peak at 91.8±3 °C;
preferably, the crystal form A of the phosphate has one, two, or three of the following characteristics:
(1) a TGA curve of the crystal form A of the phosphate showing a weight loss of 3.71% at 150.0±3 °C;
(2) a DSC curve of the crystal form A of the phosphate having 2 endothermic peaks at 41.5±3 °C and 169.6±3 °C;
(3) a DSC curve of the crystal form A of the phosphate having 1 exothermic peak at 91.8±3 °C;
in some embodiments, a TGA/DSC profile of the crystal form A of the phosphate is substantially as shown in FIG. 35; a ¹H NMR spectrum of the crystal form A of the phosphate is as shown in FIG. 36.

8. The pharmaceutically acceptable salt of the compound of formula I or the crystal form thereof according to claim 3, wherein the crystal form is a crystal form of the p-toluenesulfonate of the compound of formula I; preferably, the crystal form is crystal form A of the p-toluenesulfonate of the compound of formula I, and the crystal form A of the p-toluenesulfonate has an X-ray powder diffraction pattern comprising diffraction peaks at 2θ±0.2° diffraction angles of 5.6569, 6.5865, 9.0105, 14.4962, 17.1789, and 19.7973; further, the crystal form A of the p-toluenesulfonate has an XRPD pattern substantially as shown in FIG. 38;
preferably, the crystal form A of the p-toluenesulfonate has one or two of the following characteristics:
(1) a TGA curve of the crystal form A of the p-toluenesulfonate showing a weight loss of 2-8%, preferably about 2-6% (e.g., 2.0%, 2.5%, 3.0%, 3.5%, 4.0%, 4.5%, 5.0%, 5.5%, or 6.0%) at 150.0±3 °C;
(2) a DSC curve of the crystal form A of the p-toluenesulfonate having 2 endothermic peaks at 81.6±3 °C and 156.0±3 °C;
preferably, the crystal form crystal form A of the p-toluenesulfonate has one or two of the following characteristics:
(1) a TGA curve of the crystal form A of the p-toluenesulfonate showing a weight loss of 4.15% at 150.0±3 °C;
(2) a DSC curve of the crystal form A of the p-toluenesulfonate having 2 endothermic peaks at 81.6±3 °C and 156.0±3 °C;
preferably, a TGA/DSC profile of the crystal form A of the p-toluenesulfonate is substantially as shown in FIG. 39; a ¹H NMR spectrum of the crystal form A of the p-toluenesulfonate is as shown in FIG. 40;
preferably, the crystal form is crystal form B of the p-toluenesulfonate of the compound of formula I, and the crystal form B of the p-toluenesulfonate has an X-ray powder diffraction pattern comprising diffraction peaks at 2θ±0.2° diffraction angles of 7.6660, 16.2695, 17.6681, 19.5433, 20.3312, and 21.3520; further, the crystal form B of the p-toluenesulfonate has an X-ray powder diffraction pattern comprising diffraction peaks at 2θ±0.2° diffraction angles of 7.6660, 11.1056, 14.5769, 16.2695, 17.6681, 19.5433, 20.3312, 20.7694, 21.3520, and 26.0500; furthermore, the crystal form B of the p-toluenesulfonate has an X-ray powder diffraction pattern comprising diffraction peaks at 2θ±0.2° diffraction angles of 7.6660, 11.1056, 14.5769, 14.9845, 16.2695, 17.6681, 19.5433, 20.3312, 20.7694, 21.3520, 22.8795, 24.5060, 26.0500, and 28.2460; still further, the crystal form B of the p-toluenesulfonate has an X-ray powder diffraction pattern comprising diffraction peaks at 2θ±0.2° diffraction angles of 6.8890, 7.6660, 9.9665, 11.1056, 11.8448, 12.6617, 13.1706, 13.6098, 13.8415, 14.5769, 14.9845, 16.2695, 17.6681, 18.8718, 19.5433, 19.9700, 20.3312, 20.7694, 21.3520, 21.6902, 22.8795, 24.5060, 26.0500, 26.4583, 27.2822, 28.2460, 29.2107, 30.0879, 31.8132, 32.8460, 33.7653, and 37.7617; still further, the crystal form B of the p-toluenesulfonate has an XRPD pattern substantially as shown in FIG. 41;
preferably, the crystal form B of the p-toluenesulfonate has one or two of the following characteristics:
(1) a TGA curve of the crystal form B of the p-toluenesulfonate showing a weight loss of 1-6%, preferably about 1-4% (e.g., 1.0%, 1.5%, 2.0%, 2.5%, 3.0%, 3.5%, or 4.0%) at 150.0±3 °C;
(2) a DSC curve of the crystal form B of the p-toluenesulfonate having 1 endothermic peak at 204.3±3 °C;
preferably, the crystal form B of the p-toluenesulfonate has one or two of the following characteristics:
(1) a TGA curve of the crystal form B of the p-toluenesulfonate showing a weight loss of 1.92% at 150.0±3 °C;
(2) a DSC curve of the crystal form B of the p-toluenesulfonate having 1 endothermic peak at 204.3±3 °C;
preferably, a TGA/DSC profile of the crystal form B of the p-toluenesulfonate is substantially as shown in FIG. 42; a ¹H NMR spectrum of the crystal form B of the p-toluenesulfonate is substantially as shown in FIG. 43.

9. The pharmaceutically acceptable salt of the compound of formula I or the crystal form thereof according to claim 3, wherein the crystal form is a crystal form of the methanesulfonate of the compound of formula I; preferably, the crystal form is crystal form A of the methanesulfonate of the compound of formula I, and the crystal form A of the methanesulfonate has an X-ray powder diffraction pattern comprising diffraction peaks at 2θ±0.2° diffraction angles of 7.1654, 7.7112, 10.2242, 15.4030, 16.8120, and 19.2492; further, the crystal form A of the methanesulfonate has an X-ray powder diffraction pattern comprising diffraction peaks at 2θ±0.2° diffraction angles of 7.1654, 7.7112, 10.2242, 11.4305, 15.4030, 16.8120, 17.7499, 18.3934, 18.9572, 19.2492, and 20.0359; furthermore, the crystal form A of the methanesulfonate has an X-ray powder diffraction pattern comprising diffraction peaks at 2θ±0.2° diffraction angles of 7.1654, 7.7112, 10.2242, 11.4305, 15.4030, 16.8120, 17.7499, 18.3934, 18.9572, 19.2492, 19.6796, 20.0359, 21.4943, 23.1420, and 25.5679; still further, the crystal form A of the methanesulfonate has an X-ray powder diffraction pattern comprising diffraction peaks at 2θ±0.2° diffraction angles of 6.4245, 7.1654, 7.7112, 10.2242, 11.4305, 12.1973, 14.3852, 14.9410, 15.4030, 16.8120, 17.3257, 17.7499, 18.3934, 18.9572, 19.2492, 19.6796, 20.0359, 20.7137, 21.0725, 21.4943, 22.4448, 23.1420, 23.6743, 24.4576, 25.1217, 25.5679, 27.3091, 28.2828, 30.2612, 31.6602, and 33.8876; still further, the crystal form A of the methanesulfonate has an XRPD pattern substantially as shown in FIG. 45;
preferably, the crystal form A of the methanesulfonate has one or two of the following characteristics:
(1) a TGA curve of the crystal form A of the methanesulfonate showing a weight loss of 1-6%, preferably about 2-5% (e.g., 2.0%, 2.5%, 3.0%, 3.5%, 4.0%, 4.5%, or 5.0%) at 150.0±3 °C;
(2) a DSC curve of the crystal form A of the methanesulfonate having 1 endothermic peak at 153.8±3 °C;
preferably, the crystal form A of the methanesulfonate has one or two of the following characteristics:
(1) a TGA curve of the crystal form A of the methanesulfonate showing a weight loss of 3.08% at 150.0±3 °C;
(2) a DSC curve of the crystal form A of the methanesulfonate having 1 endothermic peak at 153.8±3 °C;
preferably, a TGA/DSC profile of the crystal form A of the methanesulfonate is substantially as shown in FIG. 46; a ¹H NMR spectrum of the crystal form A of the methanesulfonate is as shown in FIG. 47;
preferably, the crystal form is crystal form B of the methanesulfonate of the compound of formula I, and the crystal form B of the methanesulfonate has an X-ray powder diffraction pattern comprising diffraction peaks at 2θ±0.2° diffraction angles of 7.9334, 17.1889, 17.9047, 19.4932, and 20.3844; further, the crystal form B of the methanesulfonate has an X-ray powder diffraction pattern comprising diffraction peaks at 2θ±0.2° diffraction angles of 7.9334, 16.2948, 17.1889, 17.9047, 18.9481, 19.4932, 20.3844, 21.2069, 23.9181, and 26.8731; furthermore, the crystal form B of the methanesulfonate has an X-ray powder diffraction pattern comprising diffraction peaks at 2θ±0.2° diffraction angles of 7.9334, 12.4699, 13.8134, 14.2662, 16.2948, 17.1889, 17.9047, 18.9481, 19.4932, 20.3844, 21.2069, 22.9645, 23.9181, and 26.8731; still further, the crystal form B of the methanesulfonate has an X-ray powder diffraction pattern comprising diffraction peaks at 2θ±0.2° diffraction angles of 7.9334, 10.5237, 11.7673, 12.4699, 13.8134, 14.2662, 16.2948, 17.1889, 17.9047, 18.9481, 19.4932, 20.3844, 21.2069, 22.3570, 22.9645, 23.9181, 25.0848, 26.8731, 28.8447, 30.5888, and 36.0120; still further, the crystal form B of the methanesulfonate has an XRPD pattern substantially as shown in FIG. 48;
preferably, the crystal form B of the methanesulfonate has one or two of the following characteristics:
(1) a TGA curve of the crystal form B of the methanesulfonate showing a weight loss of 0-3%, preferably about 0-2% (e.g., 0.5%, 1.0%, 1.5%, or 2.0%) at 150.0±3 °C;
(2) a DSC curve of the crystal form B of the methanesulfonate having 3 endothermic peaks at 161.8±3 °C, 168.3±3 °C, and 194.9±3 °C;
preferably, the crystal form B of the methanesulfonate has one or two of the following characteristics:
(1) a TGA curve of the crystal form B of the methanesulfonate showing a weight loss of 0.67% at 150.0±3 °C;
(2) a DSC curve of the crystal form B of the methanesulfonate having 3 endothermic peaks at 61.8±3 °C, 168.3±3 °C, and 194.9±3 °C;
preferably, a TGA/DSC profile of the crystal form B of the methanesulfonate is substantially as shown in FIG. 49; a ¹H NMR spectrum of the crystal form B of the methanesulfonate is substantially as shown in FIG. 50;
preferably, the crystal form is crystal form C of the methanesulfonate of the compound of formula I, and the crystal form C of the methanesulfonate has an X-ray powder diffraction pattern comprising diffraction peaks at 2θ±0.2° diffraction angles of 5.6360, 9.3088, 15.7926, 17.1524, 17.5174, and 20.4316; further, the crystal form C of the methanesulfonate has an X-ray powder diffraction pattern comprising diffraction peaks at 2θ±0.2° diffraction angles of 5.6360, 9.3088, 13.6745, 15.7926, 17.1524, 17.5174, 18.1940, 19.2689, 20.4316, 21.5116, 25.2739, and 25.6773; furthermore, the crystal form C of the methanesulfonate has an X-ray powder diffraction pattern comprising diffraction peaks at 2θ±0.2° diffraction angles of 5.6360, 9.3088, 12.6477, 13.6745, 15.7926, 16.1257, 17.1524, 17.5174, 18.1940, 19.2689, 20.4316, 21.5116, 23.0206, 23.4450, 25.2739, and 25.6773; still further, the crystal form C of the methanesulfonate has an X-ray powder diffraction pattern comprising diffraction peaks at 2θ±0.2° diffraction angles of 5.6360, 8.5661, 9.3088, 11.6453, 12.6477, 13.6745, 15.7926, 16.1257, 17.1524, 17.5174, 18.1940, 19.2689, 20.4316, 21.5116, 23.0206, 23.4450, 25.2739, 25.6773, 29.4554, and 32.4112; still further, the crystal form C of the methanesulfonate has an XRPD pattern substantially as shown in FIG. 51;
preferably, the crystal form C of the methanesulfonate has one or two of the following characteristics:
(1) a TGA curve of the crystal form C of the methanesulfonate showing a weight loss of 10-20%, preferably about 12-16% (e.g., 12.0%, 12.5%, 13.0%, 13.5%, 14.0%, 14.5%, 15.0%, 15.5%, or 16.0%) at 150.0±3 °C;
(2) a DSC curve of the crystal form C of the methanesulfonate having 3 endothermic peaks at 111.7±3 °C, 135.8±3 °C, and 139.6±3 °C;
preferably, the crystal form C of the methanesulfonate has one or two of the following characteristics:
(1) a TGA curve of the crystal form C of the methanesulfonate showing a weight loss of 14.14% at 150.0±3 °C;
(2) a DSC curve of the crystal form C of the methanesulfonate having 3 endothermic peaks at 111.7±3 °C, 135.8±3 °C, and 139.6±3 °C;
preferably, a TGA/DSC profile of the crystal form C of the methanesulfonate is substantially as shown in FIG. 52; a ¹H NMR spectrum of the crystal form C of the methanesulfonate is substantially as shown in FIG. 53;
preferably, the crystal form is crystal form D of the methanesulfonate of the compound of formula I, and the crystal form D of the methanesulfonate has an X-ray powder diffraction pattern comprising diffraction peaks at 2θ±0.2° diffraction angles of 7.3614, 14.7295, 17.0139, 17.2434, 17.5216, 19.2452, and 20.3331; further, the crystal form D of the methanesulfonate has an X-ray powder diffraction pattern comprising diffraction peaks at 2θ±0.2° diffraction angles of 7.3614, 14.7295, 14.9968, 17.0139, 17.2434, 17.5216, 19.2452, 19.8274, and 20.3331; furthermore, the crystal form D of the methanesulfonate has an X-ray powder diffraction pattern comprising diffraction peaks at 2θ±0.2° diffraction angles of 7.3614, 14.7295, 14.9968, 17.0139, 17.2434, 17.5216, 18.7631, 19.2452, 19.8274, 20.3331, and 25.3625; furthermore, the crystal form D of the methanesulfonate has an X-ray powder diffraction pattern comprising diffraction peaks at 2θ±0.2° diffraction angles of 7.3614, 8.5975, 11.1942, 12.9868, 13.4101, 14.7295, 14.9968, 17.0139, 17.2434, 17.5216, 18.0167, 18.3980, 18.7631, 19.2452, 19.8274, 20.3331, 21.8273, 22.4894, 23.0593, and 25.3625; still further, the crystal form D of the methanesulfonate has an X-ray powder diffraction pattern comprising diffraction peaks at 2θ±0.2° diffraction angles of 6.3734, 7.3614, 8.5975, 9.8950, 11.1942, 12.9868, 13.4101, 14.7295, 14.9968, 15.9124, 17.0139, 17.2434, 17.5216, 18.0167, 18.3980, 18.7631, 19.2452, 19.8274, 20.3331, 21.8273, 22.4894, 23.0593, 24.4152, and 25.3625; still further, the crystal form D of the methanesulfonate has an XRPD pattern substantially as shown in FIG. 102b;
preferably, the crystal form D of the methanesulfonate has one or two or more of the following characteristics:
(1) a TGA curve showing a weight loss of 1-4%, preferably about 1.5-3% (e.g., 1.5%, 2.0%, 2.5%, or 3.0%), and more preferably about 2.62% at 150±3 °C;
(2) a DSC curve having 2 endothermic peaks at 40.3±3 °C and 156.6±3 °C;
preferably, a TGA/DSC profile of the crystal form D of the methanesulfonate is substantially as shown in FIG. 103; a ¹H NMR spectrum of the crystal form D of the methanesulfonate is substantially as shown in FIG. 104;
preferably, the crystal form is crystal form E of the methanesulfonate of the compound of formula I, and the crystal form E of the methanesulfonate has an X-ray powder diffraction pattern comprising diffraction peaks at 2θ±0.2° diffraction angles of 7.6948, 13.5466, 18.9557, 19.4674, and 26.1850; further, the crystal form E of the methanesulfonate has an X-ray powder diffraction pattern comprising diffraction peaks at 2θ±0.2° diffraction angles of 7.6948, 13.5466, 18.9557, 19.4674, 20.0144, 23.7738, and 26.1850; furthermore, the crystal form E of the methanesulfonate has an X-ray powder diffraction pattern comprising diffraction peaks at 2θ±0.2° diffraction angles of 7.6948, 13.5466, 18.9557, 19.4674, 20.0144, 22.3698, 23.7738, and 26.1850; furthermore, the crystal form E of the methanesulfonate has an X-ray powder diffraction pattern comprising diffraction peaks at 2θ±0.2° diffraction angles of 7.6948, 13.5466, 14.5860, 16.1727, 17.5309, 17.8641, 18.9557, 19.4674, 20.0144, 20.3406, 20.8237, 21.6960, 22.3698, 23.7738, 25.1077, 26.1850, and 29.2032; still further, the crystal form E of the methanesulfonate has an X-ray powder diffraction pattern comprising diffraction peaks at 2θ±0.2° diffraction angles of 7.6948, 11.0258, 12.4963, 13.5466, 14.2591, 14.5860, 15.3154, 16.1727, 16.8137, 17.0486, 17.5309, 17.8641, 18.9557, 19.4674, 20.0144, 20.3406, 20.8237, 21.6960, 22.3698, 22.8750, 23.1733, 23.7738, 25.1077, 25.3713, 25.7306, 26.1850, 26.8900, 27.6964, 28.4667, 29.2032, 30.5281, 31.4140, 33.7897, and 35.9511; still further, the crystal form E of the methanesulfonate has an XRPD pattern substantially as shown in FIG. 112b;
preferably, the crystal form is crystal form F of the methanesulfonate of the compound of formula I, and the crystal form F of the methanesulfonate has an X-ray powder diffraction pattern comprising diffraction peaks at 2θ±0.2° diffraction angles of 7.8706, 15.7726, 17.0258, 17.8165, 20.2728, and 23.7446; further, the crystal form F of the methanesulfonate has an X-ray powder diffraction pattern comprising diffraction peaks at 2θ±0.2° diffraction angles of 7.8706, 15.7726, 17.0258, 17.8165, 20.2728, 23.7446, and 25.3530; furthermore, the crystal form F of the methanesulfonate has an X-ray powder diffraction pattern comprising diffraction peaks at 2θ±0.2° diffraction angles of 7.8706, 15.7726, 17.0258, 17.8165, 20.2728, 21.4207, 23.7446, and 25.3530; furthermore, the crystal form F of the methanesulfonate has an X-ray powder diffraction pattern comprising diffraction peaks at 2θ±0.2° diffraction angles of 7.8706, 11.8701, 15.7726, 17.0258, 17.8165, 20.2728, 21.4207, 23.7446, 25.3530, and 26.3257; still further, the crystal form F of the methanesulfonate has an X-ray powder diffraction pattern comprising diffraction peaks at 2θ±0.2° diffraction angles of 5.0171, 7.8706, 11.8701, 13.4221, 14.3209, 15.7726, 17.0258, 17.8165, 20.2728, 21.4207, 23.7446, 25.3530, and 26.3257; still further, the crystal form F of the methanesulfonate has an XRPD pattern substantially as shown in FIG. 112c;
preferably, the crystal form is crystal form G of the methanesulfonate of the compound of formula I, and the crystal form of the methanesulfonate has an X-ray powder diffraction pattern comprising diffraction peaks at 2θ±0.2° diffraction angles of 16.1377, 17.7027, 18.1620, 19.1132, and 21.0530; further, the crystal form G of the methanesulfonate has an X-ray powder diffraction pattern comprising diffraction peaks at 2θ±0.2° diffraction angles of 15.4837, 16.1377, 16.5841, 17.7027, 18.1620, 19.1132, 21.0530, and 24.3157; furthermore, the crystal form G of the methanesulfonate has an X-ray powder diffraction pattern comprising diffraction peaks at 2θ±0.2° diffraction angles of 13.4333, 15.4837, 16.1377, 16.5841, 17.7027, 18.1620, 19.1132, 21.0530, 24.3157, 24.8063, and 26.2930; furthermore, the crystal form G of the methanesulfonate has an X-ray powder diffraction pattern comprising diffraction peaks at 2θ±0.2° diffraction angles of 8.6041, 10.1340, 12.6682, 13.4333, 15.4837, 16.1377, 16.5841, 17.7027, 18.1620, 19.1132, 21.0530, 22.0640, 24.3157, 24.8063, 25.5461, 26.2930, and 28.8940; still further, the crystal form G of the methanesulfonate has an X-ray powder diffraction pattern comprising diffraction peaks at 2θ±0.2° diffraction angles of 8.6041, 10.1340, 11.2069, 12.6682, 13.4333, 14.4922, 15.4837, 16.1377, 16.5841, 17.7027, 18.1620, 19.1132, 21.0530, 22.0640, 24.3157, 24.8063, 25.5461, 26.2930, 26.9311, 27.7204, and 28.8940; still further, the crystal form G of the methanesulfonate has an XRPD pattern substantially as shown in FIG. 113b; preferably, the crystal form is crystal form H of the methanesulfonate of the compound of formula I, and the crystal form H of the methanesulfonate has an X-ray powder diffraction pattern comprising diffraction peaks at 2θ±0.2° diffraction angles of 9.5858, 16.4833, 17.2040, 19.7648, and 22.2051; further, the crystal form H of the methanesulfonate has an X-ray powder diffraction pattern comprising diffraction peaks at 2θ±0.2° diffraction angles of 9.5858, 9.8551, 12.8418, 16.4833, 17.2040, 19.7648, and 22.2051; furthermore, the crystal form H of the methanesulfonate has an X-ray powder diffraction pattern comprising diffraction peaks at 2θ±0.2° diffraction angles of 9.5858, 9.8551, 12.8418, 16.4833, 17.2040, 19.7648, 22.2051, 25.4281, and 26.1391; furthermore, the crystal form H of the methanesulfonate has an X-ray powder diffraction pattern comprising diffraction peaks at 2θ±0.2° diffraction angles of 9.5858, 9.8551, 12.8418, 16.4833, 17.2040, 17.7099, 18.5860, 19.7648, 21.8135, 22.2051, 25.4281, 26.1391, and 29.8198; still further, the crystal form H of the methanesulfonate has an X-ray powder diffraction pattern comprising diffraction peaks at 2θ±0.2° diffraction angles of 5.6472, 9.5858, 9.8551, 12.8418, 15.2067, 16.4833, 17.2040, 17.7099, 18.5860, 19.2029, 19.7648, 21.8135, 22.2051, 23.4137, 24.9851, 25.4281, 26.1391, 27.6138, 29.8198, 30.9209, 32.0327, and 35.9179; still further, the crystal form H of the methanesulfonate has an XRPD pattern substantially as shown in FIG. 113c;
preferably, the crystal form is crystal form I of the methanesulfonate of the compound of formula I, and the crystal form I of the methanesulfonate has an X-ray powder diffraction pattern comprising diffraction peaks at 2θ±0.2° diffraction angles of 7.9648, 15.9556, 17.3872, 17.7652, 18.1048, 24.0274, and 25.6562; further, the crystal form I of the methanesulfonate has an X-ray powder diffraction pattern comprising diffraction peaks at 2θ±0.2° diffraction angles of 7.9648, 15.9556, 17.3872, 17.7652, 18.1048, 22.8525, 24.0274, and 25.6562; furthermore, the crystal form I of the methanesulfonate has an X-ray powder diffraction pattern comprising diffraction peaks at 2θ±0.2° diffraction angles of 7.9648, 15.1578, 15.9556, 17.3872, 17.7652, 18.1048, 22.8525, 24.0274, and 25.6562; furthermore, the crystal form I of the methanesulfonate has an X-ray powder diffraction pattern comprising diffraction peaks at 2θ±0.2° diffraction angles of 7.9648, 11.3739, 15.1578, 15.9556, 16.6385, 17.3872, 17.7652, 18.1048, 20.2628, 22.8525, 24.0274, and 25.6562; still further, the crystal form I of the methanesulfonate has an X-ray powder diffraction pattern comprising diffraction peaks at 2θ±0.2° diffraction angles of 7.9648, 11.3739, 15.1578, 15.9556, 16.6385, 17.3872, 17.7652, 18.1048, 19.1430, 20.2628, 21.6954, 22.8525, 24.0274, 25.0167, and 25.6562; still further, the crystal form I of the methanesulfonate has an XRPD pattern substantially as shown in FIG. 113d;
preferably, the crystal form is crystal form J of the methanesulfonate of the compound of formula I, and the crystal form J of the methanesulfonate has an X-ray powder diffraction pattern comprising diffraction peaks at 2θ±0.2° diffraction angles of 8.6620, 13.5049, 16.1555, 18.1900, 21.9852, 22.2107, and 22.9624; further, the crystal form J of the methanesulfonate has an X-ray powder diffraction pattern comprising diffraction peaks at 2θ±0.2° diffraction angles of 8.6620, 10.0632, 11.4255, 13.5049, 16.1555, 18.1900, 21.9852, 22.2107, and 22.9624; furthermore, the crystal form J of the methanesulfonate has an X-ray powder diffraction pattern comprising diffraction peaks at 2θ±0.2° diffraction angles of 8.6620, 10.0632, 11.4255, 13.5049, 16.1555, 17.5647, 18.1900, 19.7621, 21.9852, 22.2107, and 22.9624; furthermore, the crystal form J of the methanesulfonate has an X-ray powder diffraction pattern comprising diffraction peaks at 2θ±0.2° diffraction angles of 8.6620, 10.0632, 11.4255, 13.5049, 16.1555, 17.5647, 18.1900, 19.7621, 21.9852, 22.2107, 22.9624, and 24.6246; still further, the crystal form J of the methanesulfonate has an X-ray powder diffraction pattern comprising diffraction peaks at 2θ±0.2° diffraction angles of 8.6620, 10.0632, 11.4255, 13.5049, 14.5059, 15.1512, 16.1555, 17.5647, 18.1900, 19.7621, 20.5741, 21.9852, 22.2107, 22.9624, 24.6246, and 30.6181; still further, the crystal form J of the methanesulfonate has an XRPD pattern substantially as shown in FIG. 113e;
preferably, the crystal form is crystal form K of the methanesulfonate of the compound of formula I, and the crystal form K of the methanesulfonate has an X-ray powder diffraction pattern comprising diffraction peaks at 2θ±0.2° diffraction angles of 15.4362, 17.2898, 17.5619, 18.6931, 21.1887, and 25.1553; further, the crystal form K of the methanesulfonate has an X-ray powder diffraction pattern comprising diffraction peaks at 2θ±0.2° diffraction angles of 15.4362, 17.2898, 17.5619, 18.6931, 19.5256, 21.1887, and 25.1553; furthermore, the crystal form K of the methanesulfonate has an X-ray powder diffraction pattern comprising diffraction peaks at 2θ±0.2° diffraction angles of 15.4362, 17.2898, 17.5619, 18.0642, 18.6931, 19.5256, 21.1887, 21.9878, and 25.1553; furthermore, the crystal form K of the methanesulfonate has an X-ray powder diffraction pattern comprising diffraction peaks at 2θ±0.2° diffraction angles of 8.6859, 15.4362, 16.5219, 17.2898, 17.5619, 18.0642, 18.6931, 19.5256, 21.1887, 21.9878, 22.9735, 25.1553, 26.0704, 28.8383, and 30.0689; still further, the crystal form K of the methanesulfonate has an X-ray powder diffraction pattern comprising diffraction peaks at 2θ±0.2° diffraction angles of 8.6859, 9.9129, 12.6815, 13.4500, 14.0343, 15.4362, 16.5219, 17.2898, 17.5619, 18.0642, 18.6931, 19.5256, 21.1887, 21.9878, 22.9735, 25.1553, 26.0704, 27.0875, 28.8383, 30.0689, 31.9147, and 33.0164; still further, the crystal form K of the methanesulfonate has an XRPD pattern substantially as shown in FIG. 113f;
preferably, the crystal form is crystal form L of the methanesulfonate of the compound of formula I, and the crystal form L of the methanesulfonate has an X-ray powder diffraction pattern comprising diffraction peaks at 2θ±0.2° diffraction angles of 8.0867, 18.6275, 19.6478, and 21.1470; further, the crystal form L of the methanesulfonate has an X-ray powder diffraction pattern comprising diffraction peaks at 2θ±0.2° diffraction angles of 8.0867, 11.8848, 18.6275, 19.6478, 21.1470, 21.6729, and 21.9361; furthermore, the crystal form L of the methanesulfonate has an X-ray powder diffraction pattern comprising diffraction peaks at 2θ±0.2° diffraction angles of 8.0867, 11.8848, 18.6275, 19.6478, 21.1470, 21.6729, 21.9361, and 29.6831; furthermore, the crystal form L of the methanesulfonate has an X-ray powder diffraction pattern comprising diffraction peaks at 2θ±0.2° diffraction angles of 8.0867, 11.8848, 18.6275, 19.6478, 20.5586, 21.1470, 21.6729, 21.9361, 23.0719, 23.4214, 27.7310, and 29.6831; still further, the crystal form L of the methanesulfonate has an X-ray powder diffraction pattern comprising diffraction peaks at 2θ±0.2° diffraction angles of 8.0867, 11.8848, 14.2858, 15.7466, 16.2087, 18.6275, 19.6478, 20.5586, 21.1470, 21.6729, 21.9361, 22.3422, 23.0719, 23.4214, 23.9149, 27.7310, 28.6368, 29.6831, and 38.4394; still further, the crystal form L of the methanesulfonate has an XRPD pattern substantially as shown in FIG. 113g; preferably, the crystal form is crystal form M of the methanesulfonate of the compound of formula I, and the crystal form M of the methanesulfonate has an X-ray powder diffraction pattern comprising diffraction peaks at 2θ±0.2° diffraction angles of 16.0880, 17.4084, 17.6442, 18.6200, 18.8984, and 20.6457; further, the crystal form M of the methanesulfonate has an X-ray powder diffraction pattern comprising diffraction peaks at 2θ±0.2° diffraction angles of 6.0854, 16.0880, 16.2048, 17.4084, 17.6442, 18.6200, 18.8984, and 20.6457; furthermore, the crystal form M of the methanesulfonate has an X-ray powder diffraction pattern comprising diffraction peaks at 2θ±0.2° diffraction angles of 6.0854, 9.8088, 15.6545, 16.0880, 16.2048, 17.4084, 17.6442, 18.2844, 18.6200, 18.8984, and 20.6457; furthermore, the crystal form M of the methanesulfonate has an X-ray powder diffraction pattern comprising diffraction peaks at 2θ±0.2° diffraction angles of 6.0854, 8.6714, 9.8088, 13.3246, 15.6545, 16.0880, 16.2048, 17.4084, 17.6442, 18.2844, 18.6200, 18.8984, 19.7051, 20.6457, 21.0719, 22.1260, 24.4928, and 25.3051; still further, the crystal form M of the methanesulfonate has an X-ray powder diffraction pattern comprising diffraction peaks at 2θ±0.2° diffraction angles of 6.0854, 8.6714, 9.8088, 12.3906, 13.3246, 13.8974, 15.6545, 16.0880, 16.2048, 16.8014, 17.4084, 17.6442, 18.2844, 18.6200, 18.8984, 19.7051, 20.6457, 21.0719, 22.1260, 22.9780, 24.4928, 25.3051, 26.3685, and 28.9539; still further, the crystal form M of the methanesulfonate has an XRPD pattern substantially as shown in FIG. 114b;
preferably, the crystal form is crystal form N of the methanesulfonate of the compound of formula I, and the crystal form N of the methanesulfonate has an X-ray powder diffraction pattern comprising diffraction peaks at 2θ±0.2° diffraction angles of 17.4887, 17.7573, 18.3532, 19.4978, 19.7328, and 27.6786; further, the crystal form N of the methanesulfonate has an X-ray powder diffraction pattern comprising diffraction peaks at 2θ±0.2° diffraction angles of 9.1585, 17.4887, 17.7573, 18.3532, 19.4978, 19.7328, and 27.6786; furthermore, the crystal form N of the methanesulfonate has an X-ray powder diffraction pattern comprising diffraction peaks at 2θ±0.2° diffraction angles of 9.1585, 17.4887, 17.7573, 18.0588, 18.3532, 19.4978, 19.7328, 20.2234, 21.1657, 25.2039, and 27.6786; furthermore, the crystal form N of the methanesulfonate has an X-ray powder diffraction pattern comprising diffraction peaks at 2θ±0.2° diffraction angles of 9.1585, 10.5913, 13.8291, 15.3223, 17.4887, 17.7573, 18.0588, 18.3532, 19.4978, 19.7328, 20.2234, 21.1657, 21.6656, 23.4530, 25.2039, 26.7244, 27.6786, and 30.5646; still further, the crystal form N of the methanesulfonate has an X-ray powder diffraction pattern comprising diffraction peaks at 2θ±0.2° diffraction angles of 9.1585, 10.5913, 13.8291, 15.3223, 17.4887, 17.7573, 18.0588, 18.3532, 19.0631, 19.4978, 19.7328, 20.2234, 21.1657, 21.6656, 22.4118, 23.4530, 25.2039, 26.7244, 27.6786, 30.5646, and 34.8974; still further, the crystal form N of the methanesulfonate has an XRPD pattern substantially as shown in FIG. 114c;
preferably, the crystal form is crystal form O of the methanesulfonate of the compound of formula I, and the crystal form O of the methanesulfonate has an X-ray powder diffraction pattern comprising diffraction peaks at 2θ±0.2° diffraction angles of 9.8632, 16.4454, 17.1724, 17.8526, 19.5652, and 19.7882; further, the crystal form O of the methanesulfonate has an X-ray powder diffraction pattern comprising diffraction peaks at 2θ±0.2° diffraction angles of 9.8632, 16.4454, 17.1724, 17.8526, 19.1922, 19.5652, and 19.7882; furthermore, the crystal form O of the methanesulfonate has an X-ray powder diffraction pattern comprising diffraction peaks at 2θ±0.2° diffraction angles of 9.8632, 16.4454, 17.1724, 17.8526, 19.1922, 19.5652, 19.7882, 21.5123, 22.6207, 25.3282, and 26.0079; furthermore, the crystal form O of the methanesulfonate has an X-ray powder diffraction pattern comprising diffraction peaks at 2θ±0.2° diffraction angles of 9.8632, 13.0102, 16.4454, 17.1724, 17.8526, 18.5219, 19.1922, 19.5652, 19.7882, 20.7837, 21.5123, 22.1595, 22.6207, 23.3625, 25.3282, 26.0079, and 29.5124; still further, the crystal form O of the methanesulfonate has an X-ray powder diffraction pattern comprising diffraction peaks at 2θ±0.2° diffraction angles of 9.8632, 11.9975, 13.0102, 14.1723, 16.4454, 17.1724, 17.8526, 18.5219, 19.1922, 19.5652, 19.7882, 20.7837, 21.5123, 22.1595, 22.6207, 23.3625, 24.6883, 25.3282, 26.0079, 29.5124, 30.2740, 31.3467, and 32.7120; still further, the crystal form O of the methanesulfonate has an XRPD pattern substantially as shown in FIG. 114d.

10. The pharmaceutically acceptable salt of the compound of formula I or the crystal form thereof according to claim 3, wherein the crystal form is a crystal form of the ethanesulfonate of the compound of formula I; preferably, the crystal form is crystal form A of the ethanesulfonate of the compound of formula I, and the crystal form A of the ethanesulfonate has an X-ray powder diffraction pattern comprising diffraction peaks at 2θ±0.2° diffraction angles of 7.4322, 14.8852, 16.6572, 17.5439, and 20.0726; further, the crystal form A of the ethanesulfonate has an X-ray powder diffraction pattern comprising diffraction peaks at 2θ±0.2° diffraction angles of 7.4322, 10.3920, 13.3770, 14.4406, 14.8852, 16.6572, 17.5439, 20.0726, and 22.0062; furthermore, the crystal form A of the ethanesulfonate has an XRPD pattern substantially as shown in FIG. 55;
preferably, the crystal form A of the ethanesulfonate has one or two of the following characteristics:
(1) a TGA curve of the crystal form A of the ethanesulfonate showing a weight loss of 5-12%, preferably about 6-10% (e.g., 6.0%, 6.5%, 7.0%, 7.5%, 8.0%, 8.5%, 9.0%, 9.5%, or 10.0%) at 150.0±3 °C;
(2) a DSC curve of the crystal form A of the ethanesulfonate having 2 endothermic peaks at 36.3±3 °C and 141.5±3 °C;
preferably, the crystal form A of the ethanesulfonate has one or two of the following characteristics:
(1) a TGA curve of the crystal form A of the ethanesulfonate showing a weight loss of 8.29% at 150.0±3 °C;
(2) a DSC curve of the crystal form A of the ethanesulfonate having 2 endothermic peaks at 36.3±3 °C and 141.5±3 °C;
in some embodiments, a TGA/DSC profile of the crystal form A of the ethanesulfonate is substantially as shown in FIG. 56; a ¹H NMR spectrum of the crystal form A of the ethanesulfonate is substantially as shown in FIG. 57; preferably, the crystal form is crystal form B of the ethanesulfonate of the compound of formula I, and the crystal form B of the ethanesulfonate has an X-ray powder diffraction pattern comprising diffraction peaks at 2θ±0.2° diffraction angles of 4.9173, 15.3679, 17.7077, 18.9091, 19.5992, and 20.7324; further, the crystal form B of the ethanesulfonate has an X-ray powder diffraction pattern comprising diffraction peaks at 2θ±0.2° diffraction angles of 4.9173, 11.9022, 15.3679, 17.7077, 18.9091, 19.5992, 19.9469, 20.7324, 22.2822, and 24.6012; furthermore, the crystal form B of the ethanesulfonate has an X-ray powder diffraction pattern comprising diffraction peaks at 2θ±0.2° diffraction angles of 4.9173, 11.9022, 14.8131, 15.3679, 17.7077, 18.9091, 19.5992, 19.9469, 20.7324, 22.2822, 23.7415, 24.6012, 25.4858, and 27.0042; still further, the crystal form B of the ethanesulfonate has an X-ray powder diffraction pattern comprising diffraction peaks at 2θ±0.2° diffraction angles of 4.9173, 10.3467, 11.9022, 13.3498, 13.9472, 14.8131, 15.3679, 16.5326, 17.7077, 18.9091, 19.5992, 19.9469, 20.7324, 21.2340, 22.2822, 23.7415, 24.6012, 25.4858, 27.0042, 27.7863, 29.2924, 29.9997, and 31.9631; still further, the crystal form B of the ethanesulfonate has an XRPD pattern substantially as shown in FIG. 58a or 58b;
preferably, the crystal form B of the ethanesulfonate has one or two of the following characteristics:
(1) a TGA curve of the crystal form B of the ethanesulfonate showing a weight loss of 3-10%, preferably about 4-8% (e.g., 4.0%, 4.5%, 5.0%, 5.5%, 6.0%, 6.5%, 7.0%, 7.5%, or 8.0%) at 150.0±3 °C;
(2) a DSC curve of the crystal form B of the ethanesulfonate having 2 endothermic peaks at 107.1±3 °C and 145.3±3 °C;
preferably, the crystal form B of the ethanesulfonate has one or two of the following characteristics:
(1) a TGA curve of the crystal form B of the ethanesulfonate showing a weight loss of 6.30% at 150.0±3 °C;
(2) a DSC curve of the crystal form B of the ethanesulfonate having 2 endothermic peaks at 107.1±3 °C and 145.3±3 °C;
preferably, a TGA/DSC profile of the crystal form B of the ethanesulfonate is substantially as shown in FIG. 59; a ¹H NMR spectrum of the crystal form B of the ethanesulfonate is substantially as shown in FIG. 60;
preferably, the crystal form is crystal form C of the ethanesulfonate of the compound of formula I, and the crystal form C of the ethanesulfonate has an X-ray powder diffraction pattern comprising diffraction peaks at 2θ±0.2° diffraction angles of 6.3040, 10.4226, 13.6779, 15.9618, 20.3279, and 20.9348; further, the crystal form C of the ethanesulfonate has an X-ray powder diffraction pattern comprising diffraction peaks at 2θ±0.2° diffraction angles of 6.3040, 8.7771, 10.4226, 13.6779, 15.9618, 19.0549, 20.3279, and 20.9348; furthermore, the crystal form C of the ethanesulfonate has an X-ray powder diffraction pattern comprising diffraction peaks at 2θ±0.2° diffraction angles of 6.3040, 8.7771, 10.4226, 13.6779, 15.9618, 17.6528, 19.0549, 19.8646, 20.3279, 20.9348, 21.7435, 23.4169, and 26.2647; still further, the crystal form C of the ethanesulfonate has an X-ray powder diffraction pattern comprising diffraction peaks at 2θ±0.2° diffraction angles of 6.3040, 8.7771, 10.4226, 12.0274, 12.5635, 13.6779, 14.9315, 15.9618, 17.6528, 18.0467, 19.0549, 19.8646, 20.3279, 20.9348, 21.7435, 23.4169, 24.3601, 26.2647, 26.8633, 28.5527, 29.9377, 32.4837, and 35.6194; still further, the crystal form C of the ethanesulfonate has an XRPD pattern substantially as shown in FIG. 121b;
preferably, the crystal form is crystal form D of the ethanesulfonate of the compound of formula I, and the crystal form D of the ethanesulfonate has an X-ray powder diffraction pattern comprising diffraction peaks at 2θ±0.2° diffraction angles of 7.9081, 15.7893, 17.4668, 17.7671, 19.9837, 20.9514, and 23.8046; further, the crystal form D of the ethanesulfonate has an XRPD pattern substantially as shown in FIG. 121c;
preferably, the crystal form D of the ethanesulfonate has one or two or more of the following characteristics:
(1) a TGA curve showing a weight loss of 0.5-3%, preferably about 0.5-2% (e.g., 0.5%, 1.0%, 1.5%, or 2.0%), and more preferably about 1.70% at 150±3 °C;
(2) a DSC curve having 3 endothermic peaks at 55.5±3 °C, 142.4±3 °C, and 184.2±3 °C;
(3) a DSC curve having 1 exothermic peak at 91.6±3 °C;
preferably, a TGA/DSC profile of the crystal form D of the ethanesulfonate is substantially as shown in FIG. 122; a ¹H NMR spectrum of the crystal form D of the ethanesulfonate is substantially as shown in FIG. 123;
preferably, the crystal form is crystal form E of the ethanesulfonate of the compound of formula I, and the crystal form E of the ethanesulfonate has an X-ray powder diffraction pattern comprising diffraction peaks at 2θ±0.2° diffraction angles of 6.4770, 13.6740, 15.4870, 18.9455, 20.0718, 21.4317, and 23.2933; further, the crystal form E of the ethanesulfonate has an X-ray powder diffraction pattern comprising diffraction peaks at 2θ±0.2° diffraction angles of 6.4770, 10.9010, 13.6740, 15.4870, 16.1908, 18.9455, 20.0718, 21.4317, and 23.2933; furthermore, the crystal form E of the ethanesulfonate has an X-ray powder diffraction pattern comprising diffraction peaks at 2θ±0.2° diffraction angles of 6.4770, 9.3320, 10.9010, 12.3962, 13.6740, 15.4870, 16.1908, 17.8603, 18.9455, 19.6221, 20.0718, 21.4317, and 23.2933; still further, the crystal form E of the ethanesulfonate has an X-ray powder diffraction pattern comprising diffraction peaks at 2θ±0.2° diffraction angles of 6.4770, 9.3320, 10.9010, 12.3962, 13.6740, 14.7088, 15.4870, 16.1908, 17.8603, 18.9455, 19.6221, 20.0718, 20.4917, 21.4317, 23.2933, 24.8830, and 27.4600; still further, the crystal form E of the ethanesulfonate has an XRPD pattern substantially as shown in FIG. 121d;
preferably, the crystal form E of the ethanesulfonate has one or two or more of the following characteristics:
(1) a TGA curve showing a weight loss of 2-7%, preferably about 3-6% (e.g., 4.0%, 4.5%, 5.0%, or 5.5%), and more preferably about 4.64% at 110±3 °C;
(2) a DSC curve having 3 endothermic peaks at 48.4±3 °C, 104.0±3 °C, and 188.3±3 °C;
preferably, a TGA/DSC profile of the crystal form E of the ethanesulfonate is substantially as shown in FIG. 124; a ¹H NMR spectrum of the crystal form E of the ethanesulfonate is substantially as shown in FIG. 125;
preferably, the crystal form is crystal form F of the ethanesulfonate of the compound of formula I, and the crystal form F of the ethanesulfonate has an X-ray powder diffraction pattern comprising diffraction peaks at 2θ±0.2° diffraction angles of 8.7081, 9.3273, and 18.7028; further, the crystal form F of the ethanesulfonate has an XRPD pattern substantially as shown in FIG. 121e.

11. The pharmaceutically acceptable salt of the compound of formula I or the crystal form thereof according to claim 3, wherein the crystal form is a crystal form of the 1,2-ethanedisulfonate of the compound of formula I; preferably, the crystal form is crystal form A of the 1,2-ethanedisulfonate of the compound of formula I, and the crystal form A of the 1,2-ethanedisulfonate has an X-ray powder diffraction pattern comprising diffraction peaks at 2θ±0.2° diffraction angles of 9.5776, 9.7934, 16.5380, 17.6744, 19.6187, 20.1184, and 20.7238; furthermore, the crystal form A of the 1,2-ethanedisulfonate has an X-ray powder diffraction pattern comprising diffraction peaks at 2θ±0.2° diffraction angles of 9.5776, 9.7934, 12.9489, 16.5380, 16.9718, 17.6744, 19.6187, 20.1184, and 20.7238; furthermore, the crystal form A of the 1,2-ethanedisulfonate has an X-ray powder diffraction pattern comprising diffraction peaks at 2θ±0.2° diffraction angles of 9.5776, 9.7934, 12.9489, 16.3117, 16.5380, 16.9718, 17.6744, 18.7068, 19.6187, 20.1184, 20.7238, and 22.2198; furthermore, the crystal form A of the 1,2-ethanedisulfonate has an X-ray powder diffraction pattern comprising diffraction peaks at 2θ±0.2° diffraction angles of 9.5776, 9.7934, 12.9489, 13.3845, 16.3117, 16.5380, 16.9718, 17.6744, 18.7068, 19.6187, 20.1184, 20.7238, 22.2198, 23.1294, 25.3534, 26.0706, 26.5387, and 27.8458; still further, the crystal form A of the 1,2-ethanedisulfonate has an X-ray powder diffraction pattern comprising diffraction peaks at 2θ±0.2° diffraction angles of 9.5776, 9.7934, 11.0447, 12.1309, 12.9489, 13.3845, 14.5687, 15.5203, 16.3117, 16.5380, 16.9718, 17.6744, 18.7068, 19.6187, 20.1184, 20.7238, 22.2198, 23.1294, 24.1339, 25.3534, 26.0706, 26.5387, 27.8458, 30.3527, and 31.4653; still further, the crystal form A of the 1,2-ethanedisulfonate has an XRPD pattern substantially as shown in FIG. 126b;
preferably, the crystal form is crystal form B of the 1,2-ethanedisulfonate of the compound of formula I, and the crystal form B of the 1,2-ethanedisulfonate has an X-ray powder diffraction pattern comprising diffraction peaks at 2θ±0.2° diffraction angles of 9.5593, 16.2913, 16.5067, 17.0648, 17.7815, and 20.1011; further, the crystal form B of the 1,2-ethanedisulfonate has an X-ray powder diffraction pattern comprising diffraction peaks at 2θ±0.2° diffraction angles of 5.6887, 9.5593, 12.9139, 16.2913, 16.5067, 17.0648, 17.7815, and 20.1011; furthermore, the crystal form B of the 1,2-ethanedisulfonate has an X-ray powder diffraction pattern comprising diffraction peaks at 2θ±0.2° diffraction angles of 5.6887, 9.5593, 10.0235, 12.9139, 16.2913, 16.5067, 17.0648, 17.7815, 18.6648, 20.1011, and 22.2426; furthermore, the crystal form B of the 1,2-ethanedisulfonate has an X-ray powder diffraction pattern comprising diffraction peaks at 2θ±0.2° diffraction angles of 5.6887, 9.5593, 10.0235, 12.9139, 14.9480, 16.2913, 16.5067, 17.0648, 17.7815, 18.6648, 20.1011, 21.5441, 22.2426, 22.8020, 25.8424, 26.4853, 29.8463, and 30.3500; still further, the crystal form B of the 1,2-ethanedisulfonate has an X-ray powder diffraction pattern comprising diffraction peaks at 2θ±0.2° diffraction angles of 5.6887, 7.5361, 9.5593, 10.0235, 12.9139, 14.9480, 16.2913, 16.5067, 17.0648, 17.7815, 18.6648, 20.1011, 21.5441, 21.9422, 22.2426, 22.8020, 23.3535, 23.8750, 25.8424, 26.4853, 27.9265, 29.8463, 30.3500, and 32.6082; still further, the crystal form B of the 1,2-ethanedisulfonate has an XRPD pattern substantially as shown in FIG. 126c;
preferably, the crystal form is crystal form C of the 1,2-ethanedisulfonate of the compound of formula I, and the crystal form C of the 1,2-ethanedisulfonate has an X-ray powder diffraction pattern comprising diffraction peaks at 2θ±0.2° diffraction angles of 17.8877, 19.3330, 19.8489, 20.7131, and 21.1631; further, the crystal form C of the 1,2-ethanedisulfonate has an X-ray powder diffraction pattern comprising diffraction peaks at 2θ±0.2° diffraction angles of 13.3305, 16.7417, 17.8877, 19.3330, 19.8489, 20.7131, and 21.1631; furthermore, the crystal form C of the 1,2-ethanedisulfonate has an X-ray powder diffraction pattern comprising diffraction peaks at 2θ±0.2° diffraction angles of 10.9129, 13.3305, 16.7417, 17.8877, 18.3757, 19.3330, 19.8489, 20.7131, 21.1631, and 23.0842; furthermore, the crystal form C of the 1,2-ethanedisulfonate has an X-ray powder diffraction pattern comprising diffraction peaks at 2θ±0.2° diffraction angles of 10.9129, 12.0262, 13.3305, 16.7417, 17.8877, 18.3757, 19.3330, 19.8489, 20.7131, 21.1631, 22.3700, 23.0842, 24.1491, and 27.8056; still further, the crystal form C of the 1,2-ethanedisulfonate has an X-ray powder diffraction pattern comprising diffraction peaks at 2θ±0.2° diffraction angles of 7.9970, 9.8985, 10.9129, 12.0262, 13.3305, 15.5409, 16.7417, 17.8877, 18.3757, 19.3330, 19.8489, 20.7131, 21.1631, 22.3700, 23.0842, 24.1491, 25.2521, 27.8056, and 31.2456; still further, the crystal form C of the 1,2-ethanedisulfonate has an XRPD pattern substantially as shown in FIG. 126d.

12. A preparation method for the pharmaceutically acceptable salt of the compound of formula I or the crystal form thereof according to any one of claims 3-11, wherein
a preparation method for the free crystal form A of the compound of formula I, comprising the following steps: placing the compound of formula I in an organic solvent, stirring, concentrating, drying, adding water, heating at reflux, stirring at room temperature overnight, filtering, and drying, wherein the organic solvent is selected from acetonitrile;
a preparation method for the free crystal form B of the compound of formula I, comprising the following steps: placing the compound of formula I in an organic solvent, slurrying for 1-5 days, centrifuging to separate the solid, and drying, wherein the organic solvent is selected from an alcohol, preferably, the organic solvent is ethanol; the drying is selected from vacuum drying at a drying temperature of 40-60 °C, preferably 50 °C;
a preparation method for the free crystal form C of the compound of formula I, comprising the following steps: placing the compound of formula I in an organic solvent, slurrying, centrifuging to separate the solid, and drying, wherein the organic solvent is selected from MTBE; the drying is selected from vacuum drying at a drying temperature of 40-60 °C, preferably 50 °C; the slurrying is performed for 1-5 days, e.g., 1 day, 2 days, 3 days, 4 days, or 5 days;
a preparation method for the free crystal form D of the compound of formula I, comprising the following steps:
step 1. 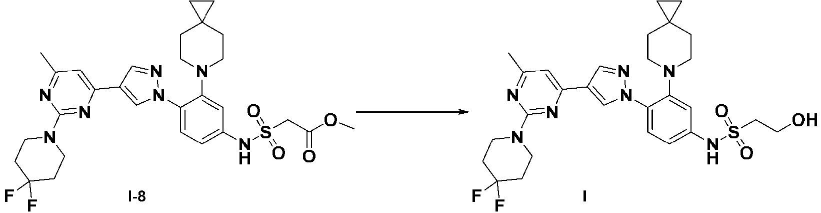 adding compound I-8, THF, and absolute ethanol, stirring to dissolve completely, adding anhydrous calcium chloride, stirring to dissolve completely, cooling to 0 °C, adding sodium borohydride, allowing to react at room temperature overnight, pouring the reaction liquid into water, adding ethyl acetate, allowing to stand for phase separation, pouring out the supernatant, adding ethyl acetate to wash the bottom solid, concentrating the organic phase and purifying by silica gel column to obtain the compound of formula I, adding acetonitrile to dissolve completely, concentrating until no liquid drops fell, adding water and heating to 90-120 °C for slurrying for 4-6 h, filtering, washing the filter cake, then adding water again and heating to 90-120 °C for slurrying for 4-6 h, slurrying overnight at room temperature, filtering, and lyophilizing the solid to obtain a mixed crystal of the compound of formula I;
step 2. slurrying the mixed crystal of the compound of formula I in IPA/H₂O (volume ratio of 982:18) at room temperature for about 5 days, centrifuging to separate the solid, and air-drying in an open container at room temperature to obtain the crystal form D;
a preparation method for the free crystal form E of the compound of formula I, comprising the following steps: placing the mixed crystal of the compound of formula I under THF atmosphere for gas-solid diffusion, and air-drying in an open container at room temperature to obtain the target product;
a preparation method for the free crystal form F of the compound of formula I, comprising the following steps:
heating the mixed crystal of the compound of formula I to 160-180 °C, and cooling to room temperature and room humidity to obtain the target product;
a preparation method for the free crystal form G of the compound of formula I, comprising the following steps: first dissolving the mixed crystal of the compound of formula I in 1,4-dioxane under H₂O atmosphere for gas-liquid diffusion to precipitate a solid, and drying in an open container in air to obtain the target product;
a preparation method for the free crystal form H of the compound of formula I, comprising the following step:
placing the mixed crystal of the compound of formula I under DMSO atmosphere for gas-solid diffusion to obtain the target product;
a preparation method for the free crystal form I of the compound of formula I, comprising the following steps:
placing the mixed crystal of the compound of formula I in MeOH, slurrying at room temperature for about 5 days, and air-drying in an open container at room temperature to obtain the target product;
a preparation method for the free crystal form J of the compound of formula I, comprising the following steps:
heating the mixed crystal of the compound of formula I to 100 °C, and cooling to room temperature and room humidity to obtain the target product;
a preparation method for the free crystal form K of the compound of formula I, comprising the following steps:
slurrying the mixed crystal of the compound of formula I in EtOH at room temperature for about 2 days, centrifuging to separate the solid, and drying under vacuum at room temperature to obtain the target product;
a preparation method for the free crystal form L of the compound of formula I, comprising the following step:
slurrying the compound of formula I in MeOH at room temperature for about 3 days to obtain the target product;
the preparation of the pharmaceutically acceptable salt of the compound of formula I comprises the following methods:
method I comprising the following steps: slurrying the compound of formula I or the mixed crystal of the compound of formula I with a salt-forming reagent (e.g., a corresponding acid) in solvent A, centrifuging to separate the solid, and drying, wherein the solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water; the alcohol is selected from methanol and ethanol; the drying is selected from vacuum drying at a drying temperature of 40-60 °C, preferably 50 °C; the slurrying is performed for 1-5 days, e.g., 1 day, 2 days, 3 days, 4 days, or 5 days;
method II comprising the following steps: slurrying the compound of formula I or the mixed crystal of the compound of formula I with a salt-forming reagent (e.g., a corresponding acid) in solvent A, adding solvent B for crystallization and gelling, performing temperature cycling between 50 °C and 5 °C to obtain a suspension, centrifuging to separate the solid, and drying, wherein the solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water; the alcohol is selected from methanol and ethanol; the solvent B is selected from n-heptane; the drying is selected from vacuum drying at a drying temperature of 40-60 °C, preferably 50 °C; the slurrying is performed for 1-5 days, e.g., 1 day, 2 days, 3 days, 4 days, or 5 days;
method III comprising the following steps: subjecting the compound of formula I or the mixed crystal of the compound of formula I and a salt-forming reagent (e.g., a corresponding acid) to temperature cycling between 50 °C and 5 °C in solvent A to obtain a solid, centrifuging to separate the solid, and drying, wherein the solvent A is selected from one or a mixture of more of an alcohol, MTBE, MIBK, MeOAc, 2-MeTHF, DMSO, IPAc, toluene, CPME, DCM, acetone, ACN, THF, ethyl formate, anisole, EtOAc, IPE, 1,4-dioxane, and water; the alcohol is selected from methanol and ethanol; the drying is selected from vacuum drying at a drying temperature of 40-60 °C, preferably 50 °C; the slurrying is performed for 1-5 days, e.g., 1 day, 2 days, 3 days, 4 days, or 5 days.

13. A pharmaceutical composition, comprising one or more of the crystal form of the compound of formula I according to any one of claims 1-2 and the pharmaceutically acceptable salt of the compound of formula I or the crystal form thereof according to any one of claims 3-11.

14. Use of the crystal form of the compound of formula I according to any one of claims 1-2, the pharmaceutically acceptable salt of the compound of formula I or the crystal form thereof according to any one of claims 3-11, or the pharmaceutical composition according to claim 13 for the manufacturing of a medicament, wherein
the medicament may inhibit KIF18A; and/or prevent and/or treat a disease associated with KIF18A;
preferably, the medicament is expected to be used for preventing and/or treating a proliferative disorder of a cancer, psoriasis, atopic dermatitis, an autoimmune disease, or an inflammatory bowel disease;
preferably, the cancer comprises mesothelioma, neuroblastoma, rectal cancer, colon cancer, familiar adenomatous polyposis and hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid carcinoma, kidney cancer, renal parenchymal carcinoma, ovarian cancer, cervical cancer, corpus uteri cancer, endometrial cancer, choriocarcinoma, pancreatic cancer, prostate cancer, bladder cancer, testicular cancer, breast cancer, urinary cancer, melanoma, brain tumor, lymphoma, head and neck cancer, acute lymphoblastic leukemia, chronic lymphoblastic leukemia, acute myeloid leukemia, chronic myeloid leukemia, hepatocellular carcinoma, gallbladder cancer, bronchial cancer, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma; preferably, the autoimmune disease comprises rheumatoid arthritis, systemic lupus erythematosus, Sjogren's syndrome, scleroderma, mixed connective tissue disease, dermatomyositis, polymyositis, Reiter's syndrome, autoimmune lymphoproliferative syndrome, multiple sclerosis, myasthenia gravis, and encephalomyelitis; preferably, the inflammatory bowel disease comprises ulcerative colitis or Crohn's disease.
